# EUROPÄISCHE PATENTANMELDUNG

(11) **EP 1 114 862 A2**
(43) Veröffentlichungstag der Anmeldung: **11.07.2001**
(21) Anmeldenummer: 00124303.9
(22) Anmeldetag: 15.11.2000
(51) Int. Cl.: C12N 15/12, A61K 38/17, G01N 33/50, A61K 48/00, C12N 5/10, A01K 67/027, C07K 16/18, A61K 39/395, C12Q 1/68

(54) **Verwendung von Polypeptiden oder diese kodierende Nukleinsäuren zur Diagnose oder Behandlung von Hauterkrankungen sowie ihre Verwendung zur Identifizierung von pharmakoligisch aktiven Substanzen**

(30) Priorität: 17.11.1999 DE 19955349; 17.12.1999 US 172511 P; 20.06.2000 DE 10030149
(71) Anmelder: Switch Biotech Aktiengesellschaft, 82152 Martinsried (DE)
(72) Erfinder: Wolf, Eckard, Prof. Dr., 85764 Oberschleissheim (DE); Werner, Sabine, Prof. Dr., 8032 Zürich (CH); Halle, Jörn-Peter, Dr., 82377 Penzberg (DE); Regenbogen; Johannes, Dr., 82152 Martinsried (DE); Goppelt, Andreas, Dr., 80636 München (DE)
(74) Vertreter: Bösl, Raphael, Dr. rer. nat., Dipl.-Chem.

(57) **Zusammenfassung**

Verwendung von Polypeptiden oder diese kodierende Nukleinsäuren zur Diagnose und/oder Prävention und/oder Behandlung von Erkrankungen und/oder der Wundheilung sowie ihre Verwendung zur Identifizierung von pharmakologisch aktiven Substanzen.

## Beschreibung

Die Erfindung betrifft die Verwendung von Polypeptiden oder diese kodierende Nukleinsäuren zur Diagnose und/oder Prävention und/oder Behandlung von Erkrankungen und/oder der Wundheilung sowie ihre Verwendung zur Identifizierung von pharmakologisch aktiven Substanzen. Insbesondere betrifft die vorliegende Erfindung die Verwendung in Zusammenhang mit Erkrankungen von Hautzellen und bei der Wundheilung.

Wunden heilen im allgemeinen ohne therapeutischen Eingriff ab. Es gibt jedoch zahlreiche Erkrankungen, bei denen die Wundheilung eine Rolle spielt, wie z.B. Diabetes mellitus, arterielle Verschlußkrankheiten, Schuppenflechte (Psoriasis), atopische Dermatitis, Kontaktdermatitis, Morbus Crohn, Epidermolysis bullosa, altersbedingte Hautveränderungen oder Innervationsstörungen. Wundheilungsstörungen führen zu einer verzögerten Wundheilung oder zu chronischen Wunden. Diese Störungen können durch die Art der Verwundung (z.B. großflächige Wunden, tiefgehende und mechanisch gedehnte Operationswunden, Verbrennungen, Trauma, Dekubitus), medikamentöse Behandlung der Patienten (z.B. mit Corticoiden) aber auch durch die Art der Erkrankung selber verursacht werden. So leiden z.B. 25% der Patienten mit Typ-II-Diabetes häufig an chronischen Ulzera ("diabetischer Fuß"), von denen etwa die Hälfte aufwendige stationäre Behandlungen erfordern und letztlich doch schlecht heilen. Der diabetische Fuß verursacht mehr Klinikaufenthalte als jede andere mit Diabetes assoziierte Komplikation. Die Zahl dieser Fälle bei Diabetes Typ I und II ist im Steigen begriffen und repräsentiert 2,5% aller Klinikeinweisungen. Zudem heilen Wunden mit zunehmendem Alter der Patienten schlechter. Oft ist auch eine Beschleunigung des natürlichen Wundheilungsprozesses wünschenswert, um z.B. die Gefahr von bakteriellen Infektionen oder die Liegezeiten der Patienten zu verringern.

Auch nach erfolgtem Wundverschluß kann es zu weiteren Störungen kommen. Während Wunden fötaler Haut ohne Narbenbildung heilen, kommt es nach Verletzungen in der Postnatalperiode stets zu Narbenbildungen, die oft ein großes kosmetisches Problem darstellen. Bei Patienten mit großflächigen Brandwunden kann zudem die Lebensqualität dramatisch beeinträchtigt werden, zumal vernarbter Haut auch die Anhangsgebilde, wie Haarfollikel, Schweiß- und Talgdrüsen fehlen. Bei entsprechender genetischer Disposition kann es auch zu Keloiden kommen, hypertrophischen Narben, die in die umgebende Haut einwuchern.

Der Vorgang der Wundheilung erfordert komplexe koordiniert ablaufende Wirkungen und Interaktionen verschiedener Zelltypen. Im Wundheilungsprozeß unterscheidet man folgende Schritte: die Blutgerinnung im Bereich der Wunde, die Rekrutierung von Entzündungszellen, die Reepithelialisierung, die Bildung von Granulationsgewebe sowie die Restrukturierung von Matrix. Die exakten Reaktionsmuster der beteiligten Zelltypen während der Phasen der Proliferation, der Migration, der Matrixsynthese und der Kontraktion sind, ebenso wie die Regulation von Genen wie z.B. Wachstumsfaktoren, Rezeptoren und Matrixproteinen, bislang wenig bekannt.

So sind bisher nur wenig zufriedenstellende Therapien entwickelt worden, um bei Wundheilungstörungen eingreifen zu können. Etablierte Therapieformen beschränken sich auf physikalische Unterstützung der Wundheilung (z.B. Verbände, Kompressen, Gele) oder der Transplantation von Hautgeweben, gezüchteten Hautzellen und/oder Matrixproteinen. In den letzten Jahren sind Wachstumsfaktoren zur Verbesserung der Wundheilung erprobt worden, ohne jedoch die konventionelle Therapie entscheidend zu verbessern. Auch die Diagnose von Wundheilungsstörungen beruht auf wenig aussagekräftigen optischen Analysen der Haut, da ein tieferes Verständnis der Genregulation während der Wundheilung bisher fehlt.

Auch für andere Störungen von regenerativen Prozessen sind bisher wenig zufriedenstellende Therapien entwickelt worden. Auch hier ist die Kenntnis der Genregulation vorteilhaft für die Entwicklung von Diagnostika und Therapien. Es ist gezeigt worden (Finch et al., 1997, Am. J. Pathol. 151: 1619-28; Werner, 1998, Cytokine Growth Factor Rev. 9: 153-165), daß wundheilungsrelevante Gene auch bei dermatologischen Erkrankungen, die auf Störungen der Regeneration der Haut beruhen, und allgemein bei regenerativen Prozessen eine entscheidende Rolle spielen. So spielt der Wachstumsfaktor KGF nicht nur eine entscheidende Rolle bei der Regulation der Proliferation und Differenzierung von Keratinozyten während der Wundheilung, sondern ist auch ein wichtiger Faktor bei der Hyperproliferation der Keratinozyten bei der Schuppenflechte (Psoriasis) und Regenerationsprozessen im Darm (bei Morbus Crohn und ulcerativer colitis)

Es ist daher Aufgabe der vorliegenden Erfindung, Polypeptide und/oder diese kodierende Nukleinsäuren zur Verfügung zu stellen, die an Prozessen bei Erkrankungen in Säugetierzellen, insbesondere bei Erkrankungen von Hautzellen und/oder der Wundheilung beteiligt sind, und deren Verwendung die Diagnose und/oder Prävention und/oder Behandlung sowie die Identifizierung und Entwicklung von in Zusammenhang mit diesen Erkrankungen wirksamen Pharmazeutika entscheidend verbessert.

Bei der Analyse der Genexpression während des Wundheilungsprozesses konnten überraschenderweise Gene identifiziert werden, die bisher nicht mit Diagnose, und/oder Behandlung von Erkrankungen oder bei der Wundheilung oder zur Identifizierung von pharmakologisch aktiven Substanzen in Verbindung gebracht wurden, deren Regulation aber für den Heilungsprozeß essentiell ist und die damit im kausalen Zusammenhang mit Diagnose und/oder Prävention und/oder Behandlung von Erkrankungen oder zur Identifizierung von pharmakologisch aktiven Substanzen stehen. Die Polypeptide dieser Gene gehören nicht zu den bisher bekannten Zielen für die Diagnose - wie beispielsweise der Indikation - und/oder Prävention und/oder der Behandlung - wie beispielsweise der Modulation - von Erkrankungen oder für die Identifizierung von pharmakologisch aktiven Substanzen, so daß sich aus dieser Erfindung völlig neue Therapieansätze ergeben.

Die Aufgabe wird daher erfindungsgemäß durch die Verwendung eines oder mehrerer Polypeptide oder Varianten davon gemäß einer der SEQ ID Nr. 1 bis SEQ ID Nr. 4 und SEQ ID Nr. 7 bis SEQ ID Nr. 9 und SEQ ID Nr. 103 bis SEQ ID Nr. 104 und SEQ ID Nr. 106 oder diese kodierende Nukleinsäuren oder Varianten davon zur Diagnose und/oder Prävention und/oder Behandlung von Erkrankungen oder zur Identifizierung von pharmakologisch aktiven Substanzen gelöst.

Die genauen biologischen Funktionen der erfindungsgemäß verwendeten Polypeptide der SEQ ID Nr. 1 bis SEQ ID Nr. 4 und SEQ ID Nr. 7 bis SEQ ID Nr. 9 und SEQ ID Nr. 103 bis SEQ ID Nr. 104 und SEQ ID Nr. 106 sind unbekannt. Bei den Untersuchungen im Rahmen dieser Erfindung konnte zum ersten Mal ein Zusammenhang der vorangehend beschriebenen Polypeptide mit Erkrankungen, beispielsweise Hauterkrankungen, festgestellt werden. Die Accession Numbers der erfindungsgemäß verwendeten Polypeptidsequenzen und ihrer cDNAs sind in Tabelle 2 aufgelistet.

Erfindungsgemäß können folgende Polypeptide und/oder diese kodierende Nukleinsäuren verwendet werden:
- MBNL aus der Maus (SEQ ID Nr. 103; GeneBank: AAF72159) oder Mensch (SEQ ID Nr. 1; GeneBank: CAA74155). Zusätzlich kann das Polypeptid KIAA0428 (SEQ ID Nr. 104; trEMBL: 043311) aus dem Menschen, das eine Variante des MBNL (SEQ ID Nr. 1) ist, verwendet werden.
- Wolf-Hirschhorn Syndrom Candidate 2 Protein aus Maus (SEQ ID Nr. 2; trEMBL: Q9Z1V9) oder Mensch (SEQ ID Nr. 3; trEMBL: 095392). Das dieses Protein kodierende Gen im Menschen liegt in einer 167 kb großen Region im Genom, die kritisch für die genetische Veranlagung für das Wolf-Hirschhorn Syndrom gilt (Wright et al., 1997, Hum. Mol. Genet. 6:317-324).
- KIAA0494 aus dem Menschen (SEQ ID Nr. 4; Seki et al., 1997, DNA Res. 4:345-349).
- KIAA0614 aus dem Menschen (SEQ ID Nr. 7; GenBank: BAA31589) oder der Maus (SEQ ID Nr. 106).
- KIAA0521 aus dem Menschen (SEQ ID Nr. 8; GenBank: BAA25447).
- KIAA0261 aus dem Menschen (SEQ ID Nr. 9; GenBank: BAA13391)

Bei der Analyse der Genexpression während des Wundheilungsprozesses konnten weitere Gene identifiziert werden, deren bereits bekannte und beschriebene Funktionen bisher nicht mit Hauterkrankungen, beispielsweise bei einer gestörten Wundheilung, in Verbindung gebracht wurden, deren Regulation aber für den Wundheilungsprozeß essentiell ist und die damit erstmals in kausalen Zusammenhang mit Hauterkrankungen, beispielsweise bei einer gestörten Wundheilung, gebracht wurden. Die Polypeptide dieser Gene gehören nicht zu den bisher bekannten Zielen von Therapien von Hauterkrankungen und/oder der Wundheilung, so daß sich aus dieser Erfindung völlig neue Therapieansätze ergeben.

Die Aufgabe der Erfindung wird weiterhin durch die Verwendung eines Polypeptids oder Varianten davon gemäß einer der SEQ ID Nr. 5 bis SEQ ID Nr. 6, SEQ ID Nr. 10 bis SEQ ID Nr. 48,SEQ ID Nr. 55 bis SEQ ID Nr. 58, SEQ ID Nr. 89 bis SEQ ID Nr. 94 oder SEQ ID Nr. 105 oder SEQ ID Nr. 109 bis SEQ ID Nr. 114 oder diese kodierende Nukleinsäuren oder Varianten davon zur Diagnose und/oder Prävention und/oder Behandlung, beispielsweise zur therapeutischen und/oder prophylaktischen Behandlung, von Hauterkrankungen oder zur Identifizierung von pharmakologisch aktiven Substanzen gelöst.

Erfindungsgemäß können folgende Polypeptide und/oder diese kodierende Nukleinsäuren verwendet werden:
- KIAA0585 aus dem Menschen (SEQ ID Nr. 5; Nagase et al., 1998, DNA Res. 5:31-39). Eine verkürzte Variante des Proteins ist aus WO 98/57985 als Protein bekannt, das an den "receptor interacting protein" (RIP; ein Protein, das bei dem Signalweg des Fas-induzierten Zelltods beteiligt ist) bindet. Die Variante ist im Nukleus humaner Zellen lokalisiert.
- HSPC028 aus dem Menschen (SEQ ID Nr. 6; GenBank: AAD39844) oder der Maus (SEQ ID Nr. 105). Das Polypeptid aus dem Menschen ist bereits aus WO 99/15658 als Protein bekannt, das eine schwache Homologie zu Transkriptionsfaktoren aufweist.
- Das Protein Calnexin aus Maus (SEQ ID Nr. 10) oder Mensch (SEQ ID Nr. 11), das in ruhenden und sich ausdifferenzierenden Zellen negativ reguliert wird (Honore et al., 1994, Electrophoresis 15:482-90; Olsen et al., 1995, Electrophoresis 16:2241-8).
- Das CREB-binding protein (CBP) aus Maus (SEQ ID Nr. 12) oder Mensch (SEQ ID Nr. 13), welches - wie das in der US 5,658,784 beschriebene und eng verwandte Gen für p300 - als Coaktivator für eine Vielzahl von an der Wachstumskontrolle und Differenzierung beteiligten Transkriptionsfaktoren, wie zum Beispiel GATA-1, p53 und E2F beschrieben ist (Blobel et al., 1998, Proc. Natl. Acad. Sci. USA 95:2061-6).
- Das mas Onkogen aus Maus (SEQ ID Nr.14) oder das homologe MRG aus Mensch (SEQ ID Nr. 15) (Monnot et al., 1991, Mol. Endocrinol. 5:1477-1487; Metzger et al., 1995, FEBS Lett. 357:27-32), das in der US 5,320,941 im Zusammenhang mit einem Verfahren zur Behandlung von Tumoren beschrieben wird. Zusätzlich zu dem bekannten Protein aus der Maus kann auch das in dieser Arbeit erstmals erwähnte eng verwandten Polypeptid mit abweichender Sequenz verwendet werden (SEQ ID Nr. 109). Die cDNA Sequenz, die für das erfindungsgemäß verwendete Polypeptid kodiert, ist im Sequenzprotokoll gemäß SEQ ID Nr. 120 angegeben.
- Das aus der JP 3254680 bekannte acylamino acid-releasing enzyme aus dem Menschen (SEQ ID Nr. 16), das in Tumorzellen aktiviert wird (Schoenberger et al., 1986, J. Clin. Chem. Clin. Biochem. 24: 375-8).
- Das in akuten promyelozytischen Leukämiezellen identifizierte und an der Zellreifung beteiligte Protein JEM-1 aus dem Menschen (SEQ ID Nr. 17) (Tong et al., 1998, Leukemia 12:1733-40). Zuätzlich können die in dieser Arbeit erstmals erwähnten eng Verwandten Polypeptide aus Mensch (SEQ ID Nr. 110) oder Maus (SEQ ID Nr. 111 und SEQ ID Nr. 112) verwendet werden, deren kodierende Nukleinsäuren im Sequenzprotokoll gemäß SEQ ID Nr. 121 (Mensch) und SEQ ID Nr. 122 und SEQ ID Nr. 123 (Maus) angegeben sind.
- Das Cardiac Ankyrin Repeat Protein (CARP/MARP) aus Maus (SEQ ID Nr.18) oder Mensch (SEQ ID Nr. 19), das für die Entwicklung des Herzmuskels entscheidend ist (Zou et al., 1997, Development 124:793-804). Zusätzlich zu der bekannten Sequenz aus der Maus kann auch das in dieser Arbeit erstmals erwähnte Polypeptid aus der Maus (SEQ ID Nr. 113) mit abweichender Sequenz verwendet werden. Die Sequenz der dieses Polypeptid kodierenden Nukleinsäure ist im Sequenzprotokoll gemäß SEQ ID Nr. 124 angegeben.
- Der Transkriptionsfaktor Checkpoint suppressor 1 (CHES1) aus dem Menschen (SEQ ID Nr. 20), der verschiedene Mutationen in *S. cerevisiae* in Zusammenhang mit der DNA-Reparatur suprimiert und für den eine Funktion bei der Zell-Zyklus-Kontrolle vermutet wird (Pati et al., 1997, Mol. Cell. Biol 17:3037-46).
- Das kleine GTP Bindeprotein RAB2 aus Maus (SEQ ID Nr. 21) oder Mensch (SEQ ID Nr. 22), das eine wichtige Rolle bei der Neuronalentwicklung spielt (Ayala et al., 1990, Neuron 4:797-805).
- Das Nukleoporin-Precursor Protein Nup98-Nup96 aus dem Menschen (SEQ ID Nr. 23), aus dem durch proteolytische Prozessierung in vivo die Nukleoporine Nup96 und Nup98 entstehen (Fontoura et al., 1999, J. Cell Biol. 144: 1097-1112). Für Nup98 ist eine Funktion bei akuter Myeolidleukämie (AML) beschrieben ist und in funktionellem Zusammenhang mit CBP und p300 steht (Kasper et al., 1999, Mol. Cell. Biol. 19:764-76).
- Der Ribonuclease L Inhibitor (Mu-RLI) aus Maus (SEQ ID Nr. 24) oder Mensch (SEQ ID Nr. 25), der in Zusammenhang mit der Ribonuclease L eine wichtige Rolle bei der antiviralen und antiproliferativen Funktion von Interferonen spielt und gewebespezifisch reguliert wird (Benoit et al., 1998, Gene 209:149-56). Zusätzlich zu den bisher bekannten Polypeptiden von Mensch (Bisbal et al., 1995, J. Biol. Chem. 270:13308-17) und Maus (De Coignac et al., 1998, Gene 209:149-56) können auch die in dieser Arbeit erstmals erwähnten eng verwandten Polypeptide mit abweichender Sequenz verwendet werden (SEQ ID Nr. 27 und SEQ ID Nr. 26). Die Sequenzen der diese Polypeptidvarianten kodierenden Nukleinsäuren sind im Sequenzprotokoll unter SEQ ID Nr. 118 und SEQ ID Nr. 117 aufgelistet.
- Die p68 Helikase aus Maus (SEQ ID Nr. 28) oder Mensch (SEQ ID Nr. 29), die als im Zellkern lokalisierte DNA oder RNA-Helikase beschrieben ist und an der für das Zellwachstum nötigen Replikation, Transkription oder RNA-Prozessierung beteiligt sein könnte (Ford et al., 1988, Nature 332:736-8). Zusätzlich zu der bekannten Polypeptidvariante aus der Maus (SEQ ID Nr. 28) (Lemaire und Heinlein, 1993, Life Sci. 52: 917-26) kann auch das in dieser Arbeit erstmals erwähnte eng verwandte Polypeptid mit abweichender Sequenz verwendet werden (SEQ ID Nr. 30). Die Sequenz der diese Polypeptidvariante kodierenden Nukleinsäure ist im Sequenzprotokoll unter SEQ ID Nr. 119 aufgelistet.
- Das Keratin assoziierte Protein mKAP13 (auch PMG-1) (SEQ ID Nr. 31) und das homologe Protein PMG-2 (SEQ ID Nr. 32) aus der Maus. Das PMG-1 Transkript wird hautspezifisch in der keratogenen Zone der Cortikalzellen der Haarfollikel exprimiert und ist spezifisch für die Keratinisierung der cortikalen Zellage im Maushaar ist (Aoki et al., 1998, J. Invest. Dermatol. 111:804-9). Das ebenfalls in wachsenden Haarfollikeln exprimierte eng verwandte PMG-2 wurde als Protein beschrieben, das zusammen mit PMG-1 an der Differenzierung aller Epithelzellen beteiligt ist, die epidermalen Anhänge bilden (Kuhn et al., 1999, Mech. Dev 86:193-196). Zusätzlich zu dem bekannten Polypeptid aus der Maus können auch die in dieser Arbeit erstmals erwähnten Polypeptide aus dem Menschen verwendet werden (SEQ ID Nr. 55 (Beispiel 7) und SEQ ID Nr. 89 bis SEQ ID Nr. 94). Die Nukleinsäuren, die für die vorangehend beschriebenen humanen Polypeptide gemäß SEQ ID Nr. 89 bis SEQ ID Nr. 94 kodieren, sind im Sequenzprotokoll gemäß einer SEQ ID Nr. 95 bis SEQ ID Nr. 100 aufgeführt.
- Das an der Apoptose beteiligte Protein MA-3 aus Maus (SEQ ID Nr. 33) oder Mensch (SEQ ID Nr. 34) (Matsuhashi et al., 1997, Res. Commun. Biochem. Cell Mol. Biol. 1:109-120; Shibahara et al., 1995, Gene 166:297-301). Zusätzlich zu dem bekannten Polypeptid aus dem Menschen kann auch das in dieser Arbeit erhaltene Polypeptid aus dem Menschen verwendet werden (SEQ ID Nr. 58). Die Sequenz der diese Polypeptidvariante kodierenden Nukleinsäure ist im Sequenzprotokoll unter SEQ ID Nr. 116 aufgelistet.
- Das antiproliferative Protein BTG1 aus Maus (SEQ ID Nr. 35) oder Mensch (SEQ ID Nr. 36), für das eine hohe Expression in absterbenden Zellen und eine Funktion bei der Regulation des Zellstatus von fortgeschrittenen atherosklerotischen Läsionen beschrieben ist (Corjay et al., 1998, Lab. Invest. 78:847-58).
- Das in der Epidermis weit verbreitet exprimierte CD9 aus Maus (SEQ ID Nr. 37) oder Mensch (SEQ ID Nr. 38), das mit beta 1 Integrinen Komplexe bildet und für das eine Rolle bei der Regulation der Motilität und Differenzierung von Keratinozyten vermutet wird (Jones et al., 1996, Cell Adhes. Commun. 4:297-305).
- Die Stearoyl-CoA Desaturase (SCD1) aus Maus (SEQ ID Nr. 39) oder Mensch (SEQ ID Nr. 40), die durch den nuclear factor 1 (NF1) reguliert wird und an der Differenzierung von Präadipocyten zu Adipocyten beteiligt ist (Singh und Ntambi, 1998, Biochim. Biophys. Acta 1398:148-56).
- Das an der fokalen Adhäsion von Fibroblasten beteiligte Protein Syndecan 4 (Ryudocan) aus Maus (SEQ ID Nr. 41) oder Mensch (SEQ ID Nr. 42), das als Transmembran-Adhäsionskomponente beschrieben ist (Woods und Couchman, 1994, Mol. Biol. Cell., 5:183-92).
- Das zu der Klasse der E2A basischen Helix-Loop-Helix Transkriptionsfaktoren gehörende Protein ALF1 aus Maus (SEQ ID Nr. 43) oder Mensch (SEQ ID Nr. 44), das das Wachstum von Mausfibroblasten reguliert (Loveys et al., 1996, Nucleic. Acids Res. 24:2813-20).
- Die potentiell prenylierte Protein Tyrosinphosphatase (PRL) aus Maus (SEQ ID Nr. 45) oder Mensch (SEQ ID Nr. 46), die in Zusammenhang mit der Signaltransduktion in der sich regenerierenden Leber und dem Muskelwachstum insbesondere des Herzens beschrieben ist (Zeng et al., 1998, Biochem. Biophys. Res. Commun. 244:421-7).
- Nuclear factor (NF1-B) aus Maus (SEQ ID Nr. 47) oder Mensch (SEQ ID Nr. 48), der in Zusammenhang mit alternativem splicing beschrieben ist und dessen Funktion bei der Onkogenese in Huhn-Fibroblasten untersucht wurde (Schuur et al., 1995, Cell Growth Differ. 6:219-27). Zusätzlich zu der angegebenen Polypeptidsequenz von NF1-B aus Mensch (SEQ ID Nr. 48) kann die ebenfalls im Sequenzprotokoll angegebene, leicht abweichende Polypeptidsequenz aus Mensch (SEQ ID Nr. 114) verwendet werden.
- Die Cystein Proteinase Cathepsin Z aus Maus (SEQ ID Nr. 56) oder Mensch (SEQ ID Nr. 57), für die eine Teilnahme am Tumorwachstum vermutet wird (Santamaria et al., 1998, J. Biol. Chem. 273:16818-23).

Für keines dieser Polypeptide oder deren kodierende Nukleinsäuren wurde bisher ein Zusammenhang mit Hauterkrankungen, beispielsweise bei einer gestörten Wundheilung, beschrieben oder nahegelegt. Es war daher unerwartet, daß diese Verbindungen erfindungsgemäß verwendet werden können. Die Accession Numbers der erfindungsgemäß verwendeten Polypeptide und ihrer cDNAs sind in Tabelle 3 aufgeführt.

Die Aufgabe der Erfindung wird weiterhin durch die Verwendung mindestens einer Nukleinsäure der SEQ ID Nr. 49 oder SEQ ID Nr. 50 oder Varianten davon zur Diagnose und/oder Prävention und/oder Behandlung, beispielsweise zur therapeutischen und/oder prophylaktischen Behandlung, von Hauterkrankungen oder zur Identifizierung von pharmakologisch aktiven Substanzen gelöst.

Die erfindungsgemäß verwendeten Nukleinsäure sind:
- Die Nukleinsäure für den sogenannten Steroid Receptor Coaktivator aus Maus (SEQ ID Nr. 49) oder Mensch (SEQ ID Nr. 50), dessen mRNA nicht translatiert wird und als RNA funktionell ist. Die Steroid Receptor Coaktivator wird als lediglich in Zusammenhang mit Steroidhormon-Rezeptoren aktiv beschrieben (Steroid Receptor Coaktivator: Lanz et al., 1999, Cell 97:17-27). Ein Zusammenhang mit Hauterkrankungen war bisher unbekannt.

Für keine dieser Nukleinsäuren wurde bisher ein Zusammenhang mit Hauterkrankungen, beispielsweise bei einer gestörten Wundheilung, beschrieben oder nahegelegt. Es war daher unerwartet, daß diese Verbindungen erfindungsgemäß verwendet werden können. Die Accession Numbers der erfindungsgemäß verwendeten Nukleinsäuren sind in Tabelle 3 aufgeführt.

Bei der Analyse der Genexpression während des Wundheilungsprozesses konnten zusätzlich Gene identifiziert werden, deren bereits bekannte und beschriebene Funktionen bisher nicht mit der Wundheilung in Verbindung gebracht wurden, deren Regulation aber für den Wundheilungsprozeß essentiell ist und die damit erstmals in kausalen Zusammenhang mit der Wundheilung gebracht wurden. Die Polypeptide dieser Gene gehören nicht zu den bisher bekannten Zielen von Therapien in Zusammenhang mit der krankhaften Veränderung der Wundheilung, so daß sich aus dieser Erfindung völlig neue Therapieansätze ergeben.

Die Aufgabe der Erfindung wird daher zusätzlich durch die Verwendung mindestens eines Polypeptids oder Varianten davon gemäß einer der SEQ ID Nr. 51 bis SEQ ID Nr. 54 oder SEQ ID Nr. 101 bis SEQ ID Nr. 102 oder dieses kodierende Nukleinsäure oder Varianten davon zur Identifizierung von pharmakologisch aktiven Substanzen oder zur Diagnose und/oder Prävention und/oder Behandlung bei der Wundheilung gelöst.

Erfindungsgemäß können folgende Polypeptide und/oder diese kodierende Nukleinsäuren verwendet werden:
- Die SR calcium ATPase (SERCA) der Maus (SEQ ID Nr. 51) oder des Menschen (SEQ ID Nr. 52), die in Zusammenhang mit dem Muskelwachstum des Herzens beschrieben ist (Baker et al., 1998, Nucleic Acids Res., 26:1092-8). Weiterhin ist ein Zusammenhang von SERCA mit Darier's disease, einer Hautkrankheit beschrieben worden (Sakuntabhai et al., 1999, Nat. Genet. 21: 271-7).
- Das bei entzündlicher Dermatose nach Läsionen der Haut exprimierte Protein Calgranulin (MRP8) der Maus (SEQ ID Nr. 53) oder des Menschen (SEQ ID Nr.54), (Kelly et al., 1989, J. Pathol. 159:17-21), dessen Beteiligung an der Wundheilung bisher auch unbekannt war.
- Das aus DE 198 13 839, US 5,776,348 und US 5,614,397 bekannte Calciumbindende Protein MRP-14 aus Maus (SEQ ID Nr. 101) oder Mensch (SEQ ID Nr. 102) (Odink et al., 1987, Nature 330 :80-82 ; Lagasse und Weissman, 1992, Blood 79:1907-1915). Das Protein bildet Heterodimere mit MRP8 und ist in Makrophagen in akut entzündeten Geweben hochreguliert (Sorg, 1992, Behring Inst. Mitt. 91:126-137). Außerdem wurden erhöhte Mengen an MRP-14 in der Epidermis von Patienten mit Lichen planus, Lupus erythematosus und Psoriasis vulgaris beobachtet (Kunz et al., Arch. Dermatol. Res. 284:386-390). Das Heterodimer MRP8/MRP14 bindet Fettsäuren, insbesondere Arachidonsäure mit hoher Spezifität, während die Monomere keine Bindeaktivität zeigen (Kerkhoff et al., 1999, J. Biol. Chem., 274: 32672-32679). Zusätzlich zu der Rolle in inflammatorischen Prozessen konnte hier erstmals eine Funktion von MRP-8 und/oder MRP-14 in Differenzierungsprozessen von Keratinozyten während der Wundheilung nachgewiesen werden.
   Für keines dieser Polypeptide oder diese kodierende Nukleinsäuren wurde bisher ein Zusammenhang mit der Wundheilung beschrieben oder nahegelegt. Es war daher unerwartet, daß diese Polypeptide erfindungsgemäß verwendet werden können. Die Accession Numbers der vorangehend beschriebenen Polypeptide und ihrer cDNAs sind in Tabelle 4 aufgeführt.

Die erfindungsgemäß verwendeten Polypeptide können weiterhin dadurch gekennzeichnet sein, daß diese synthetisch hergestellt werden. So kann das gesamte Polypeptid oder Teile davon zum Beispiel mit Hilfe der klassischen Synthese (Merrifield-Technik) synthetisiert werden. Teile der vorangehend beschriebenen Polypeptide eignen sich insbesondere zur Gewinnung von Antiseren (siehe Beispiel 11), mit deren Hilfe geeignete Genexpressionsbanken durchsucht werden können, um so zu weiteren funktionellen Varianten eines erfindungsgemäß verwendbaren Polypeptids zu gelangen.

Der Begriff "Varianten" eines Polypeptids im Sinne der vorliegenden Erfindung unfaßt auch funktionell aktive Varianten. Unter funktionell aktiven Varianten sind Polypeptide zu verstehen, die beispielsweise wie die erfindungsgemäß verwendeten Polypeptiden während Erkrankungen, insbesondere Hauterkrankungen oder bei regenerativen Prozessen der Haut, insbesondere jedoch bei Wundheilungsstörung, reguliert werden und/oder Strukturmerkmale der Polypeptide aufweisen. Unter dem Begriff "Regulation" wird beispielsweise die Erhöhung oder Erniedrigung der Menge der Polypeptide oder diese kodierende Nukleinsäure verstanden, wobei diese Änderung beispielsweise auf transkriptioneller Ebene stattfinden kann.

Zu funktionell aktiven Varianten zählen beispielsweise auch Polypeptide, die von Nukleinsäuren kodiert werden, die aus nicht-wundheilungsspezifischen Gewebe, z. B. Embryonalgewebe, isoliert werden, jedoch nach Expression in einer an der Wundheilung beteiligten Zelle die bezeichneten Funktionen besitzen.

Varianten im Sinne der vorliegenden Erfindung sind auch Polypeptide, die eine Sequenzhomologie, insbesondere eine Sequenzidentität, von ca. 70%, vorzugsweise ca. 80%, insbesondere ca. 90%, vor allem ca. 95% zu dem Polypeptid mit der Aminosäuresequenz gemäß einer der SEQ ID Nr. 1 bis SEQ ID Nr. 48, SEQ ID Nr. 51 bis SEQ ID Nr. 58, SEQ ID Nr. 89 bis SEQ ID Nr. 94, SEQ ID Nr. 101 bis SEQ ID Nr. 106 oder SEQ ID Nr. 109 bis SEQ ID Nr. 114 aufweisen. Beispiele solcher Varianten, die auch funktionell aktiv sein können, sind demnach die entsprechenden Polypeptide, die aus anderen Organismen als dem Menschen bzw. der Maus, vorzugsweise aus nicht-menschlichen Säugetieren wie z. B. Affen, Schweinen und Ratten stammen. Andere Beispiele sind Polypeptide, die durch unterschiedliche Allele des Gens, in verschiedenen Individuen oder in verschiedenen Organen eines Organismus kodiert werden (siehe Beispiel 6).

Varianten des Polypeptids können auch Teile des erfindungsgemäß verwendeten Polypeptids mit mindestens 6 Aminosäuren Länge, vorzugsweise mit mindestens 8 Aminosäure Länge, insbesondere mit mindestens 12 Aminosäure Länge sein. Umfaßt sind auch Deletionen des Polypeptids im Bereich von ca. 1-60, vorzugsweise von ca. 1-30, insbesondere von ca. 1-15, vor allem von ca. 1-5 Aminosäuren. Beispielsweise kann die erste Aminosäure Methionin fehlen, ohne daß die Funktion des Polypeptids wesentlich verändert wird.

Der Begriff "kodierende Nukleinsäure" bezieht sich auf eine DNA-Sequenz, die für ein isolierbares bioaktives erfindungsgemäß verwendbares Polypeptid oder einen Precursor kodiert. Das Polypeptid kann durch eine Sequenz in voller Länge oder jeden Teil der kodierenden Sequenz kodiert werden, solange die spezifische, beispielsweise enzymatische Aktivität erhalten bleibt.

Es ist bekannt, daß kleine Veränderungen in der Sequenz der erfindungsgemäß verwendbaren Nukleinsäuren vorhanden sein können, zum Beispiel durch die Degenerierung des genetischen Codes, oder daß nicht translatierte Sequenzen am 5' und/oder 3'-Ende der Nukleinsäure angehängt sein können, ohne daß dessen Aktivität wesentlich verändert wird. Diese Erfindung umfaßt deshalb auch sogenannte "Varianten" der vorangehend beschriebenen Nukleinsäuren.

Mit dem Begriff "Varianten" sind alle DNA-Sequenzen bezeichnet, die komplementär zu einer DNA-Sequenz sind, die unter stringenten Bedingungen mit der Referenzsequenz hybridisieren und für ein Polypeptid kodieren, das eine ähnliche Aktivität aufweist, wie das von der Referenzsequenz kodierte Polypeptid.

Unter "stringenten Hybridisierungsbedingungen" sind solche Bedingungen zu verstehen, bei denen eine Hybridisierung bei 60°C in 2,5 x SSC-Puffer, gefolgt von mehreren Waschschritten bei 37°C in einer geringeren Pufferkonzentration erfolgt und stabil bleibt.

Varianten der Nukleinsäuren können auch Teile der erfindungsgemäß verwendeten Nukleinsäuren mit mindestens 8 Nukleotiden Länge, vorzugsweise mit mindestens 18 Nukleotiden Länge, insbesondere mit mindestens 24 Nukleotiden Länge.

Bevorzugterweise handelt es sich bei den erfindungsgemäß verwendeten Nukleinsäuren um DNA oder RNA, vorzugsweise eine DNA, insbesondere eine doppelsträngige DNA. Weiterhin kann die Sequenz der Nukleinsäuren dadurch gekennzeichnet sein, daß sie mindestens ein Intron und/oder eine polyA-Sequenz aufweist. Die erfindungsgemäß verwendeten Nukleinsäuren können auch in Form ihrer antisense-Sequenz verwendet werden.

Für die Expression des betreffenden Gens ist im allgemeinen eine doppelsträngige DNA bevorzugt, wobei der für das Polypeptid kodierende DNA-Bereich besonders bevorzugt ist. Dieser Bereich beginnt mit dem ersten in einer Kozak Sequenz (Kozak, 1987, Nucleic. Acids Res. 15:8125-48) liegenden Start-Codon (ATG) bis zum nächsten Stop-Codon (TAG, TGA bzw. TAA), das im gleichen Leseraster zum ATG liegt.

Eine weitere Verwendung der erfindungsgemäß verwendeten Nukleinsäuresequenzen ist die Konstruktion von anti-sense Oligonukleotiden (Zheng und Kemeny, 1995, Clin. Exp. Immunol. 100:380-2; Nellen und Lichtenstein, 1993, Trends Biochem. Sci. 18:419-23; Stein, 1992, Leukemia 6:967-74) und/oder Ribozymen (Amarzguioui, et al. 1998, Cell. Mol. Life Sci. 54:1175-202; Vaish, et al., 1998, Nucleic Acids Res. 26:5237-42; Persidis, 1997, Nat. Biotechnol. 15:921-2; Couture und Stinchcomb, 1996, Trends Genet. 12:510-5). Mit anti-sense Oligonukleotiden kann man die Stabilität der vorangehend beschriebenen Nukleinsäure verringern und/oder die Translation der vorangehend beschriebenen Nukleinsäure inhibieren. So kann beispielsweise die Expression der entsprechenden Gene in Zellen sowohl in vivo als auch in vitro verringert werden. Oligonukleotide können sich daher als Therapeutikum eignen. Diese Strategie eignet sich beispielsweise auch für Haut, epidermale und dermale Zellen, insbesondere, wenn die antisense Oligonukleotide mit Liposomen komplexiert werden (Smyth et al., 1997, J. Invest. Dermatol. 108:523-6; White et al., 1999, J. Invest. Dermatol. 112:699-705; White et al., 1999, J. Invest. Dermatol. 112:887-92). Für die Verwendung als Sonde oder als "antisense" Oligonukleotid ist eine einzelsträngige DNA oder RNA bevorzugt.

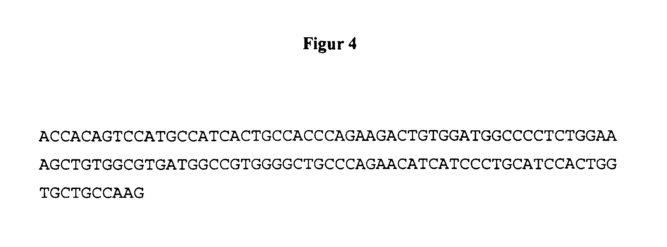

Weiterhin kann zur Durchführung der Erfindung eine Nukleinsäure verwendet werden, die synthetisch hergestellt worden ist. So kann die erfindungsgemäß verwendete Nukleinsäure beispielsweise chemisch anhand der in den Figuren 4 bis 6 beschriebenen DNA Sequenzen und/oder anhand der in diesen Figuren ebenfalls beschriebenen Proteinsequenzen unter Heranziehen des genetischen Codes z. B. nach der Phosphotriester-Methode synthetisiert werden (siehe z. B. Uhlmann, E. & Peyman, A. (1990) Chemical Revievs, 90, 543-584, No. 4).

Oligonukleotide werden in der Regel schnell durch Endo- oder Exonukleasen, insbesondere durch in der Zelle vorkommende DNasen und RNasen, abgebaut. Deshalb ist es vorteilhaft, die Nukleinsäure zu modifizieren, um sie gegen den Abbau zu stabilisieren, so daß über einen langen Zeitraum eine hohe Konzentration der Nukleinsäure in der Zelle beibehalten wird (Beigelman et al., 1995, Nucleic Acids Res. 23:3989-94; Dudycz, 1995, WO9511910; Macadam et al., 1998, WO9837240; Reese et al., 1997, WO9729116). Typischerweise kann eine solche Stabilisierung durch die Einführung von einer oder mehrerer Internukleotid-Phosphorgruppen oder durch die Einführung einer oder mehrerer Nicht-Phosphor-Internukleotide, erhalten werden.

Geeignete modifizierte Internukleotide sind in Uhlmann und Peymann (1990 Chem. Rev. 90, 544) zusammengefaßt (siehe auch Beigelman et al., 1995 Nucleic Acids Res. 23: 3989-94; Dudycz, 1995, WO 95/11910; Macadam et al., 1998, WO 98/37240; Reese et al., 1997, WO 97/29116). Modifizierte Internukleotid-Phosphatreste und/oder Nicht-Phosphorbrücken in einer Nukleinsäure, die bei einer der erfindungsgemäßen Verwendungen eingesetzt werden können, enthalten zum Beispiel Methylphosphonat, Phosphorothioat, Phosphoramidat, Phosphorodithioat, Phophatester, während Nicht-Phosphor-Internukleotid-Analoge, beispielsweise Siloxanbrücken, Carbonatbrücken, Carboxymethylester, Acetamidatbrücken und/oder Thioetherbrücken enthalten. Es ist auch beabsichtigt, daß diese Modifizierung die Haltbarkeit einer pharmazeutischen Zusammensetzung, die bei einer der erfindungsgemäßen Verwendungen eingesetzt werden kann, verbessert.

In einer weiteren Ausführungsform der Erfindung werden die erfindungsgemäß verwendeten Nukleinsäuren zur Herstellung eines Vektors, vorzugsweise in Form eines shuttle Vektors, Phagemids, Cosmids, Expressionsvektors oder gentherapeutisch wirksamen Vektors verwendet. Weiterhin können unter Verwendung der vorangehend beschriebenen Nukleinsäuren knock-out Genkonstrukte oder Expressionskassetten hergestellt werden.

So kann die erfindungsgemäß verwendete Nukleinsäure in einem Vektor vorzugsweise in einem Expressionsvektor oder gentherapeutisch wirksamen Vektor enthalten sein. Vorzugsweise enthält der gentherapeutisch wirksame Vektor wund- bzw. hautspezifische regulatorische Sequenzen, die funktionell mit der vorangehend beschriebenen Nukleinsäure verbunden sind.

Die Expressionsvektoren können prokaryotische oder eukaryotische Expressionsvektoren sein. Beispiele für prokaryotische Expressionsvektoren sind für die Expression in *E. coli* z.B. die Vektoren pGEM oder pUC-Derivate und für eukaryotische Expressionsvektoren für die Expression in *Saccharomyces cerevisiae* z. B. die Vektoren p426Met25 oder p426GAL1 (Mumberg et al. (1994) Nucl. Acids Res., 22, 5767-5768), für die Expression in Insektenzellen z. B. *Baculovirus*-Vektoren wie in EP-B1-0 127 839 oder EP-B1-0 549 721 offenbart, und für die Expression in Säugerzellen z. B. die Vektoren Rc/CMV und Rc/RSV oder SV40-Vektoren, welche alle allgemein erhältlich sind.

Im allgemeinen enthalten die Expressionsvektoren auch für die jeweilige Wirtszelle geeignete Promotoren, wie z. B. den trp-Promotor für die Expression in E. coli (siehe z. B. EP-B1-0 154 133), den Met 25, GAL 1 oder ADH2-Promotor für die Expression in Hefen (Russel et al. (1983), J. Biol. Chem. 258, 2674-2682; Mumberg, supra), den Baculovirus-Polyhedrin-Promotor, für die Expression in Insektenzellen (siehe z. 13. EP-B1-0 127 839). Für die Expression in Säugetierzellen sind beispielsweise Promotoren geeignet, die eine konstitutive, regulierbare, gewebsspezifische, zellzyklusspezifische oder metabolischspezifische Expression in eukaryotischen Zellen erlauben. Regulierbare Elemente gemäß der vorliegenden Erfindung sind Promotoren, Aktivatorsequenzen, Enhancer, Silencer und/oder Repressorsequenzen.

Beispiel für geeignete regulierbare Elemente, die konstitutive Expression in Eukaryonten ermöglichen, sind Promotoren, die von der RNA Polymerase III erkannt werden oder virale Promotoren, CMV-Enhancer, CMV-Promotor (siehe auch Beispiel 13), SV40 Promotor oder LTR-Promotoren z. B. von MMTV (mouse mammary tumour virus; Lee et al. (1981) Nature 214, 228-232) und weitere virale Promotor- und Aktivatorsequenzen, abgeleitet aus beispielsweise HBV, HCV, HSV, HPV, EBV, HTLV oder HIV.

Beispiele für regulierbare Elemente, die induzierbare Expression in Eukaryonten ermöglichen, sind der Tetracyclinoperator in Kombination mit einem entsprechenden Repressor (Gossen M. et al. (1994) Curr. Opin. Biotechnol. 5, 516-20).

Vorzugsweise erfolgt die Expression von wundheilungsrelevanten Genen unter der Kontrolle von gewebespezifischen Promotoren, wobei hautspezifische Promotoren wie beispielsweise der humane K10 Promotor (Bailleul et al., 1990. Cell 62: 697-708), der humane K14 Promotor (Vassar et al., 1989, Proc. Natl. Acad. Sci. USA 86: 1563-67) oder der bovine Cytokeratin IV Promotor (Fuchs et al., 1988; The biology of wool and hair (Hrsg.: G.E. Rogers, et al.), S. 287-309. Chapman und Hall, London/New York) besonders zu bevorzugen sind.

Weitere Beispiele für regulierbare Elemente, die gewebsspezifische Expression in Eukaryonten ermöglichen, sind Promotoren oder Aktivatorsequenzen aus Promotoren oder Enhancern von solchen Genen, die für Proteine kodieren, die nur in bestimmten Zelltypen exprimiert werden.

Beispiele für regulierbare Elemente, die zellzyklusspezifische Expression in Eukaryonten ermöglichen, sind Promotoren folgender Gene: cdc25, Cyclin A, Cyclin E, cdc2, E2F, B-myb oder DHFR (Zwicker J. und Müller R. (1997) Trends Genet. 13, 3-6).

Beispiele für regulierbare Elemente, die metabolischspezifische Expression in Eukaryonten ermöglichen, sind Promotoren, die durch Hypoxie, durch Glukosemangel, durch Phosphatkonzentration oder durch Hitzeschock reguliert werden.

Um die Einführung von erfindungsgemäß verwendeten Nukleinsäuren und damit die Expression des Polypeptids in einer eu- oder prokaryotischen Zelle durch Transfektion, Transformation oder Infektion zu ermöglichen, kann die Nukleinsäure als Plasmid, als Teil eines viralen oder nicht-viralen Vektors vorliegen. Als virale Vektoren eignen sich hierbei besonders: Baculoviren, Vakziniaviren, Adenoviren, adenoassoziierte Viren und Herpesviren. Als nicht-virale Vektoren eignen sich hierbei besonders: Virosomen, Liposomen, kationische Lipide, oder poly-Lysin konjugierte DNA.

Beispiele von gentherapeutisch wirksamen Vektoren sind Virusvektoren, beispielsweise Adenovirusvektoren oder retroviralen Vektoren (Lindemann et al., 1997, Mol. Med. 3: 466-76; Springer et al., 1998, Mol. Cell. 2: 549-58). Eukaryotische Expressionsvektoren eignen sich in isolierter Form für die gentherapeutische Anwendung, da nackte DNA bei topischer Applikation in Hautzellen eindringen kann (Hengge et al., 1996, J. Clin. Invest. 97: 2911-6; Yu et al., 1999, J. Invest. Dermatol. 112: 370-5).

Gentherapeutisch wirksame Vektoren lassen sich auch dadurch erhalten, daß man die erfindungsgemäß verwendeten Nukleinsäure mit Liposomen komplexiert, da damit eine sehr hohe Transfektionseffizienz, insbesondere von Hautzellen, erreicht werden kann (Alexander und Akhurst, 1995, Hum. Mol. Genet. 4: 2279-85). Bei der Lipofektion werden kleine unilamellare Vesikel aus kationischen Lipiden durch Ultraschallbehandlung der Liposomensuspension herstellt. Die DNA wird ionisch auf der Oberfläche der Liposomen gebunden, und zwar in einem solchen Verhältnis, daß eine positive Nettoladung verbleibt und die Plasmid-DNA zu 100% von den Liposomen komplexiert wird. Neben den von Felgner et al. (1987, supra) eingesetzten Lipidmischungen DOTMA (1,2-Dioleyloxpropyl-3-trimethylammoniumbromid) und DPOE (Dioleoylphosphatidylethanolamin) wurden inzwischen zahlreiche neue Lipidformulierungen synthetisiert und auf ihre Effizienz der Transfektion verschiedener Zellinien getestet (Behr, J.P. et al. (1989), Proc. Natl. Acad. Sci. USA 86, 6982-6986; Felgner, J.H. et al. (1994) J. Biol. Chem. 269, 2550-2561; Gao, X. & Huang, L. (1991), Biochim. Biophys. Acta 1189, 195-203). Beispiele der neuen Lipidformulierungen sind DOTAP N-[1-(2,3-Dioleoyloxy)propyl]-N,N,N-trimethylammoniumethylsulfat oder DOGS (TRANSFECTAM; Dioctadecylamidoglycylspermin). Hilfsstoffe, die den Transfer von Nukleinsäuren in die Zelle erhöhen, können beispielsweise Proteine oder Peptide, die an DNA gebunden sind oder synthetische Peptid-DNA-Moleküle, die den Transport der Nukleinsäure in den Kern der Zelle ermöglichen, sein (Schwartz et al. (1999) Gene Therapy 6, 282; Branden et al. (1999) Nature Biotech. 17, 784). Hilfsstoffe umfassen auch Moleküle, die die Freisetzung von Nukleinsäuren in das Cytoplasma der Zelle ermöglichen (Planck et al. (1994) J. Biol. Chem. 269, 12918; Kichler et al. (1997) Bioconj. Chem. 8, 213) oder beispielsweise Liposomen (Uhlmann und Peymann (1990) supra). Eine andere besonders geeignete Form von gentherapeutischen Vektoren läßt sich dadurch erhalten, daß man die erfindungsgemäß verwendeten Nukleinsäure auf Goldpartikeln aufbringt und diese mit Hilfe der sogenannten "Gene Gun" in Gewebe, bevorzugt in die Haut, oder Zellen schießt (Beispiel 13; Wang et al., 1999, J. Invest. Dermatol., 112:775-81, Tuting et al., 1998, J. Invest. Dermatol. 111:183-8).

Eine weitere Form eines gentherapeutisch wirksamen Vektors läßt sich durch das Einbringen von "nackten" Expressionsvektoren in eine biokompatible Matrix, beispielsweise eine Kollagenmatrix, herstellen. Diese Matrix kann in Wunden eingebracht werden, um die einwandernden Zellen mit dem Expressionsvektor zu transfizieren und die erfindungsgemäß verwendeten Polypeptide in den Zellen zu exprimieren (Goldstein und Banadio, US 5,962,427).

Für die gentherapeutische Anwendung der vorangehend beschriebenen Nukleinsäure ist es auch von Vorteil, wenn der Teil der Nukleinsäure, der für das Polypeptid kodiert, ein oder mehrere nicht kodierende Sequenzen einschließlich Intronsequenzen, vorzugsweise zwischen Promotor und dem Startcodon des Polypeptids, und/oder eine polyA-Sequenz, insbesondere die natürlich vorkommende polyA-Sequenz oder eine SV40 Virus polyA-Sequenz, vor allem am 3'-Ende des Gens enthält, da hierdurch eine Stabilisierung der mRNA erreicht werden kann (Jackson, R. J. (1993) Cell 74, 9-14 und Palmiter, R. D. et al. (1991) Proc. Natl. Acad. Sci. USA 88, 478-482).

Knock-out Genkonstrukte sind dem Fachmann zum Beispiel aus den US-Patenten 5,625,122; US 5,698,765; US 5,583,278 und US 5,750,825 bekannt.

Ein weiterer Gegenstand der vorliegenden Erfindung ist eine Wirtszelle, insbesondere eine Hautzelle, die mit einem erfindungsgemäßen Vektor oder einem knock-out Genkonstrukt transformiert ist. Wirtszellen können sowohl prokaryotische als auch eukaryotische Zellen sein, Beispiele für prokaryotische Wirtszellen sind *E. coli* und für eukaryotische Zellen *Saccharomyces cerevisiae* oder Insektenzellen.

Ein besonders bevorzugte transformierte Wirtszelle ist eine transgene embryonale nichtmenschliche Stammzelle, die dadurch gekennzeichnet ist, daß sie ein erfindungsgemäßes knock-out Genkonstrukt oder eine erfindungsgemäße Expressionskassette umfaßt. Verfahren zur Transformation von Wirtszellen und/oder Stammzellen sind dem Fachmann gut bekannt und umfassen zum Beispiel Elektroporation oder Mikroinjektion.

Ein weiterer Gegenstand der Erfindung ist ein transgenes nichtmenschliches Säugetier, dessen Genom ein vorangehend beschriebenes knock-out Genkonstrukt oder eine vorangehend beschriebene Expressionskassette umfaßt. Transgene Tiere zeigen im allgemeinen eine gewebespezifisch erhöhte Expression der Nukleinsäuren und/oder Polypeptide und lassen sich zur Analyse von Wundheilungsstörungen verwenden. So weist beispielsweise eine Activin A transgene Maus eine verbesserte Wundheilung auf (Munz et al., 1999, EMBO J. 18:5205-15) während eine transgene Maus mit dominant negativem KGF Rezeptor eine verzögerte Wundheilung aufweist (Werner et al., 1994, Science 266:819-22).

Verfahren zur Herstellung von transgenen Tieren, insbesondere der Maus, sind dem Fachmann ebenfalls aus der DE 196 25 049 und den US 4,736,866; US 5,625,122; US 5,698,765; US 5,583,278 und US 5,750,825 bekannt und umfassen transgene Tiere, die beispielsweise über direkte Injektion von Expressionsvektoren (s.o.) in Embryonen oder Spermatozyten oder über die Transfektion von Expressionsvektoren in embryonaler Stammzellen erzeugt werden können (Polites und Pinkert: DNA Microinjection and Transgenic Animal Produktion, Seite 15 bis 68 in Pinkert, 1994: Transgenic animal technology: a laboratory handbook, Academic Press, London, UK; Houdebine, 1997, Harwood Academic Publishers, Amsterdam, The Netherlands; Doetschman: Gene Transfer in Embryonic Stem Cells, Seite 115 bis 146 in Pinkert, 1994, supra; Wood: Retrovirus-Mediated Gene Transfer, Seite 147 bis 176 in Pinkert, 1994, supra; Monastersky: Gene Transfere Technology: Alternative Techniques and Applications, Seite 177 bis 220 in Pinkert, 1994, supra).

Werden erfindungsgemäß verwendete Nukleinsäuren in sogenannte Targeting Vektoren intergriert (Pinkert, 1994, supra) können nach Transfektion von embryonalen Stammzellen und homologer Rekombination beispielsweise knock-out Mäuse generiert werden, die im allgemeinen als heterozygote Mäuse verringerte Expression der Nukleinsäure zeigen, während homozygote Mäuse keine Expression der Nukleinsäure mehr aufweisen. Auch die so erzeugten Tiere lassen sich zur Analyse von Wundheilungsstörungen verwenden. So weisen beispielsweise die eNOS- (Lee et al., 1999, Am. J. Physiol. 277:H1600-H1608), Nf-1 (Atit et al., 1999, J. Invest. Dermatol. 112:835-42) und Osteopontin (Liaw et al., 1998, J. Clin. Invest. 101:967-71) knock-out Mäuse eine verschlechterte Wundheilung auf. Auch hier ist eine gewebespezifische Reduktion der Expression wundheilungsrelevanter Gene, beispielsweise in hautspezifischen Zellen unter Verwendung des Cre-loxP Systems (stat3 knock-out, Sano et al., EMBO J 1999 18:4657-68), besonders zu bevorzugen. So erzeugte transgene und knock-out Zellen oder Tiere lassen sich auch zum Screening und zur Identifizierung von pharmakologisch aktiven Substanzen bzw. gentherapeutisch aktiven Vektoren verwenden.

Ein weiterer Gegenstand der Erfindung betrifft ein Verfahren zur Herstellung eines Polypeptids zur Diagnose und/oder Prävention und/oder Behandlung von Erkrankungen, insbesondere Hauterkrankungen oder Behandlung bei der Wundheilung oder zur Identifizierung von pharmakologisch aktiven Substanzen in einer geeigneten Wirtszelle, das dadurch gekennzeichnet ist, daß eine vorangehend beschriebene Nukleinsäure verwendet wird.

Das Polypeptid wird beispielsweise durch Expression der vorangehend beschriebenen Nukleinsäure in einem geeigneten Expressionssystem, wie oben bereits dargestellt, nach dem Fachmann allgemein bekannten Methoden hergestellt. Als Wirtszellen eignen sich beispielsweise die *E. coli* Stämme DHS, HB101 oder BL21, der Hefestamm *Saccharomyces cerevisiae,* die Insektenzellinie Lepidopteran, z. B. von *Spodoptera frugiperda,* oder die tierischen Zellen COS, Vero, 293, HaCaT, und HeLa, die alle allgemein erhältlich sind.

Ein weiterer Gegenstand der Erfindung ist ein Verfahren zur Herstellung eines Fusionsproteins zur Diagnose und/oder Prävention und/oder Behandlung von Erkrankungen, insbesondere Hauterkrankungen oder Behandlung bei der Wundheilung oder zur Identifizierung von pharmakologisch aktiven Substanzen in einer geeigneten Wirtszelle, bei dem eine vorangehend beschriebene Nukleinsäure verwendet wird.

Hergestellt werden hierbei Fusionsproteine, die die oben beschriebenen Polypeptide enthalten, wobei die Fusionsproteine selbst bereits die Funktion eines vorangehend beschriebenen Polypeptids aufweisen oder erst nach Abspaltung des Fusionsanteils die spezifische Funktion funktionell aktiv sind. Vor allem zählen hierzu Fusionsproteine mit einem Anteil von ca. 1- 300, vorzugsweise ca. 1-200, besonders bevorzugt ca. 1-150, insbesondere ca. 1-100, vor allem ca. 1-50 fremden Aminosäuren. Beispiele solcher Peptidsequenzen sind prokaryotische Peptidsequenzen, die z. B. aus der Galactosidase von *E. coli* abgeleitet sein können. Weiterhin können auch virale Peptidsequenzen, wie zum Beispiel vom Bakteriophagen M13 verwendet werden, um so Fusionsproteine für das dem Fachmann bekannte "phage display"-Verfahren zu erzeugen.

Weitere bevorzute Beispiel für Peptidsequenzen für Fusionsproteine sind Peptide, die die Detektion des Fusionsproteins erleichtern, hierzu zählen beispielsweise "Green-fluorescent-protein" oder Varianten davon.

Zur Aufreinigung der vorangehend beschriebenen Proteine kann ein weiteres/weiterer Polypeptid("tag") angefügt sein. Geignete Protein-tags erlauben beispielsweise die hochaffine Absorption an eine Matrix, stringentes Waschen mit geeigneten Puffern, ohne den Komplex in nennenswertem Maße zu eluieren und anschließend gezielte Elution des absorbierten Komplexes. Beispiele der dem Fachmann bekannten Protein-tags sind ein (His)₆-tag, ein Myc-tag, eine FLAG-tag, ein Hämagluteinin-tag, Glutathion-Transferase (GST)-tag, Intein mit einem Affinitäts-Chintin-binding-tag oder Maltose-binding protein (MBP)-tag. Diese Protein-tags können sich N-, C-terminal und/oder intern befinden.

Ein weiterer Gegenstand der Erfindung betrifft ein Verfahren zur Herstellung eines Antikörpers, vorzugsweise eines polyklonalen oder monoklonalen Antikörpers zur Diagnose und/oder Prävention und/oder Behandlung von Erkrankungen, insbesondere Hauterkrankungen oder Behandlung bei der Wundheilung oder zur Identifizierung von pharmakologisch aktiven Substanzen, bei dem ein Polypeptid oder funktionelle Äquivalente davon oder Teile davon mit mindestens 6 Aminosäuren, vorzugsweise mit mindestens 8 Aminosäuren, insbesondere mit mindestens 12 Aminosäuren gemäß der vorliegenden Erfindung verwendet wird.

Das Verfahren erfolgt nach dem Fachmann allgemein bekannten Methoden durch Immunisieren eines Säugetiers, beispielsweise eines Kaninchens, mit dem vorangehend beschriebenen Polypeptid oder den genannten Teilen davon, gegebenenfalls in Anwesenheit von z. B. Freund's Adjuvant und/oder Aluminiumhydroxidgelen (siehe z. B. Diamond, B. A. et al. (1981) The New England Journal of Medicine, 1344-1349). Die im Tier aufgrund einer immunologischen Reaktion entstandenen polyklonalen Antikörper lassen sich anschließend nach allgemein bekannten Methoden leicht aus dem Blut isolieren und z. B. über Säulenchromatographie reinigen. Monoklonale Antikörper können beispielsweise nach der bekannten Methode von Winter & Milstein (Winter, G. & Milstein, C. (1991) Nature, 349, 293-299) hergestellt werden.

Ein weiterer Gegenstand der vorliegenden Erfindung ist ein Antikörper zur Diagnose und/oder Prävention und/oder Behandlung von Erkrankungen, insbesondere Hauterkrankungen oder Behandlung bei der Wundheilung oder zur Identifizierung von pharmakologisch aktiven Substanzen, der gegen ein vorangehend beschriebenes Polypeptid gerichtet ist und mit den vorangehend beschriebenen Polypeptiden spezifisch reagiert, wobei die oben genannten Teile des Polypeptids entweder selbst immunogen sind oder durch Kopplung an geeignete Träger, wie z. B. bovines Serumalbumin, immunogen gemacht bzw. in ihrer Immunogenität gesteigert werden können. Dieser Antikörper ist entweder polyklonal oder monoklonal, bevorzugt ist ein monoklonaler Antikörper. Unter dem Begriff Antikörper versteht man gemäß der vorliegenden Erfindung auch gentechnisch hergestellte und gegebenenfalls modifizierte Antikörper bzw. antigenbindende Teile davon, wie z.B. chimäre Antikörper, humanisierte Antikörper, multifunktionelle Antikörper, bi- oder oligospezifische Antikörper, einzelsträngige Antikörper, F(ab)- oder F(ab)₂-Fragmente (siehe z.B. EP-B1-0 368 684, US 4,816,567, US 4,816,397, WO 88/01649, WO 93/06213, WO 98/24884).

Die erfindungsgemäßen Antikörper können zur Diagnose und/oder Prävention und/oder Behandlung von Erkrankungen, insbesondere Hauterkrankungen oder Behandlung bei der Wundheilung oder zur Identifizierung von pharmakologisch aktiven Substanzen verwendet werden.

So kann beispielsweise die lokale Injektion von monoklonalen Antikörpern gegen TGF beta 1 im Tiermodell die Wundheilung verbessern (Ernst et al., 1996, Gut 39:172-5).

Die vorliegende Erfindung betrifft auch ein Verfahren zur Herstellung eines Arzneimittels zur Behandlung von Erkrankungen, insbesondere Hauterkrankungen und/oder Erkrankungen bei der Wundheilung, bei dem mindestens eine Nukleinsäure, mindestens ein Polypeptid oder mindestens ein Antikörper gemäß der vorliegenden Erfindung zusammen mit geeigneten Zusatz- und Hilfsstoffen kombiniert wird.

Die vorliegende Erfindung betrifft weiterhin ein nach diesem Verfahren hergestelltes Arzneimittel zur Behandlung von Erkrankungen, insbesondere Hauterkrankungen und/oder Erkrankungen bei der Wundheilung, das mindestens eine Nukleinsäure, mindestens ein Polypeptid oder mindestens einen Antikörper gemäß der vorliegenden Erfindung, gegebenenfalls zusammen mit geeigneten Zusatz- und Hilfsstoffen, enthält. Die Erfindung betrifft weiterhin die Verwendung dieses Arzneimittels zur Behandlung von Erkrankungen, insbesondere Hauterkrankungen und/oder Erkrankungen bei der Wundheilung.

Die Therapie der Erkrankungen, insbesondere Hauterkrankungen und/oder Erkrankungen bei der Wundheilung kann auf herkömmliche Weise, z.B. durch Verbände, Pflaster, Kompressen oder Gele erfolgen, die die erfindungsgemäßen Arzneimittel enthalten. So ist es möglich, die geeignete Zusatz- oder Hilfsstoffe, wie z. B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs, etc., enthaltenden Arzneimittel topisch und lokal zu verabreichen, um die Wundheilung sofort und unmittelbar zu beeinflussen. Die Verabreichung der erfindungsgemäßen Arzneimittel kann weiterhin gegebenenfalls in Form von Liposomenkomplexen bzw. Goldpartikelkomplexen ebenfalls topisch und lokal im Bereich der Wunde erfolgen. Weiterhin kann die Behandlung mittels eines transdermalen therapeutischen Systems (TTS) erfolgen, das eine zeitlich gesteuerte Abgabe der erfindungsgemäßen Arzneimittel ermöglicht. Die Behandlung mittels der erfindungsgemäßen Arzneimittel kann aber auch über orale Dosierungsformen, wie z.B. Tabletten oder Kapseln, über die Schleimhäute, zum Beispiel der Nase oder der Mundhöhle, oder in Form von unter die Haut implantierten Dispositorien erfolgen. TTS sind zum Beispiel aus den EP 0 944 398 A1, EP 0 916 336 A1, EP 0 889 723 A1 oder EP 0 852 493 A1 bekannt.

Die vorliegende Erfindung betrifft weiterhin ein Verfahren zur Herstellung eines Diagnostikums zur Diagnose von Erkrankungen, insbesondere Hauterkrankungen oder Erkrankungen bei der Wundheilung, das dadurch gekennzeichnet ist, daß mindestens eine Nukleinsäure, mindestens ein Polypeptid oder mindestens ein Antikörper gemäß der vorliegenden Erfindung, gegebenenfalls zusammen mit geeigneten Zusatz- und Hilfsstoffen, verwendet wird.

Beispielsweise kann gemäß der vorliegenden Erfindung anhand einer der vorangehend beschriebenen Nukleinsäure ein Diagnostikum auf der Basis der Polymerasekettenreaktion (Beispiele 5, 8, 9; PCR-Diagnostik, z. B. gemäß EP 0 200 362) oder eines RNase-Protection-Assays, wie in Beispiel 4 näher dargestellt, hergestellt werden. Diese Tests beruhen auf der spezifischen Hybridisierung der vorangehend beschriebenen Nukleinsäuren mit dem komplementären Gegenstrang, üblicherweise der entsprechenden mRNA. Die erfindungsgemäße Nukleinsäure kann hierbei auch modifiziert sein, wie z. B. in EP 0 063 879 beschrieben. Vorzugsweise wird ein vorangehend beschriebenes DNA-Fragment mittels geeigneter Reagenzien, z. B. radioaktiv mit α-P³²-dCTP oder nicht-radioaktiv mit Biotin oder Digoxigenin, nach allgemein bekannten Methoden markiert und mit isolierter RNA, die vorzugsweise vorher an geeignete Membranen aus z. B. Cellulose oder Nylon gebunden wurde, inkubiert. Bei gleicher Menge an untersuchter RNA aus jeder Gewebeprobe kann somit die Menge an mRNA bestimmt werden, die spezifisch durch die Sonde markiert wurde und mit der Menge an mRNA aus gesundem Gewebe veglichen werden. Alternativ kann die Bestimmung an mRNA auch in Gewebeschnitten mit Hilfe der in situ Hybridisierung (siehe z.B. Werner et al., 1992, Proc. Natl. Acad. Sci. U S A 89:6896-900) erfolgen.

Mit Hilfe des erfindungsgemäßen Diagnostikums kann somit auch eine Gewebeprobe *in vitro* auf die Expressionsstärke des korrespondierenden Gens spezifisch gemessen werden, um eine mögliche Wundheilungsstörung oder dermatologische Erkrankungen sicher diagnostizieren zu können (Beispiele 4, 5, 8, 9 und 10). Insbesondere eignet sich ein solches Verfahren zur frühzeitigen Prognose von Störungen. Dies ermöglicht einen präventiven Therapieeinsatz und die Analyse von Prädispositionen. So ist die Expression des Gens *spi*2 schon im nicht verwundeten Zustand in der intakten Haut verringert, die nach Verwundung Wundheilungsstörungen aufwies. Die Expression des Gens *spi*2 ermöglicht daher eine Voraussage der Wundheilungsstörung noch im intakten Gewebe.

Ein weiterer Gegenstand der vorliegenden Erfindung betrifft ein Diagnostikum zur Diagnose von Erkrankungen, insbesondere Hauterkrankungen oder Erkrankungen bei der Wundheilung, das mindestens eine Nukleinsäure, mindestens ein Polypeptid oder mindestens einen Antikörper gemäß der Erfindung, gegebenenfalls zusammen mit geeigneten Zusatz- und Hilfsstoffen, umfaßt.

Ein weiteres erfindungsgemäßes Diagnostikum enthält das erfindungsgemäß verwenbare Polypeptid bzw. die oben näher beschriebenen immunogenen Teile davon. Das Polypeptid bzw. die Teile davon, die vorzugsweise an eine Festphase, z. B. aus Nitrocellulose oder Nylon, gebunden sind, können beispielsweise mit der zu untersuchenden Körperflüssigkeit, z. B. Wundsekret, in vitro in Berührung gebracht werden, um so beispielsweise mit Autoimmunantikörper reagieren zu können. Der Antikörper-Peptid-Komplex kann anschließend beispielsweise anhand markierter Antihuman-IgG- oder Antihuman-IgM-Antikörper nachgewiesen werden. Bei der Markierung handelt es sich beispielsweise um ein Enzym, wie Peroxidase, das eine Farbreaktion katalysiert. Die Anwesenheit und die Menge an anwesenden Autoimmunantikörper kann somit über die Farbreaktion leicht und schnell nachgewiesen werden.

Ein anderes Diagnostikum enthält die erfindungsgemäßen Antikörper selbst. Mit Hilfe dieser Antikörper kann beispielsweise eine Gewebeprobe leicht und schnell dahingehend untersucht werden, ob das betreffende Polypeptid in einer erhöhten Menge vorhanden ist, um dadurch einen Hinweis auf eine mögliche Wundheilungsstörung zu erhalten. In diesem Fall sind die erfindungsgemäßen Antikörper beispielsweise mit einem Enzym, wie oben bereits beschrieben, markiert, Der spezifische Antikörper-Peptid-Komplex kann dadurch leicht und ebenso schnell über eine enzymatische Farbreaktion nachgewiesen werden (siehe Beispiele 11 und 12).

Ein weiteres erfindungsgemäßes Diagnostikum umfaßt eine Sonde, vorzugsweise eine DNA-Sonde, und/oder Primer. Dies eröffnet eine weitere Möglichkeit, die erfindungsgemäß verwendbaren Nukleinsäuren, zum Beispiel durch die Isolierung aus einer geeigneten Genbank, beispielsweise aus einer wundspezifischen Genbank, anhand einer geeigneten Sonde zu erhalten (siehe z. B. J. Sambrook et al., 1989, Molecular Cloning. A Laboratory Manual 2^{nd} edn., Cold Spring Harbor Laboratory, Cold Spring Harbor, NY Kapitel 8 Seite 8.1 bis 8.81, Kapitel 9 Seite 9.47 bis 9.58 und Kapitel 10 Seite 10.1 bis 10.67).

Als Sonde eignen sich beispielsweise DNA- oder RNA-Fragmente mit einer Länge von ca. 100-1000 Nukleotiden, vorzugsweise mit einer Länge von ca. 200-500 Nukleotiden, insbesondere mit einer Länge von ca. 300-400 Nukleotiden deren Sequenz aus den Polypeptidsequenzen gemäß SEQ ID Nr. 1 bis SEQ ID Nr. 48, SEQ ID Nr. 51 bis SEQ ID Nr. 58, SEQ ID Nr. 89 bis SEQ ID Nr. 94, SEQ ID Nr. 101 bis SEQ ID Nr. 106 oder SEQ ID Nr. 109 bis SEQ ID Nr. 114 des Sequenzprotokolls, den Nukleinsäuresequenzen gemäß SEQ ID Nr. 49 bis SEQ ID Nr. 50 des Sequenzprotokolls und/oder anhand der cDNA Sequenzen der in den Figuren 4 bis 6 angegebenen Datenbankeinträge abgeleitet werden kann (siehe auch Beispiele 4 und 10).

Alternativ können anhand der abgeleiteten Nukleinsäuresequenzen Oligonukleotide synthetisiert werden, die sich als Primer für eine Polymerase Kettenreaktion eignen. Mit diesen kann die erfindungsgemäß verwendbare Nukleinsäure oder Teile dieser aus cDNA, beispielsweise wundspezifischer cDNA, amplifiziert und isoliert werden (Beispiele 5, 6, 8, 9 und 10). Als Primer eignen sich beispielsweise DNA-Fragmente mit einer Länge von ca. 10-100 Nukleotiden, vorzugsweise mit einer Länge von ca. 15 bis 50 Nukleotiden, insbesondere mit einer Länge von 20-30 Nukleotiden deren Sequenz aus den Polypeptiden gemäß den SEQ ID Nr. 1 bis SEQ ID Nr. 48, SEQ ID Nr.51 bis SEQ ID Nr. 58, SEQ ID Nr. 89 bis SEQ ID Nr. 94, SEQ ID Nr. 101 bis SEQ ID Nr. 106 oder SEQ ID Nr. 109 bis SEQ ID Nr. 114 des Sequenzprotokolls, den Nukleinsäuresequenzen gemäß SEQ ID Nr. 49 bis SEQ ID Nr. 50 des Sequenzprotokolls und/oder anhand der cDNA Sequenzen der in den Figuren 4 bis 6 angegebenen Datenbankeinträge abgeleitet werden kann (Beispiel 4).

Ein weiterer Gegenstand der Erfindung betrifft ein Verfahren zur Herstellung eines Tests zur Auffindung funktioneller Interaktoren in Zusammenhang mit Erkrankungen, insbesondere Hauterkrankungen oder Behandlung bei der Wundheilung, dadurch gekennzeichnet, daß mindestens eine Nukleinsäure, mindestens ein Polypeptids oder mindestens ein Antikörper gemäß der vorliegenden Erfindung, gegebenenfalls zusammen mit geeigneten Zusatz- und Hilfsstoffen, zur Herstellung des Tests verwendet wird.

Unter dem Begriff "funktionelle Interaktoren" im Sinne der vorliegenden Erfindung sind alle diejenigen Moleküle, Verbindungen und/oder Zusammensetzungen und Stoffgemische zu verstehen, die mit den vorangehend beschriebenen Nukleinsäuren, Polypeptiden oder Antikörpern, gegebenenfalls zusammen mit geeigneten Zusatz- und Hilfsstoffen, unter geeigneten Bedingungen in Wechselwirkung treten können. Mögliche Interaktoren sind einfache chemische organische oder anorganische Moleküle oder Verbindungen, können aber auch Peptide, Proteine oder Komplexe davon umfassen. Die funktionellen Interaktoren können aufgrund ihrer Wechselwirkung die Funktion(en) der Nukleinsäuren, Polypeptide oder Antikörper in vivo oder in vitro beeinflussen oder auch nur an die vorangehend beschriebenen Nukleinsäuren, Polypeptide oder Antikörper binden oder mit ihnen andere Wechselwirkungen kovalenter oder nicht-kovalenter Weise eingehen.

Die Erfindung umfaßt weiterhin einen erfindungsgemäß hergestellten Test zur Identifizierung funktioneller Interaktoren in Zusammenhang mit Erkrankungen, insbesondere Hauterkrankungen oder Behandlung bei der Wundheilung, der mindestens eine Nukleinsäure, mindestens ein Polypeptid oder mindestens einen Antikörper gemäß der vorliegenden Erfindung, gegebenenfalls zusammen mit geeigneten Zusatz- und Hilfsstoffen, umfaßt.

Ein geeignetes System läßt sich beispielsweise durch die stabile Transformation von epidermalen bzw. dermalen Zellen mit Expressionsvektoren, die selektierbare Markergene und die vorangehend beschriebenen Nukleinsäuren enthalten, herstellen. Bei diesem Verfahren wird die Expression der erfindungsgemäßen Nukleinsäuren in den Zellen so verändert, daß sie der pathologisch gestörten Expression in vivo entspricht. Auch anti-sense Oligonukleotide, die die erfindungsgemäß verwendbaren Nukleinsäuresequenzen enthalten, können zu diesem Zweck eingesetzt werden. Von besonderem Vorteil für diese Systeme ist es daher, das Expressionsverhalten der Gene bei gestörten regenerativen Prozessen, wie in dieser Anmeldung offengelegt, zu kennen. Oft kann so das pathologische Verhalten der Zellen in vitro nachgeahmt werden und es können Substanzen gesucht werden, die das normale Verhalten der Zellen wieder herstellen und die ein therapeutisches Potential besitzen.

Für diese Testsysteme eignen sich z.B. HaCaT-Zellen, die allgemein erhältlich sind, und der Expressionsvektor pCMV4 (Anderson et al., 1989, J. Biol. Chem. 264:8222-9). Die erfindungsgemäß verwendbare Nukleinsäure kann dabei sowohl in sense als auch in anti-sense Orientierung in die Expressionsvektoren integriert werden, so daß die funktionelle Konzentration an mRNA der entsprechenden Gene in den Zellen entweder erhöht, oder durch Hybridisierung mit der antisense-RNA erniedrigt wird. Nach der Transformation und Selektion stabiler Transformanden zeigen die Zellen in Kultur im allgemeinen ein verändertes Proliferations-, Migrations, und/oder Differenzierungsverhalten im Vergleich zu Kontrollzellen. Dieses Verhalten in vitro ist häufig mit der Funktion der entsprechenden Gene bei regenerativen Prozessen im Organismus korreliert (Yu et al., 1997, Arch. Dermatol. Res. 289:352-9; Mils er al., 1997, Oncogene 14: 15555-61; Charvat et al., 1998, Exp Dermatol 7: 184-90; Mythily et al., 1999, J. Gen. Virol. 80:1707-13; Werner, 1998, Cytokine Growth Factor Rev. 9:153-65) und läßt sich mit einfachen und schnell durchzuführenden Tests nachweisen, so daß man darauf basierend Testsysteme für pharmakologisch aktive Substanzen aufbauen kann. So läßt sich das Proliferationsverhalten von Zellen sehr schnell durch z.B. den Einbau von markierten Nukleotiden in die DNA der Zellen (siehe z.B. de Fries und Mitsuhashi, 1995, J. Clin. Lab. Anal. 9:89-95; Perros und Weightman, 1991, Cell Prolif. 24:517-23; Savino und Dardenne, 1985, J. Immunol. Methods 85:221-6), durch Anfärbung der Zellen mit spezifischen Farbstoffen (Schulz et al., 1994, J. Immunol. Methods 167:1-13) oder über immunologische Verfahren (Frahm et al., 1998, J. Immunol. Methods 211:43-50) nachweisen. Die Migration läßt sich einfach durch den "Migration Index" Test (Charvat et al., supra) und vergleichbare Testsysteme (Benestad et al., 1987, Cell Tissue Kinet. 20:109-19, Junger et al., 1993, J. Immunol. Methods 160:73-9) nachweisen. Als Differenzierungsmarker eignen sich z.B. Keratin 6, 10 und 14 sowie Loricrin und Involucrin (Rosenthal et al., 1992, J, Invest, Dermatol, 98:343-50), deren Expression z.B. über allgemein erhältliche Antikörper leicht nachzuweisen ist.

Ein anderes geeignetes Testsystem basiert auf der Identifikation funktioneller Interaktionen mit dem sogenannten "Two-Hybrid System" (Fields und Sternglanz, 1994, Trends in Genetics, 10, 286-292; Colas und Brent, 1998 TIBTECH, 16, 355-363). Bei diesem Test werden Zellen mit Expressionsvektoren transformiert, die Fusionsproteine aus dem erfindungsgemäß verwendbaren Polypeptid und einer DNA-Bindungsdomäne eines Transkriptionsfaktors wie beispielsweise Gal4 oder LexA exprimieren. Die transformierten Zellen enthalten außerdem ein Reportergen, dessen Promotor Bindungsstellen für die entsprechende DNA Bindungsdomäne enthalten. Durch Transformation eines weiteren Expressionsvektors, der ein zweites Fusionsprotein aus einem bekannten bzw. unbekannten Polypeptid mit einer Aktivierungsdomäne, beispielsweise von Gal4 oder Herpes Virus VP16, exprimiert, kann die Expression des Reportergens stark gesteigert werden, wenn das zweite Fusionsprotein mit dem erfindungsgemäß verwendbaren Polypeptid funktionell interagiert. Diese Expressionssteigerung kann man ausnutzen, um neue Interaktoren zu identifizieren, beispielsweise indem man zur Konstruktion des zweiten Fusionsproteins eine cDNA-Bibliothek aus sich regenerierenden Gewebe herstellt. Zudem läßt sich dieses Testsystem zum Screening von Substanzen ausnutzen, die eine Interaktion zwischen dem erfindungsgemäß verwendbaren Polypeptid und einem funktionellen Interaktor inhibieren. Solche Substanzen verringern die Expression des Reportergens in Zellen, die Fusionsproteine des erfindungsgemäß verwendbaren Polypeptids und des Interaktors exprimieren (Vidal und Endoh, 1999, Trends in Biotechnology, 17:374-81). So lassen sich schnell neue Wirkstoffe identifizieren, die zur Therapie von Störungen regenerativer Prozesse eingesetzt werden können.

Funktionelle Interaktoren der erfindungsgemäß verwendbaren Polypeptide können auch Nukleinsäuren sein, die über Selektionsverfahren, wie beispielsweise SELEX (siehe Jayasena, 1999, Clin. Chem. 45:1628-50; Klug und Famulok, 1994, M. Mol. Biol. Rep. 20:97-107; Toole et al., 1996, US 5582981) isoliert wereden. Im SELEX-Verfahren werden typischerweise aus einem großen Pool unterschiedlicher, einzelsträngige RNA Moleküle durch wiederholte Amplifikation und Selektion diejenigen Moleküle isoliert, die an ein Polypeptide mit hoher Affinität binden (Aptamere). Aptamere können auch in ihrer spiegelbildlichen Form, beispielsweise als L-Ribonukleotid, synthetisiert und selektioniert werden (Nolte et al., 1996, Nat. Biotechnol. 14:1116-9; Klussmann et al., 1996, Nat. Biotechnol. 14:1112-5). So isolierte Formen haben den Vorteil, das sie nicht von natürlich vorkommenden Ribonukleasen abgebaut werden und daher größere Stabilität besitzen.

Ein weiterer Gegenstand der Erfindung ist ein Verfahren zur Herstellung eines auf einem Trägermaterial fixierten Arrays zur Analyse in Zusammenhang mit Erkrankungen, insbesondere Hauterkrankungen oder Erkrankungen bei der Wundheilung, bei dem mindestens eine Nukleinsäure, mindestens ein Polypeptid und/oder mindestens ein Antikörper gemäß der Erfindung zur Herstellung verwendet wird.

Verfahren zur Herstellung von solchen Arrays sind zum Beispiel aus der WO 89/10977, WO 90/15070, WO 95/35505 und US 5,744,305 mittels Spottings, Druckens oder Festphasenchemie in Verbindung mit photolabilen Schutzgruppen bekannt.

Ein weiterer Gegenstand der Erfindung ist ein auf einem Trägermaterial fixiertes Array zur Analyse in Zusammenhang mit Erkrankungen, insbesondere Hauterkrankungen oder Erkrankungen bei der Wundheilung, das dadurch gekennzeichnet ist, daß es mindestens eine Nukleinsäure und/oder mindestens ein Polypeptid und/oder mindestens einen Antikörper gemäß der vorliegenden Erfindung umfaßt.

Ein weiterer Gegenstand der Erfindung ist ein Verfahren zur Herstellung eines DNA-Chips und/oder Protein-Chips zur Analyse in Zusammenhang mit Erkrankungen und/oder in Zusammenhang mit Wundheilung, das dadurch gekennzeichnet ist, daß mindestens eine Nukleinsäure, mindestens ein Polypeptid oder mindestens ein Antikörper, wie vorangehend beschrieben, zur Herstellung verwendet wird.

Verfahren zur Herstellung solcher DNA-Chips und/oder Protein-Chips sind zum Beispiel aus der WO 89/10977, WO 90/15070, WO 95/35505 und US 5,744,305 mittels Spottings, Druckens oder Festphasenchemie in Verbindung mit photolabilen Schutzgruppen bekannt.

Ein weiterer Gegenstand der Erfindung umfaßt einen DNA-Chip und/oder Protein-Chip zur Analyse in Zusammenhang mit Erkrankungen, insbesondere Hauterkrankungen oder Erkrankungen bei der Wundheilung, der mindestens eine Nukleinsäure und/oder mindestens ein Polypeptid und/oder oder mindestens einen Antikörper gemäß der vorliegenden Erfindung umfaßt. DNA-Chips sind zum Beispiel aus der US 5,837,832 bekannt.

Ein Gegenstand der vorliegenden Erfindung ist auch ein Arzneimittel zur Indikation und Therapie, das eine erfindungsgemäß verwendbare Nukleinsäure oder ein erfindungsgemäß verwendbares Polypeptid und gegebenenfalls geeignete Zusatz- oder Hilfsstoffe enthält und ein Verfahren zur Herstellung eines solchen Arzneimittels zur Behandlung von dermatologischen Erkrankungen, insbesondere von Wundheilungsstörungen, bei dem eine erfindungsgemäß verwendbare Nukleinsäure oder ein erfindungsgemäß verwendbares Polypeptid mit einem pharmazeutisch annehmbaren Träger formuliert wird.

Für die gentherapeutische Anwendung beim Menschen ist vor allem ein Arzneimittel geeignet, das die erfindungsgemäß verwendbare Nukleinsäure in nackter Form oder in Form eines der oben beschriebenen gentherapeutisch wirksamen Vektoren oder in mit Liposomen bzw. Goldpartikeln komplexierter Form enthält. Der pharmazeutische Träger ist beispielsweise eine physiologische Pufferlösung, vorzugsweise mit einem pH von ca. 6,0-8,0, vorzugsweise von ca. 6,8-7,8. Insbesondere von ca. 7,4 und/oder einer Osmolarität von ca. 200-400 milliosmol/Liter, vorzugsweise von ca. 290-310 milliosmol/Liter. Zusätzlich kann der pharmazeutische Träger geeignete Stabilisatoren, wie z. B. Nukleaseinhibitoren, vorzugsweise Komplexbildner wie EDTA und/oder andere dem Fachmann bekannte Hilfsstoffe enthalten.

Die Verabreichung der vorangehend beschriebenen Nukleinsäure gegebenenfalls in Form der oben näher beschriebenen Virusvektoren oder als Liposomenkomplexe bzw. Goldpartikelkomplex erfolgt üblicherweise topisch und lokal im Bereich der Wunde. Es ist auch möglich, das Polypeptid selbst mit geeigneten Zusatz- oder Hilfsstoffen, wie z. B. physiologische Kochsalzlösung, entmineralisiertes Wasser, Stabilisatoren, Proteinaseinhibitoren, Gelformulierungen, wie z.B. weiße Vaseline, dünnflüssiges Paraffin und/oder gelbes Wachs, etc., zu verabreichen, um die Wundheilung sofort und unmittelbar zu beeinflussen.

Die Nukleinsäuren der erfindungsgemäß verwendbaren Polypeptide wurden aus cDNA Bibliotheken isoliert, die aus intakter und verwundeter Haut hergestellt wurden. Dabei wurden die cDNAs ausgewählt, die unterschiedliche Häufigkeiten in gut heilenden im Vergleich zu schlecht heilenden Wunden aufwiesen (Beispiel 1). Dies geschah beispielsweise mit Hilfe von subtraktiver Hybridisierung (Diatchenko et al., 1996, Proc. Natl. Acad. Sci. USA 93: 6025-30) und /oder mit dem vergleichenden Auszählen von Klonen in cDNA Bibliotheken mittels Sequenzierung ("ESTs", Adams et al., 1992, Nature 355, 632-4; Adams et al., 1991, Science 252, 1651-6). Die so ausgewählten cDNAs entstammen Genen, die bei Wundheilungsstörungen entweder stärker oder schwächer exprimiert werden als bei normal verlaufender Wundheilung.

Allgemein ist die Analyse von differentiell exprimierten Genen in Geweben mit deutlich mehr Fehlern in Form von falsch positiven Klone behaftet, als bei der Analyse von Zellkultursystemen. Dieses Problem kann nicht durch die Verwendung eines definierten Zellkultursystems umgangen werden, da ein solches auf Grund der Komplexität der Wundheilung nicht zur Verfügung steht und bestehende, einfache Zellkultursysteme das Gewebe nicht ausreichend wiederspiegeln. Das Problem besteht insbesondere bei der Haut, die aus einer Vielzahl von verschiedenen Zelltypen besteht. Darüber hinaus ist der Prozeß der Wundheilung ein höchst komplizierter Vorgang, der zeitliche und räumliche Änderungen zellulärer Vorgänge, wie Proliferation und Differenzierung bei den unterschiedlichen Zelltypen umfaßt. Der Erfolg des Screens war aufgrund dieser Schwierigkeiten wesentlich von der Wahl der experimentellen Parameter abhängig. Hier wurde durch die gezielte Wahl von Parametern erstmals eine neuartige Screening- und Verifizierungsstrategie verwendet. Beispielsweise ist der Tag der Biopsienahme kritisch für den Erfolg des Screenings: Wundheilungsstörungen und Hautkrankheiten liegen häufig Störungen bei der Zellproliferation und Zellmigration zugrunde. Diese Prozesse werden am Tag 1 nach Verwundung initiiert, weshalb eine Analyse der molekularen Prozesse vor diesem Zeitpunkt wenig Aufschluß über die Vorgänge liefern würde, die für eine normal verlaufende Wundheilung essentiell sind. Andererseits verändert sich im Verlauf der Wundheilung nach Tag 1 nach Verwundung die Zusammensetzung der Zelltypen in der Wunde stark. Dies kann dazu führen, daß eine differentielle Expression eines bestimmten Gens in der Wunde gemessen wird, die nicht auf veränderter Expression in den Zellen beruht, sondern nur auf der unterschiedlichen Zellzusammensetzung. Dies verdeutlicht, daß die Wahl des Tages der Biopsienahme entscheidend den Erfolg des Screenings beeinflußt.

Zudem gibt es enorme Variabilitäten des Wundzustands zum Zeitpunkt einer möglichen Biopsie des Patienten beim Erstkontakt mit dem Arzt.Daher wurde zur Identifikation der vorangehend beschriebenen Nukleinsäuren ein Tiermodell verwendet. Es wurden BALB/c Mäuse verwundet und zu verschiedenen Zeitpunkten Wundbiopsien entnommen. Dieses Verfahren hat den Vorteil, das sich die Randbedingungen wie genetischer Hintergrund, Art der Wunde, Zeitpunkt der Biopsie etc. exakt kontrollieren lassen und so erst eine reproduzierbare Analyse der Genexpression erlauben. Selbst unter den definierten Maus-Bedingungen ergeben sich weitere methodische Probleme wie Redundanz der analysierten Klone und Unterrepräsentation von schwach exprimierten Genen, die die Identifikation von relevanten Genen erschweren. Zudem wurde eine Überrepräsentation von Genen beobachtet, die während der Wundheilung differentiell exprimiert werden, die aber für die Verwendung in der Wundheilung oder bei Hautkrankheiten unggeeignet sind. Diese Gene umfassen beispielsweise Gene, die für Enzyme des Primärstoffwechsels, wie Glykolyse, Citratzyklus, Glukoneogenese und Atmungskette kodieren, aber auch Gene, die für ribosomale Proteine kodieren, z.B. L41 und S20.

Bei der vorliegenden Analyse der Genexpression wurden während des Wundheilungsprozesses neben Genen, deren Funktion bisher gänzlich unbekannt war, auch Gene identifiziert, die bisher nicht mit Wundheilungsstörungen in Verbindung gebracht wurden. Von einigen der bekannten Genen wurden weiterhin neuartige Varianten mit Sequenzen identifiziert, die signifikant von den bisher veröffentlichten und/oder patentierten Sequenzen abweichen.

Von dem bisher nicht mit Wundheilungsstörungen in Zusammenhang gebrachten Teil der identifizierten Gene war bisher bekannt, daß sie eine Funktion bei der Proliferation (NF1-B: Schuur et al., 1995, Cell Growth Differ. 6:219-27; potentially prenylated protein tyrosin phoshatase: Zeng et al., 1998, 244:421-7; mas onkogene: van 't Veer et al., 1988, Oncogene Res. 3:247-54; CBP: Blobel et al., 1998, Proc. Natl. Acad. Sci. USA 95:2061-6; BTG1: Rouault et al., 1992, EMBO J. 11:1663-70; Acylamino acid-releasing enzyme: Schoenberger et al., 1986, J. Clin. Chem. Clin. Biochem. 24: 375-8; ALF1: Loveys et al., 1996, Nucleic. Acids Res. 24:2813-20, p68 RNA Helicase: Ford et al., 1988, Nature 332:736-8, Nup98: Kasper et al., 1999, Mol. Cell. Biol. 19:764-76; Ribonuclease L Inhibitor: Benoit et al., 1998, Gene 209:149-56; Cathepsin Z: Santamaria et al., 1998, J. Biol. Chem. 273:16818-23) Zell-Zyklus-Kontrolle (Checkpoint suppressor 1: Pat et al., 1997, Mol. Cell. Biol 17:3037-46), Zell-Migration (Ryoducan: Woods und Couchman, 1994, Mol. Biol. Cell 5:1183-92), Differenzierung (mKAP13: Aoki et al., 1998, J. Invest. Dermatol. 111:804-9; pmg-2: Kuhn et al., 1999, Mech. Dev 86:193-196, Calnexin: Olsen et al., 1995, Electrophoresis 16:2241-8, JEM-1: Tong et al., 1998, Leukemia 12:1733-40; CD9: Jones et al., 1996, Cell Adhes. Commun. 4:297-305; rab2: Ayala et al., 1990, Neuron 4:797-805; Stearoyl-CoA Desaturase: Singh und Ntambi, 1998, Biochim. Biophys. Acta 1398:148-56; Cardiac Ankyrin Repeat Protein: Zou et al., 1997, Development 124:793-804) und/oder Apoptose (MA-3: Shibahara et al., 1995, Gene 166:297-301) haben. Diese Gene wurden bisher jedoch nicht mit Wundheilung in Verbindung gebracht (Tabelle 4).

Zusätzlich zu den bekannten Polypeptiden von Mensch (Bisbal et al., 1995, J. Biol. Chem. 270:13308-17) und Maus (De Coignac et al., 1998, Gene 209:149-56) Ribonuklease L Inhibitor und Maus p68 RNA Helikase (Lemaire und Heinlein, 1993, Life Sci. 52: 917-26) wurden eng verwandte Polypeptide mit signifikant abweichender Sequenz identifiziert. Von dem bekannten Maus mKAP13/Pmgl Polypeptid (Aoki et al., 1998, J. Invest. Dermatol. 111, 804-9) wurde erstmals die Sequenz des entsprechenden Polypeptids des Menschen identifiziert (Beispiel 7) (SEQ ID Nr. 55). Zusätzlich zum diesem Polypeptid aus dem Menschen wurden 6 weitere, bisher unbekannte Homologe von mKAP13 identifiziert (SEQ ID Nr. 89 bis SEQ ID Nr. 94). Außerdem ist unter SEQ ID Nr. 109 eine zusätzliche, bislang unbekannte Variante des Mas Onkogens aus der Maus (Metzger et al., 1995, FEBS Lett. 357:27-32) aufgeführt. Desweiteren konnte zusätzlich zu dem bekannten Jem-1 aus dem Menschen (Tong et al., 1998, Leukemia 12: 1733-1740) eine weitere, bislang unbekannte Variante im Menschen (SEQ ID Nr. 110) sowie zwei bislang unbekannte Varianten aus der Maus identifiziert werden (SEQ ID Nr. 112 und SEQ ID Nr. 111). Zudem wurden zusätzlich zu den bekannten Sequenzen von MCARP aus Maus (Zou et al., 1997, Development, 124:793-804) und NF-1B (GP: BAA92677) aus Mensch wurden eng verwandte Polypeptide mit abweichender Sequenz identifiziert (SEQ ID Nr. 113 und SEQ ID Nr. 114). Außerdem wurden zusätzlich zu den beschriebenen Polypeptiden von HSPC028 und KIAA0614 aus Mensch (Tabelle 2) erstmals die entsprechenden Sequenzen aus der Maus identifiziert (SEQ ID Nr. 107 und SEQ ID Nr.108).

Die Polypeptide dieser Gene gehören nicht zu den bisher bekannten Zielen von Therapien von Wundheilungsstörungen, so daß sich aus dieser Erfindung völlig neue Therapieansätze ergeben. Von den restlichen identifizierten Genen existiert noch keine relevante Funktionsbeschreibung (Tabelle 2). Zudem konnte ein Gen gefunden werden, dessen mRNA nicht translatiert wird und als RNA funktionell ist (SEQ ID Nr. 49 bis SEQ ID Nr. 50; Steroid Receptor Coaktivator: Lanz et al., 1999, Cell 97:17-27).

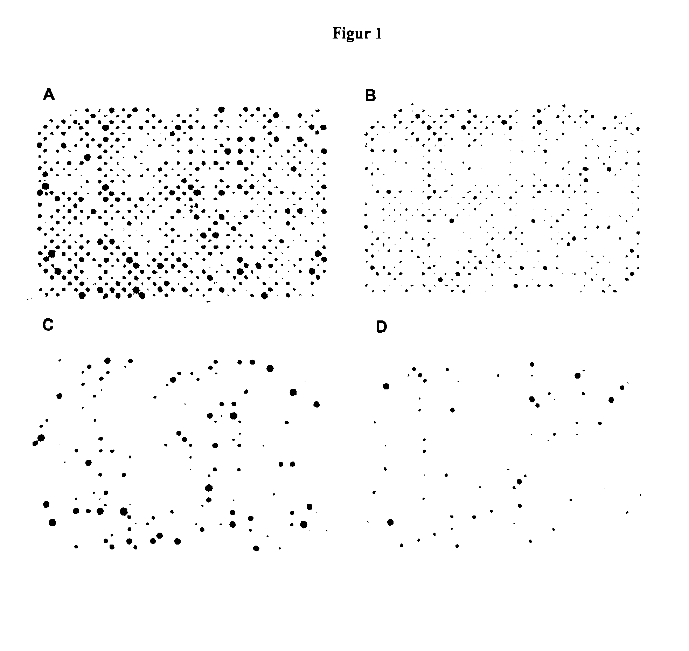

Nach der primären Identifikation der Gene ist es notwendig, die wundheilungsspezifische Expression durch eine weitere Methode zu bestätigen. Dies erfolgte mit Hilfe von sogenannten "Reverse Northern Blots", "RNase Protection Assays" oder "TaqMan Assays". Mit diesen Methoden wurde die Menge an mRNA in Gewebeextrakten aus verschiedenen Wundheilungszuständen von 10 Wochen alten Kontrollmäusen und/oder von 1 Jahr alten Mäusen und/oder von 4 Wochen alten (= jungen) Mäusen und/oder von Mäusen mit Diabetes (*db/db* Maus) bestimmt. So wurde beispielsweise die wundspezifische Expression der Klone in einer subtraktiven cDNA Bibliothek mit Hilfe eines "Reverse Northern Blots" ermittelt (Beispiel 2). Es zeigte sich, das ca. 20% aller Klone in den Bibliotheken unterschiedliche Signale mit Hybridisierungssonden aus Wund-cDNA im Vergleich zu Sonden aus intakter Haut zeigten (Figur 1). Nach der Identifikation der Klone wurden diese teilweise sequenziert, redundante Klone aussortiert, und die Sequenz mit Sequenz-Datenbanken mit dem Ziel abgeglichen, Vollängen Sequenzen der Maus-Gene und der menschlichen Gene zu identifizieren (Beispiel 3). Die Sequenzierung und Redundanzanalyse der positiven Klonen ergab, daß mehr als 75% der Klone mehrfach vorhanden waren und somit nur ca. 5% aller Ausgangsklone weiter verwendet werden konnten. Eine Minderheit von diesen wundspezifischen Genen zeigte wiederum eine verminderte Expression in schlecht heilenden Wunden. Von diesen konnte bei ca. 50% die differentielle Expression im "RNase Protection Assay", "Real time RTPCR" bzw. "TaqMan Assay" bestätigt werden (Beispiele 4 und 5). So zeigte sich, daß das als Markergen verwendete Gen *spi*2 ca. 10-fach stärker in Wundgewebe exprimiert wurde im Vergleich zu intakter Haut. Auch ergab sich, daß die Expression des Gens in schlecht heilenden Wunden von Dexamethason behandelten und von alten Tieren ca. 2-fach schwächer war, als in normal gut heilenden Wunden von Kontrolltieren (Tabelle 1).

Zur Überprüfung bzw. Generierung von Vollängen cDNA Sequenzen der erfindungsgemäß verwendbaren Nukleinsäuren wurden Vollängen-Klone mit Hilfe von Kolonie-Hybridisierung (Sambrook et al., 1989, Molecular cloning: A Laboratory Manual, Cold Spring Harbor, Cold Spring Harbor Laboratory Press, New York, Kapitel 8-10) und/oder PCR basierten Methoden ("RACE", Frohman et al., 1988, Proc. Natl. Acad. Sci. USA 85: 8998-9002, Chenchik et al., 1996, in A Laboratory Guide to RNA: Isolation, Analysis, and Synthesis, Ed. Krieg, Wiley-Liss, Seiten 272-321; "LDPCR", Barnes, 1994, Proc. Natl. Acad. Sci. USA 91: 2216-20) sowohl für die Maus-Gene als auch für die menschlichen Gene generiert und die Sequenz diese Klone bestimmt (Beispiel 6).

Mäuse eignen sich hervorragend als Modellsystem für Wundheilungsprozesse. Um zu bestätigen, daß in humanem Wundgewebe die homologen humanen Gene der in der Maus als wundheilungsrelevant identifizierten Gene differentiell exprimiert werden, wurde parallel intakte Haut und Wundgewebe aus dem Menschen und aus der Maus mittels "TaqMan Analyse" untersucht. Zusätzlich konnten durch in situ Hybridisierung bzw. Immunlokalisierung der erfindungsgemäß verwendbaren Nukleinsäuren bzw. Polypeptide in intakter Haut und Wundgewebe detaillierte Informationen darüber erhalten werden, in welchen Regionen der Haut eine wundheilungsspezifisch regulierte Expression stattfindet. Dies gibt Aufschluß über die Funktion des wundrelevanten Gens beim Wundheilungsprozeß als auch über die Bedeutung der differentiellen Expression bei gestörten Wundheilungsverläufen bzw. Hauterkrankungen. Zudem konnte die Relevanz und das therapeutische Potential wundheilungsrelevanter Gene durch *in vivo* Applikation des Gens im Tiermodell bestätigt werden.

Die Erfindung soll nun im folgenden anhand der Figuren und Beispiele weiter verdeutlicht werden, ohne daß die Erfindung hierauf eingeschränkt wird.

### Beschreibung der Tabellen, Figuren und Sequenzen:

- Tabelle 1:: Tabellarische Aufstellung der veränderten Expression verschiedener wundheilungsrelevanter Gene bei verschiedenen Wundheilungszuständen.
- Tabelle 2:: Tabellarische Übersicht über die bei der Analyse der Genexpression während des Wundheilungsprozesses identifizierten Polypeptidsequenzen mit unbekannter biologischer Funktion und ihre cDNAs und Accession Numbers.
- Tabelle 3:: Tabellarische Übersicht über die bei der Analyse der Genexpression während des Wundheilungsprozesses identifizierten Polypeptidsequenzen mit bereits bekannten und beschriebenen Funktionen und ihre cDNAs und Accession Numbers.
- Tabelle 4:: Tabellarische Übersicht über die bei der Analyse der Genexpression während des Wundheilungsprozesses zusätzlich identifizierten Polypeptidsequenzen mit bereits bekannten und beschriebenen Funktionen und ihre cDNAs und Accession Numbers.
- Tabelle 5:: Quantitative RTPCR von *spi*2. Die Anzahl von Zyklen bis zum Erreichen des Fluoreszensschwellenwerts (C_{T}) für jeweils drei unabhängige Messungen sowie der Mittelwert (C_{T} Mittel), die Differenz der C_{T} Werte von GAPDH und *spi*2 (ΔC_{T}), die daraus errechnete Abundanz von *spi*2 relativ zu GAPDH und die relative Induktion von *spi*2 relative zur intakten Haut von Kontrolltieren ist dargestellt.
- Tabelle 6:: Tabellarische Übersicht über die mittels "TaqMan Assay" bestimmte Menge an mRNA wundrelevanter Gene in verschiedenen Wundheilungszuständen der Maus.
- Tabelle 7:: Tabellarische Übersicht über die mittels "TaqMan Assay" bestimmte Menge an mRNA wundrelevanter Gene in humanen Tag 1- und Tag 5-Wunden.
- Tabelle 8:: Tabellarische Übersicht über die mittels "TaqMan Assay" bestimmte Menge an mRNA wundrelevanter Gene in intakter Haut von Ulkus-Patienten, am Wundrand von Ulkus-Patienten und am Wundgrund von Ulkus-Patienten.
- Figur 1:: Autoradiogramme von Hybridisierungen von Membranen mit gleichem Muster von aufgebrachten cDNA-Fragmenten mit vier verschiedenen Sonden. Die cDNA Fragmente entstammten alle einer wundspezifischen, subtraktiven cDNA-Bibliothek, die für solche cDNAs angereichert war, die in Wundgewebe stärker im Vergleich zur intakten Haut exprimiert wurden. Alle Sonden wurden aus cDNAs hergestellt, die aus subtraktiven Hybridisierungen stammten. A: wundspezifische Sonde (Subtraktion Wunde versus intakte Haut), B: hautspezifische Sonde (Subtraktion intakte Haut versus Wunde), C: Sonde spezifisch für gut heilende Wunden (Subtraktion Wunde Kontrolltiere versus Wunde Dexamethason behandelte Tiere), D: Sonde spezifisch für schlecht heilende Wunden (Subtraktion Wunde Dexamethason behandelte Tiere versus Wunde Kontrolltiere).

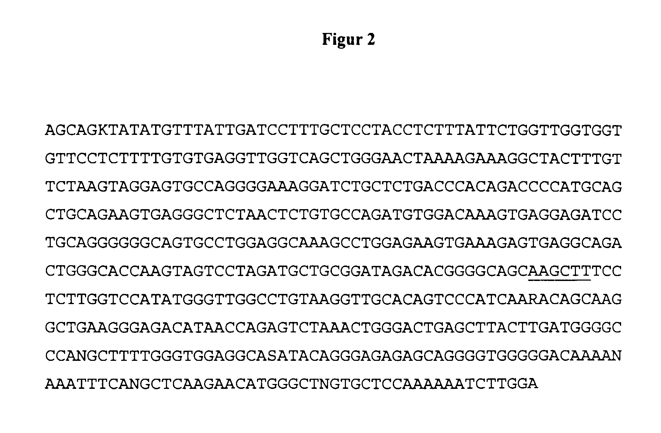

- Figur 2:: Teil der cDNA-Sequenz des Markergens *spi*2 (SEQ ID Nr. 61). Die Sequenz ist die Konsensussequenz von zwei Sequenzierungen von Klonen, die in der wundspezifischen, subtraktiven cDNA-Bibliothek enthalten waren. Vektor- und Primersequenzen der cDNA Synthese wurden entfernt, so daß nur die Sequenzen der Inserts für die Konsensussequenz berücksichtigt wurden. Die Qualität der Sequenzanalyse (<10% Fehler) erlaubt nur die Identifikation der Vollängen-cDNA, läßt aber keinen exakte Feststellung der Sequenz der Inserts zu. Die Position der Erkennungsstelle der Restriktionsendonuklease HindIII, die zur Linearisierung des Plasmids bei der Herstellung der Sonde für den RNAse Protection Assay diente, ist unterstrichen.

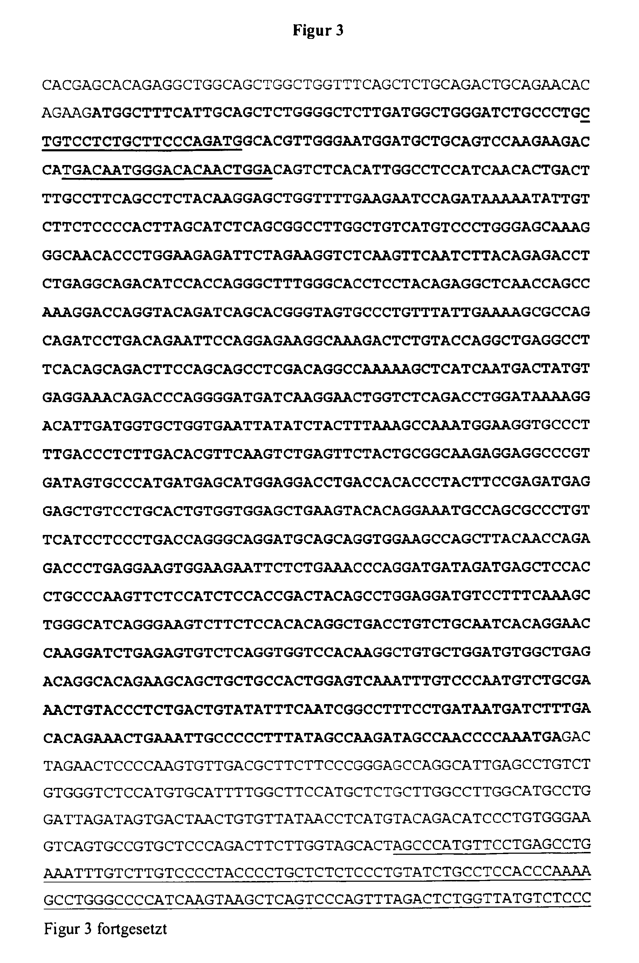

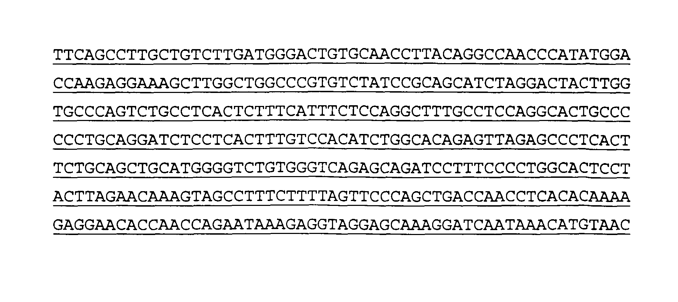

- Figur 3:: Vollängensequenz der *spi*2-cDNA (SEQ ID Nr. 62, EMBL Datenbankeintrag MMSPI201, Accession No. M64086). Die Sequenz der in der wundspezifischen cDNA-Bibliothek enthaltenen Nukleinsäuren ist unterstrichen (Vergleiche Figur 2, komplementäre Sequenz). Der kodierende Bereich (fett) der cDNA geht vom Startcodon (ATG) an Position 61 bis zum Stopcodon (TGA) an Position 1317. Die Position der Primer für die quantitative RTPCR (Beispiel 5) im kodierenden Bereich ist unterstrichen.
- Figur 4:: Teilsequenz der Maus-cDNA von GAPDH (SEQ ID Nr. 63), als Referenzsequenz, beim RNAse Protection Assay verwendet.

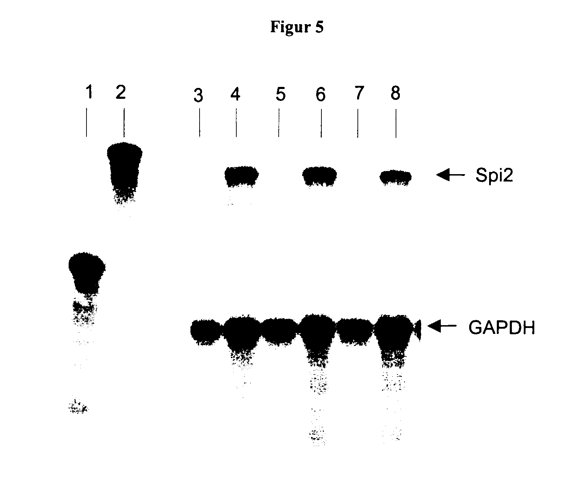

- Figur 5:: RNase Protection Assay von *spi2* mit RNA verschiedener Wundheilungszustände. Radioaktiv markierte Sonden von GAPDH (Spur 1: intakte Sonde) und *spi*2 (Spur2: intakte Sonde) wurde mit Gesamt-RNA, gewonnen aus intakter Haut (Spuren 3, 5 und 7) oder aus Wunden (Spur 4, 6, und 8) von Kontrollmäusen (Spuren 3 und 4), von Dexamethason-behandelten Mäusen (Spuren 5 und 6) oder von alten Mäusen (Spuren 7 und 8) hybridisiert und nach RNase Behandlung gelelektrophoretisch aufgetrennt. In jeder Spur wurde die Signalintensitäten der Spi2 Signale mit den Signalen der GAPDH Sonde normalisiert. Die relativen Intensitäten in Bezug auf intakte Haut von Kontrolltieren sind Spur 3: 1,0; Spur4: 13,28; Spur 5: 0,84; Spur 6: 7,72; Spur 7: 0,54; Spur 8: 5,2.

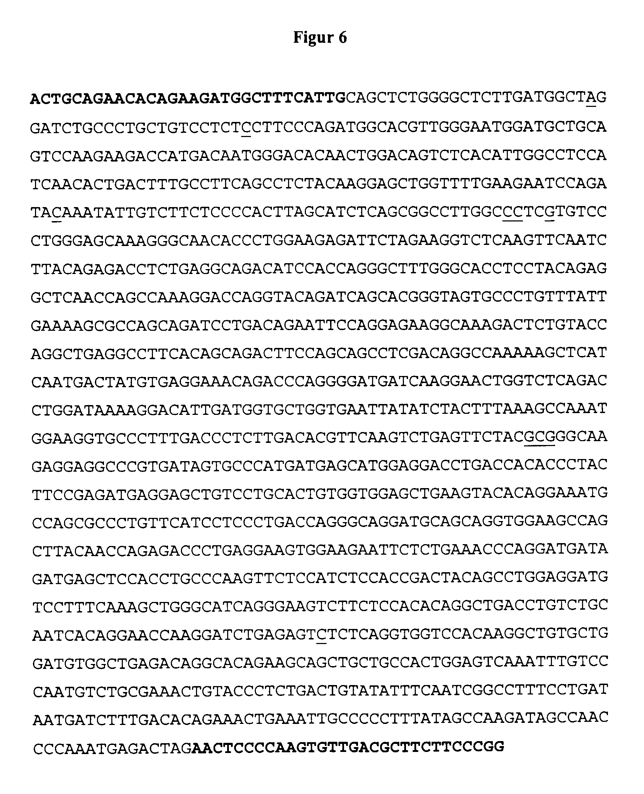

- Figur 6:: Korrektur der Sequenz von *spi*2 (SEQ ID Nr. 64). Die Primer der PCR sind fett dargestellt, Abweichungen von der veröffentlichten Datenbanksequenz sind unterstrichen.

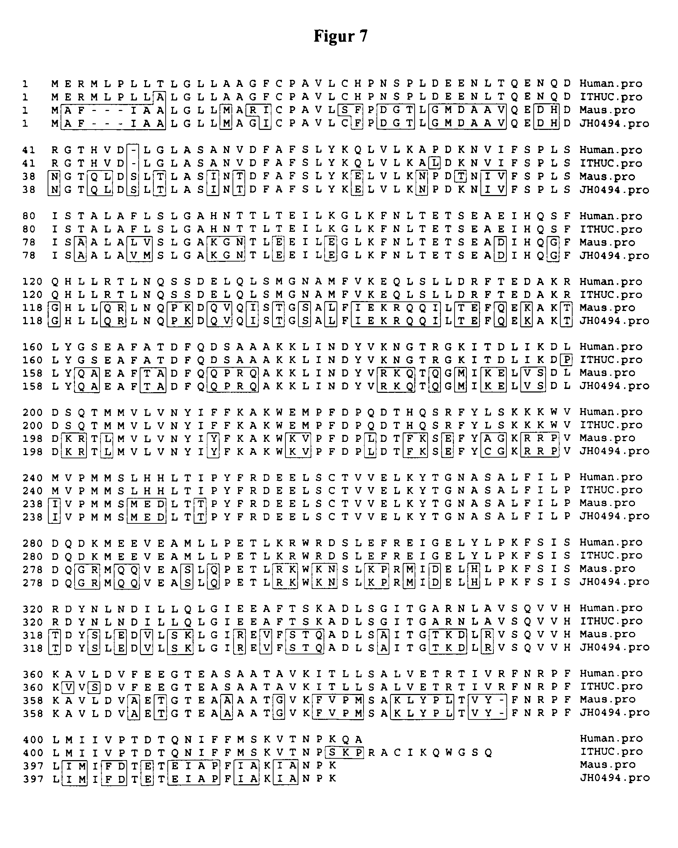

- Figur 7:: Vergleich der ermittelten Aminosäuresequenz von *spi*2 (*spi*2-Consensus.pro) mit der veröffentlichten Aminosäuresequenz des Datenbankeintrags (JH0494.pro).

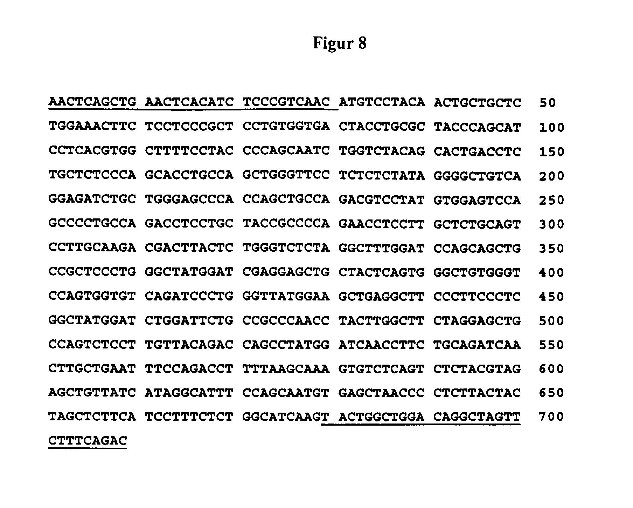

- Figur 8:: Sequenz des menschlichen Homologs von mKAP13/Pmg-1. Die Primer der PCR sind unterstrichen dargestellt.

SEQ ID Nr. 1 bis SEQ ID Nr. 58 und SEQ ID Nr. 89 bis SEQ ID Nr. 124 zeigen die erfindungsgemäß verwendbaren Polypeptid- oder cDNA-Sequenzen aus Mensch oder Maus.

SEQ ID Nr. 59 bis SEQ ID Nr. 64 zeigen Polypeptid- und cDNA-Sequenzen der Markergene *spi*2 und GADPH.

SEQ ID Nr. 65 bis SEQ ID Nr. 84 und SEQ ID Nr. 125 und SEQ ID Nr. 128 zeigen DNA-Sequenzen von Oligonukleotiden, die für die Versuche der vorliegenden Erfindung verwendet wurden.

SEQ ID Nr. 85 bis SEQ ID Nr. 88 zeigen Aminosäure-Sequenzen von Oligopeptiden, die für die Versuche der vorliegenden Erfindung verwendet wurden.

### Beispiele

### Beispiel 1: Herstellung von cDNA-Bibliotheken mittels subtraktiver Hybridisierung

Aus intakter Haut und aus Wundgewebe (Verwundung am Rücken 1 Tag vor Gewebeentnahme durch Scherenschnitt) von Balb/c Mäusen wurde durch Standardmethoden (Chomczynski und Sacchi, 1987, Anal. Biochem. 162: 156-159, Chomczynski und Mackey, 1995, Anal. Biochem. 225: 163-164) Gesamt-RNA isoliert.

Die RNAs wurden dann mit Hilfe einer reversen Transkriptase in cDNA umgeschrieben. Die cDNA Synthese erfolgte mit dem "SMART PCR cDNA Synthesis Kit" der Firma Clontech Laboratories GmbH, Heidelberg, nach Anweisungen des entsprechenden Manuals. Um diejenigen cDNAs zu identifizieren, die mit unterschiedlicher Häufigkeit in den beiden cDNA Pools vorkamen, wurde eine subtraktive Hybridisierung (Diatchenko et al., 1996, Proc. Natl. Acad. Sci. USA 93: 6025-30) durchgeführt. Dies erfolgte mit dem "PCR-Select cDNA Subtraction Kit" der Firma Clontech Laboratories GmbH, Heidelberg, nach Anweisungen des entsprechenden Manuals, wobei die Abtrennung überschüssiger Oligonukleotide nach der cDNA Synthese über Agarose-Gelelekrophorese erfolgte. Die resultierenden cDNA Fragmente wurden mittels T/A- Klonierung in den Vektor pT-Adv (Clontech Laboratories GmbH) integriert und mittels Elektroporation in E. coli (SURE electroporation-competent cells, Firma Stratagene) transformiert. Es wurden zwei cDNA Bibliotheken angelegt, wobei eine angereichert für cDNA Fragmente war, die im Wundgewebe im Vergleich zu intakter Haut stärker exprimiert sind ("wundspezifische cDNA Bibliothek"), während die andere angereichert war an cDNA Fragmenten, die in intakter Haut im Vergleich zu Wundgewebe stärker exprimiert sind ("hautspezifische cDNA Bibliothek").

### Beispiel 2: Identifizierung von wundheilungsregulierten cDNA-Fragmenten

Um diejenigen Klone der beiden cDNA-Bibliotheken im Beispiel 1 zu identifizieren, die wundheilungsrelevante cDNA-Fragmente enthielten, wurde die Expression der entsprechenden cDNAs in intakter und verwundeter Haut sowie in gut und schlecht heilenden Wunden im "Reverse Northern Blot" analysiert. Hier werden die cDNA-Fragmente auf Membranen in Form von Arrays von vielen verschiedenen cDNAs fixiert, und mit einem komplexen Gemisch radioaktiv markierter cDNA hybridisiert (Sambrook et al., 1989, Molecular cloning: A Laboratory Manual, Cold Spring Harbor, Cold Spring Harbor Laboratory Press, New York, kapitel 9 Seite 9.47 bis9.58 und kapitel 10 Seite 10.38 bis 10.50; Anderson and Young: Quantitative filter hybridisation; in: Nucleic Acids Hybridisation, A Practical Approach, 1985, Eds. Hames and Higgins, IRL Press Ltd.; Oxford, Kapitel 4, Seite 73 bis 112).

Um geeignete Membranen für die Analyse herzustellen, wurden aus jeder Bibliothek 1536 Klone isoliert und mittels Polymerase Ketten Reaktion die cDNA-Insertionen amplifiziert. Je 2 µl Suspensionkultur der Klone wurden in 40 µl TE Puffer (10 mM Tris-HCl, lmM EDTA, pH = 8.0) für 1 Minute bei 96°C lysiert und eine 20 µl PCR-Reaktion ("Advantage cDNA Polymerase Mix Kit" der Firma Clontech) mit 1 µl Lysat angeimpft. Die Amplifikation erfolgte in 35 PCR Zyklen (1 min 96°C gefolgt von 35 Zyklen 2-Stufen-PCR: 96°C/30" und 68°C/3') mit universellen Primern (TCGAGCGGCCGCCCGGGCAGGT (SEQ ID Nr. 65) und AGCGTGGTCGCGGCCGAGGT (SEQ ID Nr. 66)), die im "PCR-Select cDNA Subtraction Kit" zur Gewinnung der cDNA Fragmente verwendet werden und somit alle cDNA Insertionen umschließen. 5 µl PCR-Reaktion wurde in 384 well Mikrotiterplatten mit 35 µl Puffer (1.16 M NaCl, 20 mM Tris, pH = 7.5, 0.001 % Bromphenolblau) versetzt und mit dem Nunc 384 Pin Replikator unter Verwendung des Nunc Replicator Systems (Nunc GmbH & CO.KG, Wiesbaden) in 20 Wiederholungen auf Qiabrane Membranen (Qiagen, Hilden) aufgestempelt. Die Membranen wurden mit 0,4 M NaOH für 30 Minuten zur Denaturierung der DNA inkubiert und mit 1 M Tris /HCl, pH = 7,5 für 3 Minuten neutralisiert. Die DNA wurde anschließend durch UV-Bestrahlung (120 mJ/cm²) gefolgt von Backen für 30 min bei 80°C fixiert.

Zur Herstellung geeigneter Hybridisierungssonden wurden RNA aus intakter und verwundeter Haut (siehe oben) sowie aus gut und schlecht heilenden Wundgewebe isoliert. Um Gewebe von Mäuse mit schlecht heilenden Wunden zu gewinnen, wurden BALB/c Mäuse vor der Verwundung mit Dexamethason (Injektion von 0,5 mg Dexamethason in isotonischer Salzlösung pro kg Körpergewicht zwei mal pro Tag für 5 Tage) behandelt bzw. alte Mäuse (12 Monate) verwundet. Die RNA aus Wundgewebe dieser Tiere sowie aus Kontrollen wurde wie oben beschrieben isoliert und jeweils cDNA synthetisiert. Danach wurden wie oben beschrieben zwei subtraktive Hybridisierungen (cDNA aus Dexamethason behandelten Tieren versus cDNA aus Kontrolltieren und umgekehrt) durchgeführt. Dies erfolgte wie oben beschrieben mit dem "PCR-Select cDNA Subtraction Kit". Diese subtrahierten cDNAs sowie die oben beschriebenen subtraktiven cDNAs (intakte Haut versus Wunde und umgekehrt) wurde mit der Restriktionsendonuklease RsaI behandelt und über Agarosegelelektrophorese gereinigt (Sambrook et al., supra, Kapitel 6, seite 6.1 bis 6.35), um die cDNA- Synthese- und Amplifikationsprimer (siehe Manual "PCR-Select cDNA Subtraction Kit", Clonetech) abzutrennen. Die cDNAs wurde dann mit der "random-hexamer priming" Methode (Feinberg und Vogelstein, 1983, Anal. Biochem. 132:6-13) radioaktiv markiert, um Hybridisierungssonden herzustellen.

Die Membran wurde für 30 min bei 65 °C in 25 ml Hybridisierungslösung vorinkubiert (25 mM Natriumphosphat, pH = 7,5, 125 mM NaCl, 7% SDS). Die Hybridisierungssonde wurde 10 min bei 100°C denaturiert, anschließend auf Eis abgekühlt, ca. 100 CPM pro ml zur Hybridisierungslösung gegeben und die Hybridisierung für 16 Stunden bei 65 °C im Hybridisierungsofen durchgeführt. Danach wurde die Membran zweimal für 10 min mit der Hybridisierungslösung ohne Sonde bei 65 °C gewaschen. Anschließend wurde die Membran mehrfach für jeweils 10 min in Waschlösung (2,5 mM Natriumphosphat, pH = 7,5, 12,5 mM NaCI, 0,7% SDS) bei 65°C gewaschen, bis in der abgegossenen Lösung keine Aktivität mehr nachgewiesen werden konnte. Die radioaktiven Signale wurden mit einem Phosphoimager (BioRad, Quantitiy One®) ausgewertet (Figur 1). Danach wurden diejenigen cDNAs ausgewählt, die mit den verschiedenen Sonden unterschiedliche Signalintensitäten ergaben. Von den entsprechenden Klonen wurden nach Standardmethoden (Sambrook et al., supra) Plasmid-DNA Präparationen angefertigt und die DNA Sequenz der Inserts analysiert. Figur 1 zeigt eine Übersicht über die Expressionsmuster verschiedener Gene, die durch diese Methode ermittelt werden konnten.

### Beispiel 3: Analyse von wundheilungsrelevanten cDNA Sequenzen

Die cDNA Sequenz der Inserts aus Beispiel 2 wurden mit dem Programm SeqMan 4.01 der Firma DNAStar Inc. (GATC GmbH, Konstanz) auf Redundanz analysiert und nicht redundante Sequenzen wurden mit dem Programm BLAST 2.0 (Altschul et al., 1997, Nucleic Acids Res. 25:3389-402) mit der aktuellen EMBL-(Stoesser et al., 1999, Nucleic Acids Res., 27:18-24), TREMBL-(Bairoch und Apweiler, 1997, Nucleic Acids Res. 25:31-26) und PIR- (George et al., 1996, Nucleic Acids Res. 24:17-20) und/oder GenBank- (Benson et al., 1999, Nucleic. Acids Res. 27:12-7) Datenbank verglichen. Dabei stellte sich heraus, daß einige wundheilungsrelevante cDNA-Sequenzen von bekannten Genen stammen, die bisher aber noch nicht mit regenerativen Prozessen in Verbindung gebracht wurden.

So konnte das Gen *spi*2 als wundheilungsrelevantes Gen identifiziert werden. In Figur 2 ist die Ausgangssequenz dargestellt, die zweimal in der wundspezifischen cDNA Bibliothek (s.o.) vorhanden war, in Figur 3 die Vollängensequenz von *spi*2 aus der EMBL Datenbank (Eintrag MMSPI201, Accession No. M64086 (SEQ ID Nr. 60), die mit hoher Signifikanz (BLAST Score = 883) mit der Ausgangssequenz übereinstimmt. Ein Vergleich der beiden Sequenzen zeigt, das die Ausgangssequenz das 3'-Ende der *spi*2-cDNA enthält (unterstrichene Sequenz in Figur 3). Die Spi2 *spi*2-cDNA codiert für das Protein "alpha-1-antichymotrypsin-like protein EB22/4" (PIR: JH0494), dessen funktionelle Variante im Mensch "alpha1-antichymotrypsin" ist (Datenbankeinträge: Protein: PIR: E55627, cDNA: EMBL: K01500 (SEQ ID Nr. 71)). Die funktionellen Varianten des Proteins in Maus und Mensch haben ca. 60% Sequenzhomologie. Entsprechende Analysen wurden mit allen anderen ermittelten cDNA Fragmenten durchgeführt und eine Liste derjenigen gefundenen Datenbankeinträge erstellt, die ein signifikantes diagnostisches oder therapeutisches Potential haben (Tabelle 2 bis 4).

### Beispiel 4: Verifikation des Expressionsmusters wundheilungsrelevanter cDNAs mittels "RNAse Protection Assays"

Die differenzielle Expression der Gene wurde mit Hilfe des "RNAse Protection Assays" verifiziert. Der Test wurde wie in der Literatur beschrieben durchgeführt (Sambrook et al., supra Kapitel 7, Seite 7.71 bis 7.78; Werner et al., 1992; Growth Factors and Receptors: A Practical Approach 175-197, Werner, 1998, Proc. Natl. Acad. Sci. USA 89: 6896-6900). Es wurde in vitro transkribierte und radioaktiv markierte Gegenstrang RNA der isolierten Klone als Hybridisierungssonde eingesetzt. Als interne Kontrolle diente eine Gegenstrang RNA Sonde der murinen GAPDH-cDNA (Länge des Transkripts ohne Vektorsequenz: 120 Basenpaare, Figur 4), kloniert in pBluescript II KS (+) (Stratagene) mit Hilfe der Restriktionsendonukleasen XbaI und HindII. Dieses Plasmid wurde vor der Transkription mit XhoI linearisiert. Die Plasmide der klonierten cDNA-Fragmeten der identifizierten cDNAs wurden ebenfalls vor der Transkription mit Restriktionsendonukleasen linearisiert. So wurde beispielsweise der *spi2* Klon mit HindIII linearisiert (Länge des Transkripts ohne Vektorsequenz: 322 Basenpaare, Figur 3). Die Transkriptionen wurde mit T7 Polymerase (Roche Diagnostics, Mannheim) in Gegenwart von ³²P-UTP (35µCi/Ansatz) (Amersham, Braunschweig) nach Angaben des Herstellers durchgeführt. Nach einem DNase Verdau (Boehringer, 40U/Ansatz) wurden die nicht eingebauten Nukleotide mit Hilfe einer Säulenchromatographie (Pharmacia, Microspin Combi-Pack S200) nach Angaben des Herstellers entfernt und die Sonden durch eine Gelelektrophorese und Elution aufgereinigt (Sambrook et al., supra, Kapitel 6, Seite 6.36 bis 6.48). Für die Hybridisierungsreaktion wurden je ca. 10⁵ CPM des markierten Transskripts eingesetzt. 20µg Gesamt-RNA wurden hierfür mit entsprechenden Mengen Transkript (spezifische Probe *spi*2 und interne Kontrolle GAPDH) gefällt, in 30 µl Hybridisierungspuffer (80% entionisiertes Formamid, 400 mM NaCl, 40 mM Pipes pH 4,6, 1 mM EDTA) aufgenommen und über Nacht bei 42°C hybridisiert. Anschließend wurde ein RNase T1 Verdau (Boehringer, 200 U/Ansatz) durchgeführt. Nach Inaktivierung der RNase durch Proteinase K Verdau (Boehringer, 44 µg/Ansatz) und einer Phenolextraktion wurden die Proben mit Isopropanol nach Standardmethoden (Sambrook et al., supra) präzipitiert. Die Proben wurden anschließend gelektrophoretisch auf einem denaturierendem 5% Acrylamidgel (6M Harnstoff) aufgetrennt. Das Gel wurde getrocknet und die radioaktiven Signale mit einem Phosphoimager (BioRad, Quantitiy One®) ausgewertet (Figur 5).

### Beispiel 5: Verifikation des Expressionsmusters wundheilungsrelevanter cDNAs mittels "real time quantitative RT-PCR"

Eine weitere Verifikation der differentiellen Expression der erfindungsgemäß verwendbaren Nukleinsäuren erfolgte über eine real-time RTPCR im ABI Prism 7700 Sequence Detection System (PE Applied Biosystems). Das Gerät war mit der ABI Prism 7200/7700 SDS-Software Version 1.6.3 (1998) ausgestattet. Der Nachweis von PCR Produkten erfolgte während der Amplifikation der cDNA mit Hilfe des Farbstoffs SYBR Green 1, dessen Fluoreszenz durch Bindung an doppelsträngige DNA stark erhöht wird (Karlsen et al. 1995, J. Virol. Methods. 55: 153-6; Wittwer et al., 1997, BioTechniques 22:130-8, Morrison et al., 1998, BioTechniques 24: 954-62). Basis für die Quantifizierung ist der PCR Zyklus ("threshold cycle", C_{T}-Wert), der erreicht ist, wenn das Fluoreszenzsignal einen definierten Schwellenwert übersteigt. Die Auswertung erfolgt über die ΔΔ-CTC_{T}-Methode (User Bulletin #2, Relative Quantitation of Gene Expression, PE Applied Biosystems, 1997). Die Abundanzen der cDNAs wurden relativ zu einer endogenen Referenz (GAPDH) bestimmt. Die Ergebnisse sind in Tabelle 5 dargestellt.

Normal heilende Tag 1-Wunden und intakte Haut wurde aus mit isotonischer Kochsalzlösung behandelten, 10 Wochen alten BALB/c Mäusen durch Scherenschnitt wie oben beschrieben gewonnen. Um Gewebe von Mäusen mit schlecht heilenden Wunden zu gewinnen, wurden BALB/c Mäuse vor der Verwundung mit Dexamethason (0,5 mg Dexamethason in isotonischer Salzlösung pro kg Körpergewicht i.p. zwei mal pro Tag für 5 Tage) behandelt.

Gesamt-RNA wurde wie oben beschrieben aus Haut und Wundgewebe gewonnen und 1 µg Gesamt-RNA wurde mit dem TaqMan Reverse Transcription Reagents Kit (PE) nach den Empfehlungen des Herstellers (SYBR Green PCR and RT-PCR Reagents-Protokol, PE Applied Biosystems, 1998) in einem Thermocycler (GeneAmp PCR-System 9700, PE) revers transkribiert. Die Primer für die Amplifikation der Spi2 cDNA *(spi2 spi*2-Primer 1: CTGTCCTCTGCTTCCCAGATG (SEQ ID Nr. 67), *spi*2-Primer 2: TCCAGTTGTGTCCCATTGTCA (SEQ ID Nr. 68) und der Referenz (GAPDH-Primer1: ATCAACGGGAAGCCCATCA (SEQ ID Nr. 69), GAPDH-Primer2: GACATACTCAGCACCGGCCT (SEQ ID Nr. 70)) wurden anhand der erfindungsgemäß verwendbaren Nukleinsäure und der bekannten Sequenz von GAPDH mit der Primer-Express-Software für Macintosh PPC Version 1.0 (PE Applied Biosystems, P/N 402089, 1998) ausgewählt). Für die PCR wurde das SYBR Green PCR Core Reagents Kit (4304886, PE Applied Biosystems) verwendet. Die Konzentration der Primer in der PCR wurde zunächst im Bereich von 50 nM bis 600 nM optimiert und die Spezifität der PCR durch Analyse der Länge der amplifizierten Produkte in einer Agarose-GelElekrophorese geprüft. Anschließend wurde mittels einer Verdünnungsreihe die Effizienz der PCR-Systeme ermittelt (User Bulletin #2, Relative Quantitation of Gene Expression, PE Applied Biosystems, 1997). Dabei ergab sich, daß für beide cDNAs die Effizienz der Amplifikation bei 100% lag, d. h. bei jeder 1:2 Verdünnung der cDNA wurde ein Zyklus mehr benötigt, um den Fluoreszensschwellenwert zu überschreiten.

Für die Quantifizierung wurden je Ansatz cDNA aus 10 ng revers transkribierter Gesamt-RNA in einem Gesamtvolumen von 25 µl amplifiziert. Die Laufbedingungen für die PCR entsprachen den Angaben des Herstellers (PE Applied Biosystems, SYBR Green PCR and RT-PCR Reagents Protocol, 1998). Die C_{T} Werte wurden analysiert und die Abundanz von *spi*2 wurde berechnet. Auch mit dem zweiten Assay konnte die Induktion von *spi*2 in gut heilenden Wunden, die Reduktion der Induktion in schlecht heilenden Wunden Dexamethason behandelter Tiere und die Reduktion der Expression in intakter Haut von Dexamethason behandelten Tieren bestätigt werden. Die leichten Abweichungen der absoluten Werte sind auf die unterschiedlichen Testsysteme zurückzuführen.

### Beispiel 6: Verifikation der Sequenz der cDNA von spi2

Um die cDNA Sequenz von *spi2* zu verifizieren, wurden anhand der Datenbanksequenz Oligonukleotide synthetisiert (Primer1: ACTGCAGAACACAGAAGATGGCTTTCATTG (SEQ ID Nr. 71), Primer 2: CCGGGAAGAAGCGTCAACACTTGGGGAGTT (SEQ ID Nr. 72)). Mit diesen Primern wurde mit Hilfe des "Advantage cDNA PCR Kit" startend von Maus Wund-cDNA (s.o.) der kodierende Bereich der cDNA von *spi*2 (Figur 3) amplifiziert. Die PCR Reaktion wurde nach Angaben des Herstellers angesetzt und die Amplifikation erfolgte für 40 Zyklen (30" 94°C, 30" 64°C, 1,5' 72°C) wobei die Denaturierungszeit im ersten Zyklus auf 1,5 Minuten und die Synthesezeit im letzten Zyklus auf 6,5 Minuten ausgedehnt wurde. Das PCR Produkt (1309 bp) wurde über Agarose- Gelelektrophorese gereinigt (Sambrook et al., supra) und mit Hilfe des pCR2.1TOPO Klonierungskits (Invitrogen) nach Anweisungen des Herstellers in den Vektor pCR2 kloniert. Die Sequenz von 3 unabhängigen Klonen wurde mit Standardmethoden analysiert und die Konsensussequenz mit Hilfe des Programms SeqMan2 (s.o.) bestimmt (Figur 6). Dabei zeigte sich, das die hier bestimmte cDNA Sequenz in Wundgewebe leicht von der veröffentlichten Sequenz (Inglis et al., 1991, Gene 106:213-20) abweicht, ebenso wie die kodierten Proteine beider Sequenzen (Figur 7). Beide Polypeptide haben vergleichbare Homologiegrade von 60% zur menschlichen Sequenz, so daß davon ausgegangen werden kann, daß die Polypeptide funktionellen Varianten des Gens darstellen. Gründe für die Abweichung könnten in dem unterschiedlichen Ausgangsmaterial für die cDNA Synthese (Wundgewebe versus Kondrozyten Zellinie) oder in unterschiedlichen Allelen des Gens im Spender begründet liegen.

### Beispiel 7: Klonierung des menschlichen Homologs zu mKAP13

Mit der veröffentlichten Proteinsequenz von mKAP13 (Aoki et al., 1998, J. Invest. Dermatol. 111, 804-9) wurden die menschliche EST-Datenbank GenBank dbest (Benson et al., supra) mit Hilfe des BLAST Programms (Altschil et al., supra) nach homologen Sequenzen durchsucht. Es fanden sich vier Sequenzeinträge (W76571, W72002, AA055832 und AA055833) die mit dem Maus mKAP13 und/oder mit den gefundenen ESTs Ähnlichkeiten aufwiesen. Aus den vier Sequenzen wurde mit Hilfe des Programms SeqMan die Konsensussequenz ermittelt und PCR Primer konstruiert (AACTCAGCTGAACTCACATCTCCCGTCAAC (SEQ ID Nr. 73) und GTCTGAAAGAACTAGCCTGTCCAGCCAGTA (SEQ ID Nr. 74). Mit diesen Primern wurde wie oben beschrieben (siehe Beispiel 6) mit Hilfe der PCR aus cDNA, die aus intakter menschlicher Haut gewonnen wurde (siehe Beispiel 1), das menschliche Homolog von mKAP13 amplifiziert, kloniert und sequenziert (Figur 8). Dabei ergab sich, daß durch die allgemein bekannte schlechte Qualität der EST Sequenzen, die tatsächliche Sequenz deutlich von der durch die EST-Assemblierung vorhergesagte Sequenz abwich, so daß die Auswahl der Primer sehr kritisch für den Erfolg der Klonierung war.

### Beispiel 8: Analyse des Expressionsmusters wundheilungsrelevanter cDNAs in verschiedenen Wundheilungszuständen der Maus mittels "TaqMan Analyse"

Die Regulation der Expression wundheilungsrelevanter cDNAs in verschiedenen Wundheilungszuständen der Maus wurde mittels "TaqMan Analyse" im Gene-Amp5700 von Applied Biosystems untersucht. Normal heilende Tag 1-Wunden und intakte Haut wurde aus mit isotonischer Kochsalzlösung behandelten 10 Wochen alten BALB/c Mäusen durch Scherenschnitt wie oben beschrieben gewonnen. Um Gewebe von Mäusen mit schlecht heilenden Wunden zu gewinnen, wurden BALB/c Mäuse vor der Verwundung mit Dexamethason (0,5 mg Dexamethason in isotonischer Salzlösung pro kg Körpergewicht i.p. zwei mal pro Tag für 5 Tage) behandelt. Als Kontrolle wurde intakte Haut von Mäusen verwendet, die mit Dexamethason wie oben beschrieben behandelt wurden. Um Gewebe von jungen und alten Mäusen zu gewinnen, wurden unbehandelte Tag 1-Wunden und intakte Hautproben von 4 Wochen alten und 12 Monate alten BALB/c Mäusen verwendet. Wundgewebe und intakte Haut von Mäusen mit Diabetes (*db/db* Maus) wurde erhalten, indem unbehandelte Tag 1-Wunden und intakte Haut von 10 Wochen alten C57B/Ks-*db*/*db*/Ola Mäusen mittels Scherenschnitt isoliert wurden. C57B/Ks wildtyp Mäuse wurden dabei als Kontrolltiere eingesetzt. Von letzteren Tieren wurde ebenfalls intakte Haut sowie unbehandelte Tag 1-Wunden gewonnen.

Die Isolierung der RNA erfolgte durch Homogenisieren der Biopsien in RNAclean Puffer (AGS, Heidelberg), dem 1/100 Volumenanteil 2-Mercaptoethanol zugesetzt worden war unter Verwendung eines Dispersers. Anschließend wurde die RNA durch zweimaliges Phenolisieren mittels mit Wasser gesättigtem saurem Phenol und in Gegenwart von 1-Brom-3-Chlor-Propan extrahiert. Anschließend wurden eine Isopropanol- und eine Ethanol-Fällung durchgeführt und die RNA mit 75% Ethanol gewaschen. Danach wurde ein DNase I Verdau der RNA durchgeführt. Hierzu wurden 20 µg RNA (ad 50 µl mit DEPC-behandeltem Wasser) mit 5,7 µl Transkriptions-Puffer (Roche), 1 µl RNase Inhibitor (Roche; 40 U/µl) und 1 µl DNase I (Roche; 10 U/µl) 20 min bei 37°C inkubiert. Dann wurde wiederum 1 µl DNase I zugegeben und weitere 20 min bei 37°C inkubiert. Anschließend wurde die RNA phenolisiert, Ethanol-gefällt und gewaschen. Alle oben aufgeführten Schritte wurden mit DEPC (Diethylpyrocarbonat)-behandelten Lösungen bzw. Flüssigkeiten durchgeführt soweit diese keine reaktiven Aminogruppen enthielten. Anschließend erfolgte die Herstellung von cDNA aus der extrahierten RNA. Dies erfolgte in Gegenwart von 1 x TaqMan RT-Puffer (Perkin Elmer), 5,5 mM MgCl₂ (Perkin Elmer), je 500 µM dNTPs (Perkin Elmer), 2,5 µM Random Hexamere (Perkin Elmer), 1,25 U/µl MultiScribe Reverse Transcriptase (50 U/µl Perkin Elmer), 0,4 U/µl RNase Inhibitor (20 U/µl, Perkin Elmer), 20 µl RNA (50 ng/µl) und DEPC-behandeltem Wasser (ad 100 µl Volumen). Nach Zugabe der RNA und guter Durchmischung wurde die Lösung auf 2 0,2 ml Gefäße verteilt (je 50 µl) und die reverse Transkription in einem Temperatur-Cycler durchgeführt (10 min bei 25°C; 30 min bei 48°C und 5 min bei 95°C). Die nachfolgende Quantifizierung der cDNA erfolgte mittels quantitativer PCR unter Verwendung des SYBR Green PCR Master Mixes (Perkin Elmer), wobei für jede zu bestimmende cDNA Spezies eine Dreifachbestimmung (jeweils mit Target-Primern und GAPDH-Primern) durchgeführt wurde. Die Stammlösung für jedes Triplett enthielt bei 57 µl Gesamtvolumen 37,5 µl 2 x SYBR Master Mix, 0,75 µl AmpErase UNG (1 U/µl) und 18,75 µl DEPC-behandeltes Wasser. Pro Dreifachbestimmung wurden zu 57 µl Stammlösung je 1,5 µl Vorwärts- und Rüchwärts-Primer in einem zuvor optimierten Konzentrationsverhältnis hinzugefügt. Je 60 µl der Stammlösung/Primer-Mischung wurde mit 15 µl cDNA-Lösung (2 ng/µl) gemischt und auf 3 wells verteilt. Parallel dazu wurde eine Stammlösung mit Primern zur Bestimmung von GAPDH (SEQ ID Nr. 69 und SEQ ID Nr. 70) als Referenz hergestellt, mit weiteren 15 µl der gleichen cDNA-Lösung gemischt und auf 3 wells verteilt. Außerdem wurden um eine Standardkurve für die GAPDH-PCR zu erstellen verschiedene cDNA-Lösungen als Verdünnungsreihe hergestellt (4 ng/µl; 2 ng/µl; 1 ng/µl; 0,5 ng/µl und 0,25 ng/µl). Je 15 µl dieser cDNA-Löungen wurden mit 60 µl Stammlösung/Primer-Mischung zur Bestimmung von GAPDH gemischt und auf 3 wells verteilt. Ebenso wurde eine Standardkurve für die PCR des Targetgens erstellt; dabei wurden dieselben Verdünnungen, die auch für die GAPDH-Standardkurve zum Einsatz kamen, verwendet. Als Kontrolle diente ein PCR Ansatz ohne cDNA. Zu jeweils 60 µl Stammlösung/Primer-Mischung von jeweils Target und GAPDH wurden je 15 µl DEPC-Wasser gegeben, gemischt und jeweils auf 3 wells verteilt. Die Amplifikation der Ansätze wurde im Gene-Amp 5700 durchgeführt (2 min bei 50°C; 10 min bei 95°C, gefolgt von 3 Zyklen mit 15 sek bei 96°C und 2 min bei 60°C; danach 37 Zyklen mit 15 sek bei 95°C und 1 min bei 60°C). Die Auswertung erfolgte durch die Bestimmung der relativen Abundanz jedes Targetgens im Bezug auf die GAPDH Referenz. Dazu wurde zuerst eine Standardkurve erstellt, indem die C_{T}-Werte der Verdünnungsreihe gegen den Logarithmus der cDNA-Menge im PCR-Ansatz (in ng umgeschriebener RNA) aufgetragen wurde und die Steigung (s) der Geraden ermittelt wurde. Die Effizienz (E) der PCR ergibt sich dann wie folgt: E = 10^{-1/s} - 1. Die relative Abundanz (X) der untersuchten cDNA Spezies (Y) im Bezug auf GAPDH ist dann: X=(1+E_{GAPDH})^{C}_{T}^{(GAPDH)}/(1+E_{Y})^{C}_{T}^{(Y)}. Anschließend wurden die Zahlenwerte normiert, indem die Menge an cDNA aus intakte Haut von 10 Wochen alten BALB/c Kontrolltieren bzw. die intakte Haut der C57B/Ks Kontrolltiere gleich 1 gesetzt wurden. Die relativen Änderungen der Expression in verschiedenen Wundheilungszuständen sind in Tabelle 6 zusammengestellt.

So konnte beispielsweise unter Verwendung geeigneter Primer (p68-Primer 1: CCTTATCTCTGTGCTTCGGGAA (SEQ ID Nr. 83); p68-Primer 2: CGACCTGAACCTCTGTCTTCG (SEQ ID Nr. 84)) gezeigt werden, daß deutlich weniger *p68 Helikase* mRNA in Wunden von Kontrollmäusen im Vergleich zu intakter Haut vorhanden ist. Zudem wurde auch in Wunden von alten und jungen Mäusen eine deutlich verringerte Expression beobachtet. Dagegen war die Menge an *p68 Helikase* mRNA in schlecht heilenden Wunden von mit Dexamethason behandelten Tieren stark erhöht. Außerdem wurde auch in Wunden von diabetischen Mäusen eine Erhöhung der Expression im Vergleich zu intakter Haut festgestellt, während in Wunden der Kontrolltiere eine Erniedrigung der Expression beobachtet wurde. Dies verdeutlicht, daß die p68 Helikase während der Wundheilung differentiell exprimiert wird. Darüber hinaus zeigt dieses Experiment, daß eine Erhöhung der p68 Helikase Expression mit einer gestörten Wundheilung einhergeht und daß bevorzugt die Verringerung der p68 Helikase Expression und/oder Proteinaktivität für eine normal verlaufende Wundheilung essentiell ist.

### Beispiel 9: Nachweis der differentiellen Expression von wundrelevanten Genen in humanem Wundgewebe mittels "TaqMan Assay"

Mäuse haben sich als geeignetes Modellsystem für die Untersuchung der Wundheilung im Menschen erwiesen. Trotzdem sollte überprüft werden, ob das in der Maus beobachtete, wundheilungsspezifische Expressionsmuster der erfindungsgemäß verwendbaren Nukleinsäuren auch im Menschen nachgewiesen werden kann. Dazu wurden von gesunden Probanden aus unbehandelter intakter Haut, von Tag 1- Wunden oder von Tag 5-Wunden mittels 4 mm bzw. 6 mm Stanzen Hautproben entnommen. Von jeder Gruppe (intakte Haut, Tag 1-Wunde, Tag 5-Wunde) wurden die Biopsien von jeweils 14 Probanden gepoolt. Außerdem wurde von Patienten mit chronischen venösen Ulzera (Ulcera cruris venosum) Stanzbiopsien gleichzeitig sowohl von intakter Haut als vom Wundgrund und Wundrand entnommen. Von jeder Gruppe (intakte Haut, Wundrand, Wundgrund) wurden die Biopsien von jeweils 6 Probanden gepoolt. Aus allen Biopsien wurde, wie im Beispiel 8 beschrieben, RNA isoliert, danach DNase I verdaut und anschließend in cDNA umgeschrieben. Die Quantifizierung wundheilungsrelevanter cDNAs erfolgte ebenfalls wie in Beispiel 8 beschrieben. Die Ergebnisse der Experimente sind in Tabelle 7 zusammengestellt. Für die Analyse der p68 Helikase wurden die Primer für die Amplifikation (hGAPDH-Primer 1: CATGGGTGTGAACCATGAGAAG (SEQ ID Nr. 75); hGAPDH-Primer 2: CTAAGCAGTTGGTGGTGCAGG (SEQ ID Nr. 76), hp68-Primer 1: GAGGCCATTTCCAGCGACT (SEQ ID Nr. 77), hp68-Primer 2: GAATAACCCGACATGGCGTC (SEQ ID Nr. 78)) anhand der bekannten Sequenzen von humanem GAPDH (GenBank: M17851) und humaner p68 Helikase (Embl: X15729) ausgewählt. Für die Quantifizierung wurden je Ansatz cDNA aus 10 ng revers transkribierter Gesamt-RNA in einem Gesamtvolumen von 25 µl amplifiziert. Die PCR wurde entsprechend den Angaben des Herstellers (PE Applied Biosystems, SYBR Green PCR and RT-PCR Reagents Protocol, 1998) durchgeführt. Die C_{T}-Werte wurden analysiert und daraus wurde die Häufigkeit von hp68 Helikase mRNA relativ zu GAPDH mRNA berechnet. Es zeigte sich, daß die Menge an hp68 Helikase mRNA in humanen Tag 1-Wunden um ca. 60 % im Vergleich zur Menge in intakter Haut reduziert war (Tabelle 7). Dies korrelierte mit der Regulation der Expression des murinen p68 Helikase Homologs in normal heilenden Wunden von 10 Wochen alten Kontrollmäusen als auch in Wunden von jungen und alten BALB/c Mäusen im Vergleich jeweils zu intakter Haut (Tabelle 6). Bei diesen Versuchen wurde eine um 30% bis 70% verminderte Expression in Tag 1-Wunden beobachtet.

In humanen Tag 5-Wunden wurde ein Wiederanstieg der Menge an hp68 Helikase mRNA auf 60% des Wertes in intakter Haut beobachtet, was darauf hinweist, daß die Regulation der Expression in einer sehr frühen Phase der Wundheilung essentiell ist. Die Analyse von normal heilenden Tag 5-Wunden von 10 Wochen alten BALB/c Mäusen ergab, daß auch im murinen Zellsystem die Menge an muriner p68 Helikase mRNA auf 67% des Wertes in intakter Haut ansteigt. Somit ist auch die Kinetik der Expression von p68 Helikase in normal heilenden Wunden sowohl in der Maus als auch im Menschen vergleichbar. Somit konnte in diesem Versuch gezeigt werden, daß die im Maussystem als wundrelevant identifizierten Gene auch im Menschen differentiell exprimiert werden. Dies untermauert das Ergebnis des Versuchs aus Beispiel 8, bei dem nachgewiesen werden konnte, daß p68 Helikase essentiell für die Wundheilung ist. Zudem zeigte sich, daß im Ulkus, der einen gestörten Wunheilungsprozeß darstellt, im Bezug auf die intakte Haut dieser Patienten keine Verringerung der p68 Helikase Expression wie in der normal verlaufenden Wundheilung feststellbar ist, sondern vielmehr eine leichte Zunahme der Expression beobachtbar ist. Diese ist vor allem im Wundgrund besonders deutlich. Dies beweist, daß die Regulation der Expression nicht nur eine essentielle Rolle für die Wundheilung im Menschen spielt, sondern darüber hinaus für den normalen Verlauf der Wundheilung essentiell ist. Eine gestörte, vorzugsweise eine verstärkte Expression der p68 Helikase kann zu schweren Wundheilungsstörungen führen.

### Beispiel 10: Lokalisation wundrelevanter Gene in humaner intakter Haut und in Wunden mittels in situ Hybridisierung

Um weitere Informationen über die Bedeutung der als wundrelevant identifizierten Gene zu erhalten, wurde die mRNA in Schnitten von humanen Wunden und von intakter Haut mittels radioaktiver oder nicht-radioaktiver *in situ* Hybridisierung nachgewiesen. Der Erfolg einer *in situ* Hybridisierung hängt kritisch von einer Vielzahl von Parametern ab, z.B. von der Abundanz der mRNA im Gewebe, einer optimalen Hybridisierungstemperatur oder der Stringenz der Wasch-Schritte im Anschluß an die Hybridisierung.. Auch die Wahl einer geeigneten Sondensequenz ist essentiell. Für das Gelingen des Experiments sind daher aufwändige Optimierungsschritte nötig. Trotz der experimentellen Hindernisse konnte mittels *in situ* Hybrisierung die Wundrelevanz von MRP-8, MRP-14 und p68 Helikase, die bereits in Beispiel 8 gezeigt wurde, untermauert werden.

Für das Experiment wurden gesunden Patienten Biopsien von intakter Haut als auch von normal heilenden Tag 5-Wunden wie in Beispiel 9 beschrieben entnommen. Gewebeschnitte wurden in 4% Paraformaldehyd fixiert, mit Proteinase K (1µg/ml isotonischer Salzlösung) für 10 min bei 37°C behandelt, wiederum mit Paraformaldehyd und anschließend mit Acetanhydrid (0,5 ml in 0,1 M Triethanolamine, pH 8,0) behandelt.

Die Lokalisierung der mRNA von humanem MRP8 und MRP14 erfolgte durch nicht-radioaktive *in situ* Hybridisierung. Dazu wurde ein partielles humanes MRP8 cDNA-Fragment mittels PCR amplifiziert. Die dabei verwendeten Primer enthielten dabei zusätzlich zum MRP8-homologen Abschnitt einen RNA-Polymerase-Promoter zur Herstellung von Ribosonden (verwendeter antisense Primer (T7-MRP8): TAATACGACTCACTATAGGGCCCACGCCCATCTTTATCACCAG (SEQ ID Nr. 79); verwendeter sense Primer (T3-MRP8): AATTAACCCTCACTAAAGGGGGAATTTCCATGCCGTCTACAGG (SEQ ID Nr. 80). Das amplifizierte cDNA-Fragments wurde in den pCR 2.1 TOPO Vektor (Invitrogen) kloniert und anschließend durch Sequenzierung verifiziert. Die Herstellung der antisense Ribosonde und der sense-Kontrolle erfolgte unter Verwendung des DIG RNA Labelling Mixes (Roche) und der jeweiligen RNA Polymerase nach den Angaben des Herstellers. Die anschließende in situ Hybridisierung wurde wie bei Komminoth et al. (1992, Histochemistry 98:217-228) beschrieben durchgeführt.

Im Vergleich zur intakten Haut konnte in humanen Tag 5-Wunden mit der antisense-Sonde eine starke Induktion der mRNA Expression in den suprabasalen Zellschichten des hyperproliferativen Epithels festgestellt werden, während mit der sense-Sonde keine Färbung beobachtet wurde. Dies ist in Übereinstimmung mit dem Ergebnis der quantitativen Analyse der Menge an muriner *Mrp8* mRNA mittels "TaqMan Analyse", bei der eine deutliche Hochregulation in Tag 1-Wunden (Tabelle 6) und auch in Tag 5-Wunden festgestellt wurde. Zusätzlich zur Verifikation der Hochregulation der Genexpression verdeutlicht die Loklisierung im hyperproliferativen Epithel, daß das Gen eine wichtige Rolle bei einem zentralen Prozeß der Wundheilung spielt. Somit konnte in diesem Experiment die Wundrelevanz des im Maussystem identifizierten Mrp8 im Menschen untermauert werden.

Die Lokalisierung von MRP-14 erfolgte analog zur Lokalisierung von MRP-8, wobei als antisense Primer der Sp6-MRP-14 Primer (ATTTAGGTGACACTATAGAATAC CCC GAG GCC TGG CTT ATG GT; SEQ ID Nr. 125) und als Kontroll-sense Primer der T3-MRP-14 Primer (AATTAACCCTCACTAAAGGGG GTG GCT CCT CGG CTT TGA CA; SEQ ID NR. 126) verwendet wurden. Die Lokalisierung in intakter Haut und in Tag 5-Wunden erfolgte wie für MRP-8 beschrieben.

Es zeigte sich, daß auch die MRP-14 mRNA stark in den suprabasalen Zellschichten des hyperproliferativen Epithels exprimiert wird, während mit der sense-Sonde keine Färbung beobachtet wurde. Dies ist in Übereinstimmung mit dem Ergebnis der quantitativen Analyse der Menge an muriner *Mrp14* mRNA mittels "TaqMan Analyse", bei der eine deutliche Hochregulation in Tag 1-Wunden (Tabelle 6) und auch in Tag 5-Wunden festgestellt wurde. Somit konnte in diesem Experiment die Wundrelevanz des im Maussystem identifizierten Mrp-14 im Menschen untermauert werden. Darüber hinaus beweist dieses Experiment, daß beide Monomere des Heterodimers MRP-14/MRP-8 in gleicher Weise wundreguliert sind. Die Expression von MRP-14/MRP-8 in den suprabasalen Keratinozyten des hyperproliferativen Epithel zeigt zudem, dass das Dimer nicht nur wie bisher vermutet an inflammatorischen Prozessen beteiligt ist, sondern eine essentielle Funktion bei der Proliferation und/oder Differenzierung der Keratinozyten im Verlauf der Wundheilung hat.

Die Lokalisierung der p68 Helikase in humanen Haut- und Wundbiopsien erfolgte durch radioaktive *in situ* Hybridisierung. Dazu wurden Paraformaldehyd-fixierte Schnitte von Proben intakter Haut und Tag 5-Wunden in Paraffin eingebettet. Die Herstellung der Hybridisierungssonde basierte auf der *in vitro* Transkription von einem partiellen cDNA-Fragment der humanen p68 Helikase in Gegenwart von α-³⁵S-UTP. Die Herstellung des PCR-Produkts erfolgte mit Primern, an die die Promotersequenzen für eine Transkription in sense und antisense Richtung angefügt wurden. (T3-p68-Primer: AATTAACCCTCACTAAAGGGGGCAACATTACTTCCATATTGC (antisense-Primer mit T3-Promotor; SEQ ID Nr. 81), T7-p68-Primer: TAATACGACTCACTATAGGGCGAGACAGGGAAAACTATGAC (sense-Kontrollprimer mit T7-Promotor; SEQ ID Nr. 82). Für die *in vitro* Transkription wurden 60 µCi ³⁵S-UTP und je 5 mM ATP, GTP und CTP, sowie entweder 25 U T3 oder T7 RNA Polymerase (Roche), 1 µg PCR-Produkt, 10 mM Dithiothreitol, 40 U RNAse Inhibitor (Roche) und IX TB-Puffer (Roche) verwendet.

Die humanen Gewebeschnitte (siehe oben) wurden auf Objektträgern mit Proteinase K-behandelt, mit Paraformaldehyd fixiert und anschließend acetyliert. Anschließend wurde die Schnitte in einer Kammer, in der feuchte, mit 50% Formamid/4x SSC getränkte Whatman Papiere lagen, mit 30 µl Hybridisierungslösung bedeckt und 2,5 h bei 60°C inkubiert. Danach wurden die Schnitte mit 0,7 x 10⁶ cpm radioaktiv markierter Ribosonde in 30 µl Hybridisierungslösung 16 h bei 60°C inkubiert. Anschließend wurden die Schnitte unter stringenten Bedingungen gewaschen, mit RNAse A inkubiert und mit Ethanol dehydratisiert. Die Schnitte wurden dann mit Photoemulsion (Kodak IBO 1433) überschichtet, unter Ausschluß von Licht und Sauerstoff für 2-6 Wochen bei 4°C gelagert und anschließend mittels Entwickler und Fixierer (Kodak IBO 1433) nach Angaben des Herstellers entwickelt.

Es zeigte sich, daß p68 Helikase in den basalen Zellschichten des Epithels in intakter Haut exprimiert wird, während mit der sense-Kontrollribosonde keine Hybridisierung nachweisbar war. In humanen Tag 5-Wunden wurde eine deutlich verminderte Expression in den basalen Zellschichten des Epithels beobachtet. Dies ist in Übereinstimmung mit dem Ergebnis der "TaqMan Analyse" humaner intakter Haut und humaner Tag 5-Wunden (Beispiel 9; Tabelle 7) und der "TaqMan Analyse" muriner Gewebeproben (Beispiel 8; Tabelle 6). Die Lokalisierung in den stark proliferierenden basalen Zellschichten des Epithels bestätigt zudem, daß die differentielle Expression der p68 Helikase eine wichtige Rolle bei der Wundheilung spielt. Der Versuch untermauert daher zusätzlich das Ergebnis von Beispiel 8, das zeigt, daß die Regulation der p68 Helikase Expression, vorzugsweise die Inhibition, für Wundheilung essentiell ist.

### Beispiel 11: Verifikation der differentiellen Expression wundheilungsrelevanter Polypeptide

Um die essentielle Funktion der Polypeptide, die man im Maussystem als wundheilungsrelevant identifiziert hat, durch eine weitere Methode zu bestätigen, wurden Gewebeschnitte von humaner intakter Haut und humaner normal heilender Wunden mittels Immunfärbung untersucht. Der Erfolg einer Immunfärbung hängt, wie auch schon die in situ Hybridisierung (Beispiel 10) entscheidend von den Versuchsbedingungen ab. Im Falle der Immunfärbung sind beispielsweise die Vorbehandlung der Gewebeschnitte, die Affinität des Antikörpers und die Nachweismethode kritisch für das Gelingen. Trotz dieser vielfältigen experimentellen Probleme, die sich ergeben können, konnte die wundheilungsspezifische Expression von MBNL und Rab2 und CBP (Beispiel 8) mittels Immunfärbung verifiziert werden.

Die Lokalisierung von humanem MBNL erfolgte mit einem polyklonalen Kaninchen Antikörper (Eurogenetec), der gegen die Peptide ArgGluPheGlnArgGlyThrCysSerArgProAspThrGluCys (SEQ ID Nr. 85; Eurogenetec) und ArgGluTyrGlnArgGlyAsnCysAsnArgGlyGluAsnAspCysArg (SEQ ID Nr. 86; Eurogenetec) gerichtet war. Native Kryoschnitte von intakter Haut und Tag 5-Wunden gesunder Patienten wurden durch Schneiden der Biopsien im Gefriermikrotom bei -20°C hergestellt. Die Schnitte wurden 10 min mit Aceton fixiert. Die Inkubation mit dem spezifischen Antiserum erfolgte eine Stunde bei Raumtemperatur in einer feuchten Kammer. Dafür wurden die Schnitte mit 100 µl verdünntem Serum (1:200 in PBS, 1% BSA) inkubiert. Als sekundärer Antikörper wurde ein Peroxidase-gekoppelter anti-Kaninchen Antikörper aus Ziege (Dianova) verwendet, der in einer 1:1000 Verdünnung (in PBS, 1% BSA) eingesetzt wurde. Die Schnitte wurden mit 100 µl der sekundären Antikörperlösung bedeckt und eine Stunde in der feuchten Kammer inkubiert. Anschließend wurden die Objektträger 3 Mal mit PBS gewaschen und die Nachweisreaktion durchgeführt. Dazu wurden die Schnitte 10 Minuten in Färbelösung (frisch hergestellt aus: 10 ml AEC-Lösung (5 mg/ml 3-Amino-9-Ethylcarbazol in Dimethylformamid); 200 ml 50 mM Natriumacetat, pH 5,2; 30 µl H202) inkubiert und kurz mit PBS gewaschen. Die Gegenfärbung erfolgte mit Mayer's Hematoxylin Lösung (Sigma) für 5 min bei Raumtemperatur. Nach dem Waschen mit Leitungswasser wurden die Schnitte 1 min in "Scott's Tap Water Substitute" (Sigma) inkubiert, wiederum mit Leitungswasser gewaschen und mit Immu-Mount (Shandon) eingebettet.

Das MBNL Protein ist in den basalen Zellschichten der intakten Haut stark exprimiert. Im Vergleich dazu weisen die suprabasalen Zellschichten eine wesentlich schwächere Expression auf. Im hyperproliferativen Epithel einer Wunde (am Tag 5 nach Wundsetzung) und in der wundnahen Epidermis findet man eine gleichmäßige Verteilung des Proteins über alle Zellschichten der Epidermis hinweg. Eine stärkere Konzentration des Proteins in der basalen Zellschicht ist hier nicht erkennbar. Das Experiment verdeutlicht, daß die Expression des wundrelevanten Gens MBNL nicht nur zeitlich sondern auch räumlich differentiell reguliert ist, was ein Wechselspiel zwischen verschiedensten Zelltypen impliziert. Dieses Beispiel zeigt klar, daß durch die Komplexität des Wundheilungsprozesses, die z.B. die zeitliche und räumliche Änderung der beteiligten Zelltypen umfasst, große Schwierigkeiten bei der Identifizierung wundrelevanter cDNAs auftreten können. Dies verdeutlicht umso mehr, daß es überraschend war, MBNL als wundrelevant identifizieren zu können und dass das richtige Design des Screens entscheidend zur Identifikation erfindungsgemäß verwendbarer, wundrelevanter Gene beigetragen hat.

Die Immunlokalisierung von sowohl Rab2 als auch CBP in humaner intakter Haut und humanen Tag 5-Wunden wurde an nativen Kryoschnitten durchgeführt, wie oben dargelegt. Die Immunfärbungsreaktion basierte auf dem Vectastain Universal Elite ABC Kit (Vector Laboratories, Inc., # PK-6200). Hierzu wurden die Schnitte zunächst mit Blockierungslösung (Pferde Serum in PBS) bedeckt und bei Raumtemperatur 20 min in einer feuchten Kammer inkubiert. Diese Lösung wurde anschließend abgezogen. Dann wurden die Schnitte mit 100 µl des polyklonalen Kaninchen anti-Rab2 Antikörpers (Santa Cruz, # sc-307; Verdünnung: 1:200 in PBS/1% BSA) bzw. des polyklonalen Kaninchen anti-CBP Antikörpers (Upstate Biotechnology, # 06-294; Verdünnung: 1:1500 in PBS/1% BSA) bedeckt. Die Inkubationsdauer betrug 30 min in einer feuchten Kammer. Danach wurden die Objektträger 3 Mal mit PBS gewaschen und anschließend für 30 min mit 100 µl des sekundären Antikörpers (Peroxidase gekoppelter Pferd anti Kaninchen und anti Maus Antikörper; Vector Laboratories, Inc.; # BA-1400) inkubiert. Die Objektträger wurden wiederum 3 Mal mit PBS gewaschen und anschließend mit jeweils 100 µl Vectastain Elite ABC Reagent (Vector Laboratories, Inc.) für 30 min bedeckt. Danach wurden die Objektträger mit PBS gewaschen und die Färbeschritte (Peroxidase-Substrat-Färbung und Hematoxylin Gegenfärbung) wie im oberen Abschnitt beschrieben durchgeführt.

Die Immunfärbung mit dem anti-Rab2 Antikörper zeigte eine starke Induktion der Proteinexpression in humanen Tag 5-Wunden in den suprabasalen Zellschichten des hyperproliferativen Epithels. Dies steht scheinbar im Gegensatz zu den Ergebnissen der "TaqMan Analyse" im murinen System, die eine leichte Erniedrigung der Menge an mRNA in Tag 1-Wunden ergaben (Tabelle 6). Die genauere Analyse der Kinetik der Expression in murinen Wunden ergab jedoch, daß ab Tag 3 nach Wundsetzung ein stetiger Anstieg der mRNA Menge mittels "TaqMan Analyse" zu beobachten ist, was bei Tag 5 in einer Verdoppelung und bei Tag 7 in einer Vervierfachung der *rab2* mRNA in Bezug auf die Menge bei Tag 3 resultiert. Dies wiederum korreliert mit dem beobachteten Anstieg der Proteinexpression in humanen Tag 5-Wunden. Wie schon im Falle der MBNL-Expression (siehe oberer Abschnitt) verdeutlichen die Daten wiederum die Komplexität des Wundheilungsprozesses, in diesem Falle die Komplexität der Expressionskinetik, die eine Identifizierung wundrelevanter Gene wesentlich erschwert. Somit konnte die essentielle Funktion von Rab2 im Verlauf der Wundheilung durch die Immunfärbung bestätigt werden.

Die Immunfärbung mit dem anti-CBP-Antikörper ergab, daß das "CREB binding protein" (CBP) in intakter Haut vor allem in den Basalzellen des Epithels exprimiert wird. Im hyperproliferativem Epithel am Tag 5 nach Verwundung finden sich deutlich weniger Zellen, die dieses Protein exprimieren. Dies stimmt mit der Beobachtung mittels "TaqMan Analyse" überein, daß die Menge an *CBP* mRNA in Wunden von 10 Wochen alten Kontrolltieren, als auch in Tag 1-Wunden von jungen und alten Mäusen deutlich geringer ist als in der intakten Haut (Tabelle 6). Diese wundspezifische Runterregulation konnte auch in murinen Tag 5-Wunden mittels "TaqMan Analyse" bestätigt werden. Daher konnte in diesem Experiment erneut die essentielle Funktion eines erfindungsgemäß verwendbaren Gens in der Wundheilung bestätigt werden.

### Beispiel 12: Untersuchung der differentiellen Proteinexpression von p68 Helikase in intakter Haut, in normal heilenden Wunden und im Ulkus von Menschen mittels Immunfärbung

Es konnte gezeigt werden, daß die mRNA der *p68 Helikase* bei der Wundheilung sowohl im Menschen als auch in der Maus differentiell reguliert wird (Beispiele 8, 9). Dadurch konnte bewiesen werden, daß die p68 Helikase essentiell für die Wundheilung ist (Beispiel 8). Ein weiteres Experiment, durch das die Bedeutung der p68 Helikase verdeutlicht werden konnte, ist die Immunlokalisierung von p68 Helikase in Stanzbiopsien von humaner intakter Haut, von humanen Tag 5-Wunden gesunder Patienten und in Stanzbiopsien humaner Haut-Ulzera (Ulcera cruris venosum).

Dies erfolgte mit einem polyklonalen Kaninchen Antikörper (Eurogenetec), der gegen die Peptide CysAspGluLeuThrArgLysMetArgArgAspGlyTrpProAla (SEQ ID Nr. 87; Eurogenetec) und AsnThrPheArgAspArgGluAsnTyrAspArgGlyTyrSerSerCys (SEQ ID Nr. 88; Eurogenetec) gerichtet war. Die nativen Kryoschnitte der Gewebeproben wurden durch Schneiden der Biopsien im Gefriermikrotom bei -20°C hergestellt. Die Schnitte wurden anschließend 10 min mit Aceton fixiert. Die Inkubation mit dem spezifischen Antiserum erfolgte eine Stunde bei Raumtemperatur in einer feuchten Kammer. Dafür wurden die Schnitte mit 100 µl verdünntem Serum (1:200 in PBS, 1% BSA) inkubiert. Als sekundärer Antikörper wurde ein Peroxidase-gekoppelter anti-Kaninchen Antikörper aus Ziege (Dianova) verwendet, der in einer 1:1000 Verdünnung (in PBS, 1% BSA) eingesetzt wurde. Die Schnitte wurden mit 100 µl der sekundären Antikörperlösung bedeckt und eine Stunde in der feuchten Kammer inkubiert. Anschließend wurden die Objektträger 3 Mal mit PBS gewaschen und die Nachweisreaktion durchgeführt. Dazu wurden die Schnitte 10 Minuten in Färbelösung (frisch hergestellt aus: 10 ml AEC-Lösung (5 mg/ml 3-Amino-9-Ethylcarbazol in Dimethylformamid); 200 ml 50 mM Natriumacetat, pH 5,2; 30 µl H₂O₂) inkubiert und kurz mit PBS gewaschen. Die Gegenfärbung erfolgte mit Mayer's Hematoxylin Lösung (Sigma) für 5 min bei Raumtemperatur. Nach dem Waschen mit Leitungswasser wurden die Schnitte 1 min in "Scott's Tap Water Substitute" (Sigma) inkubiert, wiederum mit Leitungswasser gewaschen und mit Immu-Mount (Shandon) eingebettet.

Die Auswertung der Immunfärbung ergab, daß die p68 Helikase in intakter Haut stärker in den basalen Keratinozyten als in den suprabasalen Zellschichten exprimiert wird. Dagegen wird das Protein in normal heilenden Tag 5-Wunden im Bereich des Wundrands deutlich weniger stark exprimiert, wodurch ein räumlicher Protein-Gradient entsteht. Im Ulkus mit verzögerter Wundheilung wiederum findet diese Gradientenbildung und Verringerung der Expression in Richtung des Wundrandes nicht statt. Dies bestätigt, daß p68 Helikase im Verlauf der Wundheilung differentiell exprimiert wird, und daß die genaue Regulation für eine normal verlaufende Wundheilung essentiell ist. Somit konnte exemplarisch bestätigt werden, daß die Fehlregulation der wundrelevanten p68 Helikase zu Wundheilungsstörungen im Menschen führt und daß die p68 Helikase als therapeutisches Target geeignet ist: Wundheilungsstörungen können durch Modulation vorzugsweise durch Inhibition der p68 Helikase Aktivität behandelt werden.

### Beispiel 13: Nachweis der Wundheilungsrelevanz von MRP8 mittels in vivo Applikation des Gens in Kaninchen-Wunden

Um zu bestätigen, daß MRP8 für den Prozess der normal verlaufenden Wundheilung eine entscheidende Rolle spielt, wurde der Einfluß von MRP8 auf die Wundheilung *in vivo* in männlichen, weißen Neuseeland Kaninchen untersucht. Die Wundheilung wurde anhand der Menge an Kollagen im Granulationsgewebe bestimmt, wobei eine verminderte Menge an Kollagen bezüglich einer normal heilenden Wunde auf eine verschlechterte Wundheilung hinweist. Dazu wurde ein Expressionsplasmid pMHintMRP8 hergestellt, das die kodierende Sequenz von MRP8 enthielt. Zunächst wurde ein geeigneter Expressionsvektor pMHint konstruiert, der ausgehend von pMH (Roche) hergestellt wurde, indem zwischen dem CMV Promotor und der Multiple Cloning Site in die HindIII Schnittstelle das Intron II des Insulingens aus der Ratte eingefügt wurde. Unter Verwendung der Multiple Cloning Site wurde dann die MRP8 cDNA in pMHint kloniert. Hierzu wurde der kodierende Bereich der MRP8 cDNA mittels PCR amplifiziert (MRP8-Primer 1: GAG AGA GGT ACC ATG CCG TCT GAA CTG GAG (SEQ ID Nr. 127) und MRP8-Primer 2: GAG AGA GAC ACG TGC TAC TCC TTG TGG CTG TCT TTG (SEQ ID NR. 128)), anschließend mit Kpnl und Pmll geschnitten, mit dem mit KpnI und Pmll geschnittenen Expressionsvektor pMHint ligiert und somit das Expressionplasmid pMHintMRP8 erhalten.

Für die Analyse des Einflusses von MRP8 auf die Wundheilung wurden zwei Kaninchen verwendet, die mit Xylazin und Ketamin (13 bzw. 87 mg/kg i.m.) betäubt und mit Enthaarungscreme behandelt wurden. Anschließend wurde Adrenalinlösung (2 % Xylocain und Adrenalin, 1:100,000, ASTRA) intradermal eingespritzt um Gefäßverengung zu erreichen und um die Haut vom darunterliegenden Ohrknorpel zu trennen. Mittels Stanzen wurden dann vier 8 mm große Wunden auf der inneren Seite des Ohrs gesetzt. Jede Wunde wurde unter Verwendung der Helios Gene Gun (BioRad) entweder mit pMHintMRP8 oder als Kontrolle mit pMHint, in das das Luziferase-Gen kloniert worden war, bei einem Druck von 500 psi beschossen, wobei pro Schuß 0,5 µg Expressionsplasmid eingesetzt wurde, das auf Goldpartikeln (BioRad) immobilisiert worden waren. Anschließend wurden die Wunden mit einem semi-okklusiven Verband abgedeckt. Am Tag 10 nach Verwundung wurden die Tiere eingeschläfert, Wundbiopsien entnommen und Granulationsgewebe untersucht. Das Gewebe wurde in 1 N NH₄OH homogenisiert und mittels HPLC auf die Menge an Kollagen hin untersucht. Dabei wurde bei der Kontrolle ein Gesamtkollagengehalt von 195 µg/g Gewebe gemessen, während in Gewebe, das mit pHMintMRP8 beschossen wurde eine um 74 % geringere Menge an Kollagen bestimmt wurde. Dies verdeutlicht, daß eine erhöhte Menge an MRP8 im Wundgewebe zu verschlechterter Wundheilung führt, wenn die Menge des Bindungspartners MRP-14 nicht beeinflußt wird. Dies läßt auf einen negativen Effekt des MRP-8-Homodimers auf die Wundheilung schließen. Da Bedingungen, die die Homodimerbildung verhindern, die Wundheilung positiv beeinflussen, ist die Expression und/oder Aktivität von MRP-8 zu modulieren, vorzugsweise zu inhibieren: so kann die Bildung von MRP-8-Homodimeren durch die Erniedrigung der MRP-8-Menge im Gewebe beispielsweise durch Verwendung von MRP-8-antisense-Oligonukleotiden verhindert werden. Auch die Verwendung eines anti-MRP-8 Antikörpers oder eines funktionellen Interaktors zur Sequestrierung der Monomere hätte diesen Effekt. Außerdem bietet sich Verwendung des MRP-8/MRP-14 Heterodimers als therapeutisch wirksames Agens an, das zur Erhöhung der Menge des Heterodimers im Vergleich zum negativ auf die Wundheilung wirkenden Homodimers führt. Die Applikation kann dabei beispielsweise durch rekombinant hergestellte Polypeptide oder durch gentherapeutische Expressionsplasmide erfolgen.

**Tabelle 1**

| **Verändert exprimierte Gene** | Intakte Haut Kontrolltiere | Wunde Kontrolltiere | Intakte Haut Dexamethason | Wunde Dexamethason |
|---|---|---|---|---|
| KIAA0494 | 2,38E-03 | 1,15E-03 | 2,20E-03 | 2,71E-03 |
| mas Onkogen | 1,44E-05 | 2,00E-02 | 2,61E-05 | 1,68E-02 |
| basic-leucine zipper nuclear factor JEM-1 | 7,33E-04 | 2,08E-03 | 4,20E-04 | 1,05E-03 |
| checkpoint suppressor | 1,98E-01 | 1,39E-01 | 9,44E-02 | 6,56E-02 |
| Ribonuklease L inhibitor (Mu-RLI) | 1,07E-02 | 3,13E-02 | 7,70E-03 | 3,61E-02 |
| p68 RNA Helikase | 1,29E-01 | 2,39E-01 | 5,86E-02 | 8,39E-02 |
| mKAP13/pmg-1 | 5,04E+00 | 2,98E+00 | 1,51E+00 | 2,88E-01 |
| pmg-2 | 8,60E-02 | 7,43E-02 | 1,29E-02 | 1,74E-03 |
| MA-3 (apoptosis-related gene) | 1,02E-03 | 2,02E-03 | 3,51E-04 | 4,42E-04 |
| BTG1 | 1,43E-05 | 1,23E-05 | 2,60E-06 | 3,87E-06 |
| helix-loop-helix transcription factor | 1,14E-05 | 2,40E-05 | 7,15E-06 | 1,88E-05 |
| potentially prenylated protein tyrosine phosphatase | 9,31E-03 | 1,85E-02 | 5,60E-03 | 1,49E-02 |

| **Markergen** | | | | |
|---|---|---|---|---|
| spi2 proteinase inhibitor (spi2/eb4) | 3,59E-02 | 1,80E-01 | 3,21E-02 | 9,67E-02 |

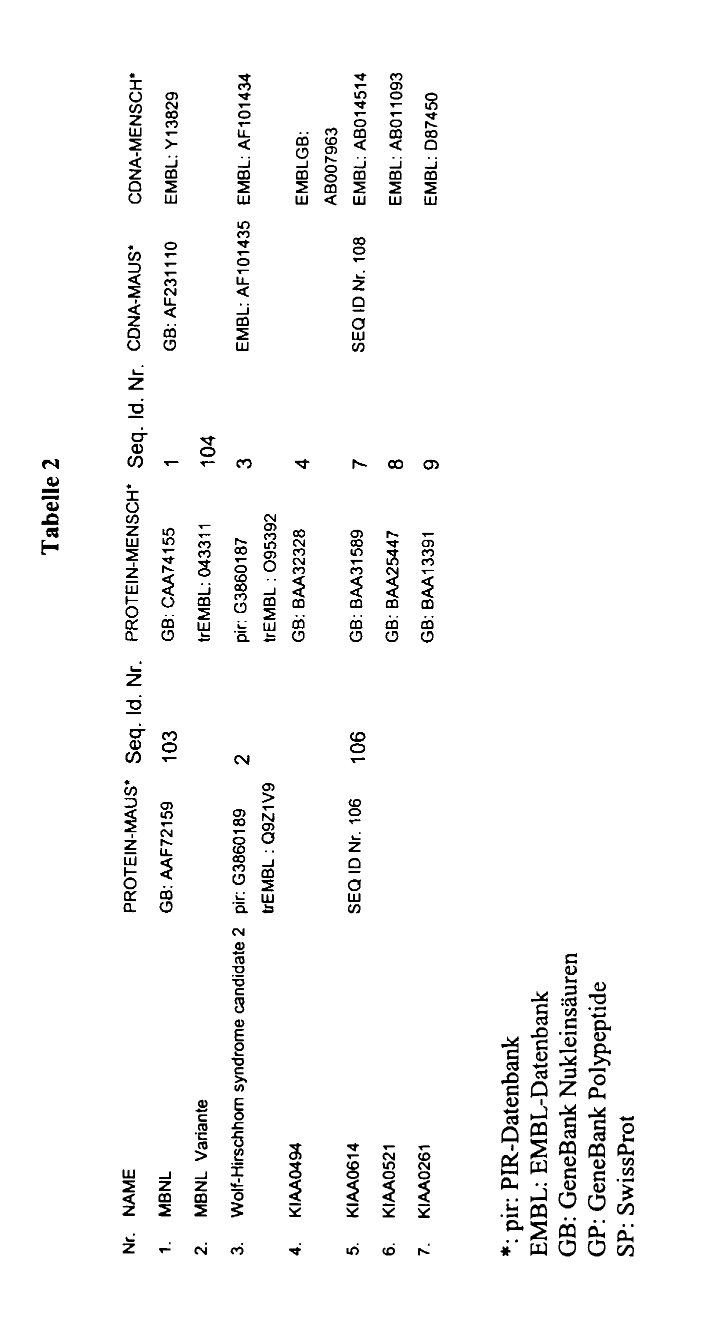

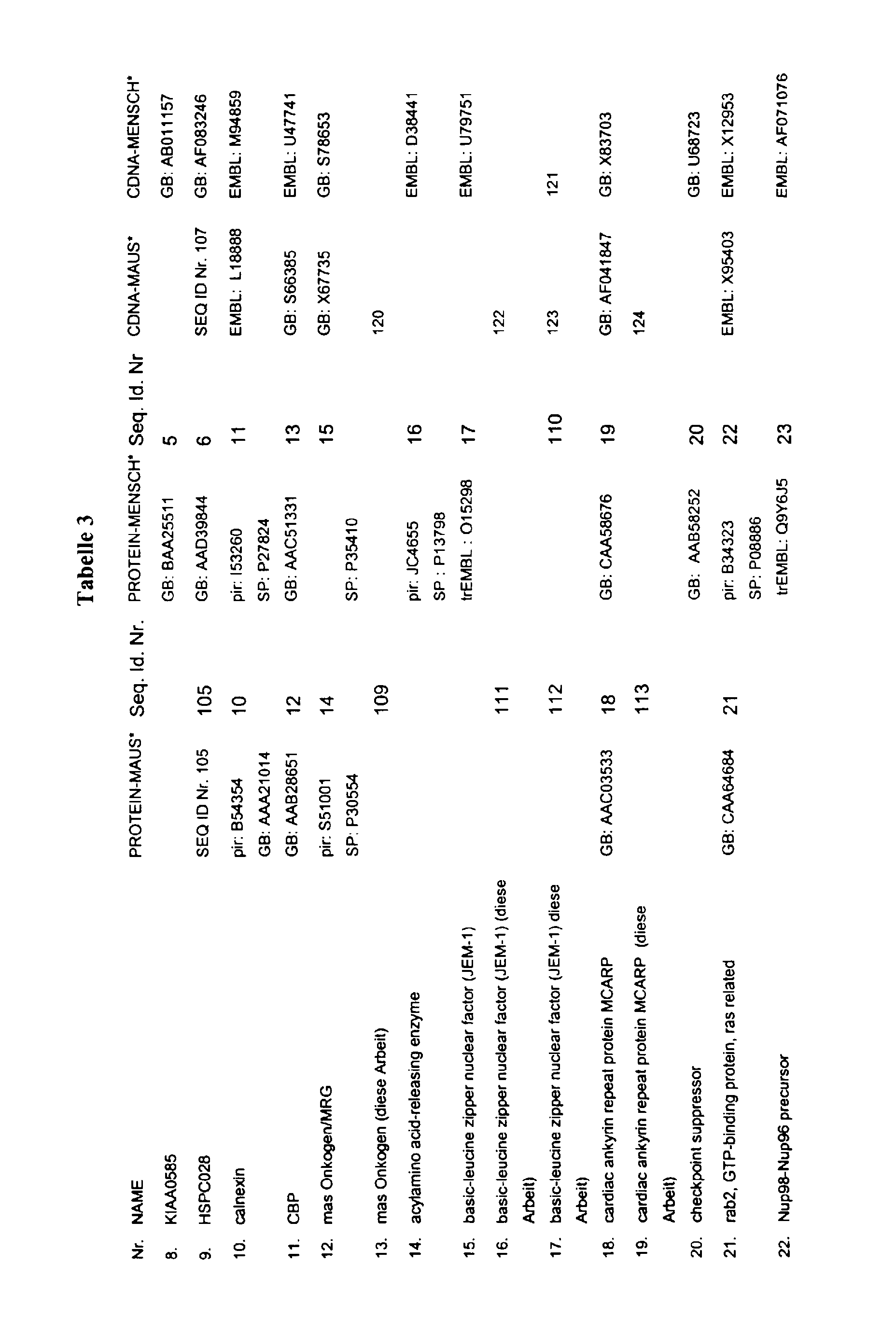

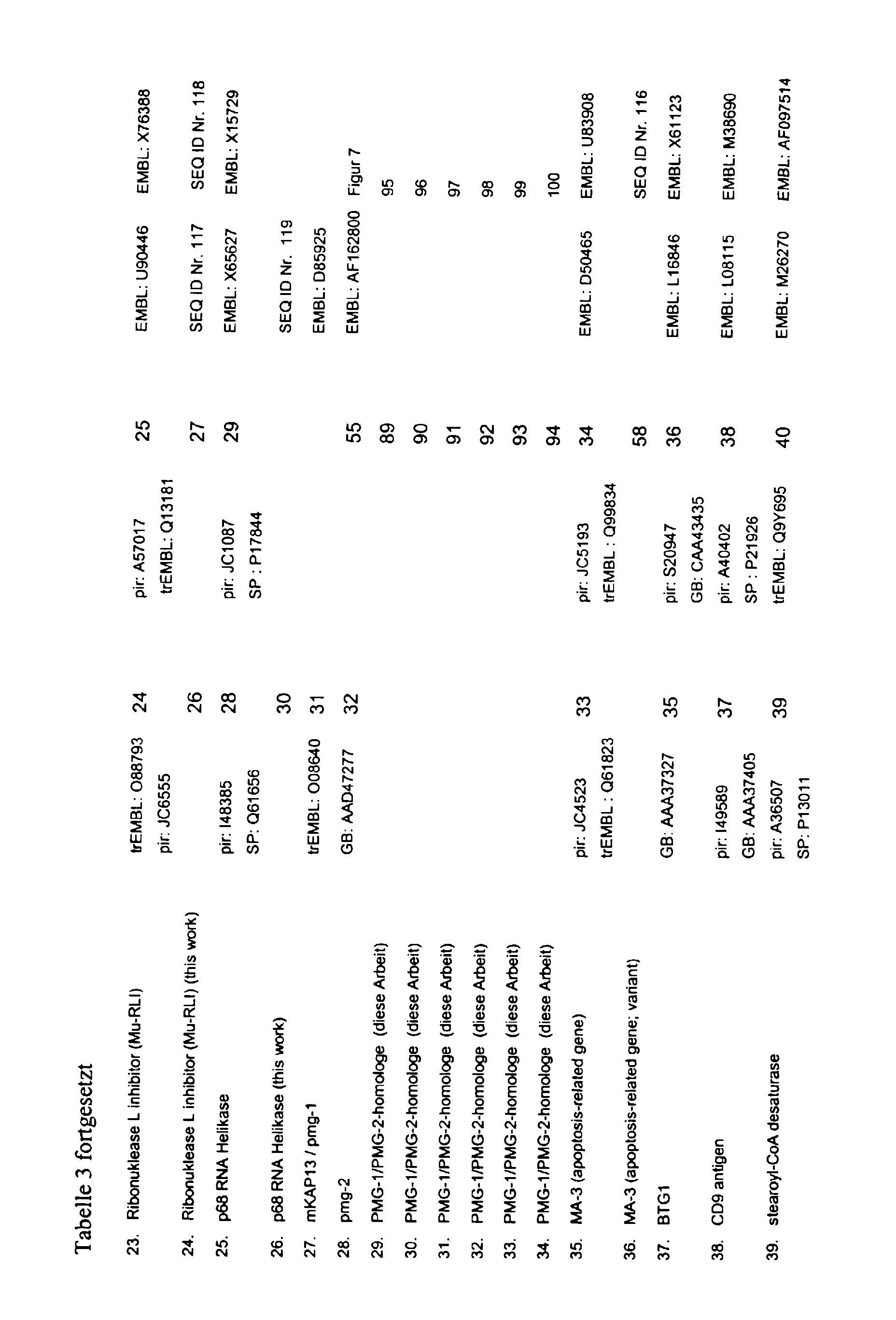

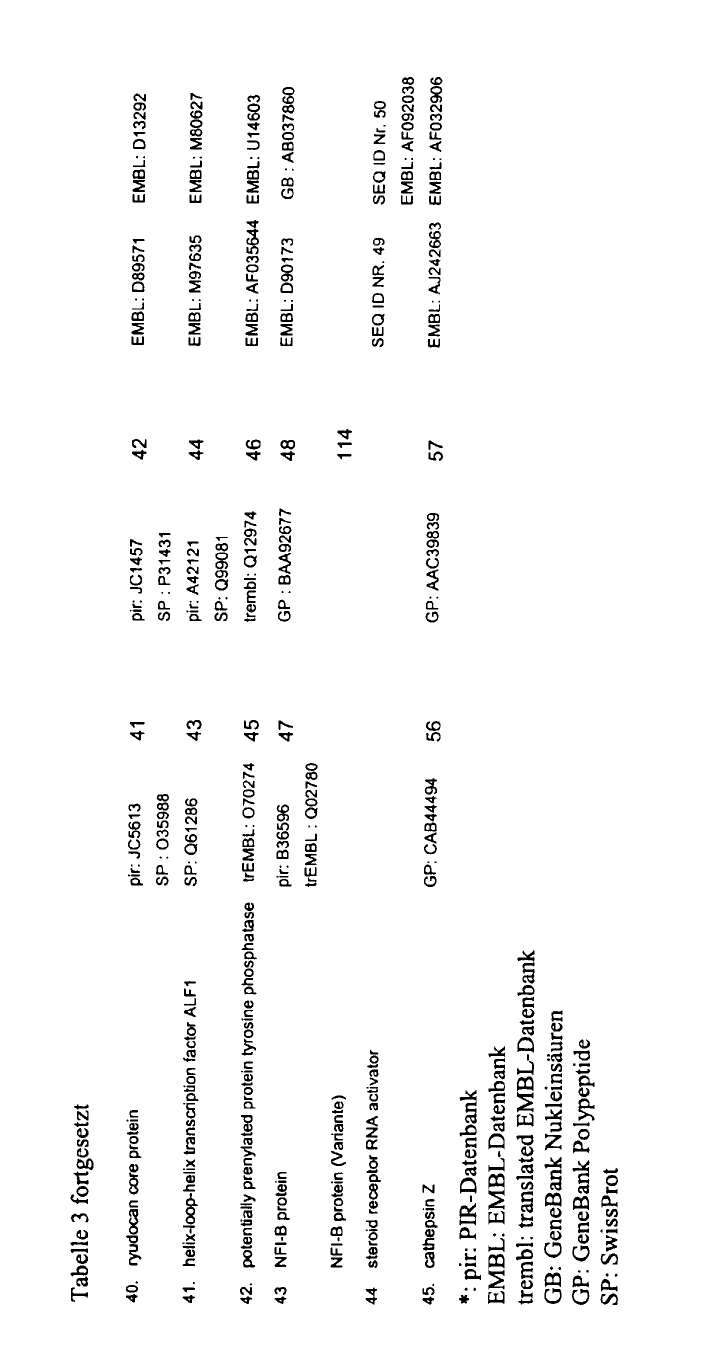

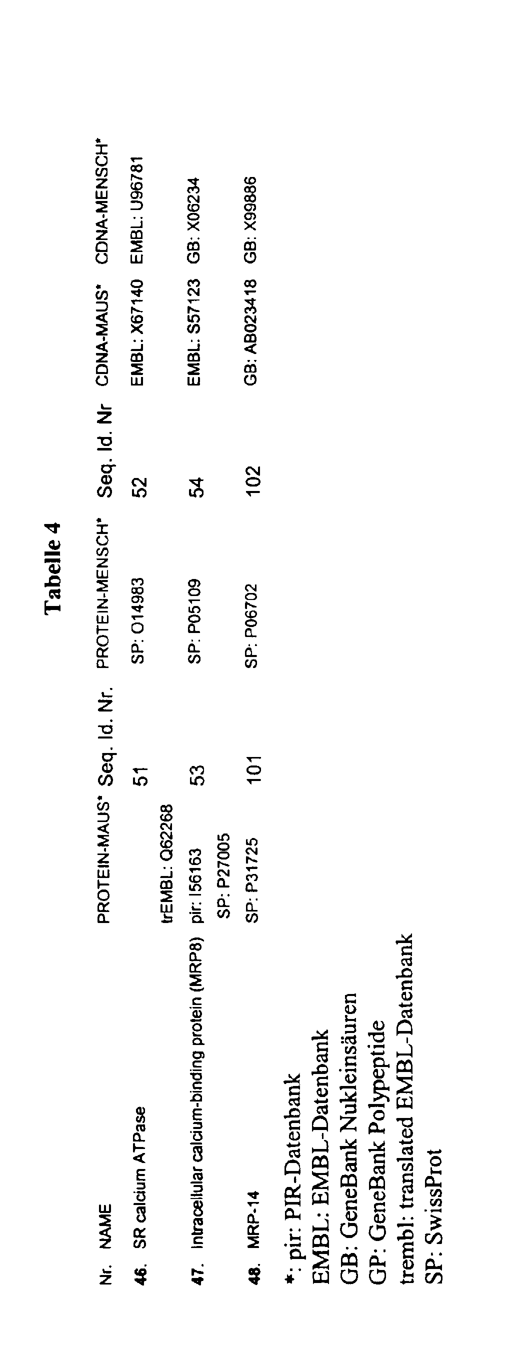

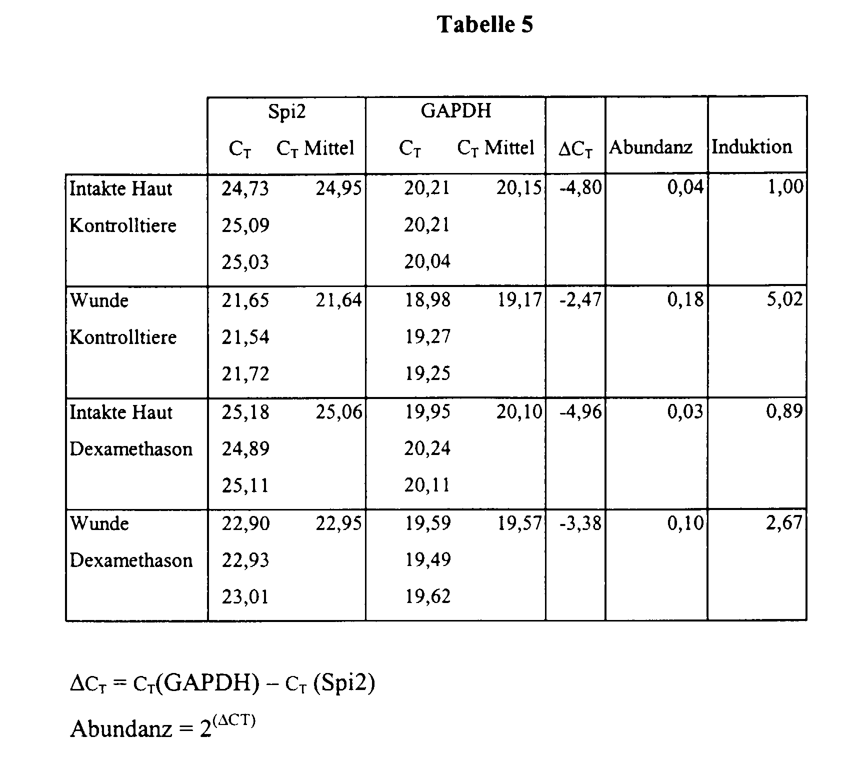

**Tabelle 6**

| Verändert exprimierte Gene | Intakte Haut Kontrolltiere | Wunde Kontrolltiere | Intakte Haut Dexamethason | Wunde Dexamethason |
|---|---|---|---|---|
| KIAA0494 | 1,00 | 0, 67 | 1,03 | 1,39 |
| mas Onkogen | 1,00 | 0,22 | 0,37 | 0,46 |
| basic-leucine zipper nuclear factor JEM-1 | 1,00 | 0,45 | 0,58 | 0,45 |
| checkpoint suppressor | 1,00 | 0,42 | 0,88 | 0,69 |
| Ribonuklease L inhibitor (Mu-RLI) | 1,00 | 0,37 | 0,72 | 2,90 |
| p68 RNA Helikase | 1,00 | 0,67 | 0,74 | 2,22 |
| mKAP13/pmg-1 | 1,00 | 0,36 | 0,27 | 0,64 |
| pmg-2 | 1,00 | 0,45 | 0,39 | 0,91 |
| MA-3 (apoptosis-related gene) | 1,00 | 0,11 | 0,58 | 0,16 |
| BTG1 | 1,00 | 0,53 | 0,57 | 2,50 |
| helix-loop-helix transcription factor | 1,00 | 0,34 | 0,78 | 0,48 |
| potentially prenylated protein tyrosine phosphatase | 1,00 | 0,56 | 0,36 | 1,91 |
| MBNL | 1,00 | 0,47 | 0,70 | 1,73 |
| Acylamino acid releasing enzyme | 1,00 | 1,12 | 0,72 | 1,41 |
| KIAA0585 | 1,00 | 0,35 | 0,39 | 1,12 |
| HSPC028 | 1,00 | 0,16 | 0,36 | 1,07 |
| Rab2 | 1,00 | 0,65 | 0,96 | 0,66 |
| Nup98-Nup96 Precursor | 1,00 | 1,66 | 1,76 | 1,80 |
| MRP-8 | 1,00 | 68,10 | 0,38 | 190,7 |
| MRP-14 | 1,00 | 205,60 | 0,77 | 875,96 |
| NF1-B | 1,00 | 0,61 | 1,43 | 0,79 |
| CBP | 1,00 | 0,72 | 1,45 | 0,92 |
| KIAA0261 | 1,00 | 0,38 | 0,57 | 1,70 |
| MCARP | 1,00 | 29,81 | 1,62 | 56,17 |
| Calnexin | 1,00 | 1,18 | 0,88 | 1,67 |
| Steroid Receptor Coactivator | 1,00 | 1,27 | 8,16 | 2,72 |
| Wolf-Hirschhorn-Syndrom Candidate 2 | 1,00 | 0,98 | 1,31 | 0,95 |
| Cathepsin Z | 1,00 | 1,26 | 0,83 | 0,58 |
| SR Calcium ATPase | 1,00 | 0,71 | 1,90 | 0,29 |
| CD9 | 1,00 | 0,84 | 1,19 | 1,41 |
| Acyl-CoA Desaturase 2 | 1,00 | 0,46 | 0,47 | 0,41 |
| Ryodocan Core Protein | 1,00 | 1,16 | 0,87 | 2,24 |
| KIAA0614 | 1,00 | 0,66 | 1,14 | 0,25 |
| KIAA0521 | 1,00 | 0,23 | 0,63 | 0,01 |

| Verändert exprimierte Gene | Intakte Haut junge Tiere | Wunde junge Tiere | Intakte Haut alte Tiere | Wunde alte Tiere |
|---|---|---|---|---|
| KIAA0494 | 01,23 | 0,36 | 0,41 | 0,32 |
| basic-leucine zipper nuclear factor JEM-1 | 2,00 | 0,29 | 0,61 | 0,29 |
| checkpoint suppressor | 1,58 | 0,42 | 0,85 | 0,27 |
| Ribonuklease L inhibitor (Mu-RLI) | 2,97 | 1,03 | 0,73 | 0,68 |
| p68 RNA Helikase | 0,98 | 0,22 | 0,61 | 0,30 |
| mKAP13/pmg-1 | 5,27 | 0,001 | 2,09 | 0,41 |
| pmg-2 | 5,11 | 0,0005 | 0,67 | 0,20 |
| MA-3 (apoptosis-related gene) | 1,57 | 0,20 | 0,61 | 0,18 |
| BTG1 | 1,86 | 0,75 | 0,82 | 0,64 |
| helix-loop-helix transcription factor | 1,61 | 0,35 | 0,67 | 0,33 |
| potentially prenylated protein tyrosine phosphatase | 0,95 | 0,26 | 0,49 | 0,32 |
| MBNL | 0,36 | 0,32 | 0,38 | 0,25 |
| Acylamino acid releasing enzyme | 1,79 | 0,92 | 1,60 | 0,59 |
| KIAA0585 | 1,52 | 0,34 | 1,05 | 0,55 |
| HSPC028 | 0,90 | 0,46 | 0,38 | 0,29 |
| Rab2 | 1,98 | 0,98 | 1,21 | 0,48 |
| Nup98-Nup96 Precursor | 2,63 | 1,33 | 0,83 | 1,37 |
| MRP-8 | 0,85 | 30,00 | 1,21 | 37,24 |
| MRP-14 | 0,86 | 100,42 | 2,30 | 128,69 |
| NF1-B | 1,19 | 0,81 | 1,24 | 0,36 |
| CBP | 1,45 | 0,79 | 0,78 | 0,46 |
| KIAA0261 | 0,65 | 0,18 | 0,30 | 0,15 |
| MCARP | 0,30 | 5,58 | 0,004 | 64,11 |
| Calnexin | 2,46 | 0,79 | 1,08 | 0,83 |
| Steroid Receptor Coactivator | 1,90 | 0,81 | 0,98 | 0,52 |
| Wolf-Hirschhorn-Syndrom Cantidate 2 | 1,50 | 1,12 | 1,17 | 0,58 |
| Cathepsin Z | 1,00 | 0,80 | 0,58 | 0,31 |
| SR Calcium ATPase | 0,29 | 0,65 | 1,30 | 1,36 |
| CD9 | 1,55 | 0,90 | 1,14 | 0,59 |
| Acyl-CoA Desaturase 2 | 0,49 | 0,32 | 0,77 | 0,48 |
| Ryodocan Core Protein | 0,91 | 0,70 | 1,04 | 0,66 |
| KIAA0614 | 0,97 | 0,13 | 0,71 | 0,26 |
| KIAA0521 | 0,48 | 0,44 | 0,56 | 0,33 |

| Verändert exprimierte Gene | Intakte Haut Diabetes-Kontrolltiere | Wunde Diabetes-Kontrolltiere | Intakte Haut Diabetesmäuse | Wunde Diabetesmäuse |
|---|---|---|---|---|
| KIAA0494 | 1,00 | 0,41 | 0,95 | 0,36 |
| Ribonuklease L inhibitor (Mu-RLI) | 1,00 | 0,60 | 1,22 | 1,06 |
| p68 RNA Helikase | 1,00 | 0,83 | 1,48 | 1,69 |
| mKAP13/pmg-1 | 1,00 | 0,13 | 0,61 | 0,09 |
| pmg-2 | 1,00 | 0,10 | 0,45 | 0,19 |
| MA-3 (apoptosis-related gene) | 1,00 | 0,16 | 0,99 | 0,24 |
| BTG1 | 1,00 | 1,72 | 1,79 | 3,39 |
| helix-loop-helix transcription factor | 1,00 | 0,58 | 1,63 | 0,81 |
| potentially prenylated protein tyrosine phosphatase | 1,00 | 0,35 | 1,19 | 0,87 |
| MBNL | 1,00 | 0,20 | 0,76 | 0,24 |
| Acylamino acid releasing enzyme | 1,00 | 0,56 | 1,47 | 0,67 |
| KIAA0585 | 1,00 | 0,75 | 0,61 | 0,99 |
| Rab2 | 1,00 | 0,43 | 2,16 | 0,13 |
| Nup98-Nup96 Precursor | 1,00 | 1,36 | 1,41 | 1,19 |
| MRP-8 | 1,00 | 47,21 | 5,26 | 40,94 |
| MRP-14 | 1,00 | 105,44 | 0,79 | 127,29 |
| NF1-B | 1,00 | 0,39 | 1,42 | 0,34 |
| CBP | 1,00 | 1,05 | 0,93 | 0,63 |
| KIAA0261 | 1,00 | 0,32 | 0,77 | 0,62 |
| MCARP | 1,00 | 171,54 | 13,18 | 515,81 |
| Acyl-CoA Desaturase 2 | 1,00 | 1,10 | 2,80 | 0,83 |
| Ryodocan Core Protein | 1,00 | 1,28 | 1,27 | 1,86 |
| KIAA0614 | 1,00 | 0,69 | 1, 31 | 0,59 |
| KIAA0521 | 1,00 | 0,42 | 1,50 | 0,61 |

**Tabelle 7**

| **Verändert exprimierte Gene** | **Patientenpool 1 Intakte Haut** | **Patientenpool 1 Tag 1-Wunde** | **Patientenpool 1 Tag 5-Wunde** |
|---|---|---|---|
| p68 Helikase | 1,00 | 0,32 | 0,59 |
| Ribonuklease L Inhibitor | 1,00 | 0,17 | 0,54 |
| MRP-8 | 1,00 | 6,40 | 38,13 |
| potentially prenylated protein tyrosine phosphatase | 1,00 | 0,29 | 0,86 |
| MA-3 Variante (SEQ ID Nr. 58) | 1,00 | 0, 17 | 0,46 |
| MA-3 (apoptosis-related gene) | 1,00 | 0,20 | 0,44 |
| human Checkpoint Suppressor | 1,00 | 0,34 | 0,41 |
| HSPC028 | 1,00 | 0,35 | 0,36 |
| helix-loop-helix transcription factor | 1,00 | 0,45 | 0,45 |
| KIAA0261 | 1,00 | 0,21 | 0,52 |
| basic-leucine zipper nuclear factor (JEM-1) | 1,00 | 0,13 | 0,36 |
| KIAA0494 | 1,00 | 0, 29 | 0,42 |
| MNBL | 1,00 | 0,43 | 0,46 |
| Acylamino acid releasing enzyme | 1,00 | 0,98 | 0,76 |
| KIAA0585 | 1,00 | 0,53 | 0,54 |

**Tabelle 8**

| **Verändert exprimierte Gene** | Intakte Haut Ulkus-Patienten | Wundrand Ulkus-Patienten | Wundgrund Ulkus-Patienten |
|---|---|---|---|
| p68 Helikase | 1,00 | 1,09 | 1,44 |
| RNAse L Inhibitor | 1,00 | 0,20 | 0,26 |
| MRP8 | 1,00 | 70,7 | 8,9 |
| potentiell prenylierte Tyrosin Phosphatase | 1,00 | 0,31 | 0,55 |
| MA-3 Variante (SEQ ID Nr. 58) | 1,00 | 0,16 | 0,18 |
| MA-3 (apoptosis-related gene) | 1,00 | 0,20 | 0,24 |
| Checkpoint Suppressor | 1,00 | 0,39 | 0,45 |
| Helix-Loop-Helix transcription factor | 1,00 | 0,46 | 0,65 |
| KIAA0261 | 1,00 | 0,61 | 0,96 |
| basic-leucine zipper nuclear factor | 1,00 | 0,59 | 0,56 |
| KIAA0494 | 1,00 | 0,17 | 0,25 |
| MNBL | 1,00 | 0,24 | 0,35 |
| Acylamino acid releasing enzyme | 1,00 | 1,35 | 1,36 |
| KIAA0585 | 1,00 | 3,23 | 2,72 |

## Patentansprüche

1. Verwendung mindestens eines Polypeptids oder Varianten davon gemäß einer der SEQ ID Nr. 1 bis SEQ ID Nr. 4, SEQ ID Nr. 7 bis SEQ ID Nr. 9,SEQ ID Nr. 103 bis SEQ ID Nr. 104 oder SEQ ID Nr. 106 oder dieses kodierende Nukleinsäuren oder Varianten davon zur Diagnose und/oder Prävention und/oder Behandlung von Erkrankungen oder zur Identifizierung von pharmakologisch aktiven Substanzen.

2. Verwendung mindestens eines Polypeptids oder Varianten davon gemäß einer der SEQ ID Nr. 5 bis SEQ ID Nr. 6, SEQ ID Nr. 10 bis SEQ ID Nr. 48, SEQ ID Nr. 55 bis SEQ ID Nr. 58, SEQ ID Nr. 89 bis SEQ ID Nr. 94, SEQ ID Nr.105 oder SEQ ID Nr. 109 bis SEQ ID Nr. 114 oder dieses kodierende Nukleinsäuren oder Varianten davon zur Diagnose und/oder Prävention und/oder Behandlung von Hauterkrankungen und/oder Behandlung bei der Wundheilung oder zur Identifizierung von pharmakologisch aktiven Substanzen.

3. Verwendung mindestens eines Polypeptids oder Varianten davon gemäß einer der SEQ ID Nr. 51 bis SEQ ID Nr. 54 oder SEQ ID Nr. 101 bis SEQ ID Nr. 102 oder dieses kodierende Nukleinsäure oder Varianten davon zur Diagnose und/oder Prävention und/oder Behandlung bei der Wundheilung oder zur Identifizierung von pharmakologisch aktiven Substanzen.

4. Verwendung mindestens einer Nukleinsäure oder Varianten davon gemäß einer der SEQ ID Nr. 49 oder SEQ ID Nr. 50 zur Diagnose und/oder Prävention und/oder Behandlung von Hauterkrankungen und/oder Behandlung bei der Wundheilung oder zur Identifizierung von pharmakologisch aktiven Substanzen.

5. Verwendung einer Nukleinsäure nach einem der Ansprüche 1 bis 4, dadurch gekennzeichnet, daß die Nukleinsäure eine DNA oder RNA, vorzugsweise eine DNA, insbesondere eine doppelsträngige DNA ist.

6. Verwendung einer Nukleinsäure nach einem der Ansprüche 1 bis 5, dadurch gekennzeichnet, daß die Sequenz der Nukleinsäure mindestens ein Intron und/oder eine polyA-Sequenz aufweist.

7. Verwendung einer Nukleinsäure nach einem der Ansprüche 1 bis 6 in Form ihrer antisense-Sequenz.

8. Verwendung einer Nukleinsäure nach einem der Ansprüche 1 bis 7, dadurch gekennzeichnet, daß die Nukleinsäure synthetisch hergestellt worden ist.

9. Verwendung eines Polypeptids nach einem der Ansprüche 1 bis 3, dadurch gekennzeichnet, daß das Polypeptid synthetisch hergestellt worden ist.

10. Verwendung eines Polypeptids nach einem der Ansprüche 1 bis 3, dadurch gekennzeichnet, daß das Polypeptid ein Fusionsprotein ist.

11. Verwendung einer Nukleinsäure nach einem der Ansprüche 1 bis 8 zur Herstellung eines Vektors, vorzugsweise in Form eines Plasmids, shuttle Vektors, Phagemids, Cosmids, Expressionsvektors oder gentherapeutisch wirksamen Vektors.

12. Verwendung einer Nukleinsäure nach einem der Ansprüche 1 bis 8 zur Herstellung eines knock-out Genkonstrukts oder einer Expressionskassette.

13. Wirtszelle, transformiert mit einem Vektor oder einem knock-out Genkonstrukts nach einem der Ansprüche 11 oder 12.

14. Wirtszelle nach Anspruch 13, dadurch gekennzeichnet, daß es sich um eine Hautzelle handelt.

15. Transgene embryonale nichtmenschliche Stammzelle, dadurch gekennzeichnet, daß sie ein knock-out Genkonstrukt oder eine Expressionskassette nach Anspruch 12 enthält.

16. Verfahren zur Herstellung eines transgenen nichtmenschlichen Säugetiers, dadurch gekennzeichnet, daß eine embryonale nichtmenschliche Stammzelle nach Anspruch 15 zu einem transgenen nichtmenschlichen Säugetier regeneriert wird.

17. Transgenes nichtmenschliches Säugetier, dadurch gekennzeichnet, daß sein Genom ein knock-out Genkonstrukt oder eine Expressionskassette nach Anspruch 12 enthält.

18. Verfahren zur Herstellung eines Polypeptids zur Diagnose und/oder Prävention und/oder Behandlung von Erkrankungen, insbesondere Hauterkrankungen oder Behandlung bei der Wundheilung oder zur Identifizierung von pharmakologisch aktiven Substanzen in einer geeigneten Wirtszelle, dadurch gekennzeichnet, daß eine Nukleinsäure nach einem der Ansprüche 1 bis 8 exprimiert wird.

19. Verfahren zur Herstellung eines Fusionsproteins zur Diagnose und/oder Prävention und/oder Behandlung von Erkrankungen, insbesondere Hauterkrankungen oder Behandlung bei der Wundheilung oder zur Identifizierung von pharmakologisch aktiven Substanzen in einer geeigneten Wirtszelle, dadurch gekennzeichnet, daß eine Nukleinsäure nach einem der Ansprüche 1 bis 8 exprimiert wird.

20. Verfahren zur Herstellung eines Antikörpers, vorzugsweise eines polyklonalen oder monoklonalen Antikörpers zur Diagnose und/oder Prävention und/oder Behandlung von Erkrankungen, insbesondere Hauterkrankungen oder Behandlung bei der Wundheilung oder zur Identifizierung von pharmakologisch aktiven Substanzen, dadurch gekennzeichnet, daß ein Antikörper produzierender Organismus mit einem Polypeptid oder funktionelle Äquivalente davon oder Teile davon mit mindestens 6 Aminosäuren, vorzugsweise mit mindestens 8 Aminosäuren, insbesondere mit mindestens 12 Aminosäuren nach einem der Ansprüche 1 bis 3 und 9 oder 10 immunisiert wird.

21. Antikörper zur Diagnose und/oder Prävention und/oder Behandlung von Erkrankungen, insbesondere Hauterkrankungen oder Behandlung bei der Wundheilung oder zur Identifizierung von pharmakologisch aktiven Substanzen, dadurch gekennzeichnet, daß er gegen ein Polypeptid nach einem der Ansprüche 1 bis 3 und 9 oder 10 gerichtet ist.

22. Verwendung eines Antikörpers nach Anspruch 21 zur Diagnose und/oder Prävention und/oder Behandlung von Erkrankungen, insbesondere Hauterkrankungen oder Behandlung bei der Wundheilung oder zur Identifizierung von pharmakologisch aktiven Substanzen.

23. Verfahren zur Herstellung eines Diagnostikums zur Diagnose von Erkrankungen, insbesondere Hauterkrankungen und/oder Erkrankungen bei der Wundheilung, dadurch gekennzeichnet, daß mindestens eine Nukleinsäure, mindestens ein Polypeptid oder mindestens ein Antikörpers nach einem der vorgenannten Ansprüche zusammen mit geeigneten Zusatz- und Hilfsstoffen kombiniert wird.

24. Diagnostikum zur Diagnose von Erkrankungen, insbesondere Hauterkrankungen und/oder Erkrankungen bei der Wundheilung, dadurch gekennzeichnet, daß es mindestens eine Nukleinsäure, mindestens ein Polypeptid oder mindestens einen Antikörper nach einem der vorgenannten Ansprüche, gegebenenfalls zusammen mit geeigneten Zusatz- und Hilfsstoffen, enthält.

25. Diagnostikum nach Anspruch 24, dadurch gekennzeichnet, daß es eine Sonde, vorzugsweise eine DNA-Sonde, enthält.

26. Verfahren zur Herstellung eines Arzneimittels zur Behandlung von Erkrankungen, insbesondere Hauterkrankungen und/oder Erkrankungen bei der Wundheilung, dadurch gekennzeichnet, daß mindestens eine Nukleinsäure, mindestens ein Polypeptid oder mindestens ein Antikörpers nach einem der vorgenannten Ansprüche zusammen mit geeigneten Zusatz- und Hilfsstoffen kombiniert wird.

27. Arzneimittel zur Behandlung von Erkrankungen, insbesondere Hauterkrankungen und/oder Erkrankungen bei der Wundheilung, dadurch gekennzeichnet, daß es mindestens eine Nukleinsäure, mindestens ein Polypeptid oder mindestens einen Antikörper nach einem der vorgenannten Ansprüche, gegebenenfalls zusammen mit geeigneten Zusatz- und Hilfsstoffen, enthält.

28. Verwendung eines Arzneimittels nach Anspruch 27 zur Behandlung von Erkrankungen, insbesondere Hauterkrankungen und/oder Erkrankungen bei der Wundheilung.

29. Verfahren zur Herstellung eines Tests zur Auffindung funktioneller Interaktoren in Zusammenhang mit Erkrankungen, insbesondere Hauterkrankungen und/oder Erkrankungen bei der Wundheilung, dadurch gekennzeichnet, daß mindestens eine Nukleinsäure, mindestens ein Polypeptids oder mindestens ein Antikörpers nach einem der vorgenannten Ansprüche zusammen mit geeigneten Zusatz- und Hilfsstoffen kombiniert wird.

30. Test zur Identifizierung funktioneller Interaktoren in Zusammenhang mit Erkrankungen, insbesondere Hauterkrankungen und/oder Erkrankungen bei der Wundheilung, dadurch gekennzeichnet, daß er mindestens eine Nukleinsäure, mindestens ein Polypeptid oder mindestens einen Antikörper nach einem der vorgenannten Ansprüche, gegebenenfalls zusammen mit geeigneten Zusatz- und Hilfsstoffen, enthält.

31. Verfahren zur Herstellung eines auf einem Trägermaterial fixierten Arrays zur Analyse in Zusammenhang mit Erkrankungen, insbesondere Hauterkrankungen oder Erkrankungen bei der Wundheilung, dadurch gekennzeichnet, daß mindestens eine Nukleinsäure, mindestens ein Polypeptid oder mindestens ein Antikörper, nach einem der vorgenannten Ansprüche auf einem Trägermaterial fixiert wird.

32. Auf einem Trägermaterial fixiertes Array zur Analyse in Zusammenhang mit Erkrankungen, insbesondere Hauterkrankungen oder Erkrankungen bei der Wundheilung, dadurch gekennzeichnet, daß es mindestens eine Nukleinsäure und/oder mindestens ein Polypeptid und/oder oder mindestens einen Antikörper nach einem der vorgenannten Ansprüche enthält.

33. Verfahren zur Herstellung eines DNA-Chips und/oder Protein-Chips zur Analyse in Zusammenhang mit Erkrankungen insbesondere Hauterkrankungen oder Erkrankungen bei der Wundheilung, dadurch gekennzeichnet, daß mindestens eine Nukleinsäure, mindestens ein Polypeptid oder mindestens ein Antikörper, nach einem der vorgenannten Ansprüche auf einem Trägermaterial fixiert wird.

34. DNA-Chip und/oder Protein-Chip zur Analyse in Zusammenhang mit Erkrankungen, insbesondere Hauterkrankungen oder Erkrankungen bei der Wundheilung, dadurch gekennzeichnet, daß er mindestens eine Nukleinsäure und/oder mindestens ein Polypeptid und/oder oder mindestens einen Antikörper nach einem der vorgenannten Ansprüche enthält.


SEQUENZPROTOKOLL

<110> Switch Biotech AG

<120> Verwendung von Polypeptiden und diese kodierende
      Nukleinsäuren zur Diagnose oder Behandlung von
      Hauterkrankungen sowie ihre Verwendung zur
      Identifizierung von pharmakologisch aktiven Substanzen

<130> S29829EP

<140>
<141>

<150> DE 199 55 349.1
<151> 1999-11-17

<150> US 60/172,511
<151> 1999-12-17

<150> DE 100 30 149.5
<151> 2000-06-20

<160> 128

<170> PatentIn Ver. 2.1

<210> 1
<211> 388
<212> PRT
<213> Homo sapiens

<400> 1
Met Ala Val Ser Val Thr Pro Ile Arg Asp Thr Lys Trp Leu Thr Leu
  1               5                  10                  15

Glu Val Cys Arg Glu Phe Gln Arg Gly Thr Cys Ser Arg Pro Asp Thr
             20                  25                  30

Glu Cys Lys Phe Ala His Pro Ser Lys Ser Cys Gln Val Glu Asn Gly
         35                  40                  45

Arg Val Ile Ala Cys Phe Asp Ser Leu Lys Gly Arg Cys Ser Arg Glu
     50                  55                  60

Asn Cys Lys Tyr Leu His Pro Pro Pro His Leu Lys Thr Gln Leu Glu
 65                  70                  75                  80

Ile Asn Gly Arg Asn Asn Leu Ile Gln Gln Lys Asn Met Ala Met Leu
                85                  90                  95

Ala Gln Gln Met Gln Leu Ala Asn Ala Met Met Pro Gly Ala Pro Leu
            100                 105                 110

Gln Pro Val Pro Met Phe Ser Val Ala Pro Ser Leu Ala Thr Asn Ala
        115                 120                 125

Ser Ala Ala Ala Phe Asn Pro Tyr Leu Gly Pro Val Ser Pro Ser Leu
    130                 135                 140

Val Pro Ala Glu Ile Leu Pro Thr Ala Pro Met Leu Val Thr Gly Asn
145                 150                 155                 160

Pro Gly Val Pro Val Pro Ala Ala Ala Ala Ala Ala Ala Gln Lys Leu
                165                 170                 175

Met Arg Thr Asp Arg Leu Glu Val Cys Arg Glu Tyr Gln Arg Gly Asn
            180                 185                 190

Cys Asn Arg Gly Glu Asn Asp Cys Arg Phe Ala His Pro Ala Asp Ser
        195                 200                 205

Thr Met Ile Asp Thr Asn Asp Asn Thr Val Thr Val Cys Met Asp Tyr
    210                 215                 220

Ile Lys Gly Arg Cys Ser Arg Glu Lys Cys Lys Tyr Phe His Pro Pro
225                 230                 235                 240

Ala His Leu Gln Ala Lys Ile Lys Ala Ala Gln Tyr Gln Val Asn Gln
                245                 250                 255

Ala Ala Ala Ala Gln Ala Ala Ala Thr Ala Ala Ala Met Thr Gln Ser
            260                 265                 270

Ala Val Lys Ser Leu Lys Arg Pro Leu Glu Ala Thr Phe Asp Leu Gly
        275                 280                 285

Ile Pro Gln Ala Val Leu Pro Pro Leu Pro Lys Arg Pro Ala Leu Glu
    290                 295                 300

Lys Thr Asn Gly Ala Thr Ala Val Phe Asn Thr Gly Ile Phe Gln Tyr
305                 310                 315                 320

Gln Gln Ala Leu Ala Asn Met Gln Leu Gln Gln His Thr Ala Phe Leu
                325                 330                 335

Pro Pro Val Pro Met Val His Gly Ala Thr Pro Ala Thr Val Ser Ala
            340                 345                 350

Ala Thr Thr Ser Ala Thr Ser Val Pro Phe Ala Ala Thr Ala Thr Ala
        355                 360                 365

Asn Gln Ile Pro Ile Ile Ser Ala Glu His Leu Thr Ser His Lys Tyr
    370                 375                 380

Val Thr Gln Met
385

<210> 2
<211> 521
<212> PRT
<213> Mus musculus

<400> 2
Asp Thr Gly Leu Trp Leu His Asn Lys Leu Gly Ala Thr Asp Glu Leu
  1               5                  10                  15

Trp Ala Pro Pro Ser Ile Ala Ser Leu Leu Thr Ala Ala Val Ile Asp
            20                  25                  30

Asn Ile Arg Leu Cys Phe His Arg Leu Ser Ser Ala Val Lys Leu Lys
        35                  40                  45

Leu Leu Leu Gly Thr Leu His Leu Pro Arg Arg Thr Val Asp Glu Met
     50                  55                  60

Asn Ala Ala Leu Met Asp Ile Ile Gln Leu Ala Thr Leu Asp Ser Asp
 65                  70                  75                  80

Pro Trp Val Leu Met Val Ala Asp Ile Leu Lys Ser Phe Pro Asp Thr
                 85                  90                  95

Gly Ser Leu Asn Leu Asp Leu Glu Glu Gln Asn Pro Asn Val Gln Glu
            100                 105                 110

Ile Leu Gly Glu Leu Arg Glu Lys Val Ser Glu Cys Glu Ala Ser Ala
        115                 120                 125

Met Leu Pro Leu Glu Cys Gln Tyr Leu Asn Lys Asn Ala Leu Thr Thr
    130                 135                 140

Leu Ala Gly Pro Leu Thr Pro Pro Val Lys His Phe Gln Leu Lys Arg
145                 150                 155                 160

Lys Pro Lys Ser Ala Thr Leu Arg Ala Glu Leu Leu Gln Ile Ser Thr
                165                 170                 175

Glu Thr Ala Gln Gln Leu Lys Arg Ser Ala Gly Val Pro Phe His Ala
            180                 185                 190

Lys Gly Arg Gly Leu Leu Arg Lys Met Asp Thr Thr Thr Pro Leu Lys
        195                 200                 205

Gly Ile Pro Lys Gln Ala Pro Phe Arg Ser Pro Thr Thr Pro Ser Val
    210                 215                 220

Phe Ser Pro Ser Gly Asn Arg Thr Pro Ile Pro Pro Ser Arg Thr Pro
225                 230                 235                 240

Leu Gln Lys Glu Arg Gly Val Lys Leu Leu Asp Ile Ser Glu Leu Asn
                245                 250                 255

Thr Val Gly Ala Gly Arg Glu Ala Lys Arg Arg Arg Lys Thr Leu Asp
            260                 265                 270

Thr Glu Val Val Glu Lys Pro Thr Lys Glu Glu Thr Val Val Glu Asn
        275                 280                 285

Ala Thr Pro Asp Tyr Ala Ala Gly Leu Val Ser Thr Gln Lys Leu Gly
    290                 295                 300

Ser Leu Asn Ser Glu Pro Thr Leu Pro Ser Thr Ser Tyr Leu Pro Ser
305                 310                 315                 320

Thr Pro Ser Val Val Pro Ala Ser Ser Tyr Ile Pro Ser Ser Glu Thr
                325                 330                 335

Pro Pro Ala Thr Pro Ser Arg Glu Ala Ser Arg Pro Pro Glu Glu Pro
            340                 345                 350

Ser Ala Pro Ser Pro Thr Leu Pro Thr Gln Phe Lys Gln Arg Ala Pro
        355                 360                 365

Met Tyr Asn Ser Gly Val Ser Pro Ala Thr Pro Ala Ala Pro Thr Ser
    370                 375                 380

Pro Arg Thr His Thr Thr Pro Pro Ala Val Thr Pro Thr Ala Gln Thr
385                 390                 395                 400

Pro Pro Val Ala Met Val Ala Pro Gln Thr Gln Ala Pro Ala Pro Val
                405                 410                 415

Gln Gln Gln Pro Lys Lys Asn Leu Ser Phe Thr Arg Glu Gln Met Phe
            420                 425                 430

Ala Ala Gln Glu Met Phe Lys Thr Ala Asn Lys Val Thr Arg Pro Glu
        435                 440                 445

Lys Ala Leu Ile Leu Gly Phe Met Ala Gly Ser Arg Glu Asn Pro Cys
    450                 455                 460

Pro Glu Gln Gly Asp Val Ile Gln Ile Lys Leu Ser Glu His Thr Glu
465                 470                 475                 480

Asp Leu Pro Lys Ala Asp Gly Gln Gly Ser Thr Thr Met Leu Val Asp
                485                 490                 495

Thr Val Phe Glu Met Asn Tyr Ala Thr Gly Gln Trp Thr Arg Phe Lys
            500                 505                 510

Lys Tyr Lys Pro Met Thr Asn Val Ser
        515                 520

<210> 3
<211> 525
<212> PRT
<213> Homo sapiens

<300>
<301> Wright, T. J.
<302> Comparative analysis of a novel gene from the
      Wolf-Hirschhorn/Pitt-Rogers-Danks syndrome critical
      region
<303> Genomics
<304> 1999
<305> 59
<306> 203-212
<308> PIR G3860187
<309> 1998-10-26
<313> 1 - 525

<400> 3
Met Arg Glu Ser Asp Thr Gly Leu Trp Leu His Asn Lys Leu Gly Ala
  1               5                  10                  15

Thr Asp Glu Leu Trp Ala Pro Pro Ser Ile Ala Ser Leu Leu Thr Ala
             20                  25                  30

Ala Val Ile Asp Asn Ile Arg Leu Cys Phe His Gly Leu Ser Ser Ala
        35                  40                  45

Val Lys Leu Lys Leu Leu Leu Gly Thr Leu His Leu Pro Arg Arg Thr
    50                  55                  60

Val Asp Glu Met Lys Gly Ala Leu Met Glu Ile Ile Gln Leu Ala Ser
65                  70                  75                  80

Leu Asp Ser Asp Pro Trp Val Leu Met Val Ala Asp Ile Leu Lys Ser
                85                  90                  95

Phe Pro Asp Thr Gly Ser Leu Asn Leu Glu Leu Glu Glu Gln Asn Pro
            100                 105                 110

Asn Val Gln Asp Ile Leu Gly Glu Leu Arg Glu Lys Val Gly Glu Cys
        115                 120                 125

Glu Ala Ser Ala Met Leu Pro Leu Glu Cys Gln Tyr Leu Asn Lys Asn
    130                 135                 140

Ala Leu Thr Thr Leu Ala Gly Pro Leu Thr Pro Pro Val Lys His Phe
145                 150                 155                 160

Gln Leu Lys Arg Lys Pro Lys Ser Ala Thr Leu Arg Ala Glu Leu Leu
                165                 170                 175

Gln Lys Ser Thr Glu Thr Ala Gln Gln Leu Lys Arg Ser Ala Gly Val
            180                 185                 190

Pro Phe His Ala Lys Gly Arg Gly Leu Leu Arg Lys Met Asp Thr Thr
        195                 200                 205

Thr Pro Leu Lys Gly Ile Pro Lys Gln Ala Pro Phe Arg Ser Pro Thr
    210                 215                 220

Ala Pro Ser Val Phe Ser Pro Thr Gly Asn Arg Thr Pro Ile Pro Pro
225                 230                 235                 240

Ser Arg Thr Leu Leu Arg Lys Glu Arg Gly Val Lys Leu Leu Asp Ile
                245                 250                 255

Ser Glu Leu Asp Met Val Gly Ala Gly Arg Glu Ala Lys Arg Arg Arg
            260                 265                 270

Lys Thr Leu Asp Ala Glu Val Val Glu Lys Pro Ala Lys Glu Glu Thr
        275                 280                 285

Val Val Glu Asn Ala Thr Pro Asp Tyr Ala Ala Gly Leu Val Ser Thr
    290                 295                 300

Gln Lys Leu Gly Ser Leu Asn Asn Glu Pro Ala Leu Pro Ser Thr Ser
305                 310                 315                 320

Tyr Leu Pro Ser Thr Pro Ser Val Val Pro Ala Ser Ser Tyr Ile Pro
                325                 330                 335

Ser Ser Glu Thr Pro Pro Ala Pro Ser Ser Arg Glu Ala Ser Arg Pro
            340                 345                 350

Pro Glu Glu Pro Ser Ala Pro Ser Pro Thr Leu Pro Ala Gln Phe Lys
        355                 360                 365

Gln Arg Ala Pro Met Tyr Asn Ser Gly Leu Ser Pro Ala Thr Pro Thr
    370                 375                 380

Pro Ala Ala Pro Thr Ser Pro Leu Thr Pro Thr Thr Pro Pro Ala Val
385                 390                 395                 400

Ala Pro Thr Thr Gln Thr Pro Pro Val Ala Met Val Ala Pro Gln Thr
                405                 410                 415

Gln Ala Pro Ala Gln Gln Gln Pro Lys Lys Asn Leu Ser Leu Thr Arg
            420                 425                 430

Glu Gln Met Phe Ala Ala Gln Glu Met Phe Lys Thr Ala Asn Lys Val
        435                 440                 445

Thr Arg Pro Glu Lys Ala Leu Ile Leu Gly Phe Met Ala Gly Ser Arg
    450                 455                 460

Glu Asn Pro Cys Gln Glu Gln Gly Asp Val Ile Gln Ile Lys Leu Ser
465                 470                 475                 480

Glu His Thr Glu Asp Leu Pro Lys Ala Asp Gly Gln Gly Ser Thr Thr
                485                 490                 495

Met Leu Val Asp Thr Val Phe Glu Met Asn Tyr Ala Thr Gly Gln Trp
            500                 505                 510

Thr Arg Phe Lys Lys Tyr Lys Pro Met Thr Asn Val Ser
        515                 520                 525

<210> 4
<211> 495

<212> PRT
<213> Homo sapiens

<400> 4
Met Lys Lys Arg Lys Glu Leu Asn Ala Leu Ile Gly Leu Ala Gly Asp
1               5                   10                  15

Ser Arg Arg Lys Lys Pro Lys Lys Gly Pro Ser Ser His Arg Leu Leu
            20                  25                  30

Arg Thr Glu Pro Pro Asp Ser Asp Ser Glu Ser Ser Ser Glu Glu Glu
        35                  40                  45

Glu Glu Phe Gly Val Val Gly Asn Arg Ser Arg Phe Ala Lys Gly Asp
    50                  55                  60

Tyr Leu Arg Cys Cys Lys Ile Cys Tyr Pro Leu Cys Gly Phe Val Ile
65                  70                  75                  80

Leu Ala Ala Cys Val Val Ala Cys Val Gly Leu Val Trp Met Gln Val
                85                  90                  95

Ala Leu Lys Glu Asp Leu Asp Ala Leu Lys Glu Lys Phe Arg Thr Met
            100                 105                 110

Glu Ser Asn Gln Lys Ser Ser Phe Gln Glu Ile Pro Lys Leu Asn Glu
        115                 120                 125

Glu Leu Leu Ser Lys Gln Lys Gln Leu Glu Lys Ile Glu Ser Gly Glu
    130                 135                 140

Met Gly Leu Asn Lys Val Trp Ile Asn Ile Thr Glu Met Asn Lys Gln
145                 150                 155                 160

Ile Ser Leu Leu Thr Ser Ala Val Asn His Leu Lys Ala Asn Val Lys
                165                 170                 175

Ser Ala Ala Asp Leu Ile Ser Leu Pro Thr Thr Val Glu Gly Leu Gln
            180                 185                 190

Lys Ser Val Ala Ser Ile Gly Asn Thr Leu Asn Ser Val His Leu Ala
        195                 200                 205

Val Glu Ala Leu Gln Lys Thr Val Asp Glu His Lys Lys Thr Met Glu
    210                 215                 220

Leu Leu Gln Ser Asp Met Asn Gln His Phe Leu Lys Glu Thr Pro Gly
225                 230                 235                 240

Ser Asn Gln Ile Ile Pro Ser Pro Ser Ala Thr Ser Glu Leu Asp Asn
                245                 250                 255

Lys Thr His Ser Glu Asn Leu Lys Gln Asp Ile Leu Tyr Leu His Asn
            260                 265                 270

Ser Leu Glu Glu Val Asn Ser Ala Leu Val Gly Tyr Gln Arg Gln Asn
        275                 280                 285

Asp Leu Lys Leu Glu Gly Met Asn Glu Thr Val Ser Asn Leu Thr Gln
    290                 295                 300

Arg Val Asn Leu Ile Glu Ser Asp Val Val Ala Met Ser Lys Val Glu
305                 310                 315                 320

Lys Lys Ala Asn Leu Ser Phe Ser Met Met Gly Asp Arg Ser Ala Thr
                325                 330                 335

Leu Lys Arg Gln Ser Leu Asp Gln Val Thr Asn Arg Thr Asp Thr Val
            340                 345                 350

Lys Ile Gln Ser Ile Lys Lys Glu Asp Ser Ser Asn Ser Gln Val Ser
        355                 360                 365

Lys Leu Arg Glu Lys Leu Gln Leu Ile Ser Ala Leu Thr Asn Lys Pro
    370                 375                 380

Glu Ser Asn Arg Pro Pro Glu Thr Ala Asp Glu Glu Gln Val Glu Ser
385                 390                 395                 400

Phe Thr Ser Lys Pro Ser Ala Leu Pro Lys Phe Ser Gln Phe Leu Gly
                405                 410                 415

Asp Pro Val Glu Lys Ala Ala Gln Leu Arg Pro Ile Ser Leu Pro Gly
            420                 425                 430

Val Ser Ser Thr Glu Asp Leu Gln Asp Leu Phe Arg Lys Thr Gly Gln
        435                 440                 445

Asp Val Asp Gly Lys Leu Thr Tyr Gln Glu Ile Trp Thr Ser Leu Gly
    450                 455                 460

Ser Ala Met Pro Glu Pro Glu Ser Leu Arg Ala Phe Asp Ser Asp Gly
465                 470                 475                 480

Asp Gly Arg Tyr Ser Phe Leu Glu Leu Arg Val Ala Leu Gly Ile
                485                 490                 495

<210> 5
<211> 416
<212> PRT
<213> Homo sapiens

<400> 5
Arg Arg Met Asn His Lys Ser Lys Lys Arg Ile Arg Glu Ala Lys Arg
1               5                   10                  15

Ser Ala Arg Pro Glu Leu Lys Asp Ser Leu Asp Trp Thr Arg His Asn
            20                  25                  30

Tyr Tyr Glu Ser Phe Ser Leu Ser Pro Ala Ala Val Ala Asp Asn Val
        35                  40                  45

Glu Arg Ala Asp Ala Leu Gln Leu Ser Val Glu Glu Phe Val Glu Arg
    50                  55                  60

Tyr Glu Arg Pro Tyr Lys Pro Val Val Leu Leu Asn Ala Gln Glu Gly
65                  70                  75                  80

Trp Ser Ala Gln Glu Lys Trp Thr Leu Glu Arg Leu Lys Arg Lys Tyr
                85                  90                  95

Arg Asn Gln Lys Phe Lys Cys Gly Glu Asp Asn Asp Gly Tyr Ser Val
            100                 105                 110

Lys Met Lys Met Lys Tyr Tyr Ile Glu Tyr Met Glu Ser Thr Arg Asp
        115                 120                 125

Asp Ser Pro Leu Tyr Ile Phe Asp Ser Ser Tyr Gly Glu His Pro Lys
    130                 135                 140

Arg Arg Lys Leu Leu Glu Asp Tyr Lys Val Pro Lys Phe Phe Thr Asp
145                 150                 155                 160

Asp Leu Phe Gln Tyr Ala Gly Glu Lys Arg Arg Pro Pro Tyr Arg Trp
                165                 170                 175

Phe Val Met Gly Pro Pro Arg Ser Gly Thr Gly Ile His Ile Asp Pro
            180                 185                 190

Leu Gly Thr Ser Ala Trp Asn Ala Leu Val Gln Gly His Lys Arg Trp
        195                 200                 205

Cys Leu Phe Pro Thr Ser Thr Pro Arg Glu Leu Ile Lys Val Thr Arg

210                 215                 220

Asp Glu Gly Gly Asn Gln Gln Asp Glu Ala Ile Thr Trp Phe Asn Val
225                 230                 235                 240

Ile Tyr Pro Arg Thr Gln Leu Pro Thr Trp Pro Pro Glu Phe Lys Pro
                245                 250                 255

Leu Glu Ile Leu Gln Lys Pro Gly Glu Thr Val Phe Val Pro Gly Gly
            260                 265                 270

Trp Trp His Val Val Leu Asn Leu Asp Thr Thr Ile Ala Ile Thr Gln
        275                 280                 285

Asn Phe Ala Ser Ser Thr Asn Phe Pro Val Val Trp His Lys Thr Val
    290                 295                 300

Arg Gly Arg Pro Lys Leu Ser Arg Lys Trp Tyr Arg Ile Leu Lys Gln
305                 310                 315                 320

Glu His Pro Glu Leu Ala Val Leu Ala Asp Ser Val Asp Leu Gln Glu
                325                 330                 335

Ser Thr Gly Ile Ala Ser Asp Ser Ser Ser Asp Ser Ser Ser Ser Ser
            340                 345                 350

Ser Ser Ser Ser Ser Asp Ser Asp Ser Glu Cys Glu Ser Gly Ser Glu
        355                 360                 365

Gly Asp Gly Thr Val His Arg Arg Lys Lys Arg Arg Thr Cys Ser Met
    370                 375                 380

Val Gly Asn Gly Asp Thr Thr Ser Gln Asp Asp Cys Val Ser Lys Glu
385                 390                 395                 400

Arg Ser Ser Ser Arg Ile Arg Asp Thr Cys Gly Gly Arg Ala His Pro
                405                 410                 415

<210> 6
<211> 419
<212> PRT
<213> Homo sapiens

<300>

<301> Zhang, Q.
<302> Human HSPC028 gene, complete cds

<400> 6
Met Asn Lys His Gln Lys Pro Val Leu Thr Gly Gln Arg Phe Lys Thr
1               5                   10                  15

Arg Lys Arg Asp Glu Lys Glu Lys Phe Glu Pro Thr Val Phe Arg Asp
            20                  25                  30

Thr Leu Val Gln Gly Leu Asn Glu Ala Gly Asp Asp Leu Glu Ala Val
        35                  40                  45

Ala Lys Phe Leu Asp Ser Thr Gly Ser Arg Leu Asp Tyr Arg Arg Tyr
    50                  55                  60

Ala Asp Thr Leu Phe Asp Ile Leu Val Ala Gly Ser Met Leu Ala Pro
65                  70                  75                  80

Gly Gly Thr Arg Ile Asp Asp Gly Asp Lys Thr Lys Met Thr Asn His
                85                  90                  95

Cys Val Phe Ser Ala Asn Glu Asp His Glu Thr Ile Arg Asn Tyr Ala
            100                 105                 110

Gln Val Phe Asn Lys Leu Ile Arg Arg Tyr Lys Tyr Leu Glu Lys Ala
        115                 120                 125

Phe Glu Asp Glu Met Lys Lys Leu Leu Leu Phe Leu Lys Ala Phe Ser
    130                 135                 140

Glu Thr Glu Gln Thr Lys Leu Ala Met Leu Ser Gly Ile Leu Leu Gly
145                 150                 155                 160

Asn Gly Thr Leu Pro Ala Thr Ile Leu Thr Ser Leu Phe Thr Asp Ser
                165                 170                 175

Leu Val Lys Glu Gly Ile Ala Ala Ser Phe Ala Val Lys Leu Phe Lys
            180                 185                 190

Ala Trp Met Ala Glu Lys Asp Ala Asn Ser Val Thr Ser Ser Leu Arg
        195                 200                 205

Lys Ala Asn Leu Asp Lys Arg Leu Leu Glu Leu Phe Pro Val Asn Arg
    210                 215                 220

Gln Ser Val Asp His Phe Ala Lys Tyr Phe Thr Asp Ala Gly Leu Lys
225                 230                 235                 240

Glu Leu Ser Asp Phe Leu Arg Val Gln Gln Ser Leu Gly Thr Arg Lys
                245                 250                 255

Glu Leu Gln Lys Glu Leu Gln Glu Arg Leu Ser Gln Glu Cys Pro Ile
            260                 265                 270

Lys Glu Val Val Leu Tyr Val Lys Glu Glu Met Lys Arg Asn Asp Leu
        275                 280                 285

Pro Glu Thr Ala Val Ile Gly Leu Leu Trp Thr Cys Ile Met Asn Ala
    290                 295                 300

Val Glu Trp Asn Lys Lys Glu Glu Leu Val Ala Glu Gln Ala Leu Lys
305                 310                 315                 320

His Leu Lys Gln Tyr Ala Pro Leu Leu Ala Val Phe Ser Ser Gln Gly
                325                 330                 335

Gln Ser Glu Leu Ile Leu Leu Gln Lys Val Gln Glu Tyr Cys Tyr Asp
            340                 345                 350

Asn Ile His Phe Met Lys Ala Phe Gln Lys Ile Val Val Leu Phe Tyr
        355                 360                 365

Lys Ala Asp Val Leu Ser Glu Glu Ala Ile Leu Lys Trp Tyr Lys Glu
    370                 375                 380

Ala His Val Ala Lys Gly Lys Ser Val Phe Leu Asp Gln Met Lys Lys
385                 390                 395                 400

Phe Val Glu Trp Leu Gln Asn Ala Glu Glu Glu Ser Glu Ser Glu Gly
                405                 410                 415

Glu Glu Asn

<210> 7
<211> 1630
<212> PRT
<213> Homo sapiens

<400> 7
   Lys Thr Phe Gly Leu His Gly Val Val Leu Asp Val Asp Ser Val Asn
   1 5 10 15
   Glu Leu Val Gln Val Glu Thr Tyr Leu Arg Ser Glu Gly Val Leu Val
   20 25 30
   Arg Tyr Trp Tyr Pro Ile Asp Met Leu Glu Arg Pro Pro Ala Gly Tyr
   35 40 45
   Arg Arg Thr Ala Thr Asn Gly Leu Val Thr Leu Asp Asn Thr Asn Leu
   50 55 60
   Gln Ile His Arg Glu Leu Leu Arg Cys Glu Ala Ala Leu Ala Arg Leu
   65 70 75 80
   Tyr Cys Arg Met Ala Leu Leu Asn Ile Phe Ala Pro Lys Leu Pro His
   85 90 95
   Leu Phe Thr Arg Leu Phe His Ile Pro Ala Ile Arg Asp Ile Thr Leu
   100 105 110
   Glu His Leu Gln Leu Leu Ser Asn Gln Leu Leu Ala Pro Pro Leu Pro
   115 120 125
   Asp Gly Thr Ile Ser Ser Ser Ser Ile Leu Leu Ala Gln Ser Leu Gln
   130 135 140
   His Cys Ile His Ser Gln Asn Cys Ser Ala Thr Asp Leu Phe Tyr Gln
   145 150 155 160
   Gly Asn Ser Gln Thr Val Arg Glu Trp Leu Asn Val Ala Ile Thr Arg
   165 170 175
   Thr Leu His Gln Gly Glu Glu Ser Leu Leu Glu Leu Thr Lys Gln Ile
   180 185 190
   Cys Ser Phe Leu Gln Thr Ala Pro Glu Gln Phe Pro Ser Glu Glu Phe
   195 200 205
   Pro Ile Ser Glu Ser Lys Val Asn Met Asp Val Asn Phe Pro Gly Ala
   210 215 220
   Ala Phe Val Val Val Ser Cys Lys Glu Ser Gln Ser Gly Phe Arg Lys
   225 230 235 240
   Asp Ser Ser Leu Tyr Lys Ala Pro Trp Ala Arg Val Leu Val Tyr Gly
   245 250 255
   Leu Gly His Lys Val Lys Arg Asn Gly Gln Leu Asn Leu Ile Glu Ala
   260 265 270
   Ala Cys Tyr Pro Arg Asp Ala Ser Pro Ala Asn Thr Gly Leu Ala Pro
   275 280 285
   Pro Pro Thr Ala Asp Gln Tyr Pro Ser Val Val Leu Ser Thr Asp Arg
   290 295 300
   Val His Ile Lys Leu Gly Val Ser Pro Pro Pro Gly Ala Val Leu Val
   305 310 315 320
   Leu His Ser Leu Pro Leu Glu Phe Pro Leu Ala Met Ala Phe Ala Glu
   325 330 335
   Gln Leu Leu Ser Trp Lys Ser Glu Asp Ser Glu Gly Lys Ser Glu Asp
   340 345 350
   Glu Pro Asp Thr Ile Pro Thr Ser Val Leu Leu Gln Val Val Glu Leu
   355 360 365
   Leu Gly Asn Phe Leu Trp Thr Thr Asp Met Ala Ala Cys Val Lys Glu
   370 375 380
   Leu Val Phe His Leu Leu Ala Glu Leu Leu Arg Thr Val His Thr Leu
   385 390 395 400
   Glu Gln Arg Arg His Pro Ala Gly Leu Ser Ser Ser Ile Ala Leu Gln
   405 410 415
   Leu Asn Pro Cys Leu Ala Met Leu Met Ala Leu Gln Ser Glu Leu His
   420 425 430
   Lys Leu Tyr Asp Glu Glu Thr Gln Asn Trp Val Ser Gly Gly Ala Cys
   435 440 445
   Gly Gly Ser Gly Gly Ala Ala Ala Gly Asp Gln Gly Arg Phe Ser Thr
   450 455 460
   Tyr Phe His Ala Leu Met Glu Gly Cys Leu Ala Val Ala Glu Val Thr
   465 470 475 480
   Leu Pro Thr Asn Met Ser Val Thr Ala Ser Gly Val Thr Ser Ala Thr
   485 490 495
   Ala Pro Asn Leu Ser Asp Ser Ser Ser Ser Ser Ser Ser Ser Pro Gly
   500 505 510
   Gln Thr Pro Gln Ser Pro Ser Leu Leu Ser Lys Arg Lys Lys Val Lys
   515 520 525
   Met Lys Arg Glu Lys Ala Ser Ser Ser Gly Lys Arg Gln Ser Ser Arg
   530                 535                 540
   Thr Val Asp Ser Asp Pro Thr Val Leu Ser Ile Gly Gly Ser Lys Pro
   545                 550                 555                 560
   Glu Asp Met Leu Trp Phe His Arg Ala Leu Thr Leu Leu Ile Ile Leu
                   565                 570                 575
   Arg His Leu Thr Arg Lys Asp Pro Gln Gly Leu Gly Val Thr Ser Asp
               580                 585                 590
   Ala Ile Ala Asp Ala Cys Gln Ala Leu Val Gly Pro Thr Ala His Ser
           595                 600                 605
   Arg Leu Leu Val Ile Ser Gly Ile Pro Thr His Leu Asp Glu Gly Val
       610                 615                 620
   Val Arg Gly Ala Ile Arg Lys Ala Cys Asn Ala His Gly Gly Val Phe
   625                 630                 635                 640
   Lys Asp Glu Ile Tyr Ile Pro Leu Gln Glu Glu Asp Thr Lys Lys Pro
                   645                 650                 655
   Lys Asp Lys Ala Glu Gly Gly Asp Gly Lys Val Glu Pro Glu Lys Thr
               660                 665                 670
   Leu Ala Phe Pro Gly Thr Asp Ser Met Glu Val Ser Thr Ser Ser Ser
           675                 680                 685
   Leu Thr Pro Ala Met Ser Ile Ser Ala Ser Ala Ser Thr Ser Gln Ala
       690                 695                 700
   Ser Ile Cys Ser Ser Gln Gly Ile Ser Gln Thr Val Ser Asp Leu Ser
   705                 710                 715                 720
   Val Asp Pro Leu Pro Ala Gly Leu Glu Leu Pro Ile Pro Pro Gly Leu
                   725                 730                 735
   Leu Glu Pro His Ala Val Ser Ser Gln Glu Ser Leu Asp Ile Ser Leu
               740                 745                 750
   Cys Ser Thr Gly Ser Leu Gly Ser Leu Gly Ser Leu Gly Glu Pro Leu
           755                 760                 765
   Asp Asn Ala Glu Thr Ala Ser Val Ser Asp Met Gly Ser Met Tyr Thr
       770                 775                 780
   Val Thr Ser Leu Asp Asn Gln Pro Leu Ala Ala Arg Pro Ile Lys Gly
   785                 790                 795                 800
   Phe Ala Val Val Glu Ile Arg Ser Arg Ala Lys Ile Glu Lys Ile Arg
                   805                 810                 815
   Ala Ser Leu Phe Asn Asn Asn Asp Leu Ile Gly Leu Ser Ser Leu Asp
               820                 825                 830
   Gly Glu Asp Glu Leu Met Glu Met Ser Thr Glu Glu Ile Leu Thr Val
           835                 840                 845
   Ser Val Val Asn Gln Ser Leu Phe Asp Thr Gln Gly Ser Pro Gly Leu
       850                 855                 860
   Glu Asp Tyr Phe Asn Asp Lys Ser Ile Lys Gly Glu Lys Leu Val Pro
   865                 870                 875                 880
   Gly Ala Arg Glu Val Leu Thr Glu Ile Phe Lys Ser Cys Ala His Ser
                   885                 890                 895
   Glu Gln Thr Leu Ser Leu Thr Pro Ala Lys Pro Ile Arg Val Ser Asp
               900                 905                 910
   Ile Tyr Leu Ser Lys Glu Gln Ile Asn Ser Gln Thr Pro Gly Asn Leu
           915                 920                 925
   Leu His Leu Phe Phe Thr Asn Val Arg Pro Pro Lys Lys Val Leu Glu
       930                 935                 940
   Asp Gln Leu Thr Gln Ile Leu Arg Lys Tyr Gly Val Pro Lys Pro Lys
   945                 950                 955                 960
   Phe Asp Lys Ser Lys Tyr Ser Lys Ala Gly Lys Glu Gln His Pro Val
                   965                 970                 975
   Lys Val Val Ser Thr Lys Arg Pro Ile Thr Lys Pro Pro Ala Lys Asp
               980                 985                 990
   Lys Ala Val Leu Asn Ser Val Ser Arg Thr Ala Leu Ser Glu Lys Lys
           995                 1000                1005
   Pro Thr Val Lys Pro Lys Ser Pro Glu Lys Ser Lys Pro Asp Glu Lys
       1010                1015                1020
   Asp Pro Glu Lys Ser Pro Thr Lys Lys Gln Glu Val Pro Glu Glu Lys
   1025                1030                1035                1040
   Tyr Leu Thr Leu Glu Gly Phe His Lys Phe Val Ile Asp Arg Ala Arg
   1045                1050                1055
   Gln Asp Ile Arg Ser Val Trp Arg Ala Ile Leu Ser Cys Gly Tyr Asp
               1060                1065                1070
   Leu His Phe Glu Arg Cys Ala Cys Ile Asp Val Arg His Ala Gln Lys
           1075                1080                1085
   Ala Ser Arg Lys Trp Thr Leu Glu Met Asp Val Ala Leu Val Gln Tyr
       1090                1095                1100
   Ile Asn Gln Leu Cys Arg His Leu Ala Ile Thr Pro Ala Arg Leu His
   1105                1110                1115                1120
   Pro His Glu Val Tyr Leu Asp Pro Ala Asp Ala Ala Asp Pro Arg Val
                   1125                1130                1135
   Ala Cys Leu Leu Asn Val Pro Ile Glu Ser Leu Arg Leu Arg Phe Ala
               1140                1145                1150
   Leu Leu Gln Ser Leu Asn Thr Thr Leu Glu Thr Phe Phe Leu Pro Leu
           1155                1160                1165
   Val Glu Leu Arg Gln Thr Pro Met Tyr Thr His Ser Ile Ala Ala Leu
       1170                1175                1180
   Leu Lys Glu Ala Lys Gly Leu Ile Phe Tyr Asp Thr Lys Val Thr Val
   1185                1190                1195                1200
   Met Asn Arg Val Leu Asn Ala Thr Val Gln Arg Thr Ala Asp His Ala
                   1205                1210                1215
   Ala Pro Glu Ile Thr Leu Asp Pro Leu Glu Ile Val Gly Gly Glu Ile
               1220                1225                1230
   Arg Ala Ser Glu Asn Ser Tyr Phe Cys Gln Ala Ala Arg Gln Leu Ala
           1235                1240                1245
   Ser Val Pro Ser Ser Gln Leu Cys Val Lys Leu Ala Ser Gly Gly Asp
       1250                1255                1260
   Pro Thr Tyr Ala Phe Asn Ile Arg Phe Thr Gly Glu Glu Val His Gly
   1265                1270                1275                1280
   Thr Ser Gly Ser Phe Arg His Phe Leu Trp Gln Val Cys Lys Glu Leu
                   1285                1290                1295
   Gln Ser Ser Ser Leu Ser Leu Leu Leu Leu Cys Pro Ser Ser Ala Val
   1300 1305 1310
   Asn Lys Asn Lys Gly Lys Tyr Ile Leu Thr Pro Ser Pro Ile Thr Tyr
   1315 1320 1325
   Gly Glu Glu Gln Leu Leu His Phe Leu Gly Gln Leu Leu Gly Ile Ala
   1330 1335 1340
   Ile Arg Ala Asp Val Pro Leu Pro Leu Asp Leu Leu Pro Ser Phe Trp
   1345 1350 1355 1360
   Lys Thr Leu Val Gly Glu Pro Leu Asp Pro Glu Gln Asp Leu Gln Glu
   1365 1370 1375
   Ala Asp Ile Leu Thr Tyr Asn Tyr Val Lys Lys Phe Glu Ser Ile Asn
   1380 1385 1390
   Asp Glu Thr Glu Leu Glu Ala Leu Cys Ala Glu Ile Ala Ser Gln His
   1395 1400 1405
   Leu Ala Thr Glu Ser Pro Asp Ser Pro Asn Lys Pro Cys Cys Arg Phe
   1410 1415 1420
   Thr Tyr Leu Thr Met Thr Gly Glu Glu Val Glu Leu Cys Ser Arg Gly
   1425 1430 1435 1440
   Arg His Ile Leu Val Ala Trp Glu Asn Lys Asp Ile Tyr Ala Ala Ala
   1445 1450 1455
   Ile Arg Ser Leu Arg Leu Arg Glu Leu Gln Asn Val Glu Cys Val Thr
   1460 1465 1470
   Ala Val Arg Ala Gly Leu Gly Ser Ile Ile Pro Leu Gln Leu Leu Thr
   1475 1480 1485
   Met Leu Ser Pro Leu Glu Met Glu Leu Arg Thr Cys Gly Leu Pro Tyr
   1490 1495 1500
   Ile Asn Leu Glu Phe Leu Lys Ala His Thr Met Tyr Gln Val Gly Leu
   1505 1510 1515 1520
   Met Glu Thr Asp Gln His Ile Glu Phe Phe Trp Gly Ala Leu Glu Met
   1525 1530 1535
   Phe Thr Gln Glu Glu Leu Cys Lys Phe Ile Lys Phe Ala Cys Asn Gln
   1540 1545 1550
   Glu Arg Ile Pro Phe Thr Cys Pro Cys Lys Asp Gly Gly Pro Asp Thr
   1555                    1560                    1565
   Ala His Val Pro Pro Tyr Pro Met Lys Ile Ala Pro Pro Asp Gly Thr
       1570                    1575                    1580
   Ala Gly Ser Pro Asp Ser Arg Tyr Ile Arg Val Glu Thr Cys Met Phe
   1585                1590                    1595                1600
   Met Ile Lys Leu Pro Gln Tyr Ser Ser Leu Glu Ile Met Leu Glu Lys
                   1605                    1610                    1615
   Leu Arg Cys Ala Ile His Tyr Arg Glu Asp Pro Leu Ser Gly
               1620                    1625                    1630

<210> 8
<211> 1050
<212> PRT
<213> Homo sapiens

<400> 8
Trp Arg Arg Ala Ala Cys Gly Arg Ser Gly Arg Ala Ser Leu Lys Glu
1               5                   10                  15

His Pro Arg Gly Thr Leu Leu Ser Asp Gly Ser Pro Ala Leu Ser Arg
            20                  25                  30

Asn Val Gly Met Thr Val Ser Gln Lys Gly Gly Pro Gln Pro Thr Pro
        35                  40                  45

Ser Pro Ala Gly Pro Gly Thr Gln Leu Gly Pro Ile Thr Gly Glu Met
    50                  55                  60

Asp Glu Ala Asp Ser Ala Phe Leu Lys Phe Lys Gln Thr Ala Asp Asp
65                  70                  75                  80

Ser Leu Ser Leu Thr Ser Pro Asn Thr Glu Ser Ile Phe Val Glu Asp
                85                  90                  95

Pro Tyr Thr Ala Ser Leu Arg Ser Glu Ile Glu Ser Asp Gly His Glu
            100                 105                 110

Phe Glu Ala Glu Ser Trp Ser Leu Ala Val Asp Ala Ala Tyr Ala Lys
        115                 120                 125

Lys Gln Lys Arg Glu Val Val Lys Arg Gln Asp Val Leu Tyr Glu Leu
    130                 135                 140

Met Gln Thr Glu Val His His Val Arg Thr Leu Lys Ile Met Leu Lys
145                 150                 155                 160

Val Tyr Ser Arg Ala Leu Gln Glu Glu Leu Gln Phe Ser Ser Lys Ala
                165                 170                 175

Ile Gly Arg Leu Phe Pro Cys Ala Asp Asp Leu Leu Glu Thr His Ser
            180                 185                 190

His Phe Leu Ala Arg Leu Lys Glu Arg Arg Gln Glu Ser Leu Glu Glu
        195                 200                 205

Gly Ser Asp Arg Asn Tyr Val Ile Gln Lys Ile Gly Asp Leu Leu Val
    210                 215                 220

Gln Gln Phe Ser Gly Glu Asn Gly Glu Arg Met Lys Glu Lys Tyr Gly
225                 230                 235                 240

Val Phe Cys Ser Gly His Asn Glu Ala Val Ser His Tyr Lys Leu Leu
                245                 250                 255

Leu Gln Gln Asn Lys Lys Phe Gln Asn Leu Ile Lys Lys Ile Gly Asn
            260                 265                 270

Phe Ser Ile Val Arg Arg Leu Gly Val Gln Glu Cys Ile Leu Leu Val
        275                 280                 285

Thr Gln Arg Ile Thr Lys Tyr Pro Val Leu Val Glu Arg Ile Ile Gln
    290                 295                 300

Asn Thr Glu Ala Gly Thr Glu Asp Tyr Glu Asp Leu Thr Gln Ala Leu
305                 310                 315                 320

Asn Leu Ile Lys Asp Ile Ile Ser Gln Val Asp Ala Lys Val Ser Glu
                325                 330                 335

Cys Glu Lys Gly Gln Arg Leu Arg Glu Ile Ala Gly Lys Met Asp Leu
            340                 345                 350

Lys Ser Ser Ser Lys Leu Lys Asn Gly Leu Thr Phe Arg Lys Glu Asp
        355                 360                 365

Met Leu Gln Arg Gln Leu His Leu Glu Gly Met Leu Cys Trp Lys Thr
    370                 375                 380

Thr Ser Gly Arg Leu Lys Asp Ile Leu Ala Ile Leu Leu Thr Asp Val
385                 390                 395                 400

Leu Leu Leu Leu Gln Glu Lys Asp Gln Lys Tyr Val Phe Ala Ser Val
                405                 410                 415

Asp Ser Lys Pro Pro Val Ile Ser Leu Gln Lys Leu Ile Val Arg Glu
            420                 425                 430

Val Ala Asn Glu Glu Lys Ala Met Phe Leu Ile Ser Ala Ser Leu Gln
        435                 440                 445

Gly Pro Glu Met Tyr Glu Ile Tyr Thr Ser Ser Lys Glu Asp Arg Asn
    450                 455                 460

Ala Trp Met Ala His Ile Gln Arg Ala Val Glu Ser Cys Pro Asp Glu
465                 470                 475                 480

Glu Glu Gly Pro Phe Ser Leu Pro Glu Glu Glu Arg Lys Val Val Glu
                485                 490                 495

Ala Arg Ala Thr Arg Leu Arg Asp Phe Gln Glu Arg Leu Ser Met Lys
            500                 505                 510

Asp Gln Leu Ile Ala Gln Ser Leu Leu Glu Lys Gln Gln Ile Tyr Leu
        515                 520                 525

Glu Met Ala Glu Met Gly Gly Leu Glu Asp Leu Pro Gln Pro Arg Gly
    530                 535                 540

Leu Phe Arg Gly Gly Asp Pro Ser Glu Thr Leu Gln Gly Glu Leu Ile
545                 550                 555                 560

Leu Lys Ser Ala Met Ser Glu Ile Glu Gly Ile Gln Ser Leu Ile Cys
                565                 570                 575

Arg Arg Leu Gly Ser Ala Asn Gly Gln Ala Glu Asp Gly Gly Ser Ser
            580                 585                 590

Thr Gly Pro Pro Arg Arg Ala Glu Thr Phe Ala Gly Tyr Asp Cys Thr
        595                 600                 605

Asn Ser Pro Thr Lys Asn Gly Ser Phe Lys Lys Lys Val Ser Ser Thr
    610                 615                 620

Asp Pro Arg Pro Arg Asp Trp Arg Gly Pro Pro Asn Ser Pro Asp Leu
625                 630                 635                 640

Lys Leu Ser Asp Ser Asp Ile Pro Gly Ser Ser Glu Glu Ser Pro Gln
                645                 650                 655

Val Val Glu Ala Pro Gly Thr Glu Ser Asp Pro Arg Leu Pro Thr Val
            660                 665                 670

Leu Glu Ser Glu Leu Val Gln Arg Ile Gln Thr Leu Ser Gln Leu Leu
        675                 680                 685

Leu Asn Leu Gln Ala Val Ile Ala His Gln Asp Ser Tyr Val Glu Thr
    690                 695                 700

Gln Arg Ala Ala Ile Gln Glu Arg Glu Lys Gln Phe Arg Leu Gln Ser
705                 710                 715                 720

Thr Arg Gly Asn Leu Leu Leu Glu Gln Glu Arg Gln Arg Asn Phe Glu
                725                 730                 735

Lys Gln Arg Glu Glu Arg Ala Ala Leu Glu Lys Leu Gln Ser Gln Leu
            740                 745                 750

Arg His Glu Gln Gln Arg Trp Glu Arg Glu Arg Gln Trp Gln His Gln
        755                 760                 765

Glu Leu Glu Arg Ala Gly Ala Arg Leu Gln Glu Arg Glu Gly Glu Ala
    770                 775                 780

Arg Gln Leu Arg Glu Arg Leu Glu Gln Glu Arg Ala Glu Leu Glu Arg
785                 790                 795                 800

Gln Arg Gln Ala Tyr Gln His Asp Leu Glu Arg Leu Arg Glu Ala Gln
                805                 810                 815

Arg Ala Val Glu Arg Glu Arg Glu Arg Leu Glu Leu Leu Arg Arg Leu
            820                 825                 830

Lys Lys Gln Asn Thr Ala Pro Gly Ala Leu Pro Pro Asp Thr Leu Ala
        835                 840                 845

Glu Ala Gln Pro Pro Ser His Pro Pro Ser Phe Asn Gly Glu Gly Leu
    850                 855                 860

Glu Gly Pro Arg Val Ser Met Leu Pro Ser Gly Val Gly Pro Glu Tyr
865                 870                 875                 880

Ala Glu Arg Pro Glu Val Ala Arg Arg Asp Ser Ala Pro Thr Glu Ser
                885                 890                 895

Arg Leu Ala Lys Ser Asp Val Pro Ile Gln Leu Leu Ser Ala Thr Asn
            900                 905                 910

Gln Phe Gln Arg Gln Ala Ala Val Gln Gln Gln Ile Pro Thr Lys Leu
        915                 920                 925

Ala Ala Ser Thr Lys Gly Gly Lys Asp Lys Gly Gly Lys Ser Arg Gly
    930                 935                 940

Ser Gln Arg Trp Glu Ser Ser Ala Ser Phe Asp Leu Lys Gln Gln Leu
945                 950                 955                 960

Leu Leu Asn Lys Leu Met Gly Lys Asp Glu Ser Thr Ser Arg Asn Arg
                965                 970                 975

Arg Ser Leu Ser Pro Ile Leu Pro Gly Arg His Ser Pro Ala Pro Pro
            980                 985                 990

Pro Asp Pro Gly Phe Pro Ala Pro Ser Pro Pro Pro Ala Asp Ser Pro
        995                 1000                1005

Ser Glu Gly Phe Ser Leu Lys Ala Gly Gly Thr Ala Leu Leu Pro Gly
   1010                1015                1020

Pro Pro Ala Pro Ser Pro Leu Pro Ala Thr Pro Leu Ser Ala Lys Glu
1025                1030                1035                1040

Asp Ala Ser Lys Glu Asp Val Ile Phe Phe
               1045                1050

<210> 9
<211> 1287
<212> PRT
<213> Homo sapiens

<400> 9
Lys Glu Val Gly Ala Gly Ser Ala Asn Gly Val Glu Met Val Gln Gly
  1               5                  10                  15

Pro Val Gln Thr Pro Ala Leu Thr Ile His Arg Arg Lys Arg Arg Arg
             20                  25                  30

Ser Cys Pro Pro Asn Arg Ala Ser Tyr Leu Pro Lys Ala Glu Ser Leu
         35                  40                  45

Ala Ser Leu Gly Ser His Leu Pro Ala Leu Leu Ser Arg Ala Arg Val
     50                  55                  60

Pro Arg Pro Pro Ala Gly Arg Arg Glu Arg Glu Arg Arg Arg Arg Pro
 65                  70                  75                  80

Val Ala Lys Ala Pro Ala Arg Leu Arg Gly Glu Tyr Glu Thr Gly Val
                85                  90                  95

Lys Met Thr Ser Arg Phe Gly Lys Thr Tyr Ser Arg Lys Gly Gly Asn
            100                 105                 110

Gly Ser Ser Lys Phe Asp Glu Val Phe Ser Asn Lys Arg Thr Thr Leu
        115                 120                 125

Ser Thr Lys Trp Gly Glu Thr Thr Phe Met Ala Lys Leu Gly Gln Lys
    130                 135                 140

Arg Pro Asn Phe Lys Pro Asp Ile Gln Glu Ile Pro Lys Lys Pro Lys
145                 150                 155                 160

Val Glu Glu Glu Ser Thr Gly Asp Pro Phe Gly Phe Asp Ser Asp Asp
                165                 170                 175

Glu Ser Leu Pro Val Ser Ser Lys Asn Leu Ala Gln Val Lys Cys Ser
            180                 185                 190

Ser Tyr Ser Glu Ser Ser Glu Ala Ala Gln Leu Glu Glu Val Thr Ser
        195                 200                 205

Val Leu Glu Ala Asn Ser Lys Ile Ser His Val Val Val Glu Asp Thr
    210                 215                 220

Val Val Ser Asp Lys Cys Phe Pro Leu Glu Asp Thr Leu Leu Gly Lys
225                 230                 235                 240

Glu Lys Ser Thr Asn Arg Ile Val Glu Asp Asp Ala Ser Ile Ser Ser
                245                 250                 255

Cys Asn Lys Leu Ile Thr Ser Asp Lys Val Glu Asn Phe His Glu Glu
            260                 265                 270

His Glu Lys Asn Ser His His Ile His Lys Asn Ala Asp Asp Ser Thr
        275                 280                 285

Lys Lys Pro Asn Ala Glu Thr Thr Val Ala Ser Glu Ile Lys Glu Thr
    290                 295                 300

Asn Asp Thr Trp Asn Ser Gln Phe Gly Lys Arg Pro Glu Ser Pro Ser
305                 310                 315                 320

Glu Ile Ser Pro Ile Lys Gly Ser Val Arg Thr Gly Leu Phe Glu Trp
                325                 330                 335

Asp Asn Asp Phe Glu Asp Ile Arg Ser Glu Asp Cys Ile Leu Ser Leu
            340                 345                 350

Asp Ser Asp Pro Leu Leu Glu Met Lys Asp Asp Asp Phe Lys Asn Arg
        355                 360                 365

Leu Glu Asn Leu Asn Glu Ala Ile Glu Glu Asp Ile Val Gln Ser Val
    370                 375                 380

Leu Arg Pro Thr Asn Cys Arg Thr Tyr Cys Arg Ala Asn Lys Thr Lys
385                 390                 395                 400

Ser Ser Gln Gly Ala Ser Asn Phe Asp Lys Leu Met Asp Gly Thr Ser
                405                 410                 415

Gln Ala Leu Ala Lys Ala Asn Ser Glu Ser Ser Lys Asp Gly Leu Asn
            420                 425                 430

Gln Ala Lys Lys Gly Gly Val Ser Cys Gly Thr Ser Phe Arg Gly Thr
        435                 440                 445

Val Gly Arg Thr Arg Asp Tyr Thr Val Leu His Pro Ser Cys Leu Ser
    450                 455                 460

Val Cys Asn Val Thr Ile Gln Asp Thr Met Glu Arg Ser Met Asp Glu
465                 470                 475                 480

Phe Thr Ala Ser Thr Pro Ala Asp Leu Gly Glu Ala Gly Arg Leu Arg
                485                 490                 495

Lys Lys Ala Asp Ile Ala Thr Ser Lys Thr Thr Thr Arg Phe Arg Pro
            500                 505                 510

Ser Asn Thr Lys Ser Lys Lys Asp Val Lys Leu Glu Phe Phe Gly Phe
        515                 520                 525

Glu Asp His Glu Thr Gly Gly Asp Glu Gly Gly Ser Gly Ser Ser Asn
    530                 535                 540

Tyr Lys Ile Lys Tyr Phe Gly Phe Asp Asp Leu Ser Glu Ser Glu Asp
545                 550                 555                 560

Asp Glu Asp Asp Asp Cys Gln Val Glu Arg Lys Thr Ser Lys Lys Arg
                565                 570                 575

Thr Lys Thr Ala Pro Ser Pro Ser Leu Gln Pro Pro Pro Glu Ser Asn
            580                 585                 590

Asp Asn Ser Gln Asp Ser Gln Ser Gly Thr Asn Asn Ala Glu Asn Leu
        595                 600                 605

Asp Phe Thr Glu Asp Leu Pro Gly Val Pro Glu Ser Val Lys Lys Pro
    610                 615                 620

Ile Asn Lys Gln Gly Asp Lys Ser Lys Glu Asn Thr Arg Lys Ile Phe
625                 630                 635                 640

Ser Gly Pro Lys Arg Ser Pro Thr Lys Ala Val Tyr Asn Ala Arg His
                645                 650                 655

Trp Asn His Pro Asp Ser Glu Glu Leu Pro Gly Pro Pro Val Val Lys
            660                 665                 670

Pro Gln Ser Val Thr Val Arg Leu Ser Ser Lys Glu Pro Asn Gln Lys
        675                 680                 685

Asp Asp Gly Val Phe Lys Ala Pro Ala Pro Pro Ser Lys Val Ile Lys
    690                 695                 700

Thr Val Thr Ile Pro Thr Gln Pro Tyr Gln Asp Ile Val Thr Ala Leu
705                 710                 715                 720

Lys Cys Arg Arg Glu Asp Lys Glu Leu Tyr Thr Val Val Gln His Val
                725                 730                 735

Lys His Phe Asn Asp Val Val Glu Phe Gly Glu Asn Gln Glu Phe Thr
            740                 745                 750

Asp Asp Ile Glu Tyr Leu Leu Ser Gly Leu Lys Ser Thr Gln Pro Leu
        755                 760                 765

Asn Thr Arg Cys Leu Ser Val Ile Ser Leu Ala Thr Lys Cys Ala Met
    770                 775                 780

Pro Ser Phe Arg Met His Leu Arg Ala His Gly Met Val Ala Met Val
785                 790                 795                 800

Phe Lys Thr Leu Asp Asp Ser Gln His His Gln Asn Leu Ser Leu Cys
                805                 810                 815

Thr Ala Ala Leu Met Tyr Ile Leu Ser Arg Asp Arg Leu Asn Met Asp
            820                 825                 830

Leu Asp Arg Ala Ser Leu Asp Leu Met Ile Arg Leu Leu Glu Leu Glu
        835                 840                 845

Gln Asp Ala Ser Ser Ala Lys Leu Leu Asn Glu Lys Asp Met Asn Lys
    850                 855                 860

Ile Lys Glu Lys Ile Arg Arg Leu Cys Glu Thr Val His Asn Lys His
865                 870                 875                 880

Leu Asp Leu Glu Asn Ile Thr Thr Gly His Leu Ala Met Glu Thr Leu
                885                 890                 895

Leu Ser Leu Thr Ser Lys Arg Ala Gly Asp Trp Phe Lys Glu Glu Leu
            900                 905                 910

Arg Leu Leu Gly Gly Leu Asp His Ile Val Asp Lys Val Lys Glu Cys
        915                 920                 925

Val Asp His Leu Ser Arg Asp Glu Asp Glu Glu Lys Leu Val Ala Ser
    930                 935                 940

Leu Trp Gly Ala Glu Arg Cys Leu Arg Val Leu Glu Ser Val Thr Val
945                 950                 955                 960

His Asn Pro Glu Asn Gln Ser Tyr Leu Ile Ala Tyr Lys Asp Ser Gln
                965                 970                 975

Leu Ile Val Ser Ser Ala Lys Ala Leu Gln His Cys Glu Glu Leu Ile
            980                 985                 990

Gln Gln Tyr Asn Arg Ala Glu Asp Ser Ile Cys Leu Ala Asp Ser Lys
        995                 1000                1005

Pro Leu Pro His Gln Asn Val Thr Asn His Val Gly Lys Ala Val Glu
    1010                1015                1020

Asp Cys Met Arg Ala Ile Ile Gly Val Leu Leu Asn Leu Thr Asn Asp
1025                1030                1035                1040

Asn Glu Trp Gly Ser Thr Lys Thr Gly Glu Gln Asp Gly Leu Ile Gly
                1045                1050                1055

Thr Ala Leu Asn Cys Val Leu Gln Val Pro Lys Tyr Leu Pro Gln Glu
            1060                1065                1070

Gln Arg Phe Asp Ile Arg Val Leu Gly Leu Gly Leu Leu Ile Asn Leu
        1075                1080                1085

Val Glu Tyr Ser Ala Arg Asn Arg His Cys Leu Val Asn Met Glu Thr
    1090                1095                1100

Ser Cys Ser Phe Asp Ser Ser Ile Cys Ser Gly Glu Gly Asp Asp Ser
1105                1110                1115                1120

Leu Arg Ile Gly Gly Gln Val His Ala Val Gln Ala Leu Val Gln Leu
                1125                1130                1135

Phe Leu Glu Arg Glu Arg Ala Ala Gln Leu Ala Glu Ser Lys Thr Asp
            1140                1145                1150

Glu Leu Ile Lys Asp Ala Pro Thr Thr Gln His Asp Lys Ser Gly Glu
        1155                1160                1165

Trp Gln Glu Thr Ser Gly Glu Ile Gln Trp Val Ser Thr Glu Lys Thr
    1170                1175                1180

Asp Gly Thr Glu Glu Lys His Lys Lys Glu Glu Glu Asp Glu Glu Leu
1185                1190                1195                1200

Asp Leu Asn Lys Ala Leu Gln His Ala Gly Lys His Met Glu Asp Cys
                1205                1210                1215

Ile Val Ala Ser Tyr Thr Ala Leu Leu Leu Gly Cys Leu Cys Gln Glu
            1220                1225                1230

Ser Pro Ile Asn Val Thr Thr Val Arg Glu Tyr Leu Pro Glu Gly Asp
        1235                1240                1245

Phe Ser Ile Met Thr Glu Met Leu Lys Lys Phe Leu Ser Phe Met Asn
    1250                1255                1260

Leu Thr Cys Ala Val Gly Thr Thr Gly Gln Lys Ser Ile Ser Arg Val
1265                1270                1275                1280

Ile Glu Tyr Leu Glu His Cys
                1285

<210> 10
<211> 591
<212> PRT
<213> Mus musculus

<300>
<301> Schreiber, K. L.
<302> Class II histocompatibility molecules associate with
      calnexin duringassembly in the endoplasmatic reticulum
<303> Int. Immunol.

<304> 1994
<305> 6
<306> 101-111
<313> 1 - 591

<400> 10
   Met Glu Gly Lys Trp Leu Leu Cys Leu Leu Leu Val Leu Gly Thr Ala
   1               5                   10                  15
   Ala Val Glu Ala His Asp Gly His Asp Asp Asp Ala Ile Asp Ile Glu
               20                  25                  30
   Asp Asp Leu Asp Asp Val Ile Glu Glu Val Glu Asp Ser Lys Ser Lys
           35                  40                  45
   Ser Asp Ala Ser Thr Pro Pro Ser Pro Lys Val Thr Tyr Lys Ala Pro
       50                  55                  60
   Val Pro Thr Gly Glu Val Tyr Phe Ala Asp Ser Phe Asp Arg Gly Ser
   65                  70                  75                  80
   Leu Ser Gly Trp Ile Leu Ser Lys Ala Lys Lys Asp Asp Thr Asp Asp
                   85                  90                  95
   Glu Ile Ala Lys Tyr Asp Gly Lys Trp Glu Val Asp Glu Met Lys Glu
               100                 105                 110
   Thr Lys Leu Pro Gly Asp Lys Gly Leu Val Leu Met Ser Arg Ala Lys
           115                 120                 125
   His His Ala Ile Ser Ala Lys Leu Asn Lys Pro Phe Leu Phe Asp Thr
       130                 135                 140
   Lys Pro Leu Ile Val Gln Tyr Glu Val Asn Phe Gln Asn Gly Ile Glu
   145                 150                 155                 160
   Cys Gly Gly Ala Tyr Val Lys Leu Leu Ser Lys Thr Ala Glu Leu Ser
                   165                 170                 175
   Leu Asp Gln Phe His Asp Lys Thr Pro Tyr Thr Ile Met Phe Gly Pro
               180                 185                 190
   Asp Lys Cys Gly Glu Asp Tyr Lys Leu His Phe Ile Phe Arg His Lys
           195                 200                 205
   Asn Pro Lys Thr Gly Val Tyr Glu Glu Lys His Ala Lys Arg Pro Asp
       210                 215                 220
   Ala Asp Leu Lys Thr Tyr Phe Thr Asp Lys Lys Thr His Leu Tyr Thr
   225                 230                 235                 240
   Leu Ile Leu Asn Pro Asp Asn Ser Phe Glu Ile Leu Val Asp Gln Ser
                   245                 250                 255
   Val Val Asn Ser Gly Asn Leu Leu Asn Asp Met Thr Pro Pro Val Asn
               260                 265                 270
   Pro Ser Arg Glu Ile Glu Asp Pro Glu Asp Arg Lys Pro Glu Asp Trp
           275                 280                 285
   Asp Glu Arg Pro Lys Ile Ala Asp Pro Asp Ala Val Lys Pro Asp Asp
       290                 295                 300
   Trp Asp Glu Asp Ala Pro Ser Lys Ile Pro Asp Glu Glu Ala Thr Lys
   305                 310                 315                 320
   Pro Glu Gly Trp Leu Asp Asp Glu Pro Glu Tyr Ile Pro Asp Pro Asp
                   325                 330                 335
   Ala Glu Lys Pro Glu Asp Trp Asp Glu Asp Met Asp Gly Glu Trp Glu
               340                 345                 350
   Ala Pro Gln Ile Ala Asn Pro Lys Cys Glu Ser Ala Pro Gly Cys Gly
           355                 360                 365
   Val Trp Gln Arg Pro Met Ile Asp Asn Pro Asn Tyr Lys Gly Lys Trp
       370                 375                 380
   Lys Pro Pro Met Ile Asp Asn Pro Asn Tyr Gln Gly Ile Trp Lys Pro
   385                 390                 395                 400
   Arg Lys Ile Pro Asn Pro Asp Phe Phe Glu Asp Leu Glu Pro Phe Lys
                   405                 410                 415
   Met Thr Pro Phe Ser Ala Ile Gly Leu Glu Leu Trp Ser Met Thr Ser
               420                 425                 430
   Asp Ile Phe Phe Asp Asn Phe Ile Ile Ser Gly Asp Arg Arg Val Val
           435                 440                 445
   Asp Asp Trp Ala Asn Asp Gly Trp Gly Leu Lys Lys Ala Ala Asp Gly
       450                 455                 460
   Ala Ala Glu Pro Gly Val Val Leu Gln Met Leu Glu Ala Ala Glu Glu
   465                 470                 475                 480
   Arg Pro Trp Leu Trp Val Val Tyr Ile Leu Thr Val Ala Leu Pro Val
                   485                     490                     495
   Phe Leu Val Ile Leu Phe Cys Cys Ser Gly Lys Lys Gln Ser Asn Ala
               500                     505                     510
   Met Glu Tyr Lys Lys Thr Asp Ala Pro Gln Pro Asp Val Lys Asp Glu
           515                     520                     525
   Glu Gly Lys Glu Glu Glu Lys Asn Lys Arg Asp Glu Glu Glu Glu Glu
       530                     535                     540
   Glu Lys Leu Glu Glu Lys Gln Lys Ser Asp Ala Glu Glu Asp Gly Val
   545                     550                     555                     560
   Thr Gly Ser Gln Asp Glu Glu Asp Ser Lys Pro Lys Ala Glu Glu Asp
                   565                     570                     575
   Glu Ile Leu Asn Arg Ser Pro Arg Asn Arg Lys Pro Arg Arg Glu
               580                     585                     590


<210> 11
<211> 592
<212> PRT
<213> Homo sapiens

<400> 11

<210> 12

<211> 2441
<212> PRT
<213> Mus musculus

<300>
<301> Chrivia, J. C.
<302> Phosphorylated CREB binds specifically to the nuclear
      protein CBP
<303> Nature
<304> 1993
<305> 365
<306> 855-859
<313> 1 - 2441

<400> 12
Met Ala Glu Asn Leu Leu Asp Gly Pro Pro Asn Pro Lys Arg Ala Lys
  1               5                  10                  15

Leu Ser Ser Pro Gly Phe Ser Ala Asn Asp Asn Thr Asp Phe Gly Ser
             20                  25                  30

Leu Phe Asp Leu Glu Asn Asp Leu Pro Asp Glu Leu Ile Pro Asn Gly
         35                  40                  45

Glu Leu Ser Leu Leu Asn Ser Gly Asn Leu Val Pro Asp Ala Ala Ser
     50                  55                  60

Lys His Lys Gln Leu Ser Glu Leu Leu Arg Gly Gly Ser Gly Ser Ser
 65                  70                  75                  80

Ile Asn Pro Gly Ile Gly Asn Val Ser Ala Ser Ser Pro Val Gln Gln
                 85                  90                  95

Gly Leu Gly Gly Gln Ala Gln Gly Gln Pro Asn Ser Thr Asn Met Ala
            100                 105                 110

Ser Leu Gly Ala Met Gly Lys Ser Pro Leu Asn Gln Gly Asp Ser Ser
        115                 120                 125

Thr Pro Asn Leu Pro Lys Gln Ala Ala Ser Thr Ser Gly Pro Thr Pro
    130                 135                 140

Pro Ala Ser Gln Ala Leu Asn Pro Gln Ala Gln Lys Gln Val Gly Leu
145                 150                 155                 160

Val Thr Ser Ser Pro Ala Thr Ser Gln Thr Gly Pro Gly Ile Cys Met
                165                 170                 175

Asn Ala Asn Phe Asn Gln Thr His Pro Gly Leu Leu Asn Ser Asn Ser
            180                 185                 190

Gly His Ser Leu Met Asn Gln Ala Gln Gln Gly Gln Ala Gln Val Met
        195                 200                 205

Asn Gly Ser Leu Gly Ala Ala Gly Arg Gly Arg Gly Ala Gly Met Pro
    210                 215                 220

Tyr Pro Ala Pro Ala Met Gln Gly Ala Thr Ser Ser Val Leu Ala Glu
225                 230                 235                 240

Thr Leu Thr Gln Val Ser Pro Gln Met Ala Gly His Ala Gly Leu Asn
                245                 250                 255

Thr Ala Gln Ala Gly Gly Met Thr Lys Met Gly Met Thr Gly Thr Thr
            260                 265                 270

Ser Pro Phe Gly Gln Pro Phe Ser Gln Thr Gly Gly Gln Gln Met Gly
        275                 280                 285

Ala Thr Gly Val Asn Pro Gln Leu Ala Ser Lys Gln Ser Met Val Asn
    290                 295                 300

Ser Leu Pro Ala Phe Pro Thr Asp Ile Lys Asn Thr Ser Val Thr Thr
305                 310                 315                 320

Val Pro Asn Met Ser Gln Leu Gln Thr Ser Val Gly Ile Val Pro Thr
                325                 330                 335

Gln Ala Ile Ala Thr Gly Pro Thr Ala Asp Pro Glu Lys Arg Lys Leu
            340                 345                 350

Ile Gln Gln Gln Leu Val Leu Leu Leu His Ala His Lys Cys Gln Arg
        355                 360                 365

Arg Glu Gln Ala Asn Gly Glu Val Arg Ala Cys Ser Leu Pro His Cys
    370                 375                 380

Arg Thr Met Lys Asn Val Leu Asn His Met Thr His Cys Gln Ala Pro
385                 390                 395                 400

Lys Ala Cys Gln Val Ala His Cys Ala Ser Ser Arg Gln Ile Ile Ser
                405                 410                 415

His Trp Lys Asn Cys Thr Arg His Asp Cys Pro Val Cys Leu Pro Leu
            420                 425                 430

Lys Asn Ala Ser Asp Lys Arg Asn Gln Gln Thr Ile Leu Gly Ser Pro
        435                 440                 445

Ala Ser Gly Ile Gln Asn Thr Ile Gly Ser Val Gly Ala Gly Gln Gln
    450                 455                 460

Asn Ala Thr Ser Leu Ser Asn Pro Asn Pro Ile Asp Pro Ser Ser Met
465                 470                 475                 480

Gln Arg Ala Tyr Ala Ala Leu Gly Leu Pro Tyr Met Asn Gln Pro Gln
                485                 490                 495

Thr Gln Leu Gln Pro Gln Val Pro Gly Gln Gln Pro Ala Gln Pro Pro
            500                 505                 510

Ala His Gln Gln Met Arg Thr Leu Asn Ala Leu Gly Asn Asn Pro Met
        515                 520                 525

Ser Val Pro Ala Gly Gly Ile Thr Thr Asp Gln Gln Pro Pro Asn Leu
    530                 535                 540

Ile Ser Glu Ser Ala Leu Pro Thr Ser Leu Gly Ala Thr Asn Pro Leu
545                 550                 555                 560

Met Asn Asp Gly Ser Asn Ser Gly Asn Ile Gly Ser Leu Ser Thr Ile
                565                 570                 575

Pro Thr Ala Ala Pro Pro Ser Ser Thr Gly Val Arg Lys Gly Trp His
            580                 585                 590

Glu His Val Thr Gln Asp Leu Arg Ser His Leu Val His Lys Leu Val
        595                 600                 605

Gln Ala Ile Phe Pro Thr Pro Asp Pro Ala Ala Leu Lys Asp Arg Arg
    610                 615                 620

Met Glu Asn Leu Val Ala Tyr Ala Lys Lys Val Glu Gly Asp Met Tyr
625                 630                 635                 640

Glu Ser Ala Asn Ser Arg Asp Glu Tyr Tyr His Leu Leu Ala Glu Lys
                645                 650                 655

Ile Tyr Lys Ile Gln Lys Glu Leu Glu Glu Lys Arg Arg Thr Arg Leu
            660                 665                 670

His Lys Gln Gly Ile Leu Gly Asn Gln Pro Ala Leu Pro Ala Ser Gly
        675                 680                 685

Ala Gln Pro Pro Val Ile Pro Pro Ala Gln Ser Val Arg Pro Pro Asn
    690                 695                 700

Gly Pro Leu Pro Leu Pro Val Asn Arg Met Gln Val Ser Gln Gly Met
705                 710                 715                 720

Asn Ser Phe Asn Pro Met Ser Leu Gly Asn Val Gln Leu Pro Gln Ala
                725                 730                 735

Pro Met Gly Pro Arg Ala Ala Ser Pro Met Asn His Ser Val Gln Met
            740                 745                 750

Asn Ser Met Ala Ser Val Pro Gly Met Ala Ile Ser Pro Ser Arg Met
        755                 760                 765

Pro Gln Pro Pro Asn Met Met Gly Thr His Ala Asn Asn Ile Met Ala
    770                 775                 780

Gln Ala Pro Thr Gln Asn Gln Phe Leu Pro Gln Asn Gln Phe Pro Ser
785                 790                 795                 800

Ser Ser Gly Ala Met Ser Val Asn Ser Val Gly Met Gly Gln Pro Ala
                805                 810                 815

Ala Gln Ala Gly Val Ser Gln Gly Gln Glu Pro Gly Ala Ala Leu Pro
            820                 825                 830

Asn Pro Leu Asn Met Leu Ala Pro Gln Ala Ser Gln Leu Pro Cys Pro
        835                 840                 845

Pro Val Thr Gln Ser Pro Leu His Pro Thr Pro Pro Pro Ala Ser Thr
    850                 855                 860

Ala Ala Gly Met Pro Ser Leu Gln His Pro Thr Ala Pro Gly Met Thr
865                 870                 875                 880

Pro Pro Gln Pro Ala Ala Pro Thr Gln Pro Ser Thr Pro Val Ser Ser
                885                 890                 895

Gly Gln Thr Pro Thr Pro Thr Pro Gly Ser Val Pro Ser Ala Ala Gln
            900                 905                 910

Thr Gln Ser Thr Pro Thr Val Gln Ala Ala Ala Gln Ala Gln Val Thr
        915                 920                 925

Pro Gln Pro Gln Thr Pro Val Gln Pro Pro Ser Val Ala Thr Pro Gln
    930                 935                 940

Ser Ser Gln Gln Gln Pro Thr Pro Val His Thr Gln Pro Pro Gly Thr
945                 950                 955                 960

Pro Leu Ser Gln Ala Ala Ala Ser Ile Asp Asn Arg Val Pro Thr Pro
                965                 970                 975

Ser Thr Val Thr Ser Ala Glu Thr Ser Ser Gln Gln Pro Gly Pro Asp
            980                 985                 990

Val Pro Met Leu Glu Met Lys Thr Glu Val Gln Thr Asp Asp Ala Glu
        995                 1000                1005

Pro Glu Pro Thr Glu Ser Lys Gly Glu Pro Arg Ser Glu Met Met Glu
    1010                1015                1020

Glu Asp Leu Gln Gly Ser Ser Gln Val Lys Glu Glu Thr Asp Thr Thr
1025                1030                1035                1040

Glu Gln Lys Ser Glu Pro Met Glu Val Glu Glu Lys Lys Pro Glu Val
                1045                1050                1055

Lys Val Glu Ala Lys Glu Glu Glu Glu Asn Ser Ser Asn Asp Thr Ala
            1060                1065                1070

Ser Gln Ser Thr Ser Pro Ser Gln Pro Arg Lys Lys Ile Phe Lys Pro
        1075                1080                1085

Glu Glu Leu Arg Gln Ala Leu Met Pro Thr Leu Glu Ala Leu Tyr Arg
    1090                1095                1100

Gln Asp Pro Glu Ser Leu Pro Phe Arg Gln Pro Val Asp Pro Gln Leu
1105                1110                1115                1120

Leu Gly Ile Pro Asp Tyr Phe Asp Ile Val Lys Asn Pro Met Asp Leu
                1125                1130                1135

Ser Thr Ile Lys Arg Lys Leu Asp Thr Gly Gln Tyr Gln Glu Pro Trp
            1140                1145                1150

Gln Tyr Val Asp Asp Val Arg Leu Met Phe Asn Asn Ala Trp Leu Tyr
        1155                1160                1165

Asn Arg Lys Thr Ser Arg Val Tyr Lys Phe Cys Ser Lys Leu Ala Glu
    1170                1175                1180

Val Phe Glu Gln Glu Ile Asp Pro Val Met Gln Ser Leu Gly Tyr Cys
1185                1190                1195                1200

Cys Gly Arg Lys Tyr Glu Phe Ser Pro Gln Thr Leu Cys Cys Tyr Gly
                1205                1210                1215

Lys Gln Leu Cys Thr Ile Pro Arg Asp Ala Ala Tyr Tyr Ser Tyr Gln
            1220                1225                1230

Asn Arg Tyr His Phe Cys Gly Lys Cys Phe Thr Glu Ile Gln Gly Glu
        1235                1240                1245

Asn Val Thr Leu Gly Asp Asp Pro Ser Gln Pro Gln Thr Thr Ile Ser
    1250                1255                1260

Lys Asp Gln Phe Glu Lys Lys Lys Asn Asp Thr Leu Asp Pro Glu Pro
1265                1270                1275                1280

Phe Val Asp Cys Lys Glu Cys Gly Arg Lys Met His Gln Ile Cys Val
                1285                1290                1295

Leu His Tyr Asp Ile Ile Trp Pro Ser Gly Phe Val Cys Asp Asn Cys
            1300                1305                1310

Leu Lys Lys Thr Gly Arg Pro Arg Lys Glu Asn Lys Phe Ser Ala Lys
        1315                1320                1325

Arg Leu Gln Thr Thr Arg Leu Gly Asn His Leu Glu Asp Arg Val Asn
    1330                1335                1340

Lys Phe Leu Arg Arg Gln Asn His Pro Glu Ala Gly Glu Val Phe Val
1345                1350                1355                1360

Arg Val Val Ala Ser Ser Asp Lys Thr Val Glu Val Lys Pro Gly Met
                1365                1370                1375

Lys Ser Arg Phe Val Asp Ser Gly Glu Met Ser Glu Ser Phe Pro Tyr
            1380                1385                1390

Arg Thr Lys Ala Leu Phe Ala Phe Glu Glu Ile Asp Gly Val Asp Val
        1395                1400                1405

Cys Phe Phe Gly Met His Val Gln Asp Thr Ala Leu Ile Ala Pro His
    1410                1415                1420

Gln Ile Gln Gly Cys Val Tyr Ile Ser Tyr Leu Asp Ser Ile His Phe
1425                1430                1435                1440

Phe Arg Pro Arg Cys Leu Arg Thr Ala Val Tyr His Glu Ile Leu Ile
                1445                1450                1455

Gly Tyr Leu Glu Tyr Val Lys Lys Leu Val Tyr Val Thr Ala His Ile
            1460                1465                1470

Trp Ala Cys Pro Pro Ser Glu Gly Asp Asp Tyr Ile Phe His Cys His
        1475                1480                1485

Pro Pro Asp Gln Lys Ile Pro Lys Pro Lys Arg Leu Gln Glu Trp Tyr
    1490                1495                1500

Lys Lys Met Leu Asp Lys Ala Phe Ala Glu Arg Ile Ile Asn Asp Tyr
1505                1510                1515                1520

Lys Asp Ile Phe Lys Gln Ala Asn Glu Asp Arg Leu Thr Ser Ala Lys
                1525                1530                1535

Glu Leu Pro Tyr Phe Glu Gly Asp Phe Trp Pro Asn Val Leu Glu Glu
            1540                1545                1550

Ser Ile Lys Glu Leu Glu Gln Glu Glu Glu Glu Arg Lys Lys Glu Glu
        1555                1560                1565

Ser Thr Ala Ala Ser Glu Thr Pro Glu Gly Ser Gln Gly Asp Ser Lys
    1570                1575                1580

Asn Ala Lys Lys Lys Asn Asn Lys Lys Thr Asn Lys Asn Lys Ser Ser
1585                1590                1595                1600

Ile Ser Arg Ala Asn Lys Lys Lys Pro Ser Met Pro Asn Val Ser Asn
                1605                1610                1615

Asp Leu Ser Gln Lys Leu Tyr Ala Thr Met Glu Lys His Lys Glu Val
            1620                1625                1630

Phe Phe Val Ile His Leu His Ala Gly Pro Val Ile Ser Thr Gln Pro
        1635                1640                1645

Pro Ile Val Asp Pro Asp Pro Leu Leu Ser Cys Asp Leu Met Asp Gly
    1650                1655                1660

Arg Asp Ala Phe Leu Thr Leu Ala Arg Asp Lys His Trp Glu Phe Ser
1665                1670                1675                1680

Ser Leu Arg Arg Ser Lys Trp Ser Thr Leu Cys Met Leu Val Glu Leu
                1685                1690                1695

His Thr Gln Gly Gln Asp Arg Phe Val Tyr Thr Cys Asn Glu Cys Lys
            1700                1705                1710

His His Val Glu Thr Arg Trp His Cys Thr Val Cys Glu Asp Tyr Asp
        1715                1720                1725

Leu Cys Ile Asn Cys Tyr Asn Thr Lys Ser His Thr His Lys Met Val
    1730                1735                1740

Lys Trp Gly Leu Gly Leu Asp Asp Glu Gly Ser Ser Gln Gly Glu Pro
1745                1750                1755                1760

Gln Ser Lys Ser Pro Gln Glu Ser Arg Arg Leu Ser Ile Gln Arg Cys
                1765                1770                1775

Ile Gln Ser Leu Val His Ala Cys Gln Cys Arg Asn Ala Asn Cys Ser
            1780                1785                1790

Leu Pro Ser Cys Gln Lys Met Lys Arg Val Val Gln His Thr Lys Gly
        1795                1800                1805

Cys Lys Arg Lys Thr Asn Gly Gly Cys Pro Val Cys Lys Gln Leu Ile
    1810                1815                1820

Ala Leu Cys Cys Tyr His Ala Lys His Cys Gln Glu Asn Lys Cys Pro
1825                1830                1835                1840

Val Pro Phe Cys Leu Asn Ile Lys His Asn Val Arg Gln Gln Gln Ile
                1845                1850                1855

Gln His Cys Leu Gln Gln Ala Gln Leu Met Arg Arg Arg Met Ala Thr
            1860                1865                1870

Met Asn Thr Arg Asn Val Pro Gln Gln Ser Leu Pro Ser Pro Thr Ser
        1875                1880                1885

Ala Pro Pro Gly Thr Pro Thr Gln Gln Pro Ser Thr Pro Gln Thr Pro
    1890                1895                1900

Gln Pro Pro Ala Gln Pro Gln Pro Ser Pro Val Asn Met Ser Pro Ala
1905                1910                1915                1920

Gly Phe Pro Asn Val Ala Arg Thr Gln Pro Pro Thr Ile Val Ser Ala
                1925                1930                1935

Gly Lys Pro Thr Asn Gln Val Pro Ala Pro Pro Pro Pro Ala Gln Pro
            1940                1945                1950

Pro Pro Ala Ala Val Glu Ala Ala Arg Gln Ile Glu Arg Glu Ala Gln
        1955                1960                1965

Gln Gln Gln His Leu Tyr Arg Ala Asn Ile Asn Asn Gly Met Pro Pro
    1970                1975                1980

Gly Arg Asp Gly Met Gly Thr Pro Gly Ser Gln Met Thr Pro Val Gly
1985                1990                1995                2000

Leu Asn Val Pro Arg Pro Asn Gln Val Ser Gly Pro Val Met Ser Ser
                2005                2010                2015

Met Pro Pro Gly Gln Trp Gln Gln Ala Pro Ile Pro Gln Gln Gln Pro
            2020                2025                2030

Met Pro Gly Met Pro Arg Pro Val Met Ser Met Gln Ala Gln Ala Ala
        2035                2040                2045

Val Ala Gly Pro Arg Met Pro Asn Val Gln Pro Asn Arg Ser Ile Ser
    2050                2055                2060

Pro Ser Ala Leu Gln Asp Leu Leu Arg Thr Leu Lys Ser Pro Ser Ser
2065                2070                2075                2080

Pro Gln Gln Gln Gln Gln Val Leu Asn Ile Leu Lys Ser Asn Pro Gln
                2085                2090                2095

Leu Met Ala Ala Phe Ile Lys Gln Arg Thr Ala Lys Tyr Val Ala Asn
            2100                2105                2110

Gln Pro Gly Met Gln Pro Gln Pro Gly Leu Gln Ser Gln Pro Gly Met
        2115                2120                2125

Gln Pro Gln Pro Gly Met His Gln Gln Pro Ser Leu Gln Asn Leu Asn
    2130                2135                2140

Ala Met Gln Ala Gly Val Pro Arg Pro Gly Val Pro Pro Pro Gln Pro
2145                2150                2155                2160

Ala Met Gly Gly Leu Asn Pro Gln Gly Gln Ala Leu Asn Ile Met Asn
                2165                2170                2175

Pro Gly His Asn Pro Asn Met Thr Asn Met Asn Pro Gln Tyr Arg Glu
            2180                2185                2190

Met Val Arg Arg Gln Leu Leu Gln His Gln Gln Gln Gln Gln Gln
        2195                2200                2205

Gln Gln Gln Gln Gln Gln Gln Gln Asn Ser Ala Ser Leu Ala Gly Gly
        2210                2215                2220

Met Ala Gly His Ser Gln Phe Gln Gln Pro Gln Gly Pro Gly Gly Tyr
2225                2230                2235                2240

Ala Pro Ala Met Gln Gln Gln Arg Met Gln Gln His Leu Pro Ile Gln
                2245                2250                2255

Gly Ser Ser Met Gly Gln Met Ala Ala Pro Met Gly Gln Leu Gly Gln
            2260                2265                2270

Met Gly Gln Pro Gly Leu Gly Ala Asp Ser Thr Pro Asn Ile Gln Gln
        2275                2280                2285

Ala Leu Gln Gln Arg Ile Leu Gln Gln Gln Met Lys Gln Gln Ile
    2290                2295                2300

Gly Ser Pro Gly Gln Pro Asn Pro Met Ser Pro Gln Gln His Met Leu
2305                2310                2315                2320

Ser Gly Gln Pro Gln Ala Ser His Leu Pro Gly Gln Gln Ile Ala Thr
                2325                2330                2335

Ser Leu Ser Asn Gln Val Arg Ser Pro Ala Pro Val Gln Ser Pro Arg
            2340                2345                2350

Pro Gln Ser Gln Pro Pro His Ser Ser Pro Ser Pro Arg Ile Gln Pro
        2355                2360                2365

Gln Pro Ser Pro His His Val Ser Pro Gln Thr Gly Thr Pro His Pro
    2370                2375                2380

Gly Leu Ala Val Thr Met Ala Ser Ser Met Asp Gln Gly His Leu Gly
2385                2390                2395                2400

Asn Pro Glu Gln Ser Ala Met Leu Pro Gln Leu Asn Thr Pro Asn Arg
                2405                2410                2415

Ser Ala Leu Ser Ser Glu Leu Ser Leu Val Gly Asp Thr Thr Gly Asp
            2420                2425                2430

Thr Leu Glu Lys Phe Val Glu Gly Leu
        2435                2440

<210> 13
<211> 2442
<212> PRT
<213> Homo sapiens

<400> 13
Met Ala Glu Asn Leu Leu Asp Gly Pro Pro Asn Pro Lys Arg Ala Lys
1               5                   10                  15

Leu Ser Ser Pro Gly Phe Ser Ala Asn Asp Ser Thr Asp Phe Gly Ser
            20                  25                  30

Leu Phe Asp Leu Glu Asn Asp Leu Pro Asp Glu Leu Ile Pro Asn Gly
        35                  40                  45

Gly Glu Leu Gly Leu Leu Asn Ser Gly Asn Leu Val Pro Asp Ala Ala
    50                  55                  60

Ser Lys His Lys Gln Leu Ser Glu Leu Leu Arg Gly Gly Ser Gly Ser
65                  70                  75                  80

Ser Ile Asn Pro Gly Ile Gly Asn Val Ser Ala Ser Ser Pro Val Gln
                85                  90                  95

Gln Gly Leu Gly Gly Gln Ala Gln Gly Gln Pro Asn Ser Ala Asn Met
            100                 105                 110

Ala Ser Leu Ser Ala Met Gly Lys Ser Pro Leu Ser Gln Gly Asp Ser
        115                 120                 125

Ser Ala Pro Ser Leu Pro Lys Gln Ala Ala Ser Thr Ser Gly Pro Thr
    130                 135                 140

Pro Ala Ala Ser Gln Ala Leu Asn Pro Gln Ala Gln Lys Gln Val Gly
145                 150                 155                 160

Leu Ala Thr Ser Ser Pro Ala Thr Ser Gln Thr Gly Pro Gly Ile Cys
                165                 170                 175

Met Asn Ala Asn Phe Asn Gln Thr His Pro Gly Leu Leu Asn Ser Asn
            180                 185                 190

Ser Gly His Ser Leu Ile Asn Gln Ala Ser Gln Gly Gln Ala Gln Val
        195                 200                 205

Met Asn Gly Ser Leu Gly Ala Ala Gly Arg Gly Arg Gly Ala Gly Met
    210                 215                 220

Pro Tyr Pro Thr Pro Ala Met Gln Gly Ala Ser Ser Ser Val Leu Ala
225                 230                 235                 240

Glu Thr Leu Thr Gln Val Ser Pro Gln Met Thr Gly His Ala Gly Leu
                245                 250                 255

Asn Thr Ala Gln Ala Gly Gly Met Ala Lys Met Gly Ile Thr Gly Asn
            260                 265                 270

Thr Ser Pro Phe Gly Gln Pro Phe Ser Gln Ala Gly Gly Gln Pro Met
        275                 280                 285

Gly Ala Thr Gly Val Asn Pro Gln Leu Ala Ser Lys Gln Ser Met Val
    290                 295                 300

Asn Ser Leu Pro Thr Phe Pro Thr Asp Ile Lys Asn Thr Ser Val Thr
305                 310                 315                 320

Asn Val Pro Asn Met Ser Gln Met Gln Thr Ser Val Gly Ile Val Pro
                325                 330                 335

Thr Gln Ala Ile Ala Thr Gly Pro Thr Ala Asp Pro Glu Lys Arg Lys
            340                 345                 350

Leu Ile Gln Gln Gln Leu Val Leu Leu Leu His Ala His Lys Cys Gln
        355                 360                 365

Arg Arg Glu Gln Ala Asn Gly Glu Val Arg Ala Cys Ser Leu Pro His
    370                 375                 380

Cys Arg Thr Met Lys Asn Val Leu Asn His Met Thr His Cys Gln Ala
385                 390                 395                 400

Gly Lys Ala Cys Gln Val Ala His Cys Ala Ser Ser Arg Gln Ile Ile
                405                 410                 415

Ser His Trp Lys Asn Cys Thr Arg His Asp Cys Pro Val Cys Leu Pro
            420                 425                 430

Leu Lys Asn Ala Ser Asp Lys Arg Asn Gln Gln Thr Ile Leu Gly Ser
        435                 440                 445

Pro Ala Ser Gly Ile Gln Asn Thr Ile Gly Ser Val Gly Thr Gly Gln
    450                 455                 460

Gln Asn Ala Thr Ser Leu Ser Asn Pro Asn Pro Ile Asp Pro Ser Ser
465                 470                 475                 480

Met Gln Arg Ala Tyr Ala Ala Leu Gly Leu Pro Tyr Met Asn Gln Pro
                485                 490                 495

Gln Thr Gln Leu Gln Pro Gln Val Pro Gly Gln Gln Pro Ala Gln Pro
            500                 505                 510

Gln Thr His Gln Gln Met Arg Thr Leu Asn Pro Leu Gly Asn Asn Pro
        515                 520                 525

Met Asn Ile Pro Ala Gly Gly Ile Thr Thr Asp Gln Gln Pro Pro Asn
    530                 535                 540

Leu Ile Ser Glu Ser Ala Leu Pro Thr Ser Leu Gly Ala Thr Asn Pro
545                 550                 555                 560

Leu Met Asn Asp Gly Ser Asn Ser Gly Asn Ile Gly Thr Leu Ser Thr
                565                 570                 575

Ile Pro Thr Ala Ala Pro Pro Ser Ser Thr Gly Val Arg Lys Gly Trp
            580                 585                 590

His Glu His Val Thr Gln Asp Leu Arg Ser His Leu Val His Lys Leu
        595                 600                 605

Val Gln Ala Ile Phe Pro Thr Pro Asp Pro Ala Ala Leu Lys Asp Arg
    610                 615                 620

Arg Met Glu Asn Leu Val Ala Tyr Ala Lys Lys Val Glu Gly Asp Met
625                 630                 635                 640

Tyr Glu Ser Ala Asn Ser Arg Asp Glu Tyr Tyr His Leu Leu Ala Glu
                645                 650                 655

Lys Ile Tyr Lys Ile Gln Lys Glu Leu Glu Glu Lys Arg Arg Ser Arg
            660                 665                 670

Leu His Lys Gln Gly Ile Leu Gly Asn Gln Pro Ala Leu Pro Ala Pro
        675                 680                 685

Gly Ala Gln Pro Pro Val Ile Pro Gln Ala Gln Pro Val Arg Pro Pro
    690                 695                 700

Asn Gly Pro Leu Ser Leu Pro Val Asn Arg Met Gln Val Ser Gln Gly
705                 710                 715                 720

Met Asn Ser Phe Asn Pro Met Ser Leu Gly Asn Val Gln Leu Pro Gln
                725                 730                 735

Ala Pro Met Gly Pro Arg Ala Ala Ser Pro Met Asn His Ser Val Gln
            740                 745                 750

Met Asn Ser Met Gly Ser Val Pro Gly Met Ala Ile Ser Pro Ser Arg
        755                 760                 765

Met Pro Gln Pro Pro Asn Met Met Gly Ala His Thr Asn Asn Met Met
    770                 775                 780

Ala Gln Ala Pro Ala Gln Ser Gln Phe Leu Pro Gln Asn Gln Phe Pro
785                 790                 795                 800

Ser Ser Ser Gly Ala Met Ser Val Gly Met Gly Gln Pro Pro Ala Gln
                805                 810                 815

Thr Gly Val Ser Gln Gly Gln Val Pro Gly Ala Ala Leu Pro Asn Pro
            820                 825                 830

Leu Asn Met Leu Gly Pro Gln Ala Ser Gln Leu Pro Cys Pro Pro Val
        835                 840                 845

Thr Gln Ser Pro Leu His Pro Thr Pro Pro Pro Ala Ser Thr Ala Ala
    850                 855                 860

Gly Met Pro Ser Leu Gln His Thr Thr Pro Pro Gly Met Thr Pro Pro
865                 870                 875                 880

Gln Pro Ala Ala Pro Thr Gln Pro Ser Thr Pro Val Ser Ser Ser Gly
                885                 890                 895

Gln Thr Pro Thr Pro Thr Pro Gly Ser Val Pro Ser Ala Thr Gln Thr
            900                 905                 910

Gln Ser Thr Pro Thr Val Gln Ala Ala Ala Gln Ala Gln Val Thr Pro
        915                 920                 925

Gln Pro Gln Thr Pro Val Gln Pro Pro Ser Val Ala Thr Pro Gln Ser
    930                 935                 940

Ser Gln Gln Gln Pro Thr Pro Val His Ala Gln Pro Pro Gly Thr Pro
945                 950                 955                 960

Leu Ser Gln Ala Ala Ala Ser Ile Asp Asn Arg Val Pro Thr Pro Ser
                965                 970                 975

Ser Val Ala Ser Ala Glu Thr Asn Ser Gln Gln Pro Gly Pro Asp Val
            980                 985                 990

Pro Val Leu Glu Met Lys Thr Glu Thr Gln Ala Glu Asp Thr Glu Pro
        995                 1000                1005

Asp Pro Gly Glu Ser Lys Gly Glu Pro Arg Ser Glu Met Met Glu Glu
    1010                1015                1020

Asp Leu Gln Gly Ala Ser Gln Val Lys Glu Glu Thr Asp Ile Ala Glu
1025                1030                1035                1040

Gln Lys Ser Glu Pro Met Glu Val Asp Glu Lys Lys Pro Glu Val Lys
                1045                1050                1055

Val Glu Val Lys Glu Glu Glu Glu Ser Ser Ser Asn Gly Thr Ala Ser
            1060                1065                1070

Gln Ser Thr Ser Pro Ser Gln Pro Arg Lys Lys Ile Phe Lys Pro Glu
        1075                1080                1085

Glu Leu Arg Gln Ala Leu Met Pro Thr Leu Glu Ala Leu Tyr Arg Gln
    1090                1095                1100

Asp Pro Glu Ser Leu Pro Phe Arg Gln Pro Val Asp Pro Gln Leu Leu
1105                1110                1115                1120

Gly Ile Pro Asp Tyr Phe Asp Ile Val Lys Asn Pro Met Asp Leu Ser
                1125                1130                1135

Thr Ile Lys Arg Lys Leu Asp Thr Gly Gln Tyr Gln Glu Pro Trp Gln
            1140                1145                1150

Tyr Val Asp Asp Val Trp Leu Met Phe Asn Asn Ala Trp Leu Tyr Asn
        1155                1160                1165

Arg Lys Thr Ser Arg Val Tyr Lys Phe Cys Ser Lys Leu Ala Glu Val
    1170                1175                1180

Phe Glu Gln Glu Ile Asp Pro Val Met Gln Ser Leu Gly Tyr Cys Cys
1185                1190                1195                1200

Gly Arg Lys Tyr Glu Phe Ser Pro Gln Thr Leu Cys Cys Tyr Gly Lys
                1205                1210                1215

Gln Leu Cys Thr Ile Pro Arg Asp Ala Ala Tyr Tyr Ser Tyr Gln Asn
            1220                1225                1230

Arg Tyr His Phe Cys Glu Lys Cys Phe Thr Glu Ile Gln Gly Glu Asn
        1235                1240                1245

Val Thr Leu Gly Asp Asp Pro Ser Gln Pro Gln Thr Thr Ile Ser Lys
    1250                1255                1260

Asp Gln Phe Glu Lys Lys Lys Asn Asp Thr Leu Asp Pro Glu Pro Phe
1265                1270                1275                1280

Val Asp Cys Lys Glu Cys Gly Arg Lys Met His Gln Ile Cys Val Leu
                1285                1290                1295

His Tyr Asp Ile Ile Trp Pro Ser Gly Phe Val Cys Asp Asn Cys Leu
            1300                1305                1310

Lys Lys Thr Gly Arg Pro Arg Lys Glu Asn Lys Phe Ser Ala Lys Arg
        1315                1320                1325

Leu Gln Thr Thr Arg Leu Gly Asn His Leu Glu Asp Arg Val Asn Lys
    1330                1335                1340

Phe Leu Arg Arg Gln Asn His Pro Glu Ala Gly Glu Val Phe Val Arg
1345                1350                1355                1360

Val Val Ala Ser Ser Asp Lys Thr Val Glu Val Lys Pro Gly Met Lys
                1365                1370                1375

Ser Arg Phe Val Asp Ser Gly Glu Met Ser Glu Ser Phe Pro Tyr Arg
            1380                1385                1390

Thr Lys Ala Leu Phe Ala Phe Glu Glu Ile Asp Gly Val Asp Val Cys
        1395                1400                1405

Phe Phe Gly Met His Val Gln Glu Tyr Gly Ser Asp Cys Pro Pro Pro
    1410                1415                1420

Asn Thr Arg Arg Val Tyr Ile Ser Tyr Leu Asp Ser Ile His Phe Phe
1425                1430                1435                1440

Arg Pro Arg Cys Leu Arg Thr Ala Val Tyr His Glu Ile Leu Ile Gly
                1445                1450                1455

Tyr Leu Glu Tyr Val Lys Lys Leu Gly Tyr Val Thr Gly His Ile Trp
            1460                1465                1470

Ala Cys Pro Pro Ser Glu Gly Asp Asp Tyr Ile Phe His Cys His Pro
        1475                1480                1485

Pro Asp Gln Lys Ile Pro Lys Pro Lys Arg Leu Gln Glu Trp Tyr Lys
    1490                1495                1500

Lys Met Leu Asp Lys Ala Phe Ala Glu Arg Ile Ile His Asp Tyr Lys
1505                1510                1515                1520

Asp Ile Phe Lys Gln Ala Thr Glu Asp Arg Leu Thr Ser Ala Lys Glu
                1525                1530                1535

Leu Pro Tyr Phe Glu Gly Asp Phe Trp Pro Asn Val Leu Glu Glu Ser
            1540                1545                1550

Ile Lys Glu Leu Glu Gln Glu Glu Glu Glu Arg Lys Lys Glu Glu Ser
        1555                1560                1565

Thr Ala Ala Ser Glu Thr Thr Glu Gly Ser Gln Gly Asp Ser Lys Asn
    1570                1575                1580

Ala Lys Lys Lys Asn Asn Lys Lys Thr Asn Lys Asn Lys Ser Ser Ile
1585                1590                1595                1600

Ser Arg Ala Asn Lys Lys Lys Pro Ser Met Pro Asn Val Ser Asn Asp
                1605                1610                1615

Leu Ser Gln Lys Leu Tyr Ala Thr Met Glu Lys His Lys Glu Val Phe
            1620                1625                1630

Phe Val Ile His Leu His Ala Gly Pro Val Ile Asn Thr Leu Pro Pro
        1635                1640                1645

Ile Val Asp Pro Asp Pro Leu Leu Ser Cys Asp Leu Met Asp Gly Arg
    1650                1655                1660

Asp Ala Phe Leu Thr Leu Ala Arg Asp Lys His Trp Glu Phe Ser Ser
1665                1670                1675                1680

Leu Arg Arg Ser Lys Trp Ser Thr Leu Cys Met Leu Val Glu Leu His
                1685                1690                1695

Thr Gln Gly Gln Asp Arg Phe Val Tyr Thr Cys Asn Glu Cys Lys His
            1700                1705                1710

His Val Glu Thr Arg Trp His Cys Thr Val Cys Glu Asp Tyr Asp Leu
        1715                1720                1725

Cys Ile Asn Cys Tyr Asn Thr Lys Ser His Ala His Lys Met Val Lys
    1730                1735                1740

Trp Gly Leu Gly Leu Asp Asp Glu Gly Ser Ser Gln Gly Glu Pro Gln
1745                1750                1755                1760

Ser Lys Ser Pro Gln Glu Ser Arg Arg Val Ser Ile Gln Arg Cys Ile
                1765                1770                1775

Gln Ser Leu Val His Ala Cys Gln Cys Arg Asn Ala Asn Cys Ser Leu
            1780                1785                1790

Pro Ser Cys Gln Lys Met Lys Arg Val Val Gln His Thr Lys Gly Cys
        1795                1800                1805

Lys Arg Lys Thr Asn Gly Gly Cys Pro Val Cys Lys Gln Leu Ile Ala
    1810                1815                1820

Leu Cys Cys Tyr His Ala Lys His Cys Gln Glu Asn Lys Cys Pro Val
1825                1830                1835                1840

Pro Phe Cys Leu Asn Ile Lys His Lys Leu Arg Gln Gln Gln Ile Gln
                1845                1850                1855

His Arg Leu Gln Gln Ala Gln Leu Met Arg Arg Arg Met Ala Thr Met
            1860                1865                1870

Asn Thr Arg Asn Val Pro Gln Gln Ser Leu Pro Ser Pro Thr Ser Ala
        1875                1880                1885

Pro Pro Gly Thr Pro Thr Gln Gln Pro Ser Thr Pro Gln Thr Pro Gln
    1890                1895                1900

Pro Pro Ala Gln Pro Gln Pro Ser Pro Val Ser Met Ser Pro Ala Gly
1905                1910                1915                1920

Phe Pro Ser Val Ala Arg Thr Gln Pro Pro Thr Thr Val Ser Thr Gly
                1925                1930                1935

Lys Pro Thr Ser Gln Val Pro Ala Pro Pro Pro Pro Ala Gln Pro Pro
            1940                1945                1950

Pro Ala Ala Val Glu Ala Ala Arg Gln Ile Glu Arg Glu Ala Gln Gln
        1955                1960                1965

Gln Gln His Leu Tyr Arg Val Asn Ile Asn Asn Ser Met Pro Pro Gly
    1970                1975                1980

Arg Thr Gly Met Gly Thr Pro Gly Ser Gln Met Ala Pro Val Ser Leu
1985                1990                1995                2000

Asn Val Pro Arg Pro Asn Gln Val Ser Gly Pro Val Met Pro Ser Met
                2005                2010                2015

Pro Pro Gly Gln Trp Gln Gln Ala Pro Leu Pro Gln Gln Gln Pro Met
            2020                2025                2030

Pro Gly Leu Pro Arg Pro Val Ile Ser Met Gln Ala Gln Ala Ala Val
        2035                2040                2045

Ala Gly Pro Arg Met Pro Ser Val Gln Pro Pro Arg Ser Ile Ser Pro
    2050                2055                2060

Ser Ala Leu Gln Asp Leu Leu Arg Thr Leu Lys Ser Pro Ser Ser Pro
2065                2070                2075                2080

Gln Gln Gln Gln Gln Val Leu Asn Ile Leu Lys Ser Asn Pro Gln Leu
                2085                2090                2095

Met Ala Ala Phe Ile Lys Gln Arg Thr Ala Lys Tyr Val Ala Asn Gln
            2100                2105                2110

Pro Gly Met Gln Pro Gln Pro Gly Leu Gln Ser Gln Pro Gly Met Gln
        2115                2120                2125

Pro Gln Pro Gly Met His Gln Gln Pro Ser Leu Gln Asn Leu Asn Ala
    2130                2135                2140

Met Gln Ala Gly Val Pro Arg Pro Gly Val Pro Pro Gln Gln Gln Ala
2145                2150                2155                2160

Met Gly Gly Leu Asn Pro Gln Gly Gln Ala Leu Asn Ile Met Asn Pro
                2165                2170                2175

Gly His Asn Pro Asn Met Ala Ser Met Asn Pro Gln Tyr Arg Glu Met
            2180                2185                2190

Leu Arg Arg Gln Leu Leu Gln Gln Gln Gln Gln Gln Gln Gln Gln Gln
        2195                2200                2205

Gln Gln Gln Gln Gln Gln Gln Gln Gly Ser Ala Gly Met Ala Gly Gly
    2210                2215                2220

Met Ala Gly His Gly Gln Phe Gln Gln Pro Gln Gly Pro Gly Gly Tyr
2225                2230                2235                2240

Pro Pro Ala Met Gln Gln Gln Gln Arg Met Gln Gln His Leu Pro Leu
                2245                2250                2255

Gln Gly Ser Ser Met Gly Gln Met Ala Ala Gln Met Gly Gln Leu Gly
            2260                2265                2270

Gln Met Gly Gln Pro Gly Leu Gly Ala Asp Ser Thr Pro Asn Ile Gln
        2275                2280                2285

Gln Ala Leu Gln Gln Arg Ile Leu Gln Gln Gln Gln Met Lys Gln Gln
    2290                2295                2300

Ile Gly Ser Pro Gly Gln Pro Asn Pro Met Ser Pro Gln Gln His Met
2305                2310                2315                2320

Leu Ser Gly Gln Pro Gln Ala Ser His Leu Pro Gly Gln Gln Ile Ala
                2325                2330                2335

Thr Ser Leu Ser Asn Gln Val Arg Ser Pro Ala Pro Val Gln Ser Pro
            2340                2345                2350

Arg Pro Gln Ser Gln Pro Pro His Ser Ser Pro Ser Pro Arg Ile Gln
        2355                2360                2365

Pro Gln Pro Ser Pro His His Val Ser Pro Gln Thr Gly Ser Pro His
    2370                2375                2380

Pro Gly Leu Ala Val Thr Met Ala Ser Ser Ile Asp Gln Gly His Leu
2385                2390                2395                2400

Gly Asn Pro Glu Gln Ser Ala Met Leu Pro Gln Leu Asn Thr Pro Ser
                2405                2410                2415

Arg Ser Ala Leu Ser Ser Glu Leu Ser Leu Val Gly Asp Thr Thr Gly
            2420                2425                2430

Asp Thr Leu Glu Lys Phe Val Glu Gly Leu
        2435                2440

<210> 14
<211> 324
<212> PRT
<213> Mus musculus

<300>
<301> Metzger, R.
<302> Expression of the mouse  and rat proto-oncogene in the
      brain and peripheral tissues
<303> FEBS Lett.
<304> 1995
<305> 3570
<306> 27-32
<313> 1 - 324

<400> 14
Met Asp Gln Ser Asn Met Thr Ser Leu Ala Glu Glu Lys Ala Met Asn
  1               5                  10                  15

Thr Ser Ser Arg Asn Ala Ser Leu Gly Ser Ser His Pro Pro Ile Pro
            20                  25                  30

Ile Val His Trp Val Ile Met Ser Ile Ser Pro Leu Gly Phe Val Glu
        35                  40                  45

Asn Gly Ile Leu Leu Trp Phe Leu Cys Phe Arg Met Arg Arg Asn Pro
    50                  55                  60

Phe Thr Val Tyr Ile Thr His Leu Ser Met Ala Asp Ile Ser Leu Leu
65                  70                  75                  80

Phe Cys Ile Phe Ile Leu Ser Thr Asp Tyr Ala Leu Asp Tyr Glu Leu
                85                  90                  95

Ser Ser Gly His His Tyr Thr Ile Val Thr Leu Ser Val Thr Phe Leu
            100                 105                 110

Phe Gly Tyr Asn Thr Gly Leu Tyr Leu Leu Thr Ala Ile Ser Val Glu
        115                 120                 125

Arg Cys Leu Ser Val Leu Tyr Pro Ile Trp Tyr Thr Ser His Arg Pro
    130                 135                 140

Lys His Gln Ser Ala Phe Val Cys Ala Leu Leu Cys Ala Leu Ser Cys
145                 150                 155                 160

Leu Val Thr Thr Met Glu Tyr Val Met Cys Ile Asp Ser Gly Glu Glu
                165                 170                 175

Ser His Ser Arg Ser Asp Cys Arg Ala Val Ile Ile Phe Ile Ala Ile
            180                 185                 190

Leu Ser Phe Leu Val Phe Thr Pro Leu Met Leu Val Ser Ser Ser Ile
        195                 200                 205

Leu Val Val Lys Ile Arg Lys Asn Thr Trp Ala Ser His Ser Ser Lys
    210                 215                 220

Leu Tyr Ile Val Ile Met Val Thr Ile Ile Ile Phe Leu Ile Phe Ala
225                 230                 235                 240

Met Pro Met Arg Val Leu Tyr Leu Leu Tyr Tyr Glu Tyr Trp Ser Ala
                245                 250                 255

Phe Gly Asn Leu His Asn Ile Ser Leu Leu Phe Ser Thr Ile Asn Ser
            260                 265                 270

Ser Ala Asn Pro Phe Ile Tyr Phe Phe Val Gly Ser Ser Lys Lys Lys
        275                 280                 285

Arg Phe Arg Glu Ser Leu Lys Val Val Leu Thr Arg Ala Phe Lys Asp
    290                 295                 300

Glu Met Gln Pro Arg Arg Gln Glu Gly Asn Gly Asn Thr Val Ser Ile
305                 310                 315                 320

Glu Thr Val Val

<210> 15
<211> 378
<212> PRT
<213> Homo sapiens

<400> 15
Met Val Trp Gly Lys Ile Cys Trp Phe Ser Gln Arg Ala Gly Trp Thr
1               5                   10                  15

Val Phe Ala Glu Ser Gln Ile Ser Leu Ser Cys Ser Leu Cys Leu His
            20                  25                  30

Ser Gly Asp Gln Glu Ala Gln Asn Pro Asn Leu Val Ser Gln Leu Cys
        35                  40                  45

Gly Val Phe Leu Gln Asn Glu Thr Asn Glu Thr Ile His Met Gln Met
    50                  55                  60

Ser Met Ala Val Gly Gln Gln Ala Leu Pro Leu Asn Ile Ile Ala Pro
65                  70                  75                  80

Lys Ala Val Leu Val Ser Leu Cys Gly Val Leu Leu Asn Gly Thr Val
                85                  90                  95

Phe Trp Leu Leu Cys Cys Gly Ala Thr Asn Pro Tyr Met Val Tyr Ile
            100                 105                 110

Leu His Leu Val Ala Ala Asp Val Ile Tyr Leu Cys Cys Ser Ala Val
        115                 120                 125

Gly Phe Leu Gln Val Thr Leu Leu Thr Tyr His Gly Val Val Phe Phe
    130                 135                 140

Ile Pro Asp Phe Leu Ala Ile Leu Ser Pro Phe Ser Phe Glu Val Cys
145                 150                 155                 160

Leu Cys Leu Leu Val Ala Ile Ser Thr Glu Arg Cys Val Cys Val Leu
                165                 170                 175

Phe Pro Ile Trp Tyr Arg Cys His Arg Pro Lys Tyr Thr Ser Asn Val
            180                 185                 190

Val Cys Thr Leu Ile Trp Gly Leu Pro Phe Cys Ile Asn Ile Val Lys
        195                 200                 205

Ser Leu Phe Leu Thr Tyr Trp Lys His Val Lys Ala Cys Val Ile Phe
    210                 215                 220

Leu Lys Leu Ser Gly Leu Phe His Ala Ile Leu Ser Leu Val Met Cys
225                 230                 235                 240

Val Ser Ser Leu Thr Leu Leu Ile Arg Phe Leu Cys Cys Ser Gln Gln
                245                 250                 255

Gln Lys Ala Thr Arg Val Tyr Ala Val Val Gln Ile Ser Ala Pro Met
            260                 265                 270

Phe Leu Leu Trp Ala Leu Pro Leu Ser Val Ala Pro Leu Ile Thr Asp
        275                 280                 285

Phe Lys Met Phe Val Thr Thr Ser Tyr Leu Ile Ser Leu Phe Leu Ile
    290                 295                 300

Ile Asn Ser Ser Ala Asn Pro Ile Ile Tyr Phe Phe Val Gly Ser Leu
305                 310                 315                 320

Arg Lys Lys Arg Leu Lys Glu Ser Leu Arg Val Ile Leu Gln Arg Ala
                325                 330                 335

Leu Ala Asp Lys Pro Glu Val Gly Arg Asn Lys Lys Ala Ala Gly Ile
            340                 345                 350

Asp Pro Met Glu Gln Pro His Ser Thr Gln His Val Glu Asn Leu Leu
        355                 360                 365

Pro Arg Glu His Arg Val Asp Val Glu Thr
    370                 375

<210> 16
<211> 732
<212> PRT
<213> Homo sapiens

<400> 16
Met Glu Arg Gln Val Leu Leu Ser Glu Pro Glu Glu Ala Ala Ala Leu
1               5                   10                  15

Tyr Arg Gly Leu Ser Arg Gln Pro Ala Leu Ser Ala Ala Cys Leu Gly
            20                  25                  30

Pro Glu Val Thr Thr Gln Tyr Gly Gly Gln Tyr Arg Thr Val His Thr
        35                  40                  45

Glu Trp Thr Gln Arg Asp Leu Glu Arg Met Glu Asn Ile Arg Phe Cys
    50                  55                  60

Arg Gln Tyr Leu Val Phe His Asp Gly Asp Ser Val Val Phe Ala Gly
65                  70                  75                  80

Pro Ala Gly Asn Ser Val Glu Thr Arg Gly Glu Leu Leu Ser Arg Glu
                85                  90                  95

Ser Pro Ser Gly Ser Met Lys Ala Val Leu Arg Lys Ala Gly Gly Thr
            100                 105                 110

Gly Pro Gly Glu Glu Lys Gln Phe Leu Glu Val Trp Glu Lys Asn Arg
        115                 120                 125

Lys Leu Lys Ser Phe Asn Leu Ser Val Leu Glu Lys His Gly Pro Val
    130                 135                 140

Tyr Glu Asp Asp Cys Phe Gly Cys Leu Ser Trp Ser His Ser Glu Thr
145                 150                 155                 160

His Leu Leu Tyr Val Ala Glu Arg Lys Arg Pro Lys Ala Glu Ser Phe
                165                 170                 175

Phe Gln Thr Lys Ala Leu Asp Val Ser Ala Ser Asp Asp Glu Ile Ala
            180                 185                 190

Arg Leu Lys Lys Pro Asp Gln Pro Ile Lys Gly Asp Gln Phe Val Phe
        195                 200                 205

Tyr Glu Asp Trp Gly Glu Asn Met Val Ser Lys Ser Ile Pro Val Leu
    210                 215                 220

Cys Val Leu Asp Val Glu Ser Gly Asn Ile Ser Val Leu Glu Gly Val
225                 230                 235                 240

Pro Glu Asn Val Ser Pro Gly Gln Ala Phe Trp Ala Pro Gly Asp Ala

                245                 250                 255

Gly Val Val Phe Val Gly Trp Trp His Glu Pro Phe Arg Leu Gly Ile
            260                 265                 270

Arg Phe Cys Thr Asn Arg Arg Ser Ala Leu Tyr Tyr Val Asp Leu Ile
        275                 280                 285

Gly Gly Lys Cys Glu Leu Leu Ser Asp Asp Ser Leu Ala Val Ser Ser
    290                 295                 300

Pro Arg Leu Ser Pro Asp Gln Cys Arg Ile Val Tyr Leu Gln Tyr Pro
305                 310                 315                 320

Ser Leu Ile Pro His His Gln Cys Ser Gln Leu Cys Leu Tyr Asp Trp
                325                 330                 335

Tyr Thr Lys Val Thr Ser Val Val Val Asp Val Val Pro Arg Gln Leu
            340                 345                 350

Gly Glu Asn Phe Ser Gly Ile Tyr Cys Ser Leu Leu Pro Leu Gly Cys
        355                 360                 365

Trp Ser Ala Asp Ser Gln Arg Val Val Phe Asp Ser Ala Gln Arg Ser
    370                 375                 380

Arg Gln Asp Leu Phe Ala Val Asp Thr Gln Val Gly Thr Val Thr Ser
385                 390                 395                 400

Leu Thr Ala Gly Gly Ser Gly Gly Ser Trp Lys Leu Leu Thr Ile Asp
                405                 410                 415

Gln Asp Leu Met Val Ala Gln Phe Ser Thr Pro Ser Leu Pro Pro Thr
            420                 425                 430

Leu Lys Val Gly Phe Leu Pro Ser Ala Gly Lys Glu Gln Ser Val Leu
        435                 440                 445

Trp Val Ser Leu Glu Glu Ala Glu Pro Ile Pro Asp Ile His Trp Gly
    450                 455                 460

Ile Arg Val Leu Gln Pro Pro Pro Glu Gln Glu Asn Val Gln Tyr Ala
465                 470                 475                 480

Gly Leu Asp Phe Glu Ala Ile Leu Leu Gln Pro Gly Ser Pro Pro Asp
                485                 490                 495

Lys Thr Gln Val Pro Met Val Val Met Pro His Gly Gly Pro His Ser

500                 505                 510

Ser Phe Val Thr Ala Trp Met Leu Phe Pro Ala Met Leu Cys Lys Met
        515                 520                 525

Gly Phe Ala Val Leu Leu Val Asn Tyr Arg Gly Ser Thr Gly Phe Gly
    530                 535                 540

Gln Asp Ser Ile Leu Ser Leu Pro Gly Asn Val Gly His Gln Asp Val
545                 550                 555                 560

Lys Asp Val Gln Phe Ala Val Glu Gln Val Leu Gln Glu Glu His Phe
                565                 570                 575

Asp Ala Ser His Val Ala Leu Met Gly Gly Ser His Gly Gly Phe Ile
            580                 585                 590

Ser Cys His Leu Ile Gly Gln Tyr Pro Glu Thr Tyr Arg Ala Cys Val
        595                 600                 605

Ala Arg Asn Pro Val Ile Asn Ile Ala Ser Met Leu Gly Ser Thr Asp
    610                 615                 620

Ile Pro Asp Trp Cys Val Val Glu Ala Gly Phe Pro Phe Ser Ser Asp
625                 630                 635                 640

Cys Leu Pro Asp Leu Ser Val Trp Ala Glu Met Leu Asp Lys Ser Pro
                645                 650                 655

Ile Arg Tyr Ile Pro Gln Val Lys Thr Pro Leu Leu Leu Met Leu Gly
            660                 665                 670

Gln Glu Asp Arg Arg Val Pro Phe Lys Gln Gly Met Glu Tyr Tyr Arg
        675                 680                 685

Ala Leu Lys Thr Arg Asn Val Pro Val Arg Leu Leu Leu Tyr Pro Lys
    690                 695                 700

Ser Thr His Ala Leu Ser Glu Val Glu Val Glu Ser Asp Ser Phe Met
705                 710                 715                 720

Asn Ala Val Leu Trp Leu Arg Thr His Leu Gly Ser
                725                 730

<210> 17
<211> 400
<212> PRT

<213> Homo sapiens

<300>
<301> Duprez, ER.
<302> Direct Submissin
<303> Inserm U-301
<304> 1996
<309> 1996-11-26

<400> 17
Met Thr Thr Lys Asn Leu Glu Thr Lys Val Thr Val Thr Ser Ser Pro
1               5                   10                  15

Ile Arg Gly Ala Gly Asp Gly Met Glu Thr Glu Glu Pro Pro Lys Ser
            20                  25                  30

Val Glu Val Thr Ser Gly Val Gln Ser Arg Lys His His Ser Leu Gln
        35                  40                  45

Ser Pro Trp Lys Lys Ala Val Pro Ser Glu Ser Pro Gly Val Leu Gln
    50                  55                  60

Leu Gly Lys Met Leu Thr Glu Lys Ala Met Glu Val Lys Ala Val Arg
65                  70                  75                  80

Ile Leu Val Pro Lys Ala Ala Ile Thr His Asp Ile Pro Asn Lys Asn
                85                  90                  95

Thr Lys Val Lys Ser Leu Gly His His Lys Gly Glu Phe Leu Gly Gln
            100                 105                 110

Ser Glu Gly Val Ile Glu Pro Asn Lys Glu Leu Ser Glu Val Lys Asn
        115                 120                 125

Val Leu Glu Lys Leu Lys Asn Ser Glu Arg Arg Leu Leu Gln Asp Lys
    130                 135                 140

Glu Gly Leu Ser Asn Gln Leu Arg Val Gln Thr Glu Val Asn Arg Glu
145                 150                 155                 160

Leu Lys Lys Leu Leu Val Ala Ser Val Gly Asp Asp Leu Gln Tyr His
                165                 170                 175

Phe Glu Arg Leu Ala Arg Glu Lys Asn Gln Leu Ile Leu Glu Asn Glu
            180                 185                 190

Ala Leu Gly Arg Asn Thr Ala Gln Leu Ser Glu Gln Leu Glu Arg Met
        195                 200                 205

Ser Ile Gln Cys Asp Val Trp Arg Ser Lys Phe Leu Ala Ser Arg Val
    210                 215                 220

Met Ala Asp Glu Leu Thr Asn Ser Arg Ala Ala Leu Gln Arg Gln Asn
225                 230                 235                 240

Arg Asp Ala His Gly Ala Ile Gln Asp Leu Leu Ser Glu Arg Glu Gln
                245                 250                 255

Phe Arg Gln Glu Met Ile Ala Thr Gln Lys Leu Leu Glu Glu Leu Leu
            260                 265                 270

Val Ser Leu Gln Trp Gly Arg Glu Gln Thr Tyr Ser Pro Ser Val Gln
        275                 280                 285

Pro His Ser Thr Ala Glu Leu Ala Leu Thr Asn His Lys Leu Ala Lys
    290                 295                 300

Ala Val Asn Ser His Leu Leu Gly Asn Val Gly Ile Asn Asn Gln Lys
305                 310                 315                 320

Lys Ile Pro Ser Thr Val Glu Phe Cys Ser Thr Pro Ala Glu Lys Met
                325                 330                 335

Ala Glu Thr Val Leu Arg Ile Leu Asp Pro Val Thr Cys Lys Glu Ser
            340                 345                 350

Ser Pro Asp Asn Pro Phe Phe Glu Ser Ser Pro Thr Thr Leu Leu Ala
        355                 360                 365

Thr Lys Lys Asn Ile Gly Arg Phe His Pro Tyr Thr Arg Tyr Glu Asn
    370                 375                 380

Ile Thr Phe Asn Cys Cys Asn His Cys Arg Gly Glu Leu Ile Ala Leu
385                 390                 395                 400

<210> 18
<211> 319
<212> PRT
<213> Mus musculus

<300>
<301> Zou, Y.

<302> CARP, a cardiac ankyrin repea protein, is downstream in
      the Nkx2-5 homeobox gene pathway
<303> Development
<304> 1996
<305> 124
<306> 793-804
<313> 1 - 319

<400> 18
Met Met Val Leu Arg Val Glu Glu Leu Val Thr Gly Lys Lys Asn Ser
1               5                   10                  15

Asn Gly Ala Ala Gly Glu Phe Leu Pro Gly Glu Phe Arg Asn Gly Glu
            20                  25                  30

Tyr Glu Ala Ala Val Ala Leu Glu Lys Gln Glu Asp Leu Lys Thr Leu
        35                  40                  45

Pro Ala Asn Ser Val Lys Gln Gly Glu Glu Gln Arg Lys Ser Glu Lys
    50                  55                  60

Leu Arg Glu Ala Glu Leu Lys Lys Lys Lys Leu Glu Gln Arg Ser Lys
65                  70                  75                  80

Leu Glu Asn Leu Glu Asp Leu Glu Ile Ile Val Gln Leu Lys Lys Arg
                85                  90                  95

Lys Lys Tyr Lys Lys Thr Lys Val Pro Val Val Lys Glu Pro Glu Pro
            100                 105                 110

Glu Ile Met Thr Glu Pro Val Asp Val Pro Arg Phe Leu Lys Ala Ala
        115                 120                 125

Leu Glu Asn Lys Leu Pro Val Val Glu Lys Leu Val Ser Asp Lys Asn
    130                 135                 140

Ser Pro Asp Val Cys Asp Glu Tyr Lys Arg Thr Ala Leu His Arg Ala
145                 150                 155                 160

Cys Leu Glu Gly His Leu Ala Ile Val Glu Lys Leu Met Glu Ala Gly
                165                 170                 175

Ala Gln Ile Glu Phe Arg Asp Met Leu Glu Ser Thr Ala Ile His Trp
            180                 185                 190

Ala Cys Arg Gly Gly Asn Ala Asp Val Leu Lys Leu Leu Leu Asn Lys
        195                 200                 205

Gly Ala Lys Ile Ser Ala Arg Asp Lys Leu Leu Ser Thr Ala Leu His
    210                 215                 220

Val Ala Val Arg Thr Gly His Tyr Glu Cys Ala Glu His Leu Ile Ala
225                 230                 235                 240

Cys Glu Ala Asp Leu Asn Ala Lys Asp Arg Glu Gly Asp Thr Pro Leu
                245                 250                 255

His Asp Ala Val Arg Leu Asn Arg Tyr Lys Met Ile Arg Leu Leu Met
            260                 265                 270

Thr Phe Gly Ala Asp Leu Lys Val Lys Asn Cys Ala Gly Lys Thr Pro
        275                 280                 285

Met Asp Leu Val Leu His Trp Gln Ser Gly Thr Lys Ala Ile Phe Asp
    290                 295                 300

Ser Pro Lys Glu Asn Ala Tyr Lys Asn Ser Arg Ile Ala Thr Phe
305                 310                 315

<210> 19
<211> 319
<212> PRT
<213> Homo sapiens

<400> 19
Met Met Val Leu Lys Val Glu Glu Leu Val Thr Gly Lys Lys Asn Gly
  1               5                  10                  15

Asn Gly Glu Ala Gly Glu Phe Leu Pro Glu Asp Phe Arg Asp Gly Glu
             20                  25                  30

Tyr Glu Ala Ala Val Thr Leu Glu Lys Gln Glu Asp Leu Lys Thr Leu
         35                  40                  45

Leu Ala His Pro Val Thr Leu Gly Glu Gln Gln Trp Lys Ser Glu Lys
     50                  55                  60

Gln Arg Glu Ala Glu Leu Pro Lys Lys Lys Leu Glu Gln Arg Ser Lys
 65                  70                  75                  80

Leu Glu Asn Leu Glu Asp Leu Glu Ile Ile Ile Gln Leu Lys Lys Arg
                 85                  90                  95

Lys Lys Tyr Arg Lys Thr Lys Val Pro Val Val Lys Glu Pro Glu Pro
            100                 105                 110

Glu Ile Ile Thr Glu Pro Val Asp Val Pro Thr Phe Leu Lys Ala Ala
        115                 120                 125

Leu Glu Asn Lys Leu Pro Val Val Glu Lys Phe Leu Ser Asp Lys Asn
    130                 135                 140

Asn Pro Asp Val Cys Asp Glu Tyr Lys Arg Thr Ala Leu His Arg Ala
145                 150                 155                 160

Cys Leu Glu Gly His Leu Ala Ile Val Glu Lys Leu Met Glu Ala Gly
                165                 170                 175

Ala Gln Ile Glu Phe Arg Asp Met Leu Glu Ser Thr Ala Ile His Trp
            180                 185                 190

Ala Ser Arg Gly Gly Asn Leu Asp Val Leu Lys Leu Leu Leu Asn Lys
        195                 200                 205

Gly Ala Lys Ile Ser Ala Arg Asp Lys Leu Leu Ser Thr Ala Leu His
    210                 215                 220

Val Ala Val Arg Thr Gly His Tyr Glu Cys Ala Glu His Leu Ile Ala
225                 230                 235                 240

Cys Glu Ala Asp Leu Asn Ala Lys Asp Arg Glu Gly Asp Thr Pro Leu
                245                 250                 255

His Asp Ala Val Arg Leu Asn Arg Tyr Lys Met Ile Arg Leu Leu Ile
            260                 265                 270

Met Tyr Gly Ala Asp Leu Asn Ile Lys Asn Cys Ala Gly Lys Thr Pro
        275                 280                 285

Met Asp Leu Val Leu His Trp Gln Asn Gly Thr Lys Ala Ile Phe Asp
    290                 295                 300

Ser Leu Arg Glu Asn Ser Tyr Lys Thr Ser Arg Ile Ala Thr Phe
305                 310                 315

<210> 20
<211> 490
<212> PRT
<213> Homo sapiens

<300>
<301> Field, L. L.

<302> Susceptibility to insulin-dependent diabetes mellitus
      maps to a locus (IDDM11) on human chromosome
      14q24.3-q31
<303> Genomics
<304> 1996
<305> 33
<306> 1-8
<313> 1 - 490

<400> 20
Met Gly Pro Val Met Pro Pro Ser Lys Lys Pro Glu Ser Ser Gly Ile
1               5                   10                  15

Ser Val Ser Ser Gly Leu Ser Gln Cys Tyr Gly Gly Ser Gly Phe Ser
            20                  25                  30

Lys Ala Leu Gln Glu Asp Asp Asp Leu Asp Phe Ser Leu Pro Asp Ile
        35                  40                  45

Arg Leu Glu Glu Gly Ala Met Glu Asp Glu Glu Leu Thr Asn Leu Asn
    50                  55                  60

Trp Leu His Glu Ser Lys Asn Leu Leu Lys Ser Phe Gly Glu Ser Val
65                  70                  75                  80

Leu Arg Ser Val Ser Pro Val Gln Asp Leu Asp Asp Asp Thr Pro Pro
                85                  90                  95

Ser Pro Ala His Ser Asp Met Pro Tyr Asp Ala Arg Gln Asn Pro Asn
            100                 105                 110

Cys Lys Pro Pro Tyr Ser Phe Ser Cys Leu Ile Phe Met Ala Ile Glu
        115                 120                 125

Asp Ser Pro Thr Lys Arg Leu Pro Val Lys Asp Ile Tyr Asn Trp Ile
    130                 135                 140

Leu Glu His Phe Pro Tyr Phe Ala Asn Ala Pro Thr Gly Trp Lys Asn
145                 150                 155                 160

Ser Val Arg His Asn Leu Ser Leu Asn Lys Cys Phe Lys Lys Val Asp
                165                 170                 175

Lys Glu Arg Ser Gln Ser Ile Gly Lys Gly Ser Leu Trp Cys Ile Asp
            180                 185                 190

Pro Glu Tyr Arg Gln Asn Leu Ile Gln Ala Leu Lys Lys Thr Pro Tyr
        195                 200                 205

His Pro His Pro His Val Phe Asn Thr Pro Pro Thr Cys Pro Gln Ala
    210                 215                 220

Tyr Gln Ser Thr Ser Gly Pro Pro Ile Trp Pro Gly Ser Thr Phe Phe
225                 230                 235                 240

Lys Arg Asn Gly Ala Leu Leu Gln Asp Pro Asp Ile Asp Ala Ala Ser
                245                 250                 255

Ala Met Met Leu Leu Asn Thr Pro Pro Glu Ile Gln Ala Gly Phe Pro
            260                 265                 270

Pro Gly Val Ile Gln Asn Gly Ala Arg Val Leu Ser Arg Gly Leu Phe
        275                 280                 285

Pro Gly Val Arg Pro Leu Pro Ile Thr Pro Ile Gly Val Thr Ala Ala
    290                 295                 300

Met Arg Asn Gly Ile Thr Ser Cys Arg Met Arg Thr Glu Ser Glu Pro
305                 310                 315                 320

Ser Cys Gly Ser Pro Val Val Ser Gly Asp Pro Lys Glu Asp His Asn
                325                 330                 335

Tyr Ser Ser Ala Lys Ser Ser Asn Ala Arg Ser Thr Ser Pro Thr Ser
            340                 345                 350

Asp Ser Ile Ser Ser Ser Ser Ser Ser Ala Asp Asp His Tyr Glu Phe
        355                 360                 365

Ala Thr Lys Gly Ser Gln Glu Gly Ser Glu Gly Ser Glu Gly Ser Phe
    370                 375                 380

Arg Ser His Glu Ser Pro Ser Asp Thr Glu Glu Asp Asp Arg Lys His
385                 390                 395                 400

Ser Gln Lys Glu Pro Lys Asp Ser Leu Gly Asp Ser Gly Tyr Ala Ser
                405                 410                 415

Gln His Lys Lys Arg Gln His Phe Ala Lys Ala Arg Lys Val Pro Ser
            420                 425                 430

Asp Thr Leu Pro Leu Lys Lys Arg Arg Thr Glu Lys Pro Pro Glu Ser
        435                 440                 445

Asp Asp Glu Glu Met Lys Glu Ala Ala Gly Ser Leu Leu His Leu Ala
    450                 455                 460

Gly Ile Arg Ser Cys Leu Asn Asn Ile Thr Asn Arg Thr Ala Lys Gly
465                 470                 475                 480

Gln Lys Glu Gln Lys Glu Thr Thr Lys Asn
                485                 490

<210> 21
<211> 212
<212> PRT
<213> Mus musculus

<300>
<301> Laufer, W.
<302> GTP-binding protein (rab2)

<400> 21
Met Ala Tyr Ala Tyr Leu Phe Lys Tyr Ile Ile Ile Gly Asp Thr Gly
  1               5                  10                  15

Val Gly Lys Ser Cys Leu Leu Leu Gln Phe Thr Asp Lys Arg Phe Gln
             20                  25                  30

Pro Val His Asp Leu Thr Ile Gly Val Glu Phe Gly Ala Arg Met Ile
         35                  40                  45

Thr Ile Asp Gly Lys Gln Ile Lys Leu Gln Ile Trp Asp Thr Ala Gly
     50                  55                  60

Gln Glu Ser Phe Arg Ser Ile Thr Arg Ser Tyr Tyr Arg Gly Ala Ala
 65                  70                  75                  80

Gly Ala Leu Leu Val Tyr Asp Ile Thr Arg Arg Asp Thr Phe Asn His
                 85                  90                  95

Leu Thr Thr Trp Leu Glu Asp Ala Arg Gln His Ser Asn Ser Asn Met
            100                 105                 110

Val Ile Met Leu Ile Gly Asn Lys Ser Asp Leu Glu Ser Arg Arg Glu
        115                 120                 125

Val Lys Lys Glu Glu Gly Glu Ala Phe Ala Arg Glu His Gly Leu Ile
    130                 135                 140

Phe Met Glu Thr Ser Ala Lys Thr Ala Ser Asn Val Glu Glu Ala Phe
145                 150                 155                 160

Ile Asn Thr Ala Lys Glu Ile Tyr Glu Lys Ile Gln Glu Gly Val Phe
                165                 170                 175

Asp Ile Asn Asn Glu Ala Asn Gly Ile Lys Ile Gly Pro Gln His Ala
            180                 185                 190

Ala Thr Asn Ala Ser His Gly Ser Asn Gln Gly Gly Gln Gln Ala Gly
        195                 200                 205

Gly Gly Cys Cys
    210

<210> 22
<211> 212
<212> PRT
<213> Homo sapiens

<300>
<301> Tachibana, K.
<302> Nucleotide sequence of a new YPT1-related human cDNA
      which belongs to the ras gene superfamily
<303> Nucleic Acids Res.
<304> 1988
<305> 16
<306> 10368-
<313> 1 - 212

<400> 22

<210> 23
<211> 1712
<212> PRT
<213> Homo sapiens

<300>
<301> Fontoura, B. M.
<302> A conserved biogenesis pathway for nucleoporins:
proteolytic processing of a 186-kilodalton precursor
generates Nup98 and the novel nucleoporin , nup96
<303> J. Cell Biol.
<304> 1999
<305> 144
<306> ^1097-1112
<313> 1 - 1712

<400> 23

<210> 24
<211> 599
<212> PRT
<213> Mus musculus

<300>
<301> De Coignac, A. B.
<302> cDNA cloning and expression analysis of the murine
      ribonuclease L inhibitor
<303> Gene
<304> 1998
<305> 209
<306> 149-156
<313> 1 - 599

<400> 24
Met Ala Asp Lys Leu Thr Arg Ile Ala Ile Val Asn His Asp Lys Cys
1               5                   10                  15

Lys Pro Lys Lys Cys Arg Gln Glu Cys Lys Lys Ser Cys Pro Val Val
            20                  25                  30

Arg Met Gly Lys Leu Cys Ile Glu Val Thr Pro Gln Ser Lys Ile Ala
        35                  40                  45

Trp Ile Ser Glu Thr Leu Cys Ile Gly Cys Gly Ile Cys Ile Lys Lys
    50                  55                  60

Cys Pro Phe Gly Ala Leu Ser Ile Val Asn Leu Pro Ser Asn Leu Glu
65                  70                  75                  80

Lys Glu Thr Thr His Arg Tyr Cys Ala Asn Ala Phe Lys Leu His Arg
                85                  90                  95

Leu Pro Ile Pro Arg Pro Gly Glu Val Leu Gly Leu Val Gly Thr Asn
            100                 105                 110

Gly Ile Gly Lys Ser Thr Ala Leu Lys Ile Leu Ala Gly Lys Gln Lys
        115                 120                 125

Pro Asn Leu Gly Lys Tyr Asp Asp Pro Pro Asp Trp Gln Glu Ile Leu
    130                 135                 140

Thr Tyr Phe Arg Gly Ser Glu Leu Lys Asn Tyr Phe Thr Thr Ile Leu
145                 150                 155                 160

Glu Asp Asp Leu Lys Ala Ile Ile Lys Pro Gln Tyr Val Asp Gln Ile
                165                 170                 175

Pro Lys Ala Ala Lys Gly Thr Val Gly Ser Ile Leu Asp Arg Lys Asp
            180                 185                 190

Glu Thr Lys Thr Gln Ala Ile Val Cys Gln Gln Leu Asp Leu Thr His
        195                 200                 205

Leu Lys Glu Arg Asn Val Glu Asp Leu Ser Gly Gly Glu Leu Gln Arg
    210                 215                 220

Phe Ala Cys Ala Val Val Cys Ile Gln Lys Ala Asp Ile Phe Met Phe
225                 230                 235                 240

Asp Glu Pro Ser Ser Tyr Leu Asp Val Lys Gln Arg Leu Lys Ala Ala
                245                 250                 255

Ile Thr Ile Arg Ser Leu Ile Asn Pro Asp Arg Tyr Ile Ile Val Val
            260                 265                 270

Glu His Asp Leu Ser Val Leu Asp Tyr Leu Ser Asp Phe Ile Cys Cys
        275                 280                 285

Leu Tyr Gly Val Pro Ser Ala Tyr Gly Val Val Thr Met Pro Phe Ser
    290                 295                 300

Val Arg Glu Gly Ile Asn Ile Phe Leu Asp Gly Tyr Val Pro Thr Glu
305                 310                 315                 320

Asn Leu Arg Phe Arg Asp Ala Ser Leu Val Phe Lys Val Ala Glu Thr
                325                 330                 335

Ala Asn Glu Glu Glu Val Lys Lys Met Cys Met Tyr Lys Tyr Pro Gly
            340                 345                 350

Met Lys Lys Lys Met Gly Glu Phe Glu Leu Ala Ile Val Ala Gly Glu
        355                 360                 365

Phe Thr Asp Ser Glu Ile Met Val Met Leu Gly Glu Asn Gly Thr Gly
    370                 375                 380

Lys Thr Thr Phe Ile Arg Met Leu Ala Gly Arg Leu Lys Pro Asp Glu
385                 390                 395                 400

Gly Gly Glu Val Pro Val Leu Asn Val Ser Tyr Lys Pro Gln Lys Ile
                405                 410                 415

Ser Pro Lys Ser Thr Gly Ser Val Arg Gln Leu Leu His Glu Lys Ile
            420                 425                 430

Arg Asp Ala Tyr Thr His Pro Gln Phe Val Thr Asp Val Met Lys Pro
        435                 440                 445

Leu Gln Ile Glu Asn Ile Ile Asp Gln Glu Val Gln Thr Leu Ser Gly
    450                 455                 460

Gly Glu Leu Gln Arg Val Ala Leu Ala Leu Cys Leu Gly Lys Pro Ala
465                 470                 475                 480

Asp Val Tyr Leu Ile Asp Glu Pro Ser Ala Tyr Leu Asp Ser Glu Gln
                485                 490                 495

Arg Leu Met Ala Ala Arg Val Val Lys Arg Phe Ile Leu His Ala Lys
            500                 505                 510

Lys Thr Ala Phe Val Val Glu His Asp Phe Ile Met Ala Thr Tyr Leu
        515                 520                 525

Ala Asp Arg Val Ile Val Phe Asp Gly Val Pro Ser Lys Asn Thr Val
    530                 535                 540

Ala Asn Ser Pro Gln Thr Leu Leu Ala Gly Met Asn Lys Phe Leu Ser
545                 550                 555                 560

Gln Leu Glu Ile Thr Phe Arg Arg Asp Pro Asn Asn Tyr Arg Pro Arg
                565                 570                 575

Ile Asn Lys Leu Asn Ser Ile Lys Asp Val Glu Gln Lys Lys Ser Gly
            580                 585                 590

Asn Tyr Phe Phe Leu Asp Asp
        595

<210> 25
<211> 599
<212> PRT
<213> Homo sapiens

<300>
<301> Bisbal, C.
<302> Cloning and characterization of a RNAse L inhibitor.  A
      new component of the interferon-regulated 2-5A pathway
<303> J. Biol. Chem.
<304> 1995
<305> 270
<306> 13308-13317
<313> 1 - 599

<400> 25
Met Ala Asp Lys Leu Thr Arg Ile Ala Ile Val Asn His Asp Lys Cys
  1               5                  10                  15

Lys Pro Lys Lys Cys Arg Gln Glu Cys Lys Lys Ser Cys Pro Val Val
             20                  25                  30

Arg Met Gly Lys Leu Cys Ile Glu Val Thr Pro Gln Ser Lys Ile Ala
         35                  40                  45

Trp Ile Ser Glu Thr Leu Cys Ile Gly Cys Gly Ile Cys Ile Lys Lys
     50                  55                  60

Cys Pro Phe Gly Ala Leu Ser Ile Val Asn Leu Pro Ser Asn Leu Glu
 65                  70                  75                  80

Lys Glu Thr Thr His Arg Tyr Cys Ala Asn Ala Phe Lys Leu His Arg
                85                  90                  95

Leu Pro Ile Pro Arg Pro Gly Glu Val Leu Gly Leu Val Gly Thr Asn
            100                 105                 110

Gly Ile Gly Lys Ser Ala Ala Leu Lys Ile Leu Ala Gly Lys Gln Lys
        115                 120                 125

Pro Asn Leu Gly Lys Tyr Asp Asp Pro Pro Asp Trp Gln Glu Ile Leu
    130                 135                 140

Thr Tyr Phe Arg Gly Ser Glu Leu Gln Asn Tyr Phe Thr Lys Ile Leu
145                 150                 155                 160

Glu Asp Asp Leu Lys Ala Ile Ile Lys Pro Gln Tyr Val Ala Arg Phe
                165                 170                 175

Leu Arg Leu Ala Lys Gly Thr Val Gly Ser Ile Leu Asp Arg Lys Asp
            180                 185                 190

Glu Thr Lys Thr Gln Ala Ile Val Cys Gln Gln Leu Asp Leu Thr His
        195                 200                 205

Leu Lys Glu Arg Asn Val Glu Asp Leu Ser Gly Gly Glu Leu Gln Arg
    210                 215                 220

Phe Ala Cys Ala Val Val Cys Ile Gln Lys Ala Asp Ile Phe Met Phe
225                 230                 235                 240

Asp Glu Pro Ser Ser Tyr Leu Asp Val Lys Gln Arg Leu Lys Ala Ala
                245                 250                 255

Ile Thr Ile Arg Ser Leu Ile Asn Pro Asp Arg Tyr Ile Ile Val Val
            260                 265                 270

Glu His Asp Leu Ser Val Leu Asp Tyr Leu Ser Asp Phe Ile Cys Cys
        275                 280                 285

Leu Tyr Gly Val Pro Ser Ala Tyr Gly Val Val Thr Met Pro Phe Ser
    290                 295                 300

Val Arg Glu Gly Ile Asn Ile Phe Leu Asp Gly Tyr Val Pro Thr Glu
305                 310                 315                 320

Asn Leu Arg Phe Arg Asp Ala Ser Leu Val Phe Lys Val Ala Glu Thr
                325                 330                 335

Ala Asn Glu Glu Glu Val Lys Lys Met Cys Met Tyr Lys Tyr Pro Gly
            340                 345                 350

Met Lys Lys Lys Met Gly Glu Phe Glu Leu Ala Ile Val Ala Gly Glu
        355                 360                 365

Phe Thr Asp Ser Glu Ile Met Val Met Leu Gly Glu Asn Gly Thr Gly
    370                 375                 380

Lys Thr Thr Phe Ile Arg Met Leu Ala Gly Arg Leu Lys Pro Asp Glu
385                 390                 395                 400

Gly Gly Glu Val Pro Val Leu Asn Val Ser Tyr Lys Pro Gln Lys Ile
                405                 410                 415

Ser Pro Lys Ser Thr Gly Ser Val Arg Gln Leu Leu His Glu Lys Ile
            420                 425                 430

Arg Asp Ala Tyr Thr His Pro Gln Phe Val Thr Asp Val Met Lys Pro
        435                 440                 445

Leu Gln Ile Glu Asn Ile Ile Asp Gln Glu Val Gln Thr Leu Ser Gly
    450                 455                 460

Gly Glu Leu Gln Arg Val Arg Leu Arg Leu Cys Leu Gly Lys Pro Ala
465                 470                 475                 480

Asp Val Tyr Leu Ile Asp Glu Pro Ser Ala Tyr Leu Asp Ser Glu Gln
                485                 490                 495

Arg Leu Met Ala Ala Arg Val Val Lys Arg Phe Ile Leu His Ala Lys
            500                 505                 510

Lys Thr Ala Phe Val Val Glu His Asp Phe Ile Met Ala Thr Tyr Leu
        515                 520                 525

Ala Asp Arg Val Ile Val Phe Asp Gly Val Pro Ser Lys Asn Thr Val
    530                 535                 540

Ala Asn Ser Pro Gln Thr Leu Leu Ala Gly Met Asn Lys Phe Leu Ser
545                 550                 555                 560

Gln Leu Glu Ile Thr Phe Arg Arg Asp Pro Asn Asn Tyr Arg Pro Arg
                565                 570                 575

Ile Asn Lys Leu Asn Ser Ile Lys Asp Val Glu Gln Lys Lys Ser Gly
            580                 585                 590

Asn Tyr Phe Phe Leu Asp Asp
        595

<210> 26
<211> 599
<212> PRT
<213> Mus musculus

<400> 26
Met Ala Asp Lys Leu Thr Arg Ile Ala Ile Val Asn His Asp Lys Cys
1               5                   10                  15

Lys Pro Lys Lys Cys Arg Gln Glu Cys Lys Lys Ser Cys Pro Val Val
            20                  25                  30

Arg Met Gly Lys Leu Cys Ile Glu Val Thr Pro Gln Ser Lys Ile Ala
        35                  40                  45

Trp Ile Ser Glu Thr Leu Cys Ile Gly Cys Gly Ile Cys Ile Lys Lys
    50                  55                  60

Cys Pro Phe Gly Ala Leu Ser Ile Val Asn Leu Pro Ser Asn Leu Glu
65                  70                  75                  80

Lys Glu Thr Thr His Arg Tyr Cys Ala Asn Ala Phe Lys Leu His Arg
                85                  90                  95

Leu Pro Ile Pro Arg Pro Gly Glu Val Leu Gly Leu Val Gly Thr Asn
            100                 105                 110

Gly Ile Gly Lys Ser Thr Ala Leu Lys Ile Leu Ala Gly Lys Gln Lys
        115                 120                 125

Pro Asn Leu Gly Lys Tyr Asp Asp Pro Pro Asp Trp Gln Glu Ile Leu
    130                 135                 140

Thr Tyr Phe Arg Gly Ser Glu Leu Gln Asn Tyr Phe Thr Lys Ile Leu
145                 150                 155                 160

Glu Asp Asp Leu Lys Ala Ile Ile Lys Pro Gln Tyr Val Asp Gln Ile
                165                 170                 175

Pro Lys Ala Ala Lys Gly Thr Val Gly Ser Ile Leu Asp Arg Lys Asp
            180                 185                 190

Glu Thr Lys Thr Gln Ala Ile Val Cys Gln Gln Leu Asp Leu Thr His
        195                 200                 205

Leu Lys Glu Arg Asn Val Glu Asp Leu Ser Gly Gly Glu Leu Gln Arg
    210                 215                 220

Phe Ala Cys Ala Val Val Cys Ile Gln Lys Ala Asp Ile Phe Met Phe
225                 230                 235                 240

Asp Glu Pro Ser Ser Tyr Leu Asp Val Lys Gln Arg Leu Lys Ala Ala
                245                 250                 255

Ile Thr Ile Arg Ser Leu Ile Asn Pro Asp Arg Tyr Ile Ile Val Val
            260                 265                 270

Glu His Asp Leu Ser Val Leu Asp Tyr Leu Ser Asp Phe Ile Cys Cys
        275                 280                 285

Leu Tyr Gly Val Pro Ser Ala Tyr Gly Val Val Thr Met Pro Phe Ser
    290                 295                 300

Val Arg Glu Gly Ile Asn Ile Phe Leu Asp Gly Tyr Val Pro Thr Glu
305                 310                 315                 320

Asn Leu Arg Phe Arg Asp Ala Ser Leu Val Phe Lys Val Ala Glu Thr
                325                 330                 335

Ala Asn Glu Glu Glu Val Lys Lys Met Cys Met Tyr Lys Tyr Pro Gly
            340                 345                 350

Met Lys Lys Lys Met Gly Glu Phe Glu Leu Ala Ile Val Ala Gly Glu
        355                 360                 365

Phe Thr Asp Ser Glu Ile Met Val Met Leu Gly Glu Asn Gly Thr Gly
    370                 375                 380

Lys Thr Thr Phe Ile Arg Met Leu Ala Gly Arg Leu Lys Pro Asp Glu
385                 390                 395                 400

Gly Gly Glu Val Pro Val Leu Asn Val Ser Tyr Lys Pro Gln Lys Ile
                405                 410                 415

Ser Pro Lys Ser Thr Gly Ser Val Arg Gln Leu Leu His Glu Lys Ile
            420                 425                 430

Arg Asp Ala Tyr Thr His Pro Gln Phe Val Thr Asp Val Met Lys Pro
        435                 440                 445

Leu Gln Ile Glu Asn Ile Ile Asp Gln Glu Val Gln Thr Leu Ser Gly
    450                 455                 460

Gly Glu Leu Gln Arg Val Ala Leu Ala Leu Cys Leu Gly Lys Pro Ala
465                 470                 475                 480

Asp Val Tyr Leu Ile Asp Glu Pro Ser Ala Tyr Leu Asp Ser Glu Gln
                485                 490                 495

Arg Leu Met Ala Ala Arg Val Val Lys Arg Phe Ile Leu His Ala Lys
            500                 505                 510

Lys Thr Ala Phe Val Val Glu His Asp Phe Ile Met Ala Thr Tyr Leu
        515                 520                 525

Ala Asp Arg Val Ile Val Phe Asp Gly Val Pro Ser Lys Asn Thr Val
    530                 535                 540

Ala Asn Ser Pro Gln Thr Leu Leu Ala Gly Met Asn Lys Phe Leu Ser
545                 550                 555                 560

Gln Leu Glu Ile Thr Phe Arg Arg Asp Pro Asn Asn Tyr Arg Pro Arg
                565                 570                 575

Ile Asn Lys Leu Asn Ser Ile Lys Asp Val Glu Gln Lys Lys Ser Gly
            580                 585                 590

Asn Tyr Phe Phe Leu Asp Asp
        595

<210> 27
<211> 599
<212> PRT
<213> Homo sapiens

<400> 27
Met Ala Asp Lys Leu Thr Arg Ile Ala Ile Val Asn His Asp Lys Cys
  1               5                  10                  15

Lys Pro Lys Lys Cys Arg Gln Glu Cys Lys Lys Ser Cys Pro Val Val
             20                  25                  30

Arg Met Gly Lys Leu Cys Ile Glu Val Thr Pro Gln Ser Lys Ile Ala
         35                  40                  45

Trp Ile Ser Glu Thr Leu Cys Ile Gly Cys Gly Ile Cys Ile Lys Lys
     50                  55                  60

Cys Pro Phe Gly Ala Leu Ser Ile Val Asn Leu Pro Ser Asn Leu Glu

65                  70                  75                  80

Lys Glu Thr Thr His Arg Tyr Cys Ala Asn Ala Phe Lys Leu His Arg
                85                  90                  95

Leu Pro Ile Pro Arg Pro Gly Glu Val Leu Gly Leu Val Gly Thr Asn
            100                 105                 110

Gly Ile Gly Lys Ser Thr Ala Leu Lys Ile Leu Ala Gly Lys Gln Lys
        115                 120                 125

Pro Asn Leu Gly Lys Tyr Asp Asp Pro Pro Asp Trp Gln Glu Ile Leu
    130                 135                 140

Thr Tyr Phe Arg Gly Ser Glu Leu Gln Asn Tyr Phe Thr Lys Ile Leu
145                 150                 155                 160

Glu Asp Asp Leu Lys Ala Ile Ile Lys Pro Gln Tyr Val Asp Gln Ile
                165                 170                 175

Pro Lys Ala Ala Lys Gly Thr Val Gly Ser Ile Leu Asp Arg Lys Asp
            180                 185                 190

Glu Thr Lys Thr Gln Ala Ile Val Cys Gln Gln Leu Asp Leu Thr His
        195                 200                 205

Leu Lys Glu Arg Asn Val Glu Asp Leu Ser Gly Gly Glu Leu Gln Arg
    210                 215                 220

Phe Ala Cys Ala Val Val Cys Ile Gln Lys Ala Asp Ile Phe Met Phe
225                 230                 235                 240

Asp Glu Pro Ser Ser Tyr Leu Asp Val Lys Gln Arg Leu Lys Ala Ala
                245                 250                 255

Ile Thr Ile Arg Ser Leu Ile Asn Pro Asp Arg Tyr Ile Ile Val Val
            260                 265                 270

Glu His Asp Leu Ser Val Leu Asp Tyr Leu Ser Asp Phe Ile Cys Cys
        275                 280                 285

Leu Tyr Gly Val Pro Ser Ala Tyr Gly Val Val Thr Met Pro Phe Ser
    290                 295                 300

Val Arg Glu Gly Ile Asn Ile Phe Leu Asp Gly Tyr Val Pro Thr Glu
305                 310                 315                 320

Asn Leu Arg Phe Arg Asp Ala Ser Leu Val Phe Lys Val Ala Glu Thr

                325                 330                 335

Ala Asn Glu Glu Glu Val Lys Lys Met Cys Met Tyr Lys Tyr Pro Gly
            340                 345                 350

Met Lys Lys Lys Met Gly Glu Phe Glu Leu Ala Ile Val Ala Gly Glu
        355                 360                 365

Phe Thr Asp Ser Glu Ile Met Val Met Leu Gly Glu Asn Gly Thr Gly
    370                 375                 380

Lys Thr Thr Phe Ile Arg Met Leu Ala Gly Arg Leu Lys Pro Asp Glu
385                 390                 395                 400

Gly Gly Glu Val Pro Val Leu Asn Val Ser Tyr Lys Pro Gln Lys Ile
                405                 410                 415

Ser Pro Lys Ser Thr Gly Ser Val Arg Gln Leu Leu His Glu Lys Ile
            420                 425                 430

Arg Asp Ala Tyr Thr His Pro Gln Phe Val Thr Asp Val Met Lys Pro
        435                 440                 445

Leu Gln Ile Glu Asn Ile Ile Asp Gln Glu Val Gln Thr Leu Ser Gly
    450                 455                 460

Gly Glu Leu Gln Arg Val Ala Leu Ala Leu Cys Leu Gly Lys Pro Ala
465                 470                 475                 480

Asp Val Tyr Leu Ile Asp Glu Pro Ser Ala Tyr Leu Asp Ser Glu Gln
                485                 490                 495

Arg Leu Met Ala Ala Arg Val Val Lys Arg Phe Ile Leu His Ala Lys
            500                 505                 510

Lys Thr Ala Phe Val Val Glu His Asp Phe Ile Met Ala Thr Tyr Leu
        515                 520                 525

Ala Asp Arg Val Ile Val Phe Asp Gly Val Pro Ser Lys Asn Thr Val
    530                 535                 540

Ala Asn Ser Pro Gln Thr Leu Leu Ala Gly Met Asn Lys Phe Leu Ser
545                 550                 555                 560

Gln Leu Glu Ile Thr Phe Arg Arg Asp Pro Asn Asn Tyr Arg Pro Arg
                565                 570                 575

Ile Asn Lys Leu Asn Ser Ile Lys Asp Val Glu Gln Lys Lys Ser Gly

580                 585                 590

Asn Tyr Phe Phe Leu Asp Asp
        595

<210> 28
<211> 614
<212> PRT
<213> Mus musculus

<300>
<301> Lemaire, L.
<302> High-level expression in male germ cells of murine p68
      RNA helicase mRNA
<303> Life Sci.
<304> 1993
<305> 52
<306> 917-926
<313> 1 - 614

<400> 28
Met Ser Ser Tyr Ser Ser Asp Arg Asp Arg Gly Arg Asp Arg Gly Phe
  1               5                  10                  15

Gly Ala Pro Arg Phe Gly Gly Ser Arg Thr Gly Pro Leu Ser Gly Lys
             20                  25                  30

Lys Phe Gly Asn Pro Gly Glu Lys Leu Val Lys Lys Lys Trp Asn Leu
         35                  40                  45

Asp Glu Leu Pro Lys Phe Glu Lys Asn Phe Tyr Gln Glu His Pro Asp
     50                  55                  60

Leu Ala Arg Arg Thr Ala Gln Glu Val Asp Thr Tyr Arg Arg Ser Lys
 65                  70                  75                  80

Glu Ile Thr Val Arg Gly His Asn Cys Pro Lys Pro Val Leu Asn Phe
                 85                  90                  95

Tyr Glu Ala Asn Phe Pro Ala Asn Val Met Asp Val Ile Ala Arg His
             100                 105                 110

Asn Phe Thr Glu Pro Thr Ala Ile Gln Ala Gln Gly Trp Pro Val Ala
        115                 120                 125

Leu Ser Gly Leu Asp Met Val Gly Val Ala Gln Thr Gly Ser Gly Lys
    130                 135                 140

Thr Leu Ser Tyr Leu Leu Pro Ala Ile Val His Ile Asn His His Pro
145                 150                 155                 160

Phe Leu Glu Arg Gly Asp Gly Pro Ile Cys Leu Val Leu Ala Pro Thr
                165                 170                 175

Arg Glu Leu Ala Gln Gln Val Gln Gln Val Ala Ala Glu Tyr Cys Arg
            180                 185                 190

Ala Cys Arg Leu Lys Ser Thr Cys Ile Tyr Gly Gly Ala Pro Lys Gly
        195                 200                 205

Pro Gln Ile Arg Asp Leu Glu Arg Gly Val Glu Ile Cys Ile Ala Thr
    210                 215                 220

Pro Gly Arg Leu Ile Asp Phe Leu Glu Cys Gly Lys Thr Asn Leu Arg
225                 230                 235                 240

Arg Thr Thr Tyr Leu Val Leu Asp Glu Ala Asp Arg Met Leu Asp Met
                245                 250                 255

Gly Phe Glu Pro Gln Ile Arg Lys Ile Val Asp Gln Ile Arg Pro Asp
            260                 265                 270

Arg Gln Thr Leu Met Trp Ser Ala Thr Trp Pro Lys Glu Val Arg Gln
        275                 280                 285

Leu Ala Glu Asp Phe Leu Lys Asp Tyr Ile His Ile Asn Ile Gly Ala
    290                 295                 300

Leu Glu Leu Ser Ala Asn His Asn Ile Leu Gln Ile Val Asp Val Cys
305                 310                 315                 320

His Asp Val Glu Lys Asp Glu Lys Leu Ile Arg Leu Met Glu Glu Ile
                325                 330                 335

Met Ser Glu Lys Glu Asn Lys Thr Ile Val Phe Val Glu Thr Lys Arg
            340                 345                 350

Arg Cys Asp Glu Leu Thr Arg Lys Met Arg Arg Asp Gly Trp Pro Ala
        355                 360                 365

Met Gly Ile His Gly Asp Lys Ser Gln Gln Glu Arg Asp Trp Val Leu
    370                 375                 380

Asn Glu Phe Lys His Gly Lys Ala Pro Ile Leu Ile Ala Thr Asp Val
385                 390                 395                 400

Ala Ser Arg Gly Leu Asp Val Glu Asp Val Lys Phe Val Ile Asn Tyr
                405                 410                 415

Asp Tyr Pro Asn Ser Ser Glu Asp Tyr Ile His Arg Ile Gly Arg Thr
            420                 425                 430

Ala Arg Ser Thr Lys Thr Gly Thr Ala Tyr Thr Phe Phe Thr Pro Asn
        435                 440                 445

Asn Ile Lys Gln Val Ser Asp Leu Ile Ser Val Leu Arg Glu Ala Asn
    450                 455                 460

Gln Ala Ile Asn Pro Lys Leu Leu Gln Leu Val Glu Asp Arg Gly Ser
465                 470                 475                 480

Gly Arg Ser Arg Gly Arg Gly Gly Met Lys Asp Asp Arg Arg Asp Arg
                485                 490                 495

Tyr Ser Ala Gly Lys Arg Gly Gly Phe Asn Thr Phe Arg Asp Arg Glu
            500                 505                 510

Asn Tyr Asp Arg Gly Tyr Ser Asn Leu Leu Lys Arg Asp Phe Gly Ala
        515                 520                 525

Lys Thr Gln Asn Gly Val Tyr Ser Ala Ala Asn Tyr Thr Asn Gly Ser
    530                 535                 540

Phe Gly Ser Asn Phe Val Ser Ala Gly Ile Gln Thr Ser Phe Arg Thr
545                 550                 555                 560

Gly Asn Pro Thr Gly Thr Tyr Gln Asn Gly Tyr Asp Ser Thr Gln Gln
                565                 570                 575

Tyr Gly Ser Asn Val Ala Asn Met His Asn Gly Met Asn Gln Gln Ala
            580                 585                 590

Tyr Ala Tyr Pro Val Pro Gln Pro Ala Pro Met Ile Gly Tyr Pro Met
        595                 600                 605

Pro Thr Gly Tyr Ser Gln
    610

<210> 29
<211> 614
<212> PRT
<213> Homo sapiens

<300>
<301> Ford, M. J.
<302> Nuclear protein with sequence homology to translation
      initiation factor eIF-4A
<303> Nature
<304> 1988
<305> 332
<306> 736-738
<313> 1 - 614

<400> 29
Met Ser Gly Tyr Ser Ser Asp Arg Asp Arg Gly Arg Asp Arg Gly Phe
1               5                   10                  15

Gly Ala Pro Arg Phe Gly Gly Ser Arg Ala Gly Pro Leu Ser Gly Lys
            20                  25                  30

Lys Phe Gly Asn Pro Gly Glu Lys Leu Val Lys Lys Lys Trp Asn Leu
        35                  40                  45

Asp Glu Leu Pro Lys Phe Glu Lys Asn Phe Tyr Gln Glu His Pro Asp
    50                  55                  60

Leu Ala Arg Arg Thr Ala Gln Glu Val Glu Thr Tyr Arg Arg Ser Lys
65                  70                  75                  80

Glu Ile Thr Val Arg Gly His Asn Cys Pro Lys Pro Val Leu Asn Phe
                85                  90                  95

Tyr Glu Ala Asn Phe Pro Ala Asn Val Met Asp Val Ile Ala Arg Gln
            100                 105                 110

Asn Phe Thr Glu Pro Thr Ala Ile Gln Ala Gln Gly Trp Pro Val Ala
        115                 120                 125

Leu Ser Gly Leu Asp Met Val Gly Val Ala Gln Thr Gly Ser Gly Lys
    130                 135                 140

Thr Leu Ser Tyr Leu Leu Pro Ala Ile Val His Ile Asn His Gln Pro
145                 150                 155                 160

Phe Leu Glu Arg Gly Asp Gly Pro Ile Cys Leu Val Leu Ala Pro Thr
                165                 170                 175

Arg Glu Leu Ala Gln Gln Val Gln Gln Val Ala Ala Glu Tyr Cys Arg
            180                 185                 190

Ala Cys Arg Leu Lys Ser Thr Cys Ile Tyr Gly Gly Ala Pro Lys Gly
        195                 200                 205

Pro Gln Ile Arg Asp Leu Glu Arg Gly Val Glu Ile Cys Ile Ala Thr
    210                 215                 220

Pro Gly Arg Leu Ile Asp Phe Leu Glu Cys Gly Lys Thr Asn Leu Arg
225                 230                 235                 240

Arg Thr Thr Tyr Leu Val Leu Asp Glu Ala Asp Arg Met Leu Asp Met
                245                 250                 255

Gly Phe Glu Pro Gln Ile Arg Lys Ile Val Asp Gln Ile Arg Pro Asp
            260                 265                 270

Arg Gln Thr Leu Met Trp Ser Ala Thr Trp Pro Lys Glu Val Arg Gln
        275                 280                 285

Leu Ala Glu Asp Phe Leu Lys Asp Tyr Ile His Ile Asn Ile Gly Ala
    290                 295                 300

Leu Glu Leu Ser Ala Asn His Asn Ile Leu Gln Ile Val Asp Val Cys
305                 310                 315                 320

His Asp Val Glu Lys Asp Glu Lys Leu Ile Arg Leu Met Glu Glu Ile
                325                 330                 335

Met Ser Glu Lys Glu Asn Lys Thr Ile Val Phe Val Glu Thr Lys Arg
            340                 345                 350

Arg Cys Asp Glu Leu Thr Arg Lys Met Arg Arg Asp Gly Trp Pro Ala
        355                 360                 365

Met Gly Ile His Gly Asp Lys Ser Gln Gln Glu Arg Asp Trp Val Leu
    370                 375                 380

Asn Glu Phe Lys His Gly Lys Ala Pro Ile Leu Ile Ala Thr Asp Val
385                 390                 395                 400

Ala Ser Arg Gly Leu Asp Val Glu Asp Val Lys Phe Val Ile Asn Tyr
                405                 410                 415

Asp Tyr Pro Asn Ser Ser Glu Asp Tyr Ile His Arg Ile Gly Arg Thr
            420                 425                 430

Ala Arg Ser Thr Lys Thr Gly Thr Ala Tyr Thr Phe Phe Thr Pro Asn
        435                 440                 445

Asn Ile Lys Gln Val Ser Asp Leu Ile Ser Val Leu Arg Glu Ala Asn
    450                 455                 460

Gln Ala Ile Asn Pro Lys Leu Leu Gln Leu Val Glu Asp Arg Gly Ser
465                 470                 475                 480

Gly Arg Ser Arg Gly Arg Gly Gly Met Lys Asp Asp Arg Arg Asp Arg
                485                 490                 495

Tyr Ser Ala Gly Lys Arg Gly Gly Phe Asn Thr Phe Arg Asp Arg Glu
            500                 505                 510

Asn Tyr Asp Arg Gly Tyr Ser Ser Leu Leu Lys Arg Asp Phe Gly Ala
        515                 520                 525

Lys Thr Gln Asn Gly Val Tyr Ser Ala Ala Asn Tyr Thr Asn Gly Ser
    530                 535                 540

Phe Gly Ser Asn Phe Val Ser Ala Gly Ile Gln Thr Ser Phe Arg Thr
545                 550                 555                 560

Gly Asn Pro Thr Gly Thr Tyr Gln Asn Gly Tyr Asp Ser Thr Gln Gln
                565                 570                 575

Tyr Gly Ser Asn Val Pro Asn Met His Asn Gly Met Asn Gln Gln Ala
            580                 585                 590

Tyr Ala Tyr Pro Ala Thr Ala Ala Ala Pro Met Ile Gly Tyr Pro Met
        595                 600                 605

Pro Thr Gly Tyr Ser Gln
    610

<210> 30
<211> 615
<212> PRT
<213> Mus musculus

<400> 30
Met Ser Ser Tyr Ser Ser Asp Arg Asp Arg Gly Arg Asp Arg Gly Phe
  1               5                  10                  15

Gly Ala Pro Arg Phe Gly Gly Ser Arg Thr Gly Pro Leu Ser Gly Lys
             20                  25                  30

Lys Phe Gly Asn Pro Gly Glu Lys Leu Val Lys Lys Lys Trp Asn Leu
         35                  40                  45

Asp Glu Leu Pro Lys Phe Glu Lys Asn Phe Tyr Gln Glu His Pro Asp
    50                  55                  60

Leu Ala Arg Arg Thr Ala Gln Glu Val Asp Thr Tyr Arg Arg Ser Lys
65                  70                  75                  80

Glu Ile Thr Val Arg Gly His Asn Cys Pro Lys Pro Val Leu Asn Phe
                85                  90                  95

Tyr Glu Ala Asn Phe Pro Ala Asn Val Met Asp Val Ile Ala Arg Gln
            100                 105                 110

Asn Phe Thr Glu Pro Thr Ala Ile Gln Ala Gln Gly Trp Pro Val Ala
        115                 120                 125

Leu Ser Gly Leu Asp Met Val Gly Val Ala Gln Thr Gly Ser Gly Lys
    130                 135                 140

Thr Leu Ser Tyr Leu Leu Pro Ala Ile Val His Ile Asn His Gln Pro
145                 150                 155                 160

Phe Leu Glu Arg Gly Asp Gly Pro Ile Cys Leu Val Leu Ala Pro Thr
                165                 170                 175

Arg Glu Leu Ala Gln Gln Val Gln Gln Val Ala Ala Glu Tyr Cys Arg
            180                 185                 190

Ala Cys Arg Leu Lys Ser Thr Cys Ile Tyr Gly Gly Ala Pro Lys Gly
        195                 200                 205

Pro Gln Ile Arg Asp Leu Glu Arg Gly Val Glu Ile Cys Ile Ala Thr
    210                 215                 220

Pro Gly Arg Leu Ile Asp Phe Leu Glu Cys Gly Lys Thr Asn Leu Arg
225                 230                 235                 240

Arg Thr Thr Tyr Leu Val Leu Asp Glu Ala Asp Arg Met Leu Asp Met
                245                 250                 255

Gly Phe Glu Pro Gln Ile Arg Lys Ile Val Asp Gln Ile Arg Pro Asp
            260                 265                 270

Arg Gln Thr Leu Met Trp Ser Ala Thr Trp Pro Lys Glu Val Arg Gln
        275                 280                 285

Leu Ala Glu Asp Phe Leu Lys Asp Tyr Ile His Ile Asn Ile Gly Ala
    290                 295                 300

Leu Glu Leu Ser Ala Asn His Asn Ile Leu Gln Ile Val Asp Val Cys
305                 310                 315                 320

His Asp Val Glu Lys Asp Glu Lys Leu Ile Arg Leu Met Glu Glu Ile
                325                 330                 335

Met Ser Glu Lys Glu Asn Lys Thr Ile Val Phe Val Glu Thr Lys Arg
            340                 345                 350

Arg Cys Asp Glu Leu Thr Arg Lys Met Arg Arg Asp Gly Trp Pro Ala
        355                 360                 365

Met Gly Ile His Gly Asp Lys Ser Gln Gln Glu Arg Asp Trp Val Leu
    370                 375                 380

Asn Glu Phe Lys His Gly Lys Ala Pro Ile Leu Ile Ala Thr Asp Val
385                 390                 395                 400

Ala Ser Arg Gly Leu Asp Val Glu Asp Val Lys Phe Val Ile Asn Tyr
                405                 410                 415

Asp Tyr Pro Asn Ser Ser Glu Asp Tyr Ile His Arg Ile Gly Arg Thr
            420                 425                 430

Ala Arg Ser Thr Lys Thr Gly Thr Ala Tyr Thr Phe Phe Thr Pro Asn
        435                 440                 445

Asn Ile Lys Gln Val Ser Asp Leu Ile Ser Val Leu Arg Glu Ala Asn
    450                 455                 460

Gln Ala Ile Asn Pro Lys Leu Leu Gln Leu Val Glu Asp Arg Gly Ser
465                 470                 475                 480

Gly Arg Ser Arg Gly Arg Gly Gly Met Lys Asp Asp Arg Arg Asp Arg
                485                 490                 495

Tyr Ser Ala Gly Lys Arg Gly Gly Phe Asn Thr Phe Arg Asp Arg Glu
            500                 505                 510

Asn Tyr Asp Arg Gly Tyr Ser Asn Leu Leu Lys Arg Asp Phe Gly Ala
        515                 520                 525

Lys Thr Gln Asn Gly Val Tyr Ser Ala Ala Asn Tyr Thr Asn Gly Ser
    530                 535                 540

Phe Gly Ser Asn Phe Val Ser Ala Gly Ile Gln Thr Ser Phe Arg Thr
545                 550                 555                 560

Gly Asn Pro Thr Gly Thr Tyr Gln Asn Gly Tyr Asp Ser Thr Gln Gln
                565                 570                 575

Tyr Gly Ser Asn Val Ala Asn Met His Asn Gly Met Asn Gln Gln Ala
            580                 585                 590

Tyr Ala Tyr Pro Ala Thr Ala Ala Ala Ala Pro Met Ile Gly Tyr Pro
        595                 600                 605

Met Pro Thr Gly Tyr Ser Gln
    610                 615

<210> 31
<211> 167
<212> PRT
<213> Mus musculus

<300>
<301> Aoki, N.
<302> Anagen-specific protein mKAP13

<400> 31
Met Ser Cys Asn Ser Cys Ser Gly Thr Phe Ser Gln Ser Phe Gly Gly
  1               5                  10                  15

Gln Leu Gln Tyr Pro Ile Ser Ser Cys Gly Ser Ser Tyr Pro Asn Asn
             20                  25                  30

Val Phe Tyr Ser Thr Asp Leu Gln Thr Pro Ile Thr His Gln Leu Gly
         35                  40                  45

Ser Ser Leu His Ser Gly Cys Gln Glu Thr Phe Cys Glu Pro Thr Asn
     50                  55                  60

Cys Gln Thr Ala Tyr Val Val Ser Arg Pro Cys Gln Arg Pro Phe Tyr
 65                  70                  75                  80

Ser Gln Arg Ile Arg Gly Pro Cys Arg Pro Cys Gln Ser Thr Phe Ser
                 85                  90                  95

Gly Ser Leu Gly Phe Gly Ser Arg Gly Phe Gln Ser Phe Gly Cys Gly
            100                 105                 110

Tyr Pro Ser Gln Gly Phe Gly Ser His Gly Phe Gln Ser Val Gly Cys
        115                 120                 125

Gly Thr Pro Thr Phe Ser Ser Leu Asn Cys Gly Ser Ser Phe Tyr Arg
    130                 135                 140

Pro Thr Cys Phe Ser Thr Lys Ser Cys Gln Ser Val Ser Tyr Gln Pro
145                 150                 155                 160

Thr Cys Gly Thr Gly Phe Phe
                165

<210> 32
<211> 150
<212> PRT
<213> Mus musculus

<300>
<301> Kuhn, F.
<302> Pmg1 and Pmg2 constitute a novel family of KAP genes
      differentially expressed during skin and mammary gland
      development
<303> Mech. Dev.
<304> 1999
<313> 1 - 150

<400> 32
Met Ser Tyr Thr Cys Asn Ser Gly Asn Tyr Ser Ser Gln Ser Phe Gly
  1               5                  10                  15

Gly Phe Leu Arg Gln Pro Val Ser Thr Tyr Asn Ser Phe Tyr Pro Thr
             20                  25                  30

Ser Asn Val Val Tyr Ser Pro Lys Asn Phe Gln Leu Gly Ser Ser Phe
         35                  40                  45

Tyr Asn Gly Gln Gln Glu Thr Phe Ser Glu Pro Leu Glu Gly His Leu
     50                  55                  60

Pro Cys Val Gly Ser Ala Ser Phe His Thr Ser Cys Phe Arg Pro Lys
 65                  70                  75                  80

Gln Tyr Phe Ser Ser Pro Cys Gln Gly Gly Phe Thr Gly Ser Phe Gly
                 85                  90                  95

Tyr Gly Asn Thr Gly Phe Gly Ala Phe Gly Phe Gly Ser Ser Gly Ile
            100                 105                 110

Arg Ser Gln Gly Cys Gly Ser Asn Phe Tyr Arg Pro Gly Tyr Phe Ser
        115                 120                 125

Ser Lys Ser Ile Gln Ser Ser Tyr Tyr Gln Pro Gly Tyr Ser Ser Gly
    130                 135                 140

Phe Cys Gly Ser Asn Phe
145                 150

<210> 33
<211> 469
<212> PRT
<213> Mus musculus

<300>
<301> Shibahara, K.
<302> Isolation of a novel gene MA-3 that is induced upon
      programmed cell death
<303> Gene
<304> 1995
<305> 166
<306> 297-301
<313> 1 - 469

<400> 33
Met Asp Ile Glu Asn Glu Gln Thr Leu Asn Val Asn Pro Thr Asp Pro
  1               5                  10                  15

Asp Asn Leu Ser Asp Ser Leu Phe Ser Gly Asp Glu Glu Asn Ala Gly
             20                  25                  30

Thr Glu Glu Ile Lys Asn Glu Ile Asn Gly Asn Trp Ile Ser Ala Ser
         35                  40                  45

Thr Ile Asn Glu Ala Arg Ile Asn Ala Lys Ala Lys Arg Arg Leu Arg
     50                  55                  60

Lys Asn Ser Ser Arg Asp Ser Gly Arg Gly Asp Ser Val Ser Asp Asn
 65                  70                  75                  80

Gly Ser Glu Ala Val Arg Ser Gly Val Ala Val Pro Thr Ser Pro Lys
                 85                  90                  95

Gly Arg Leu Leu Asp Arg Arg Ser Arg Ser Gly Lys Gly Arg Gly Leu
            100                 105                 110

Pro Lys Lys Gly Gly Ala Gly Gly Lys Gly Val Trp Gly Thr Pro Gly
        115                 120                 125

Gln Val Tyr Asp Val Glu Glu Val Asp Val Lys Asp Pro Asn Tyr Asp
    130                 135                 140

Asp Asp Gln Glu Asn Cys Val Tyr Glu Thr Val Val Leu Pro Leu Asp
145                 150                 155                 160

Glu Thr Ala Phe Glu Lys Thr Leu Thr Pro Ile Ile Gln Glu Tyr Phe
                165                 170                 175

Glu His Gly Asp Thr Asn Glu Val Ala Glu Met Leu Arg Asp Leu Asn
            180                 185                 190

Leu Gly Glu Met Lys Ser Gly Val Pro Val Leu Ala Val Ser Leu Ala
        195                 200                 205

Leu Glu Gly Lys Ala Ser His Arg Glu Met Thr Ser Lys Leu Leu Ser
    210                 215                 220

Asp Leu Cys Gly Thr Val Met Ser Thr Asn Asp Val Glu Lys Ser Phe
225                 230                 235                 240

Asp Lys Leu Leu Lys Asp Leu Pro Glu Leu Ala Leu Asp Thr Pro Arg
                245                 250                 255

Ala Pro Gln Leu Val Gly Gln Phe Ile Ala Arg Ala Val Gly Asp Gly
            260                 265                 270

Ile Leu Cys Asn Thr Tyr Ile Asp Ser Tyr Lys Gly Thr Val Asp Cys
        275                 280                 285

Val Gln Ala Arg Ala Ala Leu Asp Lys Ala Thr Val Leu Leu Ser Met
    290                 295                 300

Ser Lys Gly Gly Lys Arg Lys Asp Ser Val Trp Gly Ser Gly Gly Gly
305                 310                 315                 320

Gln Gln Pro Val Asn His Leu Val Lys Glu Ile Asp Met Leu Leu Lys
                325                 330                 335

Glu Tyr Leu Leu Ser Gly Asp Ile Ser Glu Ala Glu His Cys Leu Lys
            340                 345                 350

Glu Leu Glu Val Pro His Phe His His Glu Leu Val Tyr Glu Ala Ile
        355                 360                 365

Val Met Val Leu Glu Ser Thr Gly Glu Ser Ala Phe Lys Met Ile Leu
    370                 375                 380

Asp Leu Leu Lys Ser Leu Trp Lys Ser Ser Thr Ile Thr Ile Asp Gln
385                 390                 395                 400

Met Lys Arg Gly Tyr Glu Arg Ile Tyr Asn Glu Ile Pro Asp Ile Asn
                405                 410                 415

Leu Asp Val Pro His Ser Tyr Ser Val Leu Glu Arg Phe Val Glu Glu
            420                 425                 430

Cys Phe Gln Ala Gly Ile Ile Ser Lys Gln Leu Arg Asp Leu Cys Pro
        435                 440                 445

Ser Arg Gly Arg Lys Arg Phe Val Ser Glu Gly Asp Gly Gly Arg Leu
    450                 455                 460

Lys Pro Glu Ser Tyr
465

<210> 34
<211> 458
<212> PRT
<213> Homo sapiens

<400> 34


<210> 35
<211> 171
<212> PRT
<213> Mus musculus

<300>
<301> Suk, K.
<302> Enhancement of B-cell translocation gene-1 expresion by
      prostaglandin E2 in macrophages and the relationship to
      proliferation
<303> Immunology
<304> 1997
<305> 91
<306> 121-129
<313> 1 - 171

<400> 35
Met His Pro Phe Tyr Thr Arg Ala Ala Thr Met Ile Gly Glu Ile Ala
1               5                   10                  15

Ala Ala Val Ser Phe Ile Ser Lys Phe Leu Arg Thr Lys Gly Leu Thr
            20                  25                  30

Ser Glu Arg Gln Leu Gln Thr Phe Ser Gln Ser Leu Gln Glu Leu Leu
        35                  40                  45

Ala Glu His Tyr Lys His His Trp Phe Pro Glu Lys Pro Cys Lys Gly
    50                  55                  60

Ser Gly Tyr Arg Cys Ile Arg Ile Asn His Lys Met Asp Pro Leu Ile
65                  70                  75                  80

Gly Gln Ala Ala Gln Arg Ile Gly Leu Ser Ser Gln Glu Leu Phe Arg
                85                  90                  95

Leu Leu Pro Ser Glu Leu Thr Leu Trp Val Asp Pro Tyr Glu Val Ser
            100                 105                 110

Tyr Arg Ile Gly Glu Asp Gly Ser Ile Cys Val Leu Tyr Glu Ala Ser
        115                 120                 125

Pro Ala Gly Gly Ser Thr Gln Asn Ser Thr Asn Val Gln Met Val Asp
    130                 135                 140

Ser Arg Ile Ser Cys Lys Glu Glu Leu Leu Leu Gly Arg Thr Ser Pro
145                 150                 155                 160

Ser Lys Asn Tyr Asn Met Met Thr Val Ser Gly
                165                 170

<210> 36
<211> 171
<212> PRT
<213> Homo sapiens

<400> 36
Met His Pro Phe Tyr Thr Arg Ala Ala Thr Met Ile Gly Glu Ile Ala
  1               5                  10                  15

Ala Ala Val Ser Phe Ile Ser Lys Phe Leu Arg Thr Lys Gly Leu Thr
            20                  25                  30

Ser Glu Arg Gln Leu Gln Thr Phe Ser Gln Ser Leu Gln Glu Leu Leu
        35                  40                  45

Ala Glu His Tyr Lys His His Trp Phe Pro Glu Lys Pro Cys Lys Gly
    50                  55                  60

Ser Gly Tyr Arg Cys Ile Arg Ile Asn His Lys Met Asp Pro Leu Ile
 65                  70                  75                  80

Gly Gln Ala Ala Gln Arg Ile Gly Leu Ser Ser Gln Glu Leu Phe Arg
                85                  90                  95

Leu Leu Pro Ser Glu Leu Thr Leu Trp Val Asp Pro Tyr Glu Val Ser
            100                 105                 110

Tyr Arg Ile Gly Glu Asp Gly Ser Ile Cys Val Leu Tyr Glu Ala Ser

115                 120                 125

Pro Ala Gly Gly Ser Thr Gln Asn Ser Thr Asn Val Gln Met Val Asp
    130                 135                 140

Ser Arg Ile Ser Cys Lys Glu Glu Leu Leu Leu Gly Arg Thr Ser Pro
145                 150                 155                 160

Ser Lys Asn Tyr Asn Met Met Thr Val Ser Gly
                165                 170

<210> 37
<211> 226
<212> PRT
<213> Mus musculus

<300>
<301> Rubinstein, E.
<302> Molecular cloning of the mouse equivalent of CD9
<303> Thromb. Res.
<304> 1993
<305> 71
<306> 377-383
<313> 1 - 226

<400> 37
Met Pro Val Lys Gly Gly Ser Lys Cys Ile Lys Tyr Leu Leu Phe Gly
  1               5                  10                  15

Phe Asn Phe Ile Phe Trp Leu Ala Gly Ile Ala Val Leu Ala Ile Gly
             20                  25                  30

Leu Trp Leu Arg Phe Asp Ser Gln Thr Lys Ser Ile Phe Glu Gln Glu
         35                  40                  45

Asn Asn His Ser Ser Phe Tyr Thr Gly Val Tyr Ile Leu Ile Gly Ala
     50                  55                  60

Gly Ala Leu Met Met Leu Val Gly Phe Leu Gly Cys Cys Gly Ala Val
 65                  70                  75                  80

Gln Glu Ser Gln Cys Met Leu Gly Leu Phe Phe Gly Phe Leu Leu Val
                 85                  90                  95

Ile Phe Ala Ile Glu Ile Ala Ala Ala Val Trp Gly Tyr Thr His Lys
            100                 105                 110

Asp Glu Val Ile Lys Glu Leu Gln Glu Phe Tyr Lys Asp Thr Tyr Gln
        115                 120                 125

Lys Leu Arg Ser Lys Asp Glu Pro Gln Arg Glu Thr Leu Lys Ala Ile
    130                 135                 140

His Met Ala Leu Asp Cys Cys Gly Ile Ala Gly Pro Leu Glu Gln Phe
145                 150                 155                 160

Ile Ser Asp Thr Cys Pro Lys Lys Gln Leu Leu Glu Ser Phe Gln Val
                165                 170                 175

Lys Pro Cys Pro Glu Ala Ile Ser Glu Val Phe Asn Asn Lys Phe His
            180                 185                 190

Ile Ile Gly Ala Val Gly Ile Gly Ile Ala Val Val Met Ile Phe Gly
        195                 200                 205

Met Ile Phe Ser Met Ile Leu Cys Cys Ala Ile Arg Arg Ser Arg Glu
    210                 215                 220

Met Val
225

<210> 38
<211> 228
<212> PRT
<213> Homo sapiens

<300>
<301> Higashihara, M.
<302> Purification and partial characterization of CD9
      antigen of human platelets
<303> FEBS Lett.
<304> 1990
<305> 264(2)
<306> 270-274
<313> 1 - 228

<400> 38
Met Pro Val Lys Gly Gly Thr Lys Cys Ile Lys Tyr Leu Leu Phe Gly
  1               5                  10                  15

Phe Asn Phe Ile Phe Trp Leu Ala Gly Ile Ala Val Leu Ala Ile Gly
             20                  25                  30

Leu Trp Leu Arg Phe Asp Ser Gln Thr Lys Ser Ile Phe Glu Gln Glu

35                  40                  45

Thr Asn Asn Asn Asn Ser Ser Phe Tyr Thr Gly Val Tyr Ile Leu Ile
    50                  55                  60

Gly Ala Gly Ala Leu Met Met Leu Val Gly Phe Leu Gly Cys Cys Gly
65                  70                  75                  80

Ala Val Gln Glu Ser Gln Cys Met Leu Gly Leu Phe Phe Gly Phe Leu
                85                  90                  95

Leu Val Ile Phe Ala Ile Glu Ile Ala Ala Ala Ile Trp Gly Tyr Ser
            100                 105                 110

His Lys Asp Glu Val Ile Lys Glu Val Gln Glu Phe Tyr Lys Asp Thr
        115                 120                 125

Tyr Asn Lys Leu Lys Thr Lys Asp Glu Pro Gln Arg Glu Thr Leu Lys
    130                 135                 140

Ala Ile His Tyr Ala Leu Asn Cys Cys Gly Leu Ala Gly Gly Val Glu
145                 150                 155                 160

Gln Phe Ile Ser Asp Ile Cys Pro Lys Lys Asp Val Leu Glu Thr Phe
                165                 170                 175

Thr Val Lys Ser Cys Pro Asp Ala Ile Lys Glu Val Phe Asp Asn Lys
            180                 185                 190

Phe His Ile Ile Gly Ala Val Gly Ile Gly Ile Ala Val Val Met Ile
        195                 200                 205

Phe Gly Met Ile Phe Ser Met Ile Leu Cys Cys Ala Ile Arg Arg Asn
    210                 215                 220

Arg Glu Met Val
225

<210> 39
<211> 358
<212> PRT
<213> Mus musculus

<300>
<301> Kaestner, K. H.
<302> Differentiation-induced gene expression in 3T3-L1
      preadipocytes. A second differentially expressed gene

encoding stearoyl-CoA
<303> J. Biol. Chem.
<304> 1989
<305> 264
<306> 14755-14761
<313> 1 - 358

<400> 39
Met Pro Ala His Ile Leu Gln Glu Ile Ser Gly Ala Tyr Ser Ala Thr
1               5                   10                  15

Thr Thr Ile Thr Ala Pro Pro Ser Gly Gly Gln Gln Asn Gly Gly Glu
            20                  25                  30

Lys Phe Glu Lys Ser Ser His His Trp Gly Ala Asp Val Arg Pro Glu
        35                  40                  45

Leu Lys Asp Asp Leu Tyr Asp Pro Thr Tyr Gln Asp Asp Glu Gly Pro
    50                  55                  60

Pro Pro Lys Leu Glu Tyr Val Trp Arg Asn Ile Ile Leu Met Ala Leu
65                  70                  75                  80

Leu His Leu Gly Ala Leu Tyr Gly Ile Thr Leu Val Pro Ser Cys Lys
                85                  90                  95

Leu Tyr Thr Cys Leu Phe Ala Tyr Leu Tyr Tyr Val Ile Ser Ala Leu
            100                 105                 110

Gly Ile Thr Ala Gly Ala His Arg Leu Trp Ser His Arg Thr Tyr Lys
        115                 120                 125

Ala Arg Leu Pro Leu Arg Leu Phe Leu Ile Ile Ala Asn Thr Met Ala
    130                 135                 140

Phe Gln Asn Asp Val Tyr Glu Trp Ala Arg Asp His Arg Ala His His
145                 150                 155                 160

Lys Phe Ser Glu Thr His Ala Asp Pro His Asn Ser Arg Arg Gly Phe
                165                 170                 175

Phe Phe Ser His Val Gly Trp Leu Leu Val Arg Lys His Pro Ala Val
            180                 185                 190

Lys Glu Lys Gly Gly Lys Leu Asp Met Ser Asp Leu Lys Ala Glu Lys
        195                 200                 205

Leu Val Met Phe Gln Arg Arg Tyr Tyr Lys Pro Asp Leu Leu Leu Met

210                 215                 220

Cys Phe Val Leu Pro Thr Leu Val Pro Trp Tyr Cys Trp Gly Glu Thr
225                 230                 235                 240

Phe Val Asn Ser Leu Cys Val Ser Thr Phe Leu Arg Tyr Ala Val Val
                245                 250                 255

Leu Asn Ala Thr Trp Leu Val Asn Ser Ala Ala His Leu Tyr Gly Tyr
            260                 265                 270

Arg Pro Tyr Asp Lys Asn Ile Ser Ser Arg Glu Asn Ile Leu Val Ser
        275                 280                 285

Met Gly Ala Val Gly Glu Arg Phe His Asn Tyr His His Ala Phe Pro
    290                 295                 300

Tyr Asp Tyr Ser Ala Ser Glu Tyr Arg Trp His Ile Asn Phe Thr Thr
305                 310                 315                 320

Phe Phe Ile Asp Cys Met Ala Leu Leu Gly Leu Ala Tyr Asp Arg Lys
                325                 330                 335

Arg Val Ser Arg Ala Ala Val Leu Ala Arg Ile Lys Arg Thr Gly Asp
            340                 345                 350

Gly Ser Cys Lys Ser Gly
        355

<210> 40
<211> 359
<212> PRT
<213> Homo sapiens

<300>
<301> Zhang, L.
<302> Human stearoyl-CoA desaturase: alternative transcripts
      generated from a single gene by usage of tandem
      polyadenylation sites
<303> Biochem. J.
<304> 1999
<305> 340
<306> 255-264
<313> 1 - 359

<400> 40
Met Pro Ala His Leu Leu Gln Asp Asp Ile Ser Ser Ser Tyr Thr Thr

1                   5                   10                  15

Thr Thr Thr Ile Thr Ala Pro Pro Ser Arg Val Leu Gln Asn Gly Gly
                20                  25                  30

Asp Lys Leu Glu Thr Met Pro Leu Tyr Leu Glu Asp Asp Ile Arg Pro
            35                  40                  45

Asp Ile Lys Asp Asp Ile Tyr Asp Pro Thr Tyr Lys Asp Lys Glu Gly
        50                  55                  60

Pro Ser Pro Lys Val Glu Tyr Val Trp Arg Asn Ile Ile Leu Met Ser
65                  70                  75                  80

Leu Leu His Leu Gly Ala Leu Tyr Gly Ile Thr Leu Ile Pro Thr Cys
                85                  90                  95

Lys Phe Tyr Thr Trp Leu Trp Gly Val Phe Tyr Tyr Phe Val Ser Ala
            100                 105                 110

Leu Gly Ile Thr Ala Gly Ala His Arg Leu Trp Ser His Arg Ser Tyr
        115                 120                 125

Lys Ala Arg Leu Pro Leu Arg Leu Phe Leu Ile Ile Ala Asn Thr Met
    130                 135                 140

Ala Phe Gln Asn Asp Val Tyr Glu Trp Ala Arg Asp His Arg Ala His
145                 150                 155                 160

His Lys Phe Ser Glu Thr His Ala Asp Pro His Asn Ser Arg Arg Gly
                165                 170                 175

Phe Phe Phe Ser His Val Gly Trp Leu Leu Val Arg Lys His Pro Ala
            180                 185                 190

Val Lys Glu Lys Gly Ser Thr Leu Asp Leu Ser Asp Leu Glu Ala Glu
        195                 200                 205

Lys Leu Val Met Phe Gln Arg Arg Tyr Tyr Lys Pro Gly Leu Leu Leu
    210                 215                 220

Met Cys Phe Ile Leu Pro Thr Leu Val Pro Trp Tyr Phe Trp Gly Glu
225                 230                 235                 240

Thr Phe Gln Asn Ser Val Phe Val Ala Thr Phe Leu Arg Tyr Ala Val
                245                 250                 255

Val Leu Asn Ala Thr Trp Leu Val Asn Ser Ala Ala His Leu Phe Gly

            260                 265                 270

Tyr Arg Pro Tyr Asp Lys Asn Ile Ser Pro Arg Glu Asn Ile Leu Val
        275                 280                 285

Ser Leu Gly Ala Val Gly Glu Gly Phe His Asn Tyr His His Ser Phe
    290                 295                 300

Pro Tyr Asp Tyr Ser Ala Ser Glu Tyr Arg Trp His Ile Asn Phe Thr
305                 310                 315                 320

Thr Phe Phe Ile Asp Cys Met Ala Ala Leu Gly Leu Ala Tyr Asp Arg
                325                 330                 335

Lys Lys Val Ser Lys Ala Ala Ile Leu Ala Arg Ile Lys Arg Thr Gly
            340                 345                 350

Asp Gly Asn Tyr Lys Ser Gly
        355

<210> 41
<211> 198
<212> PRT
<213> Mus musculus

<300>
<301> Tsuzuki, S.
<302> Molecular cloning, enomic organisation, promoter
activity, and tissue-specific expression of the mouse
ryudocan gene
<303> J. Biochem.
<304> 1997
<305> 122
<306> 17-24
<313> 1 - 198

<400> 41
Met Ala Pro Ala Cys Leu Leu Ala Pro Leu Leu Leu Leu Leu Gly
1               5                   10                  15

Gly Phe Pro Leu Val Pro Gly Glu Ser Ile Arg Glu Thr Glu Val Ile
                20                  25                  30

Asp Pro Gln Asp Leu Leu Glu Gly Arg Tyr Phe Ser Gly Ala Leu Pro
            35                  40                  45

Asp Asp Glu Asp Ala Gly Gly Ser Asp Asp Phe Glu Leu Ser Gly Ser

50                  55                  60

Gly Asp Leu Asp Asp Thr Glu Glu Pro Arg Pro Phe Pro Glu Val Ile
65                  70                  75                  80

Glu Pro Leu Val Pro Leu Asp Asn His Ile Pro Glu Asn Ala Gln Pro
                85                  90                  95

Gly Ile Arg Val Pro Ser Glu Pro Lys Glu Leu Glu Glu Asn Glu Val
            100                 105                 110

Ile Pro Lys Arg Ala Pro Ser Asp Val Gly Asp Asp Met Ser Asn Lys
        115                 120                 125

Val Ser Met Ser Ser Thr Ala Gln Gly Ser Asn Ile Phe Glu Arg Thr
    130                 135                 140

Glu Val Leu Ala Ala Leu Ile Val Gly Gly Val Val Gly Ile Leu Phe
145                 150                 155                 160

Ala Val Phe Leu Ile Leu Leu Leu Val Tyr Arg Met Lys Lys Lys Asp
                165                 170                 175

Glu Gly Ser Tyr Asp Leu Gly Lys Lys Pro Ile Tyr Lys Lys Ala Pro
            180                 185                 190

Thr Asn Glu Phe Tyr Ala
        195

<210> 42
<211> 198
<212> PRT
<213> Homo sapiens

<300>
<301> Kojima, T.
<302> Human ryudocan core protein: molecular cloning and
      characterization of the cDNA, and chromosomal
      localization of the gene
<303> Biochem. Biophys. Res. Commun.
<304> 1993
<305> 190
<306> 814-822
<313> 1 - 198

<400> 42
Met Ala Pro Ala Arg Leu Phe Ala Leu Leu Leu Phe Phe Val Gly Gly

1                   5                   10                  15

Val Ala Glu Ser Ile Arg Glu Thr Glu Val Ile Asp Pro Gln Asp Leu
            20                  25                  30

Leu Glu Gly Arg Tyr Phe Ser Gly Ala Leu Pro Asp Asp Glu Asp Val
        35                  40                  45

Val Gly Pro Gly Gln Glu Ser Asp Asp Phe Glu Leu Ser Gly Ser Gly
    50                  55                  60

Asp Leu Asp Asp Leu Glu Asp Ser Met Ile Gly Pro Glu Val Val His
65                  70                  75                  80

Pro Leu Val Pro Leu Asp Asn His Ile Pro Glu Arg Ala Gly Ser Gly
                85                  90                  95

Ser Gln Val Pro Thr Glu Pro Lys Lys Leu Glu Glu Asn Glu Val Ile
            100                 105                 110

Pro Lys Arg Ile Ser Pro Val Glu Glu Ser Glu Asp Val Ser Asn Lys
        115                 120                 125

Val Ser Met Ser Ser Thr Val Gln Gly Ser Asn Ile Phe Glu Arg Thr
    130                 135                 140

Glu Val Leu Ala Ala Leu Ile Val Gly Gly Ile Val Gly Ile Leu Phe
145                 150                 155                 160

Ala Val Phe Leu Ile Leu Leu Leu Met Tyr Arg Met Lys Lys Lys Asp
                165                 170                 175

Glu Gly Ser Tyr Asp Leu Gly Lys Lys Pro Ile Tyr Lys Lys Ala Pro
            180                 185                 190

Thr Asn Glu Phe Tyr Ala
        195

<210> 43
<211> 706
<212> PRT
<213> Mus musculus

<300>
<301> Nielsen, A. L.
<302> Murine helix-loop-helix transcriptional activator
      proteins to the E-box motif of the Akv murine leukemia

virus enhancer identified by cDNA cloning
<303> Mol. Cell. Biol.
<304> 1992
<305> 12
<306> 3449-3459
<313> 1 - 706

<400> 43
Met Asn Pro Gln Gln Gln Arg Met Ala Ala Ile Gly Thr Asp Lys Glu
1               5                   10                  15

Leu Ser Asp Leu Leu Asp Phe Ser Ala Met Phe Ser Pro Pro Val Asn
            20                  25                  30

Ser Gly Lys Thr Arg Pro Thr Thr Leu Gly Ser Ser Gln Phe Ser Gly
        35                  40                  45

Ser Gly Met Asp Glu Arg Gly Gly Thr Thr Ser Trp Gly Thr Ser Gly
    50                  55                  60

Gln Pro Ser Pro Ser Tyr Asp Ser Ser Arg Gly Phe Thr Asp Ser Pro
65                  70                  75                  80

His Tyr Ser Asp His Leu Asn Asp Ser Arg Leu Gly Thr His Glu Gly
                85                  90                  95

Leu Ser Pro Thr Pro Phe Met Asn Ser Asn Leu Ile Gly Lys Thr Ser
            100                 105                 110

Glu Arg Gly Ser Phe Ser Leu Tyr Ser Arg Asp Ser Ala Leu Ser Gly
        115                 120                 125

Cys Gln Ser Ser Leu Leu Arg Ala Ser Ser Arg Thr Trp Glu Pro Ala
    130                 135                 140

Gln Leu Ser Ser Ser Gly Lys Pro Gly Thr Pro Tyr Tyr Ser Phe Ser
145                 150                 155                 160

Ala Thr Ser Ser Arg Arg Arg Pro Leu His Asp Ser Val Ala Leu Asp
                165                 170                 175

Pro Leu Gln Ala Lys Lys Val Arg Lys Val Pro Pro Gly Leu Pro Ser
            180                 185                 190

Ser Val Tyr Ala Pro Ser Pro Asn Ser Asp Asp Phe Asn Arg Glu Ser
        195                 200                 205

Pro Ser Tyr Pro Ser Pro Lys Pro Pro Thr Ser Met Phe Ala Ser Thr

210                 215                 220

Phe Phe Met Gln Asp Gly Thr His Ser Ser Ser Asp Leu Trp Ser Ser
225                 230                 235                 240

Ser Asn Gly Met Ser Gln Pro Gly Phe Gly Gly Ile Leu Gly Thr Ser
                245                 250                 255

Thr Ser His Met Ser Gln Ser Ser Ser Tyr Gly Ser Leu His Ser His
            260                 265                 270

Asp Arg Leu Ser Tyr Pro Pro His Ser Val Ser Pro Thr Asp Ile Asn
        275                 280                 285

Thr Ser Leu Pro Pro Met Ser Ser Phe His Arg Gly Ser Thr Ser Ser
    290                 295                 300

Ser Pro Tyr Val Ala Ala Ser His Thr Pro Pro Ile Asn Gly Ser Asp
305                 310                 315                 320

Ser Ile Leu Gly Thr Arg Gly Asn Ala Ala Gly Ser Ser Gln Thr Gly
                325                 330                 335

Asp Ala Leu Gly Lys Ala Leu Ala Ser Ile Tyr Ser Pro Asp His Thr
            340                 345                 350

Ser Ser Ser Phe Pro Ser Asn Pro Ser Thr Pro Val Gly Ser Pro Ser
        355                 360                 365

Pro Leu Thr Gly Thr Ser Gln Trp Pro Arg Ala Gly Gly Gln Ala Pro
    370                 375                 380

Ser Ser Pro Ser Tyr Glu Asn Ser Leu His Ser Leu Lys Asn Arg Val
385                 390                 395                 400

Glu Gln Gln Leu His Glu His Leu Gln Asp Ala Met Ser Phe Leu Lys
                405                 410                 415

Asp Val Cys Glu Gln Ser Arg Met Glu Asp Arg Leu Asp Arg Leu Asp
            420                 425                 430

Asp Ala Ile His Val Leu Arg Asn His Ala Val Gly Pro Ser Thr Ser
        435                 440                 445

Leu Pro Thr Ser His Ser Asp Ile His Ser Leu Leu Gly Pro Ser His
    450                 455                 460

Asn Ala Ser Ile Gly Asn Leu Asn Ser Asn Tyr Gly Gly Ser Ser Leu

465                 470                 475                 480

Val Thr Asn Ser Arg Ser Ala Ser Met Val Gly Thr His Arg Glu Asp
                485                 490                 495

Ser Val Ser Leu Asn Gly Asn His Ser Val Leu Ser Ser Thr Val Ala
            500                 505                 510

Ala Ser Asn Thr Glu Leu Asn His Lys Thr Pro Glu Asn Phe Arg Gly
        515                 520                 525

Gly Val Gln Asn Gln Ser Gly Ser Val Val Pro Thr Glu Ile Lys Thr
    530                 535                 540

Glu Asn Lys Glu Lys Asp Glu Asn Leu His Glu Pro Pro Ser Ser Asp
545                 550                 555                 560

Asp Met Lys Ser Asp Asp Glu Ser Ser Gln Lys Asp Ile Lys Val Ser
                565                 570                 575

Ser Arg Gly Arg Thr Ser Ser Thr Asn Glu Asp Glu Asp Leu Asn Pro
            580                 585                 590

Glu Gln Lys Ile Glu Arg Glu Lys Glu Arg Arg Met Ala Asn Asn Ala
        595                 600                 605

Arg Glu Arg Leu Arg Val Arg Asp Ile Asn Glu Ala Phe Lys Glu Leu
    610                 615                 620

Gly Arg Met Cys Gln Leu His Leu Lys Ser Glu Lys Pro Gln Thr Lys
625                 630                 635                 640

Leu Leu Ile Leu His Gln Ala Val Ala Val Ile Leu Ser Leu Glu Gln
                645                 650                 655

Gln Val Arg Glu Arg Asn Leu Asn Pro Lys Ala Ala Cys Leu Lys Arg
            660                 665                 670

Arg Glu Glu Glu Lys Val Ser Ala Ala Ser Ala Glu Pro Pro Ser Thr
        675                 680                 685

Leu Pro Gly Ala His Pro Gly Leu Ser Glu Ser Thr Asn Pro Met Gly
    690                 695                 700

His Leu
705

<210> 44
<211> 682
<212> PRT
<213> Homo sapiens

<400> 44
Met Asn Pro Gln Gln Gln Arg Met Ala Ala Ile Gly Thr Asp Lys Glu
1               5                   10                  15

Leu Ser Asp Leu Leu Asp Phe Ser Ala Met Phe Ser Pro Pro Val Asn
            20                  25                  30

Ser Gly Lys Thr Arg Pro Thr Thr Leu Gly Ser Ser Gln Phe Ser Gly
        35                  40                  45

Ser Gly Ile Asp Glu Arg Gly Gly Thr Thr Ser Trp Gly Thr Ser Gly
    50                  55                  60

Gln Pro Ser Pro Ser Tyr Asp Ser Ser Arg Gly Phe Thr Asp Ser Pro
65                  70                  75                  80

His Tyr Ser Asp His Leu Asn Asp Ser Arg Leu Gly Ala His Glu Gly
                85                  90                  95

Leu Ser Pro Thr Pro Phe Met Asn Ser Asn Leu Met Gly Lys Thr Ser
            100                 105                 110

Glu Arg Gly Ser Phe Ser Leu Tyr Ser Arg Asp Thr Gly Leu Pro Gly
        115                 120                 125

Cys Gln Ser Ser Leu Leu Arg Gln Asp Leu Gly Leu Gly Ser Pro Ala
    130                 135                 140

Gln Leu Ser Ser Ser Gly Lys Pro Gly Thr Ala Tyr Tyr Ser Phe Ser
145                 150                 155                 160

Ala Thr Ser Ser Arg Arg Arg Pro Leu His Asp Ser Ala Ala Leu Asp
                165                 170                 175

Pro Leu Gln Ala Lys Lys Val Arg Lys Val Pro Pro Gly Leu Pro Ser
            180                 185                 190

Ser Val Tyr Ala Pro Ser Pro Asn Ser Asp Asp Phe Asn Arg Glu Ser
        195                 200                 205

Pro Ser Tyr Pro Ser Pro Lys Pro Pro Thr Ser Met Phe Ala Ser Thr
    210                 215                 220

Phe Phe Met Gln Asp Gly Thr His Asn Ser Ser Asp Leu Trp Ser Ser
225                 230                 235                 240

Ser Asn Gly Met Ser Gln Pro Gly Phe Gly Gly Ile Leu Gly Thr Ser
                245                 250                 255

Thr Ser His Met Ser Gln Ser Ser Ser Tyr Gly Asn Leu His Ser His
            260                 265                 270

Asp Arg Leu Ser Tyr Pro Pro His Ser Val Ser Pro Thr Asp Ile Asn
        275                 280                 285

Thr Ser Leu Pro Pro Met Ser Ser Phe His Arg Gly Ser Thr Ser Ser
    290                 295                 300

Ser Pro Tyr Val Ala Ala Ser His Thr Pro Pro Ile Asn Gly Ser Asp
305                 310                 315                 320

Ser Ile Leu Gly Thr Arg Gly Asn Ala Ala Gly Ser Ser Gln Thr Gly
                325                 330                 335

Asp Ala Leu Gly Lys Ala Leu Ala Ser Ile Tyr Ser Pro Asp His Thr
            340                 345                 350

Ser Ser Ser Phe Pro Ser Asn Pro Ser Thr Pro Val Gly Ser Pro Ser
        355                 360                 365

Pro Leu Thr Gly Thr Ser Gln Trp Pro Arg Pro Gly Gly Gln Ala Pro
    370                 375                 380

Ser Ser Pro Ser Tyr Glu Asn Ser Leu His Ser Leu Gln Ser Arg Met
385                 390                 395                 400

Glu Asp Arg Leu Asp Arg Leu Asp Asp Ala Ile His Val Leu Arg Asn
                405                 410                 415

His Ala Val Gly Pro Ser Thr Ser Leu Pro Ala Gly His Ser Asp Ile
            420                 425                 430

His Ser Leu Leu Gly Pro Ser His Asn Ala Pro Ile Gly Ser Leu Asn
        435                 440                 445

Ser Asn Tyr Gly Gly Ser Ser Leu Val Ala Ser Ser Arg Ser Ala Ser
    450                 455                 460

Met Val Gly Thr His Arg Glu Asp Ser Val Ser Leu Asn Gly Asn His
465                 470                 475                 480

Ser Val Leu Ser Ser Thr Val Thr Thr Ser Ser Thr Asp Leu Asn His
                485                 490                 495

Lys Thr Gln Glu Asn Tyr Arg Gly Gly Leu Gln Ser Gln Ser Gly Thr
            500                 505                 510

Val Val Thr Thr Glu Ile Lys Thr Glu Asn Lys Glu Lys Asp Glu Asn
        515                 520                 525

Leu His Glu Pro Pro Ser Ser Asp Asp Met Lys Ser Asp Asp Glu Ser
    530                 535                 540

Ser Gln Lys Asp Ile Lys Val Ser Ser Arg Gly Arg Thr Ser Ser Thr
545                 550                 555                 560

Asn Glu Asp Glu Asp Leu Asn Pro Glu Gln Lys Ile Glu Arg Glu Lys
                565                 570                 575

Glu Arg Arg Met Ala Asn Asn Ala Arg Glu Arg Leu Arg Val Arg Asp
            580                 585                 590

Ile Asn Glu Ala Phe Lys Glu Leu Gly Arg Met Cys Gln Leu His Leu
        595                 600                 605

Lys Ser Glu Lys Pro Gln Thr Lys Leu Leu Ile Leu His Gln Ala Val
    610                 615                 620

Ala Val Ile Leu Ser Leu Glu Gln Gln Val Arg Glu Arg Asn Leu Asn
625                 630                 635                 640

Pro Lys Ala Ala Cys Leu Lys Arg Arg Glu Glu Glu Lys Val Ser Ala
                645                 650                 655

Val Ser Ala Glu Pro Pro Thr Thr Leu Pro Gly Thr His Pro Gly Leu
            660                 665                 670

Ser Glu Thr Thr Asn Pro Met Gly His Met
        675                 680

<210> 45
<211> 167
<212> PRT
<213> Mus musculus

<300>
<301> Zeng, Q.
<302> Mouse PRL-2 and PRL-3, two potentially prenylated

protein tyrosine phosphatase homologues to PRL-1
<303> Biochem. Biophys. Res. Commun.
<304> 1998
<305> 244
<306> 421-427
<313> 1 - 167

<400> 45
Met Asn Arg Pro Ala Pro Val Glu Ile Ser Tyr Glu Asn Met Arg Phe
1               5                   10                  15

Leu Ile Thr His Asn Pro Thr Asn Ala Thr Leu Asn Lys Phe Thr Glu
            20                  25                  30

Glu Leu Lys Lys Tyr Gly Val Thr Thr Leu Val Arg Val Cys Asp Ala
        35                  40                  45

Thr Tyr Asp Lys Ala Pro Val Glu Lys Glu Gly Ile His Val Leu Asp
    50                  55                  60

Trp Pro Phe Asp Asp Gly Ala Pro Pro Pro Asn Gln Ile Val Asp Asp
65                  70                  75                  80

Trp Leu Asn Leu Leu Lys Thr Leu Phe Arg Glu Glu Pro Gly Cys Cys
                85                  90                  95

Val Ala Val His Cys Val Ala Gly Ile Gly Arg Ala Pro Val Leu Val
            100                 105                 110

Ala Leu Ala Leu Ile Glu Cys Gly Met Lys Tyr Glu Asp Ala Val Gln
        115                 120                 125

Phe Ile Arg Gln Lys Arg Arg Gly Ala Phe Asn Ser Lys Gln Leu Leu
    130                 135                 140

Tyr Leu Glu Lys Tyr Arg Pro Lys Met Arg Leu Arg Phe Arg Asp Thr
145                 150                 155                 160

Asn Gly His Cys Cys Val Gln
                165


<210> 46
<211> 167
<212> PRT
<213> Homo sapiens

<300>

<301> Montagna, M.
<302> A 100-kb physical and transcriptional map around the
      EDH17B2 gene: identification of three novel genes and a
      pseudogene of a human homologue of the rat PRL-1
      tyrosine phosphatase
<303> Hum. Genet.
<304> 1995
<305> 96
<306> 532-538

<400> 46
Met Asn Arg Pro Ala Pro Val Glu Ile Ser Tyr Glu Asn Met Arg Phe
 1               5                  10                  15

Leu Ile Thr His Asn Pro Thr Asn Ala Thr Leu Asn Lys Phe Thr Glu
            20                  25                  30

Glu Leu Lys Lys Tyr Gly Val Thr Thr Leu Val Arg Val Cys Asp Ala
        35                  40                  45

Thr Tyr Asp Lys Ala Pro Val Glu Lys Glu Gly Ile His Val Leu Asp
    50                  55                  60

Trp Pro Phe Asp Asp Gly Ala Pro Pro Pro Asn Gln Ile Val Asp Asp
65                  70                  75                  80

Trp Leu Asn Leu Leu Lys Thr Lys Phe Arg Glu Glu Pro Gly Cys Cys
                85                  90                  95

Val Ala Val His Cys Val Ala Gly Leu Gly Arg Ala Pro Val Leu Val
            100                 105                 110

Ala Leu Ala Leu Ile Glu Cys Gly Met Lys Tyr Glu Asp Ala Val Gln
        115                 120                 125

Phe Ile Arg Gln Lys Arg Arg Gly Ala Phe Asn Ser Lys Gln Leu Leu
    130                 135                 140

Tyr Leu Glu Lys Tyr Arg Pro Lys Met Arg Leu Arg Phe Arg Asp Thr
145                 150                 155                 160

Asn Gly His Cys Cys Val Gln
                165


<210> 47
<211> 532
<212> PRT

<213> Mus musculus

<300>
<301> Inoue, T.
<302> Isolation of complementary DNAs encoding a
      cerebellum-enriched nuclear factor I family that
      activates ranscription from the mouse myelin basic
      protein promoter
<303> J. Biol. Chem.
<304> 1990
<305> 265
<306> 19065-19070
<313> 1 - 532

<400> 47
Met Lys Leu Ala Asp Ser Val Met Ala Gly Lys Ala Ser Asp Gly Ser
1               5                   10                  15

Ile Lys Trp Gln Leu Cys Tyr Asp Ile Ser Ala Arg Thr Trp Trp Met
            20                  25                  30

Asp Glu Phe His Pro Phe Ile Glu Ala Leu Leu Pro His Val Arg Ala
        35                  40                  45

Phe Ala Tyr Thr Trp Phe Asn Leu Gln Ala Arg Lys Arg Lys Tyr Phe
    50                  55                  60

Lys Lys His Glu Lys Arg Met Ser Lys Glu Glu Glu Arg Ala Val Lys
65                  70                  75                  80

Asp Glu Leu Leu Ser Glu Lys Pro Glu Val Lys Gln Lys Trp Ala Ser
                85                  90                  95

Arg Leu Leu Ala Lys Leu Arg Lys Asp Ile Arg Pro Glu Tyr Arg Glu
            100                 105                 110

Asp Phe Val Leu Thr Val Thr Gly Lys Lys Pro Pro Cys Cys Val Leu
        115                 120                 125

Ser Asn Pro Asp Gln Lys Gly Lys Met Arg Arg Ile Asp Cys Leu Arg
    130                 135                 140

Gln Ala Asp Lys Val Trp Arg Leu Asp Leu Val Met Val Ile Leu Phe
145                 150                 155                 160

Lys Gly Ile Pro Leu Glu Ser Thr Asp Gly Glu Arg Leu Val Lys Ser
                165                 170                 175

Pro Gln Cys Ser Asn Pro Gly Leu Cys Val Gln Pro His His Ile Gly
            180                 185                 190

Val Ser Val Lys Glu Leu Asp Leu Tyr Leu Ala Tyr Phe Val His Ala
        195                 200                 205

Ala Asp Ser Ser Gln Ser Glu Ser Pro Ser Gln Pro Ser Glu Ala Asp
    210                 215                 220

Ile Lys Asp Gln Pro Glu Asn Gly His Leu Gly Phe Gln Asp Ser Phe
225                 230                 235                 240

Val Thr Ser Gly Val Phe Ser Val Thr Glu Leu Val Arg Val Ser Gln
                245                 250                 255

Thr Pro Ile Ala Ala Gly Thr Gly Pro Asn Phe Ser Leu Ser Asp Leu
            260                 265                 270

Glu Ser Ser Ser Tyr Tyr Ser Met Ser Pro Gly Ala Met Arg Arg Ser
        275                 280                 285

Leu Pro Ser Thr Ser Ser Thr Ser Ser Thr Lys Arg Leu Lys Ser Val
    290                 295                 300

Glu Asp Glu Met Asp Ser Pro Gly Glu Glu Pro Phe Tyr Thr Gly Gln
305                 310                 315                 320

Gly Arg Ser Pro Gly Ser Gly Ser Gln Ser Ser Gly Trp His Glu Val
                325                 330                 335

Glu Pro Gly Leu Pro Ser Pro Ser Thr Leu Lys Lys Ser Glu Lys Ser
            340                 345                 350

Gly Phe Ser Ser Pro Ser Pro Ser Gln Thr Ser Ser Leu Gly Thr Ala
        355                 360                 365

Phe Thr Gln His His Arg Pro Val Ile Thr Gly Pro Arg Ala Ser Pro
    370                 375                 380

His Ala Thr Pro Ser Thr Leu His Phe Pro Thr Ser Pro Ile Ile Gln
385                 390                 395                 400

Gln Pro Gly Pro Tyr Phe Ser His Pro Ala Ile Arg Tyr His Pro Gln
                405                 410                 415

Glu Thr Leu Lys Glu Phe Val Gln Leu Val Cys Pro Asp Ala Gly Gln
            420                 425                 430

Gln Ala Gly Gln Val Gly Phe Leu Asn Pro Asn Gly Ser Ser Gln Gly
        435                 440                 445

Lys Val His Asn Pro Phe Leu Pro Thr Pro Met Leu Pro Pro Pro Pro
    450                 455                 460

Pro Pro Pro Met Ala Arg Pro Val Pro Leu Pro Met Pro Asp Thr Lys
465                 470                 475                 480

Pro Pro Thr Thr Ser Thr Glu Gly Gly Ala Ala Ser Pro Thr Ser Pro
                485                 490                 495

Thr Tyr Ser Thr Pro Ser Thr Ser Pro Ala Asn Arg Phe Val Ser Val
            500                 505                 510

Gly Pro Arg Asp Pro Ser Phe Val Asn Ile Pro Gln Gln Thr Gln Ser
        515                 520                 525

Trp Tyr Leu Gly
    530


<210> 48
<211> 433
<212> PRT
<213> Homo sapiens

<300>
<301> Amemiy, K.
<302> Nuclear factor 1

<400> 48
Met Asp Glu Phe His Pro Phe Ile Glu Ala Leu Leu Pro His Val Arg
  1               5                  10                  15

Ala Phe Ser Tyr Thr Trp Phe Asn Leu Gln Ala Arg Lys Arg Lys Tyr
             20                  25                  30

Phe Lys Lys His Glu Lys Arg Met Ser Lys Asp Glu Glu Arg Ala Val
         35                  40                  45

Lys Asp Glu Leu Leu Gly Glu Lys Pro Glu Ile Lys Gln Lys Trp Ala
     50                  55                  60

Ser Arg Leu Leu Ala Lys Leu Arg Lys Asp Ile Arg Pro Glu Phe Arg
 65                  70                  75                  80

Glu Asp Phe Val Leu Thr Ile Thr Gly Lys Lys Pro Pro Cys Cys Val

85                  90                  95

Leu Ser Asn Pro Asp Gln Lys Gly Lys Ile Arg Arg Ile Asp Cys Leu
            100                 105                 110

Arg Gln Ala Asp Lys Val Trp Arg Leu Asp Leu Val Met Val Ile Leu
        115                 120                 125

Phe Lys Gly Ile Pro Leu Glu Ser Thr Asp Gly Glu Arg Leu Tyr Lys
    130                 135                 140

Ser Pro Gln Cys Ser Asn Pro Gly Leu Cys Val Gln Pro His His Ile
145                 150                 155                 160

Gly Val Thr Ile Lys Glu Leu Asp Leu Tyr Leu Ala Tyr Phe Val His
                165                 170                 175

Thr Pro Glu Ser Gly Gln Ser Asp Ser Ser Asn Gln Gln Gly Asp Ala
            180                 185                 190

Asp Ile Lys Pro Leu Pro Asn Gly His Leu Ser Phe Gln Asp Cys Phe
        195                 200                 205

Val Thr Ser Gly Val Trp Asn Val Thr Glu Leu Val Arg Val Ser Gln
    210                 215                 220

Thr Pro Val Ala Thr Ala Ser Gly Pro Asn Phe Ser Leu Ala Asp Leu
225                 230                 235                 240

Glu Ser Pro Ser Tyr Tyr Asn Ile Asn Gln Val Thr Leu Gly Arg Arg
                245                 250                 255

Ser Ile Thr Ser Pro Pro Ser Thr Ser Thr Thr Lys Arg Pro Lys Ser
            260                 265                 270

Ile Asp Asp Ser Glu Met Glu Ser Pro Val Asp Asp Val Phe Tyr Pro
        275                 280                 285

Gly Thr Gly Arg Ser Pro Ala Ala Gly Ser Ser Gln Ser Ser Gly Trp
    290                 295                 300

Pro Asn Asp Val Asp Ala Gly Pro Ala Ser Leu Lys Lys Ser Gly Lys
305                 310                 315                 320

Leu Asp Phe Cys Ser Ala Leu Ser Ser Gln Gly Ser Ser Pro Arg Met
                325                 330                 335

Ala Phe Thr His His Pro Leu Pro Val Leu Ala Gly Val Arg Pro Gly

            340                 345                 350

Ser Pro Arg Ala Thr Ala Ser Ala Leu His Phe Pro Ser Thr Ser Ile
        355                 360                 365

Ile Gln Gln Ser Ser Pro Tyr Phe Thr His Pro Thr Ile Arg Tyr His
    370                 375                 380

His His His Gly Gln Asp Ser Leu Lys Glu Phe Val Gln Phe Val Cys
385                 390                 395                 400

Ser Asp Gly Ser Gly Gln Ala Thr Gly Gln His Ser Gln Arg Gln Ala
                405                 410                 415

Pro Pro Leu Pro Thr Gly Leu Ser Ala Ser Asp Pro Gly Thr Ala Thr
            420                 425                 430

Phe

<210> 49
<211> 829
<212> DNA
<213> Mus musculus

<400> 49
caattctcct acgggctcca gactcagact ggtggaccca aacgcactcc ccttactaag 60
agggtcgcgg ccccacagga tggatcccct agagccccag aaacttctgg accacctcca 120
gtggatcatc cacctccttc aagtaaggct tccaggcctc cgcccatggg gagctgtcct 180
gctactggtg tggagccccc aagttcccca gtcattgagt ctgaaactct gatagaagac 240
gtgctgagac ctctggaaca ggcattggag gactgccatg gtcacacaaa gaaacaggta 300
tgtgatgata tcagccgacg cttggcgctg cttcaagaac agtgggctgg agggaagttg 360
tcaaaacctg taaaggaggg gatggcattg ctagtgcaag gaattttaan attcacccag 420
tgggatgcag cagatgacat tcaccgatca ttcatggttg accatgtgac tgaggtcagt 480
cagtggatgg tgggagtaaa aagattaatt gcagaaaagg aagagtctat cttcagagga 540
gaccaaagaa gagaaattta cagtggaacc tgagaaccag acaataccag gcttccaaca 600
gccatcataa tgcctgtggc tccccagact cacttcacct gacttcctat gccttagtgt 660
ggaaggcttc ttnttccttt ttaccaccag ggagactatt ggtcttgtgg gtcttgacca 720
aagatcctat ntagaccact gcaagatcac ttgttatgta catttcaata aacatctcaa 780
taaaatctca atggtgggaa gtgggtagga aagatgaaaa aaaaaaaag 829

<210> 50
<211> 875
<212> DNA
<213> Homo sapiens

<300>
<301> Lanz, R. B.
<302> A steroid receptor coactivator, SRA, functions as an
      RNA and is present in an SRC-1 complex
<303> Cell
<304> 1999
<305> 97
<306> 17-27
<313> 1 - 875

<400> 50
cgcttggcgg agctgtacgt gaagccgggc aacaaggaac gcggctggaa cgacccgccg 60
cagttctcat acgggctgca gacccaggcc ggcggaccca ggcgctcgct gcttaccaag 120
agggtagccg caccccagga tggatccccc agagtccccg catcagagac ttctcctggg 180
cctcccccaa tggggcctcc acctccttca agtaaggctc ccaggtcccc acctgtgggg 240
agtggtcctg cctctggcgt ggagcccaca agtttcccag tcgagtctga ggctgtgatg 300
gaggatgtgc tgagaccttt ggaacaggca ttggaagact gccgtggcca cacaaggaag 360
caggtatgtg atgacatcag ccgacgcctg gcactgctgc aggaacagtg ggctggagga 420
aagttgtcaa tacctgtaaa gaagagaatg gctctactgg tgcaagagct ttcaagccac 480
cggtgggacg cagcagatga catccaccgc tccctcatgg ttgaccatgt gactgaggtc 540
agtcagtgga tggtaggagt taaaagatta attgcagaaa agaggagtct gttttcagag 600
gaggcagcca atgaagagaa atctgcagcc acagctgaga agaaccatac cataccaggc 660
ttccagcagg cttcataatc ctcggttccc cagactcacc ggacaccatc tcctatgcct 720
tggagacctt ctgtcacttg gctcccttct taccaccacc aagactgtcc cactgggcct 780
gacccaccta tgagggaaga agtcccacct gggccagagg gagttcatgt gttactcata 840
acatgcattt caataaaaac atctctgcgg tggtg                            875


<210> 51
<211> 182
<212> PRT
<213> Mus musculus

<300>
<301> Schleef, M.
<302> Subtractive cDNA cloning as a tool to analyse secondary
      effects of a muscle disease. Characterisation of
      affected genes in the myotonic ADR mouse
<303> Neuromuscul. Disord.
<304> 1994
<305> 4
<306> 205-217
<313> 1 - 182

<400> 51
Met Gln Asp Ile Met Asp Arg Pro Pro Arg Ser Pro Lys Glu Pro Leu
  1               5                  10                  15

Ile Ser Gly Trp Leu Phe Phe Arg Tyr Met Ala Ile Gly Gly Tyr Val
            20                  25                  30

Gly Ala Ala Thr Val Gly Ala Ala Ala Trp Trp Phe Leu Tyr Ala Glu
        35                  40                  45

Asp Gly Pro His Val Ser Tyr His Gln Leu Thr His Phe Met Gln Cys
    50                  55                  60

Thr Glu His Asn Pro Glu Phe Asp Gly Leu Asp Cys Glu Val Phe Glu
65                  70                  75                  80

Ala Pro Glu Pro Met Thr Met Ala Leu Ser Glu Leu Val Thr Ile Glu
                85                  90                  95

Met Cys Asn Ala Leu Asn Ser Leu Ser Glu Asn Gln Ser Leu Leu Arg
            100                 105                 110

Met Pro Pro Trp Val Asn Ile Trp Leu Leu Gly Ser Ile Cys Leu Ser
        115                 120                 125

Met Ser Leu His Phe Leu Ile Leu Tyr Val Asp Pro Leu Pro Met Ile
    130                 135                 140

Phe Lys Leu Arg Ala Leu Asp Phe Thr Gln Trp Leu Met Val Leu Lys
145                 150                 155                 160

Ile Ser Leu Pro Val Ile Gly Leu Asp Glu Leu Leu Lys Phe Ile Ala
                165                 170                 175

Arg Asn Tyr Leu Glu Gly
            180

<210> 52
<211> 1001
<212> PRT
<213> Homo sapiens

<400> 52
Met Glu Ala Ala His Ala Lys Thr Thr Glu Glu Cys Leu Ala Tyr Phe
1               5                   10                  15

Gly Val Ser Glu Thr Thr Gly Leu Thr Pro Asp Gln Val Lys Arg Asn
            20                  25                  30

Leu Glu Lys Tyr Gly Leu Asn Glu Leu Pro Ala Glu Glu Gly Lys Thr
        35                  40                  45

Leu Trp Glu Leu Val Ile Glu Gln Phe Glu Asp Leu Leu Val Arg Ile
    50                  55                  60

Leu Leu Leu Ala Ala Cys Ile Ser Phe Val Leu Ala Trp Phe Glu Glu
65                  70                  75                  80

Gly Glu Glu Thr Ile Thr Ala Phe Val Glu Pro Phe Val Ile Leu Leu
                85                  90                  95

Ile Leu Ile Ala Asn Ala Ile Val Gly Val Trp Gln Glu Arg Asn Ala
            100                 105                 110

Glu Asn Ala Ile Glu Ala Leu Lys Glu Tyr Glu Pro Glu Met Gly Lys
        115                 120                 125

Val Tyr Arg Ala Asp Arg Lys Ser Val Gln Arg Ile Lys Ala Arg Asp
    130                 135                 140

Ile Val Pro Gly Asp Ile Val Glu Val Ala Val Gly Asp Lys Val Pro
145                 150                 155                 160

Ala Asp Ile Arg Ile Leu Ala Ile Lys Ser Thr Thr Leu Arg Val Asp
                165                 170                 175

Gln Ser Ile Leu Thr Gly Glu Ser Val Ser Val Ile Lys His Thr Glu
            180                 185                 190

Pro Val Pro Asp Pro Arg Ala Val Asn Gln Asp Lys Lys Asn Met Leu
        195                 200                 205

Phe Ser Gly Thr Asn Ile Ala Ala Gly Lys Ala Leu Gly Ile Val Ala
    210                 215                 220

Thr Thr Gly Val Gly Thr Glu Ile Gly Lys Ile Arg Asp Gln Met Ala
225                 230                 235                 240

Ala Thr Glu Gln Asp Lys Thr Pro Leu Gln Gln Lys Leu Asp Glu Phe
                245                 250                 255

Gly Glu Gln Leu Ser Lys Val Ile Ser Leu Ile Cys Val Ala Val Trp
            260                 265                 270

Leu Ile Asn Ile Gly His Phe Asn Asp Pro Val His Gly Gly Ser Trp
        275                 280                 285

Phe Arg Gly Ala Ile Tyr Tyr Phe Lys Ile Ala Val Ala Leu Ala Val
    290                 295                 300

Ala Ala Ile Pro Glu Gly Leu Pro Ala Val Ile Thr Thr Cys Leu Ala
305                 310                 315                 320

Leu Gly Thr Arg Arg Met Ala Lys Lys Asn Ala Ile Val Arg Ser Leu
                325                 330                 335

Pro Ser Val Glu Thr Leu Gly Cys Thr Ser Val Ile Cys Ser Asp Lys
            340                 345                 350

Thr Gly Thr Leu Thr Thr Asn Gln Met Ser Val Cys Lys Met Phe Ile
        355                 360                 365

Ile Asp Lys Val Asp Gly Asp Ile Cys Leu Leu Asn Glu Phe Ser Ile
    370                 375                 380

Thr Gly Ser Thr Tyr Ala Pro Glu Gly Glu Val Leu Lys Asn Asp Lys
385                 390                 395                 400

Pro Val Arg Pro Gly Gln Tyr Asp Gly Leu Val Glu Leu Ala Thr Ile
                405                 410                 415

Cys Ala Leu Cys Asn Asp Ser Ser Leu Asp Phe Asn Glu Ala Lys Gly
            420                 425                 430

Val Tyr Glu Lys Val Gly Glu Ala Thr Glu Thr Ala Leu Thr Thr Leu
        435                 440                 445

Val Glu Lys Met Asn Val Phe Asn Thr Asp Val Arg Ser Leu Ser Lys
    450                 455                 460

Val Glu Arg Ala Asn Ala Cys Asn Ser Val Ile Arg Gln Leu Met Lys
465                 470                 475                 480

Lys Glu Phe Thr Leu Glu Phe Ser Arg Asp Arg Lys Ser Met Ser Val
                485                 490                 495

Tyr Cys Ser Pro Ala Lys Ser Ser Arg Ala Ala Val Gly Asn Lys Met
            500                 505                 510

Phe Val Lys Gly Ala Pro Glu Gly Val Ile Asp Arg Cys Asn Tyr Val
        515                 520                 525

Arg Val Gly Thr Thr Arg Val Pro Leu Thr Gly Pro Val Lys Glu Lys
    530                 535                 540

Ile Met Ala Val Ile Lys Glu Trp Gly Thr Gly Arg Asp Thr Leu Arg
545                 550                 555                 560

Cys Leu Ala Leu Ala Thr Arg Asp Thr Pro Pro Lys Arg Glu Glu Met
                565                 570                 575

Val Leu Asp Asp Ser Ala Arg Phe Leu Glu Tyr Glu Thr Asp Leu Thr
            580                 585                 590

Phe Val Gly Val Val Gly Met Leu Asp Pro Pro Arg Lys Glu Val Thr
        595                 600                 605

Gly Ser Ile Gln Leu Cys Arg Asp Ala Gly Ile Arg Val Ile Met Ile
    610                 615                 620

Thr Gly Asp Asn Lys Gly Thr Ala Ile Ala Ile Cys Arg Arg Ile Gly
625                 630                 635                 640

Ile Phe Gly Glu Asn Glu Glu Val Ala Asp Arg Ala Tyr Thr Gly Arg
                645                 650                 655

Glu Phe Asp Asp Leu Pro Leu Ala Glu Gln Arg Glu Ala Cys Arg Arg
            660                 665                 670

Ala Cys Cys Phe Ala Arg Val Glu Pro Ser His Lys Ser Lys Ile Val
        675                 680                 685

Glu Tyr Leu Gln Ser Tyr Asp Glu Ile Thr Ala Met Thr Gly Asp Gly
    690                 695                 700

Val Asn Asp Ala Pro Ala Leu Lys Lys Ala Glu Ile Gly Ile Ala Met
705                 710                 715                 720

Gly Ser Gly Thr Ala Val Ala Lys Thr Ala Ser Glu Met Val Leu Ala
                725                 730                 735

Asp Asp Asn Phe Ser Thr Ile Val Ala Ala Val Glu Glu Gly Arg Ala
            740                 745                 750

Ile Tyr Asn Asn Met Lys Gln Phe Ile Arg Tyr Leu Ile Ser Ser Asn
        755                 760                 765

Val Gly Glu Val Val Cys Ile Phe Leu Thr Ala Ala Leu Gly Leu Pro
    770                 775                 780

Glu Ala Leu Ile Pro Val Gln Leu Leu Trp Val Asn Leu Val Thr Asp
785                 790                 795                 800

Gly Leu Pro Ala Thr Ala Leu Gly Phe Asn Pro Pro Asp Leu Asp Ile
                805                 810                 815

Met Asp Arg Pro Pro Arg Ser Pro Lys Glu Pro Leu Ile Ser Gly Trp
            820                 825                 830

Leu Phe Phe Arg Tyr Met Ala Ile Gly Gly Tyr Val Gly Ala Ala Thr
        835                 840                 845

Val Gly Ala Ala Ala Trp Trp Phe Leu Tyr Ala Glu Asp Gly Pro His
    850                 855                 860

Val Asn Tyr Ser Gln Leu Thr His Phe Met Gln Cys Thr Glu Asp Asn
865                 870                 875                 880

Thr His Phe Glu Gly Ile Asp Cys Glu Val Phe Glu Ala Pro Glu Pro
                885                 890                 895

Met Thr Met Ala Leu Ser Val Leu Val Thr Ile Glu Met Cys Asn Ala
            900                 905                 910

Leu Asn Ser Leu Ser Glu Asn Gln Ser Leu Leu Arg Met Pro Pro Trp
        915                 920                 925

Val Asn Ile Trp Leu Leu Gly Ser Ile Cys Leu Ser Met Ser Leu His
    930                 935                 940

Phe Leu Ile Leu Tyr Val Asp Pro Leu Pro Met Ile Phe Lys Leu Arg
945                 950                 955                 960

Ala Leu Asp Leu Thr Gln Trp Leu Met Val Leu Lys Ile Ser Leu Pro
                965                 970                 975

Val Ile Gly Leu Asp Glu Ile Leu Lys Phe Val Ala Arg Asn Tyr Leu
            980                 985                 990

Glu Asp Pro Glu Asp Glu Arg Arg Lys
        995                 1000

<210> 53
<211> 89
<212> PRT
<213> Mus musculus

<400> 53
Met Pro Ser Glu Leu Glu Lys Ala Leu Ser Asn Leu Ile Asp Val Tyr
1               5                   10                  15

His Asn Tyr Ser Asn Ile Gln Gly Asn His His Ala Leu Tyr Lys Asn

            20                  25                  30

Asp Phe Lys Lys Met Val Thr Thr Glu Cys Pro Gln Phe Val Gln Asn
        35                  40                  45

Ile Asn Ile Glu Asn Leu Phe Arg Glu Leu Asp Ile Asn Ser Asp Asn
    50                  55                  60

Ala Ile Asn Phe Glu Glu Phe Leu Ala Met Val Ile Lys Val Gly Val
65                  70                  75                  80

Ala Ser His Lys Asp Ser His Lys Glu
                85

<210> 54
<211> 93
<212> PRT
<213> Homo sapiens

<300>
<301> Dorin, J. R.
<302> A clue to the basic defect in cystic fibrosis from
cloning the CF antigen gene
<303> Nature
<304> 1987
<305> 326
<306> 614-617
<313> 1 - 93

<400> 54
Met Leu Thr Glu Leu Glu Lys Ala Leu Asn Ser Ile Ile Asp Val Tyr
1               5                   10                  15

His Lys Tyr Ser Leu Ile Lys Gly Asn Phe His Ala Val Tyr Arg Asp
            20                  25                  30

Asp Leu Lys Lys Leu Leu Glu Thr Glu Cys Pro Gln Tyr Ile Arg Lys
        35                  40                  45

Lys Gly Ala Asp Val Trp Phe Lys Glu Leu Asp Ile Asn Thr Asp Gly
    50                  55                  60

Ala Val Asn Phe Gln Glu Phe Leu Ile Leu Val Ile Lys Met Gly Val
65                  70                  75                  80

Ala Ala His Lys Lys Ser His Glu Glu Ser His Lys Glu
                85                  90

<210> 55
<211> 134
<212> PRT
<213> Homo sapiens

<400> 55
Met Ser Tyr Asn Cys Cys Ser Gly Asn Phe Ser Ser Arg Ser Cys Gly
1               5                   10                  15

Asp Tyr Leu Arg Tyr Pro Ala Ser Ser Arg Gly Phe Ser Tyr Pro Ser
            20                  25                  30

Asn Leu Val Tyr Ser Thr Asp Leu Cys Ser Pro Ser Thr Cys Gln Leu
        35                  40                  45

Gly Ser Ser Leu Tyr Arg Gly Cys Gln Glu Ile Cys Trp Glu Pro Thr
    50                  55                  60

Ser Cys Gln Thr Ser Tyr Val Glu Ser Ser Pro Cys Gln Thr Ser Cys
65                  70                  75                  80

Tyr Arg Pro Arg Thr Ser Leu Leu Cys Ser Pro Cys Lys Thr Thr Tyr
                85                  90                  95

Ser Gly Ser Leu Gly Phe Gly Ser Ser Ser Cys Arg Ser Leu Gly Tyr
            100                 105                 110

Gly Ser Arg Ser Cys Tyr Ser Val Gly Cys Gly Ser Ser Gly Val Arg
        115                 120                 125

Ser Leu Gly Tyr Gly Ser
    130


<210> 56
<211> 306
<212> PRT
<213> Mus musculus

<400> 56
Met Ala Ser Ser Gly Ser Val Gln Gln Leu Pro Leu Val Leu Leu Met
1               5                   10                  15

Leu Leu Leu Ala Ser Ala Ala Arg Ala Arg Leu Tyr Phe Arg Ser Gly
            20                  25                  30

Gln Thr Cys Tyr His Pro Ile Arg Gly Asp Gln Leu Ala Leu Leu Gly
        35                  40                  45

Arg Arg Thr Tyr Pro Arg Pro His Glu Tyr Leu Ser Pro Ala Asp Leu
    50                  55                  60

Pro Lys Asn Trp Asp Trp Arg Asn Val Asn Gly Val Asn Tyr Ala Ser
65                  70                  75                  80

Val Thr Arg Asn Gln His Ile Pro Gln Tyr Cys Gly Ser Cys Trp Ala
                85                  90                  95

His Gly Ser Thr Ser Ala Met Ala Asp Arg Ile Asn Ile Lys Arg Lys
            100                 105                 110

Gly Ala Trp Pro Ser Ile Leu Leu Ser Val Gln Asn Val Ile Asp Cys
        115                 120                 125

Gly Asn Ala Gly Ser Cys Glu Gly Gly Asn Asp Leu Pro Val Trp Glu
    130                 135                 140

Tyr Ala His Lys His Gly Ile Pro Asp Glu Thr Cys Asn Asn Tyr Gln
145                 150                 155                 160

Ala Lys Asp Gln Asp Cys Asp Lys Phe Asn Gln Cys Gly Thr Cys Thr
                165                 170                 175

Glu Phe Lys Glu Cys His Thr Ile Gln Asn Tyr Thr Leu Trp Arg Val
            180                 185                 190

Gly Asp Tyr Gly Ser Leu Ser Gly Arg Glu Lys Met Met Ala Glu Ile
        195                 200                 205

Tyr Ala Asn Gly Pro Ile Ser Cys Gly Ile Met Ala Thr Glu Met Met
    210                 215                 220

Ser Asn Tyr Thr Gly Gly Ile Tyr Ala Glu His Gln Asp Gln Ala Val
225                 230                 235                 240

Ile Asn His Ile Ile Ser Val Ala Gly Trp Gly Val Ser Asn Asp Gly
                245                 250                 255

Ile Glu Tyr Trp Ile Val Arg Asn Ser Trp Gly Glu Pro Trp Gly Glu
            260                 265                 270

Lys Gly Trp Met Arg Ile Val Thr Ser Thr Tyr Lys Gly Gly Thr Gly
        275                 280                 285

Asp Ser Tyr Asn Leu Ala Ile Glu Ser Ala Cys Thr Phe Gly Asp Pro
    290                 295                 300

Ile Val
305

<210> 57
<211> 303
<212> PRT
<213> Homo sapiens

<400> 57
Met Ala Arg Arg Gly Pro Gly Trp Arg Pro Leu Leu Leu Leu Val Leu
  1               5                  10                  15

Leu Ala Gly Ala Ala Gln Gly Gly Leu Tyr Phe Arg Arg Gly Gln Thr
             20                  25                  30

Cys Tyr Arg Pro Leu Arg Gly Asp Gly Leu Ala Pro Leu Gly Arg Thr
         35                  40                  45

Thr Tyr Pro Arg Pro His Glu Tyr Leu Ser Pro Ala Asp Leu Pro Lys
     50                  55                  60

Ser Trp Asp Trp Arg Asn Val Asp Gly Val Asn Tyr Ala Ser Ile Thr
 65                  70                  75                  80

Arg Asn Gln His Ile Pro Gln Tyr Cys Gly Ser Cys Trp Ala His Ala
                 85                  90                  95

Ser Thr Ser Ala Met Ala Asp Arg Ile Asn Ile Lys Arg Lys Gly Ala
            100                 105                 110

Trp Pro Ser Thr Leu Leu Ser Val Gln Asn Val Ile Asp Cys Gly Asn
        115                 120                 125

Ala Gly Ser Cys Glu Gly Gly Asn Asp Leu Ser Val Trp Asp Tyr Ala
    130                 135                 140

His Gln His Gly Ile Pro Asp Glu Thr Cys Asn Asn Tyr Gln Ala Lys
145                 150                 155                 160

Asp Gln Glu Cys Asp Lys Phe Asn Gln Cys Gly Thr Cys Asn Glu Phe
                165                 170                 175

Lys Glu Cys His Ala Ile Arg Asn Tyr Thr Leu Trp Arg Val Gly Asp
            180                 185                 190

Tyr Gly Ser Leu Ser Gly Arg Glu Lys Met Met Ala Glu Ile Tyr Ala
        195                 200                 205

Asn Gly Pro Ile Ser Cys Gly Ile Met Ala Thr Glu Arg Leu Ala Asn
    210                 215                 220

Tyr Thr Gly Gly Ile Tyr Ala Glu Tyr Gln Asp Thr Thr Tyr Ile Asn
225                 230                 235                 240

His Val Val Ser Val Ala Gly Trp Gly Ile Ser Asp Gly Thr Glu Tyr
                245                 250                 255

Trp Ile Val Arg Asn Ser Trp Gly Glu Pro Trp Gly Glu Arg Gly Trp
            260                 265                 270

Leu Arg Ile Val Thr Ser Thr Tyr Lys Asp Gly Lys Gly Ala Arg Tyr
        275                 280                 285

Asn Leu Ala Ile Glu Glu His Cys Thr Phe Gly Asp Pro Ile Val
    290                 295                 300

<210> 58
<211> 469
<212> PRT
<213> Homo sapiens

<400> 58
Met Asp Ile Glu Asn Glu Gln Thr Leu Asn Val Asn Pro Thr Asp Pro
  1               5                  10                  15

Asp Asn Leu Ser Asp Ser Leu Phe Ser Gly Asp Glu Glu Asn Ala Gly
             20                  25                  30

Thr Glu Glu Ile Lys Asn Glu Ile Asn Gly Asn Trp Ile Ser Ala Ser
         35                  40                  45

Thr Ile Asn Glu Ala Arg Ile Asn Ala Lys Ala Lys Arg Arg Leu Arg
     50                  55                  60

Lys Asn Ser Ser Arg Asp Ser Gly Arg Gly Asp Ser Val Ser Asp Asn
 65                  70                  75                  80

Gly Ser Glu Ala Val Arg Ser Gly Val Ala Val Pro Thr Ser Pro Lys
                 85                  90                  95

Gly Arg Leu Leu Asp Arg Arg Ser Arg Ser Gly Lys Gly Arg Gly Leu

            100                 105                 110

Pro Lys Lys Gly Gly Ala Gly Gly Lys Gly Val Trp Gly Thr Pro Gly
        115                 120                 125

Gln Val Tyr Asp Val Glu Glu Val Asp Val Lys Asp Pro Asn Tyr Asp
    130                 135                 140

Asp Asp Gln Glu Asn Cys Val Tyr Glu Thr Val Val Leu Pro Leu Asp
145                 150                 155                 160

Glu Thr Ala Phe Glu Lys Thr Leu Thr Pro Ile Ile Gln Glu Tyr Phe
                165                 170                 175

Glu His Gly Asp Thr Asn Glu Val Ala Glu Met Leu Arg Asp Leu Asn
            180                 185                 190

Leu Gly Glu Met Lys Ser Gly Val Pro Val Leu Ala Val Ser Leu Ala
        195                 200                 205

Leu Glu Gly Lys Ala Ser His Arg Glu Met Thr Ser Lys Leu Leu Ser
    210                 215                 220

Asp Leu Cys Gly Thr Val Met Ser Thr Asn Asp Val Glu Lys Ser Phe
225                 230                 235                 240

Asp Lys Leu Leu Lys Asp Leu Pro Glu Leu Ala Leu Asp Thr Pro Arg
                245                 250                 255

Ala Pro Gln Leu Val Gly Gln Phe Ile Ala Arg Ala Val Gly Asp Gly
            260                 265                 270

Ile Leu Cys Asn Thr Tyr Ile Asp Ser Tyr Lys Gly Thr Val Asp Cys
        275                 280                 285

Val Gln Ala Arg Ala Ala Leu Asp Lys Ala Thr Val Leu Leu Ser Met
    290                 295                 300

Ser Lys Gly Gly Lys Arg Lys Asp Ser Val Trp Gly Ser Gly Gly Gly
305                 310                 315                 320

Gln Gln Pro Val Asn His Leu Val Lys Glu Ile Asp Met Leu Leu Lys
                325                 330                 335

Glu Tyr Leu Leu Ser Gly Asp Ile Ser Glu Ala Glu His Cys Leu Lys
            340                 345                 350

Glu Leu Glu Val Pro His Phe His His Glu Leu Val Tyr Glu Ala Ile

355                 360                 365

Val Met Val Leu Glu Ser Thr Gly Glu Ser Ala Phe Lys Met Ile Leu
    370                 375                 380

Asp Leu Leu Lys Ser Leu Trp Lys Ser Ser Thr Ile Thr Ile Asp Gln
385                 390                 395                 400

Met Lys Arg Gly Tyr Glu Arg Ile Tyr Asn Glu Ile Pro Asp Ile Asn
                405                 410                 415

Leu Asp Val Pro His Ser Tyr Ser Val Leu Glu Arg Phe Val Glu Glu
            420                 425                 430

Cys Phe Gln Ala Gly Ile Ile Ser Lys Gln Leu Arg Asp Leu Cys Pro
        435                 440                 445

Ser Arg Gly Arg Lys Arg Phe Val Ser Glu Gly Asp Gly Gly Arg Leu
    450                 455                 460

Lys Pro Glu Ser Tyr
465

<210> 59
<211> 418
<212> PRT
<213> Mus musculus

<400> 59
Met Ala Phe Ile Ala Ala Leu Gly Leu Leu Met Ala Arg Ile Cys Pro
  1               5                  10                  15

Ala Val Leu Ser Phe Pro Asp Gly Thr Leu Gly Met Asp Ala Ala Val
             20                  25                  30

Gln Glu Asp His Asp Asn Gly Thr Gln Leu Asp Ser Leu Thr Leu Ala
         35                  40                  45

Ser Ile Asn Thr Asp Phe Ala Phe Ser Leu Tyr Lys Glu Leu Val Leu
     50                  55                  60

Lys Asn Pro Asp Thr Asn Ile Val Phe Ser Pro Leu Ser Ile Ser Ala
 65                  70                  75                  80

Ala Leu Ala Leu Val Ser Leu Gly Ala Lys Gly Asn Thr Leu Glu Glu
                 85                  90                  95

Ile Leu Glu Gly Leu Lys Phe Asn Leu Thr Glu Thr Ser Glu Ala Asp
            100                 105                 110

Ile His Gln Gly Phe Gly His Leu Leu Gln Arg Leu Asn Gln Pro Lys
        115                 120                 125

Asp Gln Val Gln Ile Ser Thr Gly Ser Ala Leu Phe Ile Glu Lys Arg
    130                 135                 140

Gln Gln Ile Leu Thr Glu Phe Gln Glu Lys Ala Lys Thr Leu Tyr Gln
145                 150                 155                 160

Ala Glu Ala Phe Thr Ala Asp Phe Gln Gln Pro Arg Gln Ala Lys Lys
                165                 170                 175

Leu Ile Asn Asp Tyr Val Arg Lys Gln Thr Gln Gly Met Ile Lys Glu
            180                 185                 190

Leu Val Ser Asp Leu Asp Lys Arg Thr Leu Met Val Leu Val Asn Tyr
        195                 200                 205

Ile Tyr Phe Lys Ala Lys Trp Lys Val Pro Phe Asp Pro Leu Asp Thr
    210                 215                 220

Phe Lys Ser Glu Phe Tyr Ala Gly Lys Arg Arg Pro Val Ile Val Pro
225                 230                 235                 240

Met Met Ser Met Glu Asp Leu Thr Thr Pro Tyr Phe Arg Asp Glu Glu
                245                 250                 255

Leu Ser Cys Thr Val Val Glu Leu Lys Tyr Thr Gly Asn Ala Ser Ala
            260                 265                 270

Leu Phe Ile Leu Pro Asp Gln Gly Arg Met Gln Gln Val Glu Ala Ser
        275                 280                 285

Leu Gln Pro Glu Thr Leu Arg Lys Trp Lys Asn Ser Leu Lys Pro Arg
    290                 295                 300

Met Ile Asp Glu Leu His Leu Pro Lys Phe Ser Ile Ser Thr Asp Tyr
305                 310                 315                 320

Ser Leu Glu Asp Val Leu Ser Lys Leu Gly Ile Arg Glu Val Phe Ser
                325                 330                 335

Thr Gln Ala Asp Leu Ser Ala Ile Thr Gly Thr Lys Asp Leu Arg Val
            340                 345                 350

Ser Gln Val Val His Lys Ala Val Leu Asp Val Ala Glu Thr Gly Thr
        355                 360                 365

Glu Ala Ala Ala Ala Thr Gly Val Lys Phe Val Pro Met Ser Ala Lys
    370                 375                 380

Leu Tyr Pro Leu Thr Val Tyr Phe Asn Arg Pro Phe Leu Ile Met Ile
385                 390                 395                 400

Phe Asp Thr Glu Thr Glu Ile Ala Pro Phe Ile Ala Lys Ile Ala Asn
                405                 410                 415

Pro Lys

<210> 60
<211> 423
<212> PRT
<213> Homo sapiens

<400> 60

<210> 61
<211> 542
<212> DNA
<213> Mus musculus

<400> 61
agcagktata tgtttattga tcctttgctc ctacctcttt attctggttg gtggtgttcc 60
tcttttgtgt gaggttggtc agctgggaac taaaagaaag gctactttgt tctaagtagg 120
agtgccaggg gaaaggatct gctctgaccc acagacccca tgcagctgca gaagtgaggg 180
ctctaactct gtgccagatg tggacaaagt gaggagatcc tgcagggggg cagtgcctgg 240
aggcaaagcc tggagaagtg aaagagtgag gcagactggg caccaagtag tcctagatgc 300
tgcggataga cacggggcag caagctttcc tcttggtcca tatgggttgg cctgtaaggt 360
tgcacagtcc catcaaraca gcaaggctga agggagacat aaccagagtc taaactggga 420
ctgagcttac ttgatggggc ccangctttt gggtggaggc asatacaggg agagagcagg 480
ggtgggggac aaaanaaatt tcangctcaa gaacatgggc tngtgctcca aaaaatcttg 540
ga                                                                542

<210> 62
<211> 2035
<212> DNA
<213> Mus musculus

<400> 62
cacgagcaca gaggctggca gctggctggt ttcagctctg cagactgcag aacacagaag 60
atggctttca ttgcagctct ggggctcttg atggctggga tctgccctgc tgtcctctgc 120
ttcccagatg gcacgttggg aatggatgct gcagtccaag aagaccatga caatgggaca 180
caactggaca gtctcacatt ggcctccatc aacactgact ttgccttcag cctctacaag 240
gagctggttt tgaagaatcc agataaaaat attgtcttct ccccacttag catctcagcg 300
gccttggctg tcatgtccct gggagcaaag ggcaacaccc tggaagagat tctagaaggt 360
ctcaagttca atcttacaga gacctctgag gcagacatcc accagggctt tgggcacctc 420
ctacagaggc tcaaccagcc aaaggaccag gtacagatca gcacgggtag tgccctgttt 480
attgaaaagc gccagcagat cctgacagaa ttccaggaga aggcaaagac tctgtaccag 540
gctgaggcct tcacagcaga cttccagcag cctcgacagg ccaaaaagct catcaatgac 600
tatgtgagga aacagaccca ggggatgatc aaggaactgg tctcagacct ggataaaagg 660
acattgatgg tgctggtgaa ttatatctac tttaaagcca aatggaaggt gccctttgac 720
cctcttgaca cgttcaagtc tgagttctac tgcggcaaga ggaggcccgt gatagtgccc 780
atgatgagca tggaggacct gaccacaccc tacttccgag atgaggagct gtcctgcact 840
gtggtggagc tgaagtacac aggaaatgcc agcgccctgt tcatcctccc tgaccagggc 900
aggatgcagc aggtggaagc cagcttacaa ccagagaccc tgaggaagtg gaagaattct 960

ctgaaaccca ggatgataga tgagctccac ctgcccaagt tctccatctc caccgactac 1020
agcctggagg atgtcctttc aaagctgggc atcagggaag tcttctccac acaggctgac 1080
ctgtctgcaa tcacaggaac caaggatctg agagtgtctc aggtggtcca caaggctgtg 1140
ctggatgtgg ctgagacagg cacagaagca gctgctgcca ctggagtcaa atttgtccca 1200
atgtctgcga aactgtaccc tctgactgta tatttcaatc ggcctttcct gataatgatc 1260
tttgacacag aaactgaaat tgcccccttt atagccaaga tagccaaccc caaatgagac 1320
tagaactccc caagtgttga cgcttcttcc cgggagccag gcattgagcc tgtctgtggg 1380
tctccatgtg cattttggct tccatgctct gcttggcctt ggcatgcctg gattagatag 1440
tgactaactg tgttataacc tcatgtacag acatccctgt gggaagtcag tgccgtgctc 1500
ccagacttct tggtagcact agcccatgtt cctgagcctg aaatttgtct tgtcccctac 1560
ccctgctctc tccctgtatc tgcctccacc caaaagcctg ggccccatca agtaagctca 1620
gtcccagttt agactctggt tatgtctccc ttcagccttg ctgtcttgat gggactgtgc 1680
aaccttacag gccaacccat atggaccaag aggaaagctt ggctggcccg tgtctatccg 1740
cagcatctag gactacttgg tgcccagtct gcctcactct ttcatttctc caggctttgc 1800
ctccaggcac tgcccccctg caggatctcc tcactttgtc cacatctggc acagagttag 1860
agccctcact tctgcagctg catggggtct gtgggtcaga gcagatcctt tcccctggca 1920
ctcctactta gaacaaagta gcctttcttt tagttcccag ctgaccaacc tcacacaaaa 1980
gaggaacacc aaccagaata aagaggtagg agcaaaggat caataaacat gtaac 2035

<210> 63
<211> 120
<212> DNA
<213> Mus musculus

<400> 63
accacagtcc atgccatcac tgccacccag aagactgtgg atggcccctc tggaaagctg 60
tggcgtgatg gccgtggggc tgcccagaac atcatccctg catccactgg tgctgccaag 120

<210> 64
<211> 1310
<212> DNA
<213> Mus musculus

<400> 64
actgcagaac acagaagatg gctttcattg cagctctggg gctcttgatg gctaggatct 60
gccctgctgt cctctccttc ccagatggca cgttgggaat ggatgctgca gtccaagaag 120
accatgacaa tgggacacaa ctggacagtc tcacattggc ctccatcaac actgactttg 180
ccttcagcct ctacaaggag ctggttttga agaatccaga tacaaatatt gtcttctccc 240
cacttagcat ctcagcggcc ttggccctcg tgtccctggg agcaaagggc aacaccctgg 300
aagagattct agaaggtctc aagttcaatc ttacagagac ctctgaggca gacatccacc 360
agggctttgg gcacctccta cagaggctca accagccaaa ggaccaggta cagatcagca 420
cgggtagtgc cctgtttatt gaaaagcgcc agcagatcct gacagaattc caggagaagg 480
caaagactct gtaccaggct gaggccttca cagcagactt ccagcagcct cgacaggcca 540
aaaagctcat caatgactat gtgaggaaac agacccaggg gatgatcaag gaactggtct 600
cagacctgga taaaaggaca ttgatggtgc tggtgaatta tatctacttt aaagccaaat 660
ggaaggtgcc ctttgaccct cttgacacgt tcaagtctga gttctacgcg ggcaagagga 720

ggcccgtgat agtgcccatg atgagcatgg aggacctgac cacaccctac ttccgagatg 780
aggagctgtc ctgcactgtg gtggagctga agtacacagg aaatgccagc gccctgttca 840
tcctccctga ccagggcagg atgcagcagg tggaagccag cttacaacca gagaccctga 900
ggaagtggaa gaattctctg aaacccagga tgatagatga gctccacctg cccaagttct 960
ccatctccac cgactacagc ctggaggatg tcctttcaaa gctgggcatc agggaagtct 1020
tctccacaca ggctgacctg tctgcaatca caggaaccaa ggatctgaga gtctctcagg 1080
tggtccacaa ggctgtgctg gatgtggctg agacaggcac agaagcagct gctgccactg 1140
gagtcaaatt tgtcccaatg tctgcgaaac tgtaccctct gactgtatat ttcaatcggc 1200
ctttcctgat aatgatcttt gacacagaaa ctgaaattgc cccctttata gccaagatag 1260
ccaaccccaa atgagactag aactccccaa gtgttgacgc ttcttcccgg 1310

<210> 65
<211> 20
<212> DNA
<213> Künstliche Sequenz

<220>
<223> Beschreibung der künstlichen Sequenz:Universal
      primer

<400> 65
agcgtggtcg cggccgaggt 20

<210> 66
<211> 21
<212> DNA
<213> Künstliche Sequenz

<220>
<223> Beschreibung der künstlichen Sequenz:Universal
      primer

<400> 66
tccagttgtg tcccattgtc a 21

<210> 67
<211> 21
<212> DNA
<213> Mus musculus

<400> 67
ctgtcctctg cttcccagat g 21

<210> 68

<211> 21
<212> DNA
<213> Mus musculus

<400> 68
tccagttgtg tcccattgtc a                                                21


<210> 69
<211> 19
<212> DNA
<213> Mus musculus

<400> 69
atcaacggga agcccatca                                                   19


<210> 70
<211> 20
<212> DNA
<213> Mus musculus

<400> 70
gacatactca gcaccggcct                                                  20


<210> 71
<211> 30
<212> DNA
<213> Mus musculus

<400> 71
actgcagaac acagaagatg gctttcattg                                       30


<210> 72
<211> 30
<212> DNA
<213> Mus musculus

<400> 72
ccgggaagaa gcgtcaacac ttggggagtt                                       30


<210> 73
<211> 30
<212> DNA
<213> Homo sapiens

<400> 73
aactcagctg aactcacatc tcccgtcaac                                        30

<210> 74
<211> 30
<212> DNA
<213> Homo sapiens

<400> 74
gtctgaaaga actagcctgt ccagccagta                                        30

<210> 75
<211> 22
<212> DNA
<213> Homo sapiens

<400> 75
catgggtgtg aaccatgaga ag                                                22

<210> 76
<211> 21
<212> DNA
<213> Homo sapiens

<400> 76
ctaagcagtt ggtggtgcag g                                                 21

<210> 77
<211> 19
<212> DNA
<213> Homo sapiens

<400> 77
gaggccattt ccagcgact                                                    19

<210> 78
<211> 20
<212> DNA
<213> Homo sapiens

<400> 78
gaataacccg acatggcgtc                                                   20

<210> 79
<211> 43
<212> DNA
<213> Künstliche Sequenz

<220>
<223> Beschreibung der künstlichen Sequenz:Antisense
      primer that fuses  the T7 RNA polymerase promoter
      with a region homologous to human MRP8

<400> 79
taatacgact cactataggg cccacgccca tctttatcac cag                    43


<210> 80
<211> 43
<212> DNA
<213> Künstliche Sequenz

<220>
<223> Beschreibung der künstlichen Sequenz:Sense primer
      that fuses  the T3  RNA polymerase promoter with a
      region homologous to human MRP8

<400> 80
aattaaccct cactaaaggg ggaatttcca tgccgtctac agg                    43


<210> 81
<211> 42
<212> DNA
<213> Künstliche Sequenz

<220>
<223> Beschreibung der künstlichen Sequenz:Antisense
      primer that fuses  the T3  RNA polymerase promoter
      with a region homologous to human p68 helicase

<400> 81
aattaaccct cactaaaggg ggcaacatta cttccatatt gc                     42


<210> 82
<211> 41
<212> DNA
<213> Künstliche Sequenz

<220>
<223> Beschreibung der künstlichen Sequenz:Sense primer
      that fuses  the T7 RNA polymerase promoter with a
      region homologous to human p68 helicase

<400> 82
taatacgact cactataggg cgagacaggg aaaactatga c                          41


<210> 83
<211> 22
<212> DNA
<213> Mus musculus

<400> 83
ccttatctct gtgcttcggg aa                                               22


<210> 84
<211> 21
<212> DNA
<213> Mus musculus

<400> 84
cgacctgaac ctctgtcttc g                                                21


<210> 85
<211> 15
<212> PRT
<213> Homo sapiens

<400> 85
Arg Glu Phe Gln Arg Gly Thr Cys Ser Arg Pro Asp Thr Glu Cys
  1               5                  10                  15


<210> 86
<211> 16
<212> PRT
<213> Homo sapiens

<400> 86
Arg Glu Tyr Gln Arg Gly Asn Cys Asn Arg Gly Glu Asn Asp Cys Arg
  1               5                  10                  15

<210> 87
<211> 15
<212> PRT
<213> Homo sapiens

<400> 87
Cys Asp Glu Leu Thr Arg Lys Met Arg Arg Asp Gly Trp Pro Ala
1               5                   10                  15

<210> 88
<211> 16
<212> PRT
<213> Homo sapiens

<400> 88
Asn Thr Phe Arg Asp Arg Glu Asn Tyr Asp Arg Gly Tyr Ser Ser Cys
1               5                   10                  15

<210> 89
<211> 175
<212> PRT
<213> Homo sapiens

<400> 89
Met Ser Tyr Asn Cys Cys Ser Gly Asn Phe Ser Ser Arg Ser Cys Gly
1               5                   10                  15

Asp Tyr Leu Arg Tyr Pro Ala Ser Ser Arg Gly Phe Ser Tyr Pro Ser
            20                  25                  30

Asn Leu Val Tyr Ser Thr Asp Leu Cys Ser Pro Ser Thr Cys Gln Leu
        35                  40                  45

Gly Ser Ser Leu Tyr Arg Gly Cys Gln Glu Ile Cys Trp Glu Pro Thr
    50                  55                  60

Ser Cys Gln Thr Ser Tyr Val Glu Ser Ser Pro Cys Gln Thr Ser Cys
65                  70                  75                  80

Tyr Arg Pro Arg Thr Ser Leu Leu Cys Ser Pro Cys Lys Thr Thr Tyr
                85                  90                  95

Ser Gly Ser Leu Gly Phe Gly Ser Ser Ser Cys Arg Ser Leu Gly Tyr
            100                 105                 110

Gly Ser Arg Ser Cys Tyr Ser Val Gly Cys Gly Ser Ser Gly Val Arg

115                 120                 125

Ser Leu Gly Tyr Gly Ser Cys Gly Phe Pro Ser Leu Gly Tyr Gly Ser
    130                 135                 140

Gly Phe Cys Arg Pro Thr Tyr Leu Ala Ser Arg Ser Cys Gln Ser Pro
145                 150                 155                 160

Cys Tyr Arg Pro Ala Tyr Gly Ser Thr Phe Cys Arg Ser Thr Cys
                165                 170                 175

<210> 90
<211> 193
<212> PRT
<213> Homo sapiens

<400> 90
Met Trp His Ala Asn Ser Glu Ser Ser Gln Cys Asn Ser Ala Glu Leu
  1               5                  10                  15

Thr Ser Pro Ile Asn Met Ser Tyr Asn Cys Cys Ser Gly Asn Phe Ser
             20                  25                  30

Ser Arg Ser Cys Gly Gly Tyr Leu His Tyr Pro Ala Ser Ser Cys Gly
         35                  40                  45

Phe Ser Tyr Pro Ser Asn Gln Val Tyr Ser Thr Asp Leu Cys Ser Pro
     50                  55                  60

Ser Thr Cys Gln Leu Gly Ser Ser Leu Tyr Arg Gly Cys Gln Gln Thr
 65                  70                  75                  80

Cys Trp Glu Pro Thr Ser Cys Gln Thr Ser Tyr Val Glu Ser Ser Pro
                 85                  90                  95

Cys Gln Thr Ser Cys Tyr Arg Pro Arg Thr Ser Leu Leu Cys Ser Pro
            100                 105                 110

Cys Gln Thr Thr Tyr Ser Gly Ser Leu Gly Phe Gly Ser Ser Ser Cys
        115                 120                 125

Arg Ser Leu Gly Tyr Gly Ser Arg Ser Cys Tyr Ser Val Gly Cys Gly
    130                 135                 140

Ser Ser Gly Phe Arg Ser Leu Gly Tyr Gly Gly Cys Gly Phe Pro Ser
145                 150                 155                 160

Leu Gly Tyr Gly Val Gly Phe Cys Arg Pro Thr Tyr Leu Ala Ser Arg
                165                 170                 175

Ser Cys Gln Ser Ser Cys Tyr Arg Pro Thr Cys Gly Ser Gly Phe Tyr
            180                 185                 190

Tyr

<210> 91
<211> 172
<212> PRT
<213> Homo sapiens

<400> 91
Met Ser Tyr Asn Cys Cys Ser Arg Asn Phe Ser Ser Cys Ser His Gly
1               5                   10                  15

Gly Tyr Leu His Tyr Pro Gly Ser Ser Cys Gly Ser Ser Tyr Pro Ser
            20                  25                  30

Asn Leu Val Tyr Ser Thr Asp Leu Cys Ser Pro Ser Thr Cys Gln Leu
        35                  40                  45

Gly Ser Ser Leu Tyr Arg Gly Cys Gln Glu Thr Cys Trp Arg Pro Asn
    50                  55                  60

Ser Cys Gln Thr Leu Cys Val Glu Ser Ser Pro Cys His Thr Ser Cys
65                  70                  75                  80

Tyr Tyr Pro Arg Thr His Met Leu Cys Asn Ser Cys Leu Thr Met His
                85                  90                  95

Val Gly Ser Arg Gly Phe Gly Ser Asn Ser Cys Cys Ser Leu Ser Cys
            100                 105                 110

Gly Ser Arg Ser Cys Ser Ser Leu Gly Cys Gly Ser Asn Gly Phe Arg
        115                 120                 125

Tyr Leu Asn Tyr Arg Ile His Thr Ser Pro Ser Gln Ser Tyr Arg Ser
    130                 135                 140

Arg Phe Cys His Pro Ile Tyr Phe Pro Pro Arg Arg Trp Phe His Ser
145                 150                 155                 160

Ser Cys Tyr Gln Pro Phe Cys Arg Ser Gly Phe Tyr
                165                 170

<210> 92
<211> 160
<212> PRT
<213> Homo sapiens

<400> 92
Met Ser Tyr Asn Cys Cys Ser Arg Asn Phe Ser Ser Arg Ser Phe Gly
1               5                   10                  15

Gly Tyr Leu Tyr Tyr Pro Gly Ser Tyr Pro Ser Ser Leu Val Tyr Ser
            20                  25                  30

Thr Ala Leu Cys Ser Pro Ser Thr Cys Gln Leu Arg Ser Ser Leu Tyr
        35                  40                  45

Arg Asp Cys Gln Lys Thr Cys Trp Glu Pro Ala Ser Cys Gln Lys Ser
    50                  55                  60

Cys Tyr Arg Pro Arg Thr Ser Ile Leu Cys Cys Pro Cys Gln Thr Thr
65                  70                  75                  80

Cys Ser Gly Ser Leu Gly Phe Arg Ser Ser Ser Cys Arg Ser Gln Gly
                85                  90                  95

Tyr Gly Ser Arg Cys Cys Tyr Ser Leu Gly Asn Gly Ser Ser Gly Phe
            100                 105                 110

Arg Phe Leu Lys Tyr Gly Gly Cys Gly Phe Pro Ser Leu Ser Tyr Gly
        115                 120                 125

Ser Arg Phe Cys Tyr Pro Asn Tyr Leu Ala Ser Gly Ala Trp Gln Ser
    130                 135                 140

Ser Cys Tyr Arg Pro Ile Cys Gly Ser Arg Phe Tyr Gln Phe Thr Cys
145                 150                 155                 160

<210> 93
<211> 137
<212> PRT
<213> Homo sapiens

<400> 93

Met Ser Tyr Asn Cys Ser Ser Gly Asn Phe Ser Ser Cys Cys Phe Gly
1               5                   10                  15

Ser Tyr Leu Arg Tyr Pro Val Ser Thr Tyr Asn Leu Phe Tyr Pro Ser
            20                  25                  30

Asn Ala Ile Tyr Ser Pro Asn Thr Cys Gln Leu Gly Ser Ser Leu Tyr
        35                  40                  45

Asn Gly Cys Gln Glu Thr Tyr Cys Glu Pro Thr Ser Cys Gln Thr Ser
    50                  55                  60

Cys Thr Leu Ala Arg Ser Tyr Gln Thr Ser Cys Tyr Cys Pro Lys Asn
65                  70                  75                  80

Ser Ile Phe Cys Ser Pro Arg Gln Thr Asn Tyr Ile Arg Ser Leu Gly
                85                  90                  95

Cys Gly Asn Thr Gly Leu Gly Ser Leu Gly Cys Gly Ser Thr Gly Phe
            100                 105                 110

Gln Ser Leu Asp Cys Gly Ser Ser Phe Tyr His Pro Thr Thr Phe Ser
        115                 120                 125

Ser Arg Asn Phe Gln Ala Thr Cys Tyr
    130                 135

<210> 94
<211> 163
<212> PRT
<213> Homo sapiens

<400> 94
Met Ser Phe Asn Cys Ser Thr Arg Asn Cys Ser Ser Arg Pro Ile Gly
1               5                   10                  15

Gly Arg Cys Ile Val Pro Val Ala Gln Val Thr Thr Thr Ser Thr Thr
            20                  25                  30

Asp Ala Asp Cys Leu Gly Gly Ile Cys Leu Pro Ser Ser Phe Gln Thr
        35                  40                  45

Gly Ser Trp Leu Leu Asp His Cys Gln Glu Thr Cys Cys Glu Pro Thr
    50                  55                  60

Ala Cys Gln Pro Thr Cys Tyr Arg Arg Thr Ser Cys Val Ser Asn Pro
65                  70                  75                  80

Cys Gln Val Thr Cys Ser Arg Gln Thr Thr Cys Ile Ser Asn Pro Cys
                85                  90                  95

Ser Thr Thr Tyr Ser Arg Pro Leu Thr Phe Val Ser Ser Gly Cys Gln
            100                 105                 110

Pro Leu Gly Gly Ile Ser Ser Val Cys Gln Pro Val Gly Gly Ile Ser
        115                 120                 125

Thr Val Cys Gln Pro Val Gly Gly Val Ser Thr Val Cys Gln Pro Ala
    130                 135                 140

Cys Gly Val Ser Arg Thr Tyr Gln Gln Ser Cys Val Ser Ser Cys Arg
145                 150                 155                 160

Arg Thr Cys

<210> 95
<211> 671
<212> DNA
<213> Homo sapiens

<400> 95
aactcacatc tcccgtcaac atgtcctaca actgctgctc tggaaacttc tcctcccgct 60
cctgtggtga ctacctgcgc tacccagcat cctcacgtgg cttttcctac cccagcaatc 120
tggtctacag cactgacctc tgctctccca gcacctgcca gctgggttcc tctctctata 180
ggggctgtca ggagatctgc tgggagccca ccagctgcca gacgtcctat gtggagtcca 240
gcccctgcca gacctcctgc taccgcccca gaacctcctt gctctgcagt ccttgcaaga 300
cgacttactc tgggtctcta ggctttggat ccagcagctg ccgctccctg ggctatggat 360
cgaggagctg ctactcagtg ggctgtgggt ccagtggtgt cagatccctg ggttatggaa 420
gctgtggctt cccttccctc ggctatggat ctggattctg ccgcccaacc tacttggctt 480
ctaggagctg ccagtctcct tgttacagac cagcctatgg atcaaccttc tgcagatcaa 540
cttgctgaat ttccagacct tttaagcaaa gtgtctcagt ctctacgtag agctgttatc 600
ataggcattt ccagcaatgt gagctaaccc ctcttactac tagctcttca tcctttctct 660
ggcatcaagt a                                                      671

<210> 96
<211> 697
<212> DNA
<213> Homo sapiens

<400> 96
atataaatgg tcctgtccag atgtggcatg caaactcaga atcttctcag tgtaactcag 60
ctgaactcac atctcccatc aacatgtcct acaactgctg ctctggaaac ttctcctccc 120

gctcctgtgg tggctacctg cactacccag cctcctcctg tggcttttcc taccccagca 180
accaggtcta cagcactgac ctctgctctc ccagcacgtg ccagctgggt tcctctctct 240
ataggggctg tcagcagacc tgctgggagc ccaccagctg ccagacatcc tatgtggagt 300
ccagcccctg ccagacctcc tgctaccgtc ccagaacctc cttgctctgc agtccctgcc 360
agacaactta ctctgggtct ctaggctttg gatccagcag ctgccgctcc ctgggctatg 420
gatcgaggag ctgctactca gtgggctgtg ggtccagtgg cttcagatcc ctgggttatg 480
gaggctgtgg cttcccttcc ctgggctatg gcgttggatt ctgccgccca acctacttgg 540
cttctaggag ctgccagtct tcttgctaca gaccaacttg tggatcaggc ttctactatt 600
gatcatcttg ttaaattgct gattttgttg gctaatgcct tcaatgcctc tactcataac 660
ctttattgtc ttcatcatgt acagaaagaa ttagcct 697

<210> 97
<211> 637
<212> DNA
<213> Homo sapiens

<400> 97
ctcacatctt cccgtcaaca tgtcctacaa ctgttgctct agaaacttct cctcctgctc 60
ccacgggggt tacttgcact acccaggctc ctcctgtggc tcttcctacc ccagcaacct 120
ggtctacagc actgacctct gctctcccag cacctgccag ctgggttcct ctctctatag 180
gggctgtcag gagacctgct ggaggcccaa cagctgtcag acattgtgtg ttgagtccag 240
cccctgccac acctcctgct actaccccag gactcacatg ctctgcaatt cttgcctgac 300
tatgcatgtt gggtctcggg gttttggatc caatagctgc tgctccctga gctgtggatc 360
caggagctgc tcctcactgg gctgtggatc caatggcttc agatatctga attatagaat 420
ccatacctcc ccttcccaga gttatagatc cagattctgc catccaatct attttccacc 480
tagaaggtgg ttccattcat cttgttatca gccattctgt agatctggtt tctactgact 540
aatgtggtga ctggtaaaac tcatttgaga aatgcatatt ctttagtaag gtcatctgtt 600
aatttcttcc tttgagaagt attctaatat tattgat 637

<210> 98
<211> 603
<212> DNA
<213> Homo sapiens

<400> 98
tgaactccca tctctcatca gcatgtccta caactgctgc tctagaaact tctcctcccg 60
ctcctttggg ggctacctgt actacccagg ctcctacccc agcagcctgg tctacagcac 120
tgccctctgc tctcccagca cctgccagct gcgttcctct ctctacaggg actgtcagaa 180
gacctgctgg gagcccgcca gctgccagaa atcctgctac cgccccagga cctccatcct 240
ctgctgtccc tgtcagacga cttgctctgg atctctaggc tttcggtcca gcagctgtcg 300
ctcccagggc tatggatcta ggtgctgcta ctcgctggga aatggatcca gtggcttcag 360
attcctgaaa tatggaggct gtggttttcc ttccctgagt tacggatcca gattctgcta 420
cccaaactac ttggcttctg gagcctggca gtcttcttgt tacagaccaa tctgtggatc 480
tcgcttctat caattcacct gctaaatttc tagatccttt tgagtattgg gatcaaagtc 540
tctactgaat gcagccatta ttttcattct tgccagatcc caatatcttt ttattcttcc 600
acc 603

<210> 99
<211> 531
<212> DNA
<213> Homo sapiens

<400> 99
gagctcagaa ctcctgttaa catgtcttac aactgcagct ctggaaactt ctcctcctgc 60
tgttttggaa gttacctgag gtatccagtt tccacttata atttgttcta ccccagcaat 120
gccatctatt ctccaaatac ctgccaactg ggctcctctc tctacaatgg ctgtcaggag 180
acctactgtg agcccaccag ctgccagaca tcctgcactt tggccagatc ctatcagaca 240
tcctgttact gcccaaagaa ttccatcttc tgcagtcccc gccagactaa ctacataaga 300
tcccttggat gtggaaacac tggccttgga tctcttggtt gtggaagcac tggcttccaa 360
tctctggact gtgggtccag cttctaccac ccaactacct tttcatccag gaatttccag 420
gcaacttgtt actaaccagc ctttgggtct cgcctttttg gatcatctta ctgaatattc 480
tccattctct catgattatt tctgtactct atggaactgc aacactcagc c 531

<210> 100
<211> 609
<212> DNA
<213> Homo sapiens

<400> 100
gcagccctct gtctgacatc atgtccttca actgctccac aagaaattgc tcttccaggc 60
ccattggagg acgctgcatt gttccagtgg cccaagttac cacgacttcc accactgatg 120
ctgactgcct gggcggcatc tgtttgccca gttccttcca gactggctct tggctcctgg 180
accactgtca agagacctgc tgtgagccca ctgcttgcca gccaacctgt taccggcgaa 240
cttcatgtgt ctccaaccct tgccaggtga cttgctctcg acaaactacc tgtatttcca 300
acccctgctc aactacctac agccggccgc tcacctttgt ctctagtgga tgtcagcccc 360
tgggaggcat ctccagtgtc tgccaaccag tgggcggcat ctctactgtc tgccaaccag 420
tgggaggagt ctctactgtc tgccagccag cctgtggggt ctccaggacg tatcagcagt 480
cctgcgtgtc cagctgccga agaacctgct aagtgtgtag gagccagtga gcgaatcaag 540
actccacgac ctgccagctg tttccaggat cttccagcat gctgcttgtc cctgaatagc 600
tcttcatag 609

<210> 101
<211> 113
<212> PRT
<213> Mus musculus

<400> 101
Met Ala Asn Lys Ala Pro Ser Gln Met Glu Arg Ser Ile Thr Thr Ile
1               5                   10                  15

Ile Asp Thr Phe His Gln Tyr Ser Arg Lys Glu Gly His Pro Asp Thr

20                  25                  30

Leu Ser Lys Lys Glu Phe Arg Gln Met Val Glu Ala Gln Leu Ala Thr
        35                  40                  45

Phe Met Lys Lys Glu Lys Arg Asn Glu Ala Leu Ile Asn Asp Ile Met
    50                  55                  60

Glu Asp Leu Asp Thr Asn Gln Asp Asn Gln Leu Ser Phe Glu Glu Cys
65                  70                  75                  80

Met Met Leu Met Ala Lys Leu Ile Phe Ala Cys His Glu Lys Leu His
                85                  90                  95

Glu Asn Asn Pro Arg Gly His Gly His Ser His Gly Lys Gly Cys Gly
            100                 105                 110

Lys

<210> 102
<211> 114
<212> PRT
<213> Homo sapiens

<400> 102
Met Thr Cys Lys Met Ser Gln Leu Glu Arg Asn Ile Glu Thr Ile Ile
1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Asn Ala Asp Lys Gln Leu Ser Phe Glu Glu Phe Ile
65                  70                  75                  80

Met Leu Met Ala Arg Leu Thr Trp Ala Ser His Glu Lys Met His Glu
                85                  90                  95

Gly Asp Glu Gly Pro Gly His His His Lys Pro Gly Leu Gly Glu Gly
            100                 105                 110

Thr Pro

<210> 103
<211> 341
<212> PRT
<213> Mus musculus

<400> 103
Met Ala Val Ser Val Thr Pro Ile Arg Asp Thr Lys Trp Leu Thr Leu
1               5                   10                  15

Glu Val Cys Arg Glu Phe Gln Arg Gly Thr Cys Ser Arg Pro Asp Thr
            20                  25                  30

Glu Cys Lys Phe Ala His Pro Ser Lys Ser Cys Gln Val Glu Asn Gly
        35                  40                  45

Arg Val Ile Ala Cys Phe Asp Ser Leu Lys Gly Arg Cys Ser Arg Glu
    50                  55                  60

Asn Cys Lys Tyr Leu His Pro Pro Pro His Leu Lys Thr Gln Leu Glu
65                  70                  75                  80

Ile Asn Gly Arg Asn Asn Leu Ile Gln Gln Lys Asn Met Ala Met Leu
                85                  90                  95

Ala Gln Gln Met Gln Leu Ala Asn Ala Met Met Pro Gly Ala Pro Leu
            100                 105                 110

Gln Pro Val Pro Met Phe Ser Val Ala Pro Ser Leu Ala Thr Ser Ala
        115                 120                 125

Ser Ala Ala Phe Asn Pro Tyr Leu Gly Pro Val Ser Pro Ser Leu Val
    130                 135                 140

Pro Ala Glu Ile Leu Pro Thr Ala Pro Met Leu Val Thr Gly Asn Pro
145                 150                 155                 160

Gly Val Pro Val Pro Ala Ala Ala Ala Ala Ala Ala Gln Lys Leu Met
                165                 170                 175

Arg Thr Asp Arg Leu Glu Val Cys Arg Glu Tyr Gln Arg Gly Asn Cys
            180                 185                 190

Asn Arg Gly Glu Asn Asp Cys Arg Phe Ala His Pro Ala Asp Ser Thr
        195                 200                 205

Met Ile Asp Thr Asn Asp Asn Thr Val Thr Val Cys Met Asp Tyr Ile
    210                 215                 220

Lys Gly Arg Cys Ser Arg Glu Lys Cys Lys Tyr Phe His Pro Pro Ala
225                 230                 235                 240

His Leu Gln Ala Lys Ile Lys Ala Ala Gln Tyr Gln Val Asn Gln Ala
                245                 250                 255

Ala Ala Ala Gln Ala Ala Ala Thr Ala Ala Ala Met Gly Ile Pro Gln
            260                 265                 270

Ala Val Leu Pro Pro Leu Pro Lys Arg Pro Ala Leu Glu Lys Thr Asn
        275                 280                 285

Gly Ala Thr Ala Val Phe Asn Thr Gly Ile Phe Gln Tyr Gln Gln Ala
    290                 295                 300

Leu Ala Asn Met Gln Leu Gln Gln His Thr Ala Phe Leu Pro Pro Gly
305                 310                 315                 320

Ser Ile Leu Cys Met Thr Pro Ala Thr Ser Val Asp Thr His Asn Ile
                325                 330                 335

Cys Arg Thr Ser Asp
            340


<210> 104
<211> 370
<212> PRT
<213> Homo sapiens

<400> 104
Met Ala Val Ser Val Thr Pro Ile Arg Asp Thr Lys Trp Leu Thr Leu
1               5                   10                  15

Glu Val Cys Arg Glu Phe Gln Arg Gly Thr Cys Ser Arg Pro Asp Thr
            20                  25                  30

Glu Cys Lys Phe Ala His Pro Ser Lys Ser Cys Gln Val Glu Asn Gly
        35                  40                  45

Arg Val Ile Ala Cys Phe Asp Ser Leu Lys Gly Arg Cys Ser Arg Glu
    50                  55                  60

Asn Cys Lys Tyr Leu His Pro Pro Pro His Leu Lys Thr Gln Leu Glu

65                  70                  75                  80

Ile Asn Gly Arg Asn Asn Leu Ile Gln Gln Lys Asn Met Ala Met Leu
                85                  90                  95

Ala Gln Gln Met Gln Leu Ala Asn Ala Met Met Pro Gly Ala Pro Leu
            100                 105                 110

Gln Pro Val Pro Met Phe Ser Val Ala Pro Ser Leu Ala Thr Asn Ala
        115                 120                 125

Ser Ala Ala Ala Phe Asn Pro Tyr Leu Gly Pro Val Ser Pro Ser Leu
    130                 135                 140

Val Pro Ala Glu Ile Leu Pro Thr Ala Pro Met Leu Val Thr Gly Asn
145                 150                 155                 160

Pro Gly Val Pro Val Pro Ala Ala Ala Ala Ala Ala Ala Gln Lys Leu
                165                 170                 175

Met Arg Thr Asp Arg Leu Glu Val Cys Arg Glu Tyr Gln Arg Gly Asn
            180                 185                 190

Cys Asn Arg Gly Glu Asn Asp Cys Arg Phe Ala His Pro Ala Asp Ser
        195                 200                 205

Thr Met Ile Asp Thr Asn Asp Asn Thr Val Thr Val Cys Met Asp Tyr
    210                 215                 220

Ile Lys Gly Arg Cys Ser Arg Glu Lys Cys Lys Tyr Phe His Pro Pro
225                 230                 235                 240

Ala His Leu Gln Ala Lys Ile Lys Ala Ala Gln Tyr Gln Val Asn Gln
                245                 250                 255

Ala Ala Ala Ala Gln Ala Ala Ala Thr Ala Ala Ala Met Gly Ile Pro
            260                 265                 270

Gln Ala Val Leu Pro Pro Leu Pro Lys Arg Pro Ala Leu Glu Lys Thr
        275                 280                 285

Asn Gly Ala Thr Ala Val Phe Asn Thr Gly Ile Phe Gln Tyr Gln Gln
    290                 295                 300

Ala Leu Ala Asn Met Gln Leu Gln Gln His Thr Ala Phe Leu Pro Pro
305                 310                 315                 320

Val Pro Met Val His Gly Ala Thr Pro Ala Thr Val Ser Ala Ala Thr

325                 330                 335

Thr Ser Ala Thr Ser Val Pro Phe Ala Ala Thr Ala Thr Ala Asn Gln
            340                 345                 350

Ile Pro Ile Ile Ser Ala Glu His Leu Thr Ser His Lys Tyr Val Thr
        355                 360                 365

Gln Met
    370



<210> 105
<211> 419
<212> PRT
<213> Mus musculus

<400> 105
Met Asn Lys His Gln Lys Pro Val Leu Thr Gly Gln Arg Phe Lys Thr
  1               5                  10                  15

Arg Lys Arg Asp Glu Lys Glu Lys Phe Glu Pro Thr Val Phe Arg Asp
             20                  25                  30

Thr Leu Val Gln Gly Leu Asn Glu Ala Gly Asp Asp Leu Glu Ala Val
         35                  40                  45

Ala Lys Phe Leu Asp Ser Thr Gly Ser Arg Leu Asp Tyr Arg Arg Tyr
     50                  55                  60

Ala Asp Thr Leu Phe Asp Ile Leu Val Ala Gly Ser Met Leu Ala Pro
 65                  70                  75                  80

Gly Gly Thr Arg Ile Asp Asp Gly Asp Lys Thr Lys Met Thr Asn His
                 85                  90                  95

Cys Val Phe Ser Ala Asn Glu Asp His Glu Thr Ile Arg Asn Tyr Ala
            100                 105                 110

Gln Val Phe Asn Lys Leu Ile Arg Arg Tyr Lys Tyr Leu Glu Lys Ala
        115                 120                 125

Phe Glu Asp Glu Met Lys Lys Leu Leu Leu Phe Leu Lys Ala Phe Ser
    130                 135                 140

Glu Ala Glu Gln Thr Lys Leu Ala Met Leu Ser Gly Ile Leu Leu Gly
145                 150                 155                 160

Asn Gly Thr Leu Pro Ala Thr Ile Leu Thr Ser Leu Phe Thr Asp Ser
                165                 170                 175

Leu Val Lys Glu Gly Ile Ala Ala Ser Phe Ala Val Lys Leu Phe Lys
            180                 185                 190

Ala Trp Met Ala Glu Lys Asp Ala Asn Ser Val Thr Ser Ser Leu Arg
        195                 200                 205

Lys Ala Asn Leu Asp Lys Arg Leu Leu Glu Leu Phe Pro Val Asn Arg
    210                 215                 220

Gln Ser Val Asp His Phe Ala Lys Tyr Phe Thr Asp Ala Gly Leu Lys
225                 230                 235                 240

Glu Leu Ser Asp Phe Leu Arg Val Gln Gln Ser Leu Gly Thr Arg Lys
                245                 250                 255

Glu Leu Gln Lys Glu Leu Gln Glu Arg Leu Ser Gln Glu Cys Pro Ile
            260                 265                 270

Lys Glu Val Val Leu Tyr Val Lys Glu Glu Met Lys Arg Asn Asp Leu
        275                 280                 285

Pro Glu Thr Ala Val Ile Gly Leu Leu Trp Thr Cys Ile Met Asn Ala
    290                 295                 300

Val Glu Trp Asn Lys Lys Glu Glu Leu Val Ala Glu Gln Ala Leu Lys
305                 310                 315                 320

His Leu Lys Gln Tyr Ala Pro Leu Leu Ala Val Phe Ser Ser Gln Gly
                325                 330                 335

Gln Ser Glu Leu Val Leu Leu Gln Lys Val Gln Glu Tyr Cys Tyr Asp
            340                 345                 350

Asn Ile His Phe Met Lys Ala Phe Gln Lys Ile Val Val Leu Phe Tyr
        355                 360                 365

Lys Ala Asp Val Leu Ser Glu Glu Ala Ile Leu Lys Trp Tyr Lys Glu
    370                 375                 380

Ala His Ala Ala Lys Gly Lys Ser Val Phe Leu Asp Gln Met Lys Lys
385                 390                 395                 400

Phe Val Glu Trp Ile Gln Asn Ala Glu Glu Glu Ser Glu Ser Glu Gly
                405                 410                 415

Glu Glu Ser

<210> 106
<211> 2988
<212> PRT
<213> Homo sapiens

<400> 106
   Met Val Glu Gly Asp Thr Val Thr Phe Ser Phe Glu Met Arg Ser Gly
   1               5                   10                  15
   Arg Glu His Asn Thr Pro Asp Lys Ala Met Trp Gly Phe Ala Cys Thr
               20                  25                  30
   Val Arg Ala Gln Glu Ser Ser Glu Asp Val Ser Gly Gly Leu Pro Phe
           35                  40                  45
   Leu Val Asp Leu Ala Leu Gly Leu Ser Val Leu Ala Cys Ser Met Leu
       50                  55                  60
   Arg Ile Leu Tyr Asn Gly Pro Glu Ile Thr Lys Glu Glu Glu Ala Cys
   65                  70                  75                  80
   Gln Glu Leu Leu Arg Ser Lys Leu Leu Gln Arg Glu Val Ile Gln Pro
                   85                  90                  95
   Asp Val Met Glu Glu Met Val Val Ser Cys Val Ile Lys His Leu Asn
               100                 105                 110
   Leu Val Asp Ala Leu Gln Ser Leu Ile Asn Phe Gln Tyr Gln Glu Glu
           115                 120                 125
   His Ala Glu Glu Tyr Asp Leu Leu Cys Lys Ile Met Gly Glu Thr Phe
       130                 135                 140
   Lys Lys Leu Asn Ala Met Glu Arg Gln Leu Gln Asn Lys Met Lys Glu
   145                 150                 155                 160
   Leu Glu Leu Leu Cys Ser Met Lys Glu Val Ser Phe Asp Gly Asn Asp
                   165                 170                 175
   Leu Glu Asn Met Val Leu Ser Leu Arg Glu Lys Phe Leu Gln Glu Val
               180                 185                 190
   Asn Ser Leu Ile Gln Lys Pro Ser His Pro Leu Ala Lys Thr Lys Thr
           195                 200                 205
   Leu Val Lys Ser Leu Met Asn Arg Ala Glu Leu Leu Leu His Val Thr
       210                 215                 220
   Ile Ala Ala Gln Ser Gly Leu Thr Arg Ser Ile Ser Gly Thr Pro Ala
   225                 230                 235                 240
   Glu Thr Pro Ala Cys Lys Ser Ala Ser Glu Thr Lys Val Ile Ser His
                   245                 250                 255
   Ala Val Arg Gln Pro Val Phe Leu Arg Ser Met Ser Ala Pro Ser Asp
               260                 265                 270
   Leu Glu Met Ile Gly Asn Glu Asp Leu Glu Phe Thr Arg Ala Asn Gln
           275                 280                 285
   Arg Arg Arg His Val Thr Ser His Arg Ser Ser Ser Phe Thr Leu Leu
       290                 295                 300
   Gln Ser Leu Ala Ile Glu Asp Ser Arg Asp Lys Pro Thr Tyr Ser Val
   305                 310                 315                 320
   Leu Leu Gly Gln Leu Phe Ala Phe Ile Gly Thr Asn Pro Asp Gln Ala
                   325                 330                 335
   Val Ser Ser Ser Ser Phe Leu Leu Ala Ala Gln Thr Arg Trp Arg Arg
               340                 345                 350
   Gly Asn Thr Arg Lys Gln Ala Leu Val His Met Arg Glu Leu Leu Thr
           355                 360                 365
   Ala Ala Val Arg Val Gly Gly Val Thr His Leu Val Gly Pro Val Thr
       370                 375                 380
   Met Val Leu Gln Gly Gly Pro Arg Ile Glu Glu Leu Thr Cys Gly Gly
   385                 390                 395                 400
   Met Val Glu Gln Val Gln Glu Ala Phe Gly Glu Thr Met Thr Ser Val
                   405                 410                 415
   Val Ser Leu Cys Ala Arg Tyr Pro Ile Ala Cys Ala Asn Ser Ile Gly
               420                 425                 430
   Leu Leu Cys Thr Ile Pro Tyr Thr Arg Ser Glu Glu Lys Cys Leu Val
           435                 440                 445
   Arg Ser Gly Leu Val Gln Leu Met Asp Arg Leu Cys Ser Leu Ser Asn
       450                 455                 460
   Gln Thr Glu Ser Ser Ser Ser Glu Lys Gln Thr Lys Lys Gln Lys Val
   465                 470                 475                 480
   Ala Thr Met Ala Trp Ala Ala Phe Gln Val Leu Ala Asn Arg Cys Val
                   485                 490                 495
   Glu Trp Glu Lys Glu Glu Glu Thr Gly Ser Tyr Tyr Val Ala Gln Leu
               500                 505                 510
   Cys Arg Ala Ala Leu Pro Leu Met Ser Val Glu Asp Cys Gly Asn Val
           515                 520                 525
   Glu Leu Pro Pro Trp Ser Tyr Ser Val Pro Ser Leu Asn Ser Glu Gln
       530                 535                 540
   Glu Asp Pro Ser Asp Pro Ala Ser Lys Ile Ala Ser Leu Leu Leu Ala
   545                 550                 555                 560
   Lys Leu Ala Asp Tyr Val Val Pro Gly Cys Gln Thr Val Leu Ser Pro
                   565                 570                 575
   Thr Ala Ser Glu Pro Asp Thr Thr Leu Thr Lys Thr Ser Pro Lys Asn
               580                 585                 590
   Ser Leu Lys Gly Asp Lys Asp Pro Gly Glu Glu Ser Glu Ala Val Asp
           595                 600                 605
   Gly Lys Leu Ser Ile Phe Ile His Lys Arg Glu Asp Gln Ser Ser His
       610                 615                 620
   Glu Val Leu Gln Pro Leu Leu Ser Ser Ser Glu Gly Arg Pro Phe Arg
   625                 630                 635                 640
   Leu Gly Thr Gly Ala Asn Met Glu Lys Val Val Lys Met Asp Arg Asp
                   645                 650                 655
   Met Thr Lys Gly Gly Cys Cys Glu Val Ile Thr Glu Glu Ala Ala Ala
               660                 665                 670
   Ala Leu Arg Lys Ala Thr Lys Trp Ala Gln Ser Gly Leu Ile Val Ser
           675                 680                 685
   Ile Gly Pro Pro Val Glu Ser Ile Asn Pro Glu Thr Val Ser Gly Leu
       690                 695                 700
   Ser Thr Gly Asp Lys Lys Lys Thr Ala Gln Thr Ser Ile Cys Arg Glu
   705                 710                 715                 720
   Arg Asn Ser Glu Leu Ala Arg Thr Asp Pro Val Arg Pro Phe Ile Ser
                   725                 730                 735
   Gly His Val Ala Asn Ser Met Ala Ala Glu Val Ile Ala Leu Leu His
               740                 745                 750
   Ser Leu Leu Met Ala Pro Glu Ser Asn Ala Ala Gln Ile Trp Thr Thr
           755                 760                 765
   Thr Ala Glu Lys Val Leu Ser Arg Ala Leu Met Tyr Ile Pro Gln Leu
       770                 775                 780
   Gly Lys Tyr Ala Glu Ser Ile Leu Glu Asn Gly Ser Ser Ser Gly Arg
   785                 790                 795                 800
   Lys Leu Ala Lys Leu Gln Arg Ile Ala Arg Gln Ala Val Ala Ala Leu
                   805                 810                 815
   Cys Ala Leu Gly Gly Phe Lys Glu Thr Ile Lys Ile Gly Ser Glu Val
               820                 825                 830
   Gln Ala Leu Pro Leu His Lys Leu Ser Ile Thr Glu Lys Val Val Gln
           835                 840                 845
   Ala Val Gln Ser Met Leu Leu Pro Gln Glu Gly Ser Leu Ser Ile His
       850                 855                 860
   Thr Ser Leu Pro Ala Thr Gly Asp Gly Ser Ala Pro Val Met Ala Val
   865                 870                 875                 880
   Val Arg Leu Leu Ala Glu Ile Arg Thr Arg Ala Cys Leu Val Met Ala
                   885                 890                 895
   Gln Leu Leu Glu Asp Ser Leu Phe Cys Glu Glu Phe Ile Gln Gln Cys
               900                 905                 910
   Pro Ala Ala Val Glu Val Leu Asn Leu Val Ala Gln Glu Cys Ser Ala
           915                 920                 925
   Gly Glu Arg Leu Ala Val Val Glu Val Gln Cys Glu Arg Leu Arg Met
       930                 935                 940
   Leu Tyr Arg Asp Cys Ala Arg Pro Pro Pro Pro Pro Leu Gln Ala Asp
   945                 950                 955                 960
   Arg Arg Gln Ala Pro Ser Phe Tyr Trp Glu Ile Glu Ile Val Ser Tyr
                   965                 970                 975
   Gly Asp Thr Asp Asp Asp Thr Gly Pro Ile Val Ser Phe Gly Phe Thr
   980 985 990
   Thr Glu Ala Glu Lys Arg Asp Gly Ala Trp Thr Asn Pro Val Gly Thr
   995 1000 1005
   Cys Leu Phe His Asn Asn Gly Arg Ala Val His Tyr Asn Gly Ser Ser
   1010 1015 1020
   Leu Leu Gln Trp Lys Ser Val Arg Leu Asp Val Thr Leu Ser Pro Gly
   1025 1030 1035 1040
   Asp Val Ala Gly Ile Gly Trp Glu Arg Thr Glu Gly Thr Pro Pro Pro
   1045 1050 1055
   Pro Gly Gln Pro Ala Lys Gly Arg Val Tyr Phe Thr Tyr Cys Gly Gln
   1060 1065 1070
   Arg Leu Ser Pro Tyr Leu Glu Asp Val Ser Gly Gly Met Trp Pro Val
   1075 1080 1085
   Val His Ile Gln Lys Lys Ala Glu Ile Lys Ile Asn Val Ser Leu Ser
   1090 1095 1100
   Lys Ser Phe Ile Trp Ser Pro Phe Gln Asn Thr Lys Thr Arg Ala Asn
   1105 1110 1115 1120
   Phe Gly Ser Arg Pro Phe Ala Tyr Ala Glu Gly Gln Ala His Arg Asn
   1125 1130 1135
   Ala Ala Asp Leu Cys Thr Asp Leu Ala Glu Glu Ile Ser Ala Asn Phe
   1140 1145 1150
   Glu Ala Leu Pro Phe Ala Met Ala Ser Asp Ser Asp Asn Asp Ala Gly
   1155 1160 1165
   Thr Ser Ile Ala Ser Asp Pro Gly Thr His Gly Pro Pro Cys Arg Ile
   1170 1175 1180
   Ala Ala Val Ala Thr Ala Gln Gln Gln Tyr Asp Ser Asp Thr Ser Cys
   1185 1190 1195 1200
   His Tyr Lys Val Glu Leu Ser Tyr Glu Asn Phe Ile Thr Ser Gly Pro
   1205 1210 1215
   Asp Pro His Pro Pro Pro Ile Ala Asp Asp Glu Ser Asp Asp Asp Asp
   1220 1225 1230
   Asp Asp Asp Ile Pro Gln Glu Asp His Tyr Ala Leu Leu Val Lys Ala
   1235 1240 1245
   Trp Glu Thr Lys Val Phe Pro Thr Ile Arg Arg Arg Phe Arg Asn Glu
   1250 1255 1260
   Ala Glu Arg Lys Ser Gly Leu Asp Gln Ile Lys Gly Ala Leu Gln Leu
   1265 1270 1275 1280
   Gly Met Val Asp Ile Ala Arg Gln Thr Val Glu Phe Leu Tyr Glu Glu
   1285 1290 1295
   Asn Gly Gly Ile Pro Arg Asp Leu Tyr Leu Pro Thr Ile Glu Asp Ile
   1300 1305 1310
   Lys Asp Glu Ala Asn Lys Phe Thr Ile Asp Lys Val Arg Lys Gly Leu
   1315 1320 1325
   Thr Val Val Thr Arg Ser Pro Asp Ser Asn Asn Val Ala Ser Ser Ala
   1330 1335 1340
   Val Gly Thr Ala Leu Pro Lys Phe Ala Ile Arg Gly Met Leu Lys Thr
   1345 1350 1355 1360
   Phe Gly Leu His Gly Val Val Leu Asp Val Asp Ser Val Asn Glu Leu
   1365 1370 1375
   Val Gln Val Glu Thr Tyr Leu Arg Ser Glu Gly Val Leu Val Arg Tyr
   1380 1385 1390
   Trp Tyr Pro Ile Asp Met Leu Glu Arg Pro Pro Ala Gly Tyr Arg Arg
   1395 1400 1405
   Thr Ala Thr Asn Gly Leu Val Thr Leu Asp Asn Thr Asn Leu Gln Ile
   1410 1415 1420
   His Arg Glu Leu Leu Arg Cys Glu Ala Ala Leu Ala Arg Leu Tyr Cys
   1425 1430 1435 1440
   Arg Met Ala Leu Leu Asn Ile Phe Ala Pro Lys Leu Pro His Leu Phe
   1445 1450 1455
   Thr Arg Leu Phe His Ile Pro Ala Ile Arg Asp Ile Thr Leu Glu His
   1460 1465 1470
   Leu Gln Leu Leu Ser Asn Gln Leu Leu Ala Pro Pro Leu Pro Asp Gly
   1475 1480 1485
   Thr Ile Ser Ser Ser Ser Ile Leu Leu Ala Gln Ser Leu Gln His Cys
   1490                1495                1500
   Ile His Ser Gln Asn Cys Ser Ala Thr Asp Leu Phe Tyr Gln Gly Asn
   1505                1510                1515                1520
   Ser Gln Thr Val Arg Glu Trp Leu Asn Val Ala Ile Thr Arg Thr Leu
                   1525                1530                1535
   His Gln Gly Glu Glu Ser Leu Leu Glu Leu Thr Lys Gln Ile Cys Ser
               1540                1545                1550
   Phe Leu Gln Thr Ala Pro Glu Gln Phe Pro Ser Glu Glu Phe Pro Ile
           1555                1560                1565
   Ser Glu Ser Lys Val Asn Met Asp Val Asn Phe Pro Gly Ala Ala Phe
       1570                1575                1580
   Val Val Val Ser Cys Lys Glu Ser Gln Ser Gly Phe Arg Lys Asp Ser
   1585                1590                1595                1600
   Ser Leu Tyr Lys Ala Pro Trp Ala Arg Val Leu Val Tyr Gly Leu Gly
                   1605                1610                1615
   His Lys Val Lys Arg Asn Gly Gln Leu Asn Leu Ile Glu Ala Ala Cys
               1620                1625                1630
   Tyr Pro Arg Asp Ala Ser Pro Ala Asn Thr Gly Leu Ala Pro Pro Pro
           1635                1640                1645
   Thr Ala Asp Gln Tyr Pro Ser Val Val Leu Ser Thr Asp Arg Val His
       1650                1655                1660
   Ile Lys Leu Gly Val Ser Pro Pro Pro Gly Ala Val Leu Val Leu His
   1665                1670                1675                1680
   Ser Leu Pro Leu Glu Phe Pro Leu Ala Met Ala Phe Ala Glu Gln Leu
                   1685                1690                1695
   Leu Ser Trp Lys Ser Glu Asp Ser Glu Gly Lys Ser Glu Asp Glu Pro
               1700                1705                1710
   Asp Thr Ile Pro Thr Ser Val Leu Leu Gln Val Val Glu Leu Leu Gly
           1715                1720                1725
   Asn Phe Leu Trp Thr Thr Asp Met Ala Ala Cys Val Lys Glu Leu Val
       1730                1735                1740
   Phe His Leu Leu Ala Glu Leu Leu Arg Thr Val His Thr Leu Glu Gln
   1745                1750                1755                1760
   Arg Arg His Pro Ala Gly Leu Ser Ser Ser Ile Ala Leu Gln Leu Asn
                   1765                1770                1775
   Pro Cys Leu Ala Met Leu Met Ala Leu Gln Ser Glu Leu His Lys Leu
               1780                1785                1790
   Tyr Asp Glu Glu Thr Gln Asn Trp Val Ser Gly Gly Ala Cys Gly Gly
           1795                1800                1805
   Ser Gly Gly Ala Ala Ala Gly Asp Gln Gly Arg Phe Ser Thr Tyr Phe
       1810                1815                1820
   His Ala Leu Met Glu Gly Cys Leu Ala Val Ala Glu Val Thr Leu Pro
   1825                1830                1835                1840
   Thr Asn Met Ser Val Thr Ala Ser Gly Val Thr Ser Ala Thr Ala Pro
                   1845                1850                1855
   Asn Leu Ser Asp Ser Ser Ser Ser Ser Ser Ser Ser Pro Gly Gln Thr
               1860                1865                1870
   Pro Gln Ser Pro Ser Leu Leu Ser Lys Arg Lys Lys Val Lys Met Lys
           1875                1880                1885
   Arg Glu Lys Ala Ser Ser Ser Gly Lys Arg Gln Ser Ser Arg Thr Val
       1890                1895                1900
   Asp Ser Asp Pro Thr Val Leu Ser Ile Gly Gly Ser Lys Pro Glu Asp
   1905                1910                1915                1920
   Met Leu Trp Phe His Arg Ala Leu Thr Leu Leu Ile Ile Leu Arg His
                   1925                1930                1935
   Leu Thr Arg Lys Asp Pro Gln Gly Leu Gly Val Thr Ser Asp Ala Ile
               1940                1945                1950
   Ala Asp Ala Cys Gln Ala Leu Val Gly Pro Thr Ala His Ser Arg Leu
           1955                1960                1965
   Leu Val Ile Ser Gly Ile Pro Thr His Leu Asp Glu Gly Val Val Arg
       1970                1975                1980
   Gly Ala Ile Arg Lys Ala Cys Asn Ala His Gly Gly Val Phe Lys Asp
   1985                1990                1995                2000
   Glu Ile Tyr Ile Pro Leu Gln Glu Glu Asp Thr Lys Lys Pro Lys Asp
   2005 2010 2015
   Lys Ala Glu Gly Gly Asp Gly Lys Val Glu Pro Glu Lys Thr Leu Ala
   2020 2025 2030
   Phe Pro Gly Thr Asp Ser Met Glu Val Ser Thr Ser Ser Ser Leu Thr
   2035 2040 2045
   Pro Ala Met Ser Ile Ser Ala Ser Ala Ser Thr Ser Gln Ala Ser Ile
   2050 2055 2060
   Cys Ser Ser Gln Gly Ile Ser Gln Thr Val Ser Asp Leu Ser Val Asp
   2065 2070 2075 2080
   Pro Leu Pro Ala Gly Leu Glu Leu Pro Ile Pro Pro Gly Leu Leu Glu
   2085 2090 2095
   Pro His Ala Val Ser Ser Gln Glu Ser Leu Asp Ile Ser Leu Cys Ser
   2100 2105 2110
   Thr Gly Ser Leu Gly Ser Leu Gly Ser Leu Gly Glu Pro Leu Asp Asn
   2115 2120 2125
   Ala Glu Thr Ala Ser Val Ser Asp Met Gly Ser Met Tyr Thr Val Thr
   2130 2135 2140
   Ser Leu Asp Asn Gln Pro Leu Ala Ala Arg Pro Ile Lys Gly Phe Ala
   2145 2150 2155 2160
   Val Val Glu Ile Arg Ser Arg Ala Lys Ile Glu Lys Ile Arg Ala Ser
   2165 2170 2175
   Leu Phe Asn Asn Asn Asp Leu Ile Gly Leu Ser Ser Leu Asp Gly Glu
   2180 2185 2190
   Asp Glu Leu Met Glu Met Ser Thr Glu Glu Ile Leu Thr Val Ser Val
   2195 2200 2205
   Val Asn Gln Ser Leu Phe Asp Thr Gln Gly Ser Pro Gly Leu Glu Asp
   2210 2215 2220
   Tyr Phe Asn Asp Lys Ser Ile Lys Gly Glu Lys Leu Val Pro Gly Ala
   2225 2230 2235 2240
   Arg Glu Val Leu Thr Glu Ile Phe Lys Ser Cys Ala His Ser Glu Gln
   2245 2250 2255
   Thr Leu Ser Leu Thr Pro Ala Lys Pro Ile Arg Val Ser Asp Ile Tyr
   2260 2265 2270
   Leu Ser Lys Glu Gln Ile Asn Ser Gln Thr Pro Gly Asn Leu Leu His
   2275 2280 2285
   Leu Phe Phe Thr Asn Val Arg Pro Pro Lys Lys Val Leu Glu Asp Gln
   2290 2295 2300
   Leu Thr Gln Ile Leu Arg Lys Tyr Gly Val Pro Lys Pro Lys Phe Asp
   2305 2310 2315 2320
   Lys Ser Lys Tyr Ser Lys Ala Gly Lys Glu Gln His Pro Val Lys Val
   2325 2330 2335
   Val Ser Thr Lys Arg Pro Ile Thr Lys Pro Pro Ala Lys Asp Lys Ala
   2340 2345 2350
   Val Leu Asn Ser Val Ser Arg Thr Ala Leu Ser Glu Lys Lys Pro Thr
   2355 2360 2365
   Val Lys Pro Lys Ser Pro Glu Lys Ser Lys Pro Asp Glu Lys Asp Pro
   2370 2375 2380
   Glu Lys Ser Pro Thr Lys Lys Gln Glu Val Pro Glu Glu Lys Tyr Leu
   2385 2390 2395 2400
   Thr Leu Glu Gly Phe His Lys Phe Val Ile Asp Arg Ala Arg Gln Asp
   2405 2410 2415
   Ile Arg Ser Val Trp Arg Ala Ile Leu Ser Cys Gly Tyr Asp Leu His
   2420 2425 2430
   Phe Glu Arg Cys Ala Cys Ile Asp Val Arg His Ala Gln Lys Ala Ser
   2435 2440 2445
   Arg Lys Trp Thr Leu Glu Met Asp Val Ala Leu Val Gln Tyr Ile Asn
   2450 2455 2460
   Gln Leu Cys Arg His Leu Ala Ile Thr Pro Ala Arg Leu His Pro His
   2465 2470 2475 2480
   Glu Val Tyr Leu Asp Pro Ala Asp Ala Ala Asp Pro Arg Val Ala Cys
   2485 2490 2495
   Leu Leu Asn Val Pro Ile Glu Ser Leu Arg Leu Arg Phe Ala Leu Leu
   2500 2505 2510
   Gln Ser Leu Asn Thr Thr Leu Glu Thr Phe Phe Leu Pro Leu Val Glu
   2515 2520 2525
   Leu Arg Gln Thr Pro Met Tyr Thr His Ser Ile Ala Ala Leu Leu Lys
   2530 2535 2540
   Glu Ala Lys Gly Leu Ile Phe Tyr Asp Thr Lys Val Thr Val Met Asn
   2545 2550 2555 2560
   Arg Val Leu Asn Ala Thr Val Gln Arg Thr Ala Asp His Ala Ala Pro
   2565 2570 2575
   Glu Ile Thr Leu Asp Pro Leu Glu Ile Val Gly Gly Glu Ile Arg Ala
   2580 2585 2590
   Ser Glu Asn Ser Tyr Phe Cys Gln Ala Ala Arg Gln Leu Ala Ser Val
   2595 2600 2605
   Pro Ser Ser Gln Leu Cys Val Lys Leu Ala Ser Gly Gly Asp Pro Thr
   2610 2615 2620
   Tyr Ala Phe Asn Ile Arg Phe Thr Gly Glu Glu Val His Gly Thr Ser
   2625 2630 2635 2640
   Gly Ser Phe Arg His Phe Leu Trp Gln Val Cys Lys Glu Leu Gln Ser
   2645 2650 2655
   Ser Ser Leu Ser Leu Leu Leu Leu Cys Pro Ser Ser Ala Val Asn Lys
   2660 2665 2670
   Asn Lys Gly Lys Tyr Ile Leu Thr Pro Ser Pro Ile Thr Tyr Gly Glu
   2675 2680 2685
   Glu Gln Leu Leu His Phe Leu Gly Gln Leu Leu Gly Ile Ala Ile Arg
   2690 2695 2700
   Ala Asp Val Pro Leu Pro Leu Asp Leu Leu Pro Ser Phe Trp Lys Thr
   2705 2710 2715 2720
   Leu Val Gly Glu Pro Leu Asp Pro Glu Gln Asp Leu Gln Glu Ala Asp
   2725 2730 2735
   Ile Leu Thr Tyr Asn Tyr Val Lys Lys Phe Glu Ser Ile Asn Asp Glu
   2740 2745 2750
   Thr Glu Leu Glu Ala Leu Cys Ala Glu Ile Ala Ser Gln His Leu Ala
   2755 2760 2765
   Thr Glu Ser Pro Asp Ser Pro Asn Lys Pro Cys Cys Arg Phe Thr Tyr
   2770 2775 2780
   Leu Thr Met Thr Gly Glu Glu Val Glu Leu Cys Ser Arg Gly Arg His
   2785 2790 2795 2800
   Ile Leu Val Ala Trp Glu Asn Lys Asp Ile Tyr Ala Ala Ala Ile Arg
   2805 2810 2815
   Ser Leu Arg Leu Arg Glu Leu Gln Asn Val Glu Cys Val Thr Ala Val
   2820 2825 2830
   Arg Ala Gly Leu Gly Ser Ile Ile Pro Leu Gln Leu Leu Thr Met Leu
   2835 2840 2845
   Ser Pro Leu Glu Met Glu Leu Arg Thr Cys Gly Leu Pro Tyr Ile Asn
   2850 2855 2860
   Leu Glu Phe Leu Lys Ala His Thr Met Tyr Gln Val Gly Leu Met Glu
   2865 2870 2875 2880
   Thr Asp Gln His Ile Glu Phe Phe Trp Gly Ala Leu Glu Met Phe Thr
   2885 2890 2895
   Gln Glu Glu Leu Cys Lys Phe Ile Lys Phe Ala Cys Asn Gln Glu Arg
   2900 2905 2910
   Ile Pro Phe Thr Cys Pro Cys Lys Asp Gly Gly Pro Asp Thr Ala His
   2915 2920 2925
   Val Pro Pro Tyr Pro Met Lys Ile Ala Pro Pro Asp Gly Thr Ala Gly
   2930 2935 2940
   Ser Pro Asp Ser Arg Tyr Ile Arg Val Glu Thr Cys Met Phe Met Ile
   2945 2950 2955 2960
   Lys Leu Pro Gln Tyr Ser Ser Leu Glu Ile Met Leu Glu Lys Leu Arg
   2965 2970 2975
   Cys Ala Ile His Tyr Arg Glu Asp Pro Leu Ser Gly
   2980 2985

<210> 107
<211> 1801
<212> DNA
<213> Mus musculus

<400> 107
cattgtctgc ctcagccact gccgccgcca ctgctgctgc aggatccgcc gcagccacca 60
gtctcgcgcg tcgtcgctcc ctccttggac agtaatttta tgaataagca tcagaagcca 120
gtactaacag gccagcggtt caaaacccgg aaaagggatg aaaaagagaa attcgaaccc 180
acagttttca gggatacact tgtccagggg cttaatgaag ctggtgatga ccttgaagct 240
gtagccaaat ttttggattc tactggctca cgattagatt accgtcgcta tgcagacaca 300
ctctttgata tcctggtggc tggcagtatg cttgcccctg gaggaacacg catagacgat 360
ggtgacaaga ccaagatgac caaccactgt gtgttttcag caaatgaaga tcatgaaacc 420
atccgaaact atgctcaggt cttcaataaa ctcatcagga gatacaagta tttggaaaag 480
gcatttgaag atgaaatgaa aaagcttctc ctcttcctta aagcattttc tgaagcagag 540
cagacaaagt tggcaatgct gtctgggatc ctgctgggca atgggaccct cccagccacc 600
atcctcacca gtctcttcac agacagctta gtcaaagaag gcatcgcagc ctcattcgct 660
gtcaagcttt tcaaagcctg gatggcagag aaagatgcca attctgttac ctcttcactg 720
agaaaagcca acttagacaa gaggctgctt gaactcttcc cagtgaacag acagagtgtg 780
gatcatttcg ctaagtactt cactgacgcg gggctgaagg agctgtcgga ctttctccga 840
gtccagcagt cgctgggcac caggaaagaa ctgcagaagg agctgcaaga gcgactgtct 900
caggaatgcc ccatcaagga ggtggtgctt tacgtcaaag aggaaatgaa aaggaacgac 960
cttccggaaa cagctgtgat cgggctgctg tggacctgca tcatgaatgc ggtggaatgg 1020
aacaagaagg aggagctggt tgcggagcag gcactgaagc acctaaagca atacgctccg 1080
ctgctggccg tgttcagttc ccaaggccag tccgagctcg tcctcctgca gaaggttcag 1140
gaatactgct acgacaacat ccacttcatg aaggccttcc agaagatcgt ggttctcttt 1200
tataaagccg acgttctgag cgaagaggcg atactgaagt ggtataaaga agcacacgcc 1260
gccaaaggca agagcgtctt tcttgaccag atgaagaaat ttgtggagtg gatacaaaat 1320
gcagaagaag aatctgaatc agaaggcgaa gaaagctaaa tgtctscrgc acwctgcmta 1380
aaaaaggaaa raaawaaytm awrsgccaca ctttgtcgcg yygscgmatg ctgrwgcgkt 1440
kggggrgggr rrcttggctt ctgtttgcac caaggaaata aatgataggc gaggcctccc 1500
ccgctccccg gggaaggcat ttttttcatt tctgttttgt ttcaatggag ccctgaggcg 1560
tcggctacta cacttgggac tctacctctc actgtacgct aactaaagcc attcaacaag 1620
ggggtcaggt aggaacctga agctcaaaac tcagtacacc ccttttttta gctgtatttt 1680
caggtactgt gtgaacgccc cactggtgtc tatgttacag ggccacttca atagttgtgt 1740
ttcttctgct cgtgtatgtg atttgacaaa ccagtttttt taaaataaac ggctttttaa 1800
a 1801

<210> 108
<211> 11157
<212> DNA
<213> Homo sapiens

<400> 108
   atggtggaag gagatacagt caccttctcc tttgaaatga gaagtggccg tgaacacaac 60
   actcctgata aagccatgtg gggctttgct tgcacagttc gcgctcagga gtcttcggag 120
   gatgtctcag gaggcttgcc ctttctggta gacctggctt taggtctgtc tgtgttagct 180
   tgttccatgt taagaatcct gtacaatgga ccagaaatta ccaaagaaga agaagcctgt 240
   caggagctat tgcggtccaa acttttacaa agagaagtga ttcagccaga cgtgatggag 300
   gaaatggtgg tatcttgtgt tattaagcac ttgaacttgg ttgatgcact gcagtctcta 360
   ataaatttcc aatatcaaga agaacatgct gaagaatatg atttattatg taaaattatg 420
   ggagagacct ttaagaaact caatgccatg gagagacagc tgcagaacaa aatgaaagaa 480
   ctagaacttt tatgttcaat gaaggaagtc tcctttgatg ggaatgatct tgaaaacatg 540
   gtcctatcac tgagggagaa gttcctacaa gaagtgaatt ctcttattca gaaaccctca 600
   cacccactgg ctaaaacaaa gacgttagtg aagagtttaa tgaaccgagc cgagctgttg 660
   ctgcatgtca ccatcgcagc ccagtcgggc ctcacgagaa gcatctctgg gacccctgct 720
   gaaacaccag cttgtaaatc agcttctgaa acaaaagtga tatctcacgc tgtcaggcag 780
   cctgtttttc ttcgcagcat gtcagctcct tctgacctgg aaatgattgg taatgaagat 840
   ttggaattta ctagagcaaa tcagaggcgt cgccacgtga ccagccaccg cagcagctcc 900
   tttacactcc tgcagtcgct ggccattgag gacagcaggg acaagcccac ctacagtgtc 960
   ctgctagggc agctgtttgc tttcatcggc accaaccctg accaagctgt gtccagcagc 1020
   agtttccttt tggctgcaca gacaaggtgg cggcggggaa acactcgcaa gcaggcactg 1080
   gtgcacatgc gggaattgct gacagctgcc gtacgagtcg ggggagtgac gcatcttgtg 1140
   ggtccagtga cgatggtcct tcagggagga cccagaattg aagaactcac atgtggtggg 1200
   atggtcgagc aggtccagga agcctttggc gagaccatga cctctgttgt gtctctgtgt 1260
   gctcgttacc ctatcgcttg cgcaaatagc attggactct tatgtaccat accttacacc 1320
   aggagcgaag agaagtgcct ggtacgcagt ggccttgtgc agctcatgga tcgactgtgt 1380
   agcttgagca atcagaccga gtccagctcc agtgagaaac agaccaagaa gcagaaggtg 1440
   gccaccatgg cctgggctgc cttccaggtg cttgccaacc gctgtgttga gtgggaaaaa 1500
   gaggaagaga caggatctta ctatgttgcc cagctgtgcc gggcggcgct gcccctgatg 1560
   agcgtagaag actgtggaaa cgtggagctc ccaccctgga gctactctgt cccctcctta 1620
   aacagtgagc aggaggatcc cagcgaccca gcttccaaga tcgcctccct gctcttagca 1680
   aagctggcag attatgtggt tccaggatgt cagacagttc tttctccaac cgcttctgaa 1740
   cctgacacca cattgacaaa aaccagtccc aagaattcct tgaaaggaga taaagatcct 1800
   ggagaagaga gtgaggctgt ggatggcaag ctctcgatat ttatccacaa gcgggaagac 1860
   cagtcatccc atgaagtcct ccagccattg ctaagtagtt cagaaggacg gcccttccga 1920
   cttggtactg gcgccaacat ggagaaagtt gtgaagatgg atcgagacat gaccaagggt 1980
   ggctgttgcg aagtgattac agaagaggct gcagccgccc tgcggaaagc caccaagtgg 2040
   gcacagtcag gcctcatcgt cagcattggg ccacctgtag agtctatcaa cccagagact 2100
   gtgagtggac tatccacagg cgacaaaaag aaaactgccc aaacttccat ttgccgagag 2160
   aggaactcgg agcttgcaag gactgatcct gtgcgtccct tcatcagtgg gcatgtggca 2220
   aacagcatgg ctgcagaagt gatcgccttg ctacatagct tgctcatggc acctgaatca 2280
   aatgctgctc aaatatggac cacaacagca gagaaggttc tgtctagagc actgatgtac 2340
   attccacaat tggggaaata cgcagaaagc attctggaaa atggcagcag cagtggcagg 2400
   aaacttgcca aacttcagag aattgcacgc caggccgttg ccgcactgtg tgcacttgga 2460
   ggcttcaaag agacaatcaa gataggatcc gaggttcagg cattgcctct tcataaactg 2520
   tccattactg agaaggtagt acaggcagtc cagtccatgt tgcttcctca ggagggaagt 2580
   ctctctattc acacctcact tcctgcaaca ggagatgggt cagctcctgt gatggcagtc 2640
   gttcggctcc ttgctgaaat aaggacaaga gcatgcctgg tcatggccca gcttttagaa 2700
   gacagcttat tttgtgaaga attcatacaa caatgtccag cagctgttga agttcttaac 2760
   ctagtagccc aggaatgtag tgctggagag cgacttgcag ttgtggaggt acagtgtgaa 2820
   cggctgagga tgctctaccg ggactgtgct cggcccccac cacctccttt gcaggctgac 2880
   cgaagacagg ccccgtcttt ttactgggaa attgaaatcg tttcctatgg agacaccgat 2940
   gatgacaccg gacccatagt ttcctttggc ttcactacag aagcagagaa gcgagatggg 3000
   gcttggacta atccagtggg cacttgtctt ttccataaca acggccgagc cgtgcactat 3060
   aatggctcca gtttgttaca gtggaagagc gttcgcttag acgtgactct ctcccccggt 3120
   gacgtggcag gaattggctg ggagagaact gaggggactc cacctcctcc gggacagcca 3180
   gccaagggcc gggtgtactt cacatactgc gggcagcgcc tctcaccata tcttgaagac 3240
   gtctctggtg gcatgtggcc agtggttcac attcagaaaa aggcagaaat aaaaataaac 3300
   gtaagtcttt ccaaatcctt catttggtct cctttccaga acaccaaaac ccgagctaac 3360
   tttggctccc ggccgtttgc ctacgcggaa gggcaggccc accgcaatgc tgctgacctg 3420
   tgcactgacc tagcagaaga gatcagcgct aactttgagg ccctgccttt cgccatggca 3480
   tctgacagtg acaatgatgc tggcaccagt attgcatcag acccaggcac tcatgggcca 3540
   ccatgccgga ttgctgccgt ggccaccgct cagcaacaat atgatagtga cacctcctgt 3600
   cattataaag tcgagctcag ctatgagaat ttcatcacct ctggcccaga ccctcacccg 3660
   cctcccattg cagatgatga aagcgatgat gatgacgatg atgacatccc tcaggaggac 3720
   cactacgccc tgctggtgaa agcatgggag accaaggttt ttcctaccat ccgaagacgc 3780
   ttccgcaatg aagcagagcg gaaatcgggc ctggaccaga taaagggggc cttacaacta 3840
   ggtatggtgg atattgcccg acagacggtt gaatttctct acgaagagaa tggtggcatc 3900
   ccaagagacc tttatcttcc caccattgaa gacattaaag acgaagcaaa caagttcaca 3960
   attgataaag ttcgaaaagg tctcacagta gtaacccgct ctccagacag caataatgta 4020
   gccagcagtg ctgttggaac tgctctgcca aaatttgcca tccgagggat gctgaaaacc 4080
   tttgggcttc atggagtcgt cttagatgtt gattcagtga atgaactggt gcaggtagaa 4140
   acgtacctcc gcagtgaagg tgtgctggtg cgatactggt atcctattga catgttggaa 4200
   aggcccccag caggctaccg aaggactgcc accaatgggc tggtcacact ggacaatacc 4260
   aaccttcaaa ttcacaggga gctgctgcgc tgcgaggctg cgctggccag gctgtactgc 4320
   cgcatggccc tgctcaatat cttcgccccg aagttgcctc acttgttcac tcgcctcttc 4380
   cacatccctg ccatccggga cattaccctg gagcacctgc aactgctgtc caatcagctc 4440
   ctcgcacctc ctctcccaga cggcaccatc agctccagct cgatcctcct ggcgcagtct 4500
   ttacagcatt gcatccattc ccagaactgc tccgccacgg acctctttta ccagggcaac 4560
   tcccagacag tgagagagtg gctcaacgtg gccatcaccc ggaccctgca ccagggcgag 4620
   gagagccttt tagagctgac gaaacagatc tgctctttcc tgcagacagc accagagcag 4680
   ttcccctccg aagagttccc aatttccgaa tccaaagtca acatggacgt gaatttcccc 4740
   ggggcagctt ttgttgttgt gtcttgtaaa gaaagtcaat ctggattccg caaagactcc 4800
   tctctgtaca aggcaccgtg ggcacgggtg ctcgtatatg gcctcggcca caaagtgaag 4860
   cgaaatggcc agctgaacct catcgaggcc gcctgttacc cgcgggacgc gtccccagcc 4920
   aacactgggc ttgcacctcc ccccaccgct gaccagtacc cctctgtggt cctctccaca 4980
   gacagggtcc acatcaaact gggggtgtct ccacctcctg gagcagtcct ggtgttacat 5040
   tccctgcccc tggagttccc actggctatg gccttcgcag agcagctgct gtcctggaaa 5100
   tcagaggaca gtgaagggaa gtccgaagat gagcctgaca ccattccgac atccgtcctc 5160
   ctgcaggtgg tggagctgct aggaaacttc ttgtggacca cggacatggc agcctgcgtg 5220
   aaggagcttg ttttccatct cctggcagag ctcctgcgca cggtgcacac cctggagcag 5280
   aggcggcacc ccgctggcct gtcctcctca atcgccctcc agctgaaccc ctgcctggcc 5340
   atgctgatgg ccttgcagtc ggagctccac aagctgtacg acgaggagac gcagaactgg 5400
   gtctcaggcg gcgcctgcgg gggctccggg ggggcggcgg ccggtgacca gggcaggttc 5460
   tctacgtatt ttcatgcact catggaaggg tgcctggctg tggccgaagt gaccctgcct 5520
   actaacatga gtgtcacagc cagtggggtg acctcagcga ccgccccaaa tctcagtgac 5580
   tcgtcctcct cctcctcgtc ctccccagga cagaccccac agagtcccag cctcctctcc 5640
   aagaggaaaa aagtcaagat gaagcgggaa aaggcctcct cgtcgggcaa gcgccagtct 5700
   tcccgcaccg tggactcgga ccccaccgtg ctcagcatcg gaggcagcaa gcccgaggac 5760
   atgctgtggt tccaccgcgc actcaccctg ctcatcatcc tccgccacct caccaggaag 5820
   gacccacagg ggctgggcgt gacgagtgac gccatcgccg atgcctgcca ggccctggtg 5880
   ggccccaccg cccacagccg cttgctggtg atctccggga tccccaccca cctggacgag 5940
   ggcgtagtca gaggcgccat ccgcaaggcc tgcaacgccc acggcggggt cttcaaagac 6000
   gagatctaca tcccgctgca ggaagaagac accaagaagc caaaagacaa ggccgagggc 6060
   ggggacggga aagttgagcc cgagaagaca ctggccttcc ccggcacaga cagcatggag 6120
   gtcagcacgt ccagcagcct gacccccgcc atgagcatca gcgcctccgc ctccaccagc 6180
   caggcctcca tctgcagctc gcagggcatc tcccaaaccg tcagcgacct ctctgtggat 6240
   ccgctgcctg ccggcctcga gctgcccatc cctccgggcc tgttggagcc ccacgcggtg 6300
   tccagccagg aaagcctgga catttccctg tgcagcaccg gcagcctggg cagcctgggc 6360
   agcctggggg agcccctgga caatgcagag acggcctcgg tgtcggacat gggctccatg 6420
   tacacagtca cttccctgga caaccagccc ctcgccgccc gccccatcaa aggcttcgca 6480
   gtcgtggaga taagatcccg agccaaaatt gagaaaattc gagcaagttt atttaacaat 6540
   aatgacttga ttggtttgtc aagtttggat ggtgaagatg aattgatgga aatgtcaaca 6600
   gaagagattc taaccgtgtc tgtagtaaat cagagtctct ttgatactca agggagccca 6660
   gggttagaag attatttcaa tgataagtca attaaagggg agaagctggt gcccggagcc 6720
   agagaggttc tgacggagat atttaagagt tgtgcccatt cagagcagac gctgagcctg 6780
   acaccagcga agcccatcag agtctctgac atttatctta gcaaagagca gatcaactcc 6840
   cagaccccag gcaacctcct ccacctcttc ttcaccaatg tccggccgcc aaaaaaggtg 6900
   ctggaggatc agctcaccca gatcctgagg aagtatggcg tgccaaagcc caagtttgac 6960
   aagagcaagt acagcaaggc cgggaaggag cagcaccccg tgaaggtggt gagcaccaag 7020
   cggcctatca ccaagccgcc cgccaaggac aaggctgtgc tcaacagcgt cagcaggact 7080
   gccttaagtg agaagaagcc aactgtgaag ccaaaatcac cagaaaagag caaaccagat 7140
   gaaaaggacc cagaaaagtc acctaccaaa aaacaagaag tccctgagga aaaatacctg 7200
   acgctggaag gatttcacaa atttgttatt gaccgagcca ggcaagatat ccgtagcgtc 7260
   tggagggcga tcttgtcctg tggttacgat cttcactttg agaggtgcgc atgcatcgat 7320
   gtccgacatg cacagaaggc ctcaagaaag tggaccctgg agatggacgt ggcacttgtg 7380
   cagtacatca accagctatg ccgccacctc gccatcacac ccgcacggct ccatccccat 7440
   gaggtgtacc tggaccccgc ggatgctgct gaccccagag tggcctgtct cctgaacgtg 7500
   cccatcgaga gcctgcgcct gcgcttcgcc ttgctgcagt ccctcaacac cacactggag 7560
   accttcttcc tgcccctggt ggagctgcgc cagacaccca tgtataccca cagcatcgcc 7620
   gccctgctga aggaggccaa agggctgatc ttctatgaca cgaaggtgac cgtgatgaat 7680
   cgagtgctga atgccactgt gcagaggaca gcggaccacg cggcccctga gatcaccttg 7740
   gacccactgg aaattgtggg aggggaaatc agagcttctg aaaactccta cttctgtcag 7800
   gctgccaggc agctggcctc agtgccgtcg tctcagctct gcgtcaagct ggccagtggc 7860
   ggtgacccca catatgcctt caacatccgc ttcactggcg aggaggtcca tggcaccagc 7920
   ggctctttcc gccacttcct gtggcaggtg tgtaaggagc tgcagagttc ctcgctgtcg 7980
   ctgctgctgc tgtgccccag ctcagctgtc aataagaaca agggcaagta tatcctgacc 8040
   ccgagcccca tcacctacgg ggaggagcag ctgctgcact tcctggggca gctgctgggg 8100
   attgcaattc gggcagacgt cccgctgccc ctggacctcc tgccctcctt ctggaagacg 8160
   ctggtgggcg agcccttgga ccctgagcaa gacctgcagg aagcggatat cctcacctac 8220
   aattacgtca agaagtttga gagcatcaat gatgagaccg agctggaggc cctgtgcgct 8280
   gagatcgcct cccagcacct ggccacggag agccctgaca gccccaacaa gccctgctgc 8340
   aggttcacct acctgaccat gacgggcgag gaggtggagc tgtgcagccg gggccggcac 8400
   atccttgtgg cgtgggagaa caaggacatc tacgcggcag ccatccggag cctgcggctg 8460
   cgggagctgc agaatgtgga gtgcgtgacg gccgtgcggg ccggcctggg ctccatcatc 8520
   cccctgcagc tgctgaccat gctcagccca ctggagatgg agctgcgcac ctgcggcctc 8580
   ccctacatca acctcgagtt cctcaaggcc cacaccatgt accaagtggg gctgatggag 8640
   acggaccagc acatcgagtt cttctggggg gccctggaga tgttcaccca ggaggagctg 8700
   tgcaagttca tcaagtttgc ctgcaaccag gagcgcatcc cgttcacctg cccctgcaaa 8760
   gatgggggtc ccgacactgc ccatgtgccc ccgtacccca tgaagatcgc ccccccagat 8820
   ggcacagcag gttccccaga ctctcgctac atccgcgtgg agacctgcat gttcatgatc 8880
   aagcttcccc agtactcctc tctggaaatc atgctggaga aacttcgttg tgccattcac 8940
   taccgtgaag accccctcag tggctgatgg gagggagccc cagaattagg ctgtcactga 9000
   ggcacccact ctgctggctt gggaagccac cactgcggcc cgtccctcca gggccctgcg 9060
   tgaggagttg gcaacatttt gcttttccaa actttcgtcc acattccagg gcctcctgga 9120
   agacttaacc ttttgtcttt gtacgtttcg tgatgggttg gttcttttgc tgcctgtttg 9180
   tggtctattt gtaggatagt ttagttccca gacagtttgt gtctaatttg atcttcttgg 9240
   acattgtccc tcatggccac cagattctaa tgccataagg ccccagctgg ttccccatca 9300
   cagaaccacc cagcaggaag ccgggggtac aatgacctcg gccacacaga gcgctcgtgc 9360
   tttcagcaga gcgcctccac agacaagcct gtggccagcc ctgccctcca gcctcgagct 9420
   ggagttgctc cagaggcagc cccagcaggc ctccctccac atagaggtgg ctgggccaat 9480
   tgcccaaaca cgtgagacac acagggagct ttatgtgttc atcttcctgc tttagaactt 9540
   gctcccggtc ttggtttttc tgaggaaaag cccacagtgg ttcccctgcc cctcccatac 9600
   ctccctatct gccgtctgtc tctttgggcc ctggtctgac agttttcatc tgaggcctcc 9660
   tcgggagcag gagacttaag ggttcatccc gaccttagtt cacagagcct agctgggtgc 9720
   atcccagccg cagggggtga gaagacaccc agagatgggg gacaatgctc aggtgggggc 9780
   ctcctccgag actctggttg agaggaaagc acatgattca agcctacccc acaaaaaagg 9840
   agcacaaaat gctcagaaat ggcccttcct ccctgcagtg acgtaggaca agggaaaggg 9900
   ccacacccat tttcaaaaga gtggactgag gtgctctgct caatttgggg tctgctttag 9960
   tgacccagac actcaagtcc agatggagac tccagggggg tccttctgcc cctctggcca 10020
   gccccaccca gcactcccca tcaccccatg taagtgtgta tgccagggct gagctgcctc 10080
   caggctcacc cactgcatct ttgccccaaa taggtctttg tccagtggta ctttgttcat 10140
   gagcagcaca ggccatcttg ttggacactg tcactttggg ccttgccagg tctcacagaa 10200
   tcacatcgct gtatttattg tataatattg tgaatattgg aagttttgag tgagtgctgt 10260
   ctagaaggtt ttggagtgtg gatgctggtc caagaaagaa gatgtgccct ggcctgctgg 10320
   aaagattgtg aattctagag atagagccgt tagcatccat ctcctccctg taactcttag 10380
   gaaatgcatc aaggaccatt atagaaagga tcccctgacc taaggatgaa atcgacctag 10440
   ggttaatcat gacagctgca tcagttgtat tggatgcaag tcaactctgg aggtgagtgc 10500
   ccgcaagcag ttcccctgct ttgaaagaag atggtcagcc cgctgcctcc ccagcggcca 10560
   cagctccttt gagtgtgggg ggtcacagcc aggatgtatt cctgacaata aggatccttt 10620
   tattttcatt tctgcataat gaaagagcct ttcgatttat tagaagtggt ttttcctccc 10680
   tacatcttta aattcttggt tctctttagt cctcatccct tgaatggact tgtcagctac 10740
   ctccccagga ggagttcaga agcttctgtt ggccatcact tgagtagatg acaagctgta 10800
   aggtatctgc tgctaacgtg gctgcttctc cgagagcaca ttgaggtcca gacagatcca 10860
   aaccttggcc ctaaaaaatg tgtgctgtaa agttacttga tgtatattta ttataattat 10920
   ttatggttgc atttaacaaa cttttcattc aatctgatgc tatttacacc atgctgtgta 10980
   aaggaagctc actaaaggaa aaaagtcaca ccaataaata accttcatct aattttgatt 11040
   taccttaaga gttgtgtgat catctactct tgctgagcaa gttcaaaggc tggcatgctt 11100
   ggatggctct gatatttaag tgctgccaag tggctaggaa ttaaagtgtt tctaatt 11157

<210> 109
<211> 324
<212> PRT
<213> Mus musculus

<400> 109
Met Asp Gln Ser Asn Met Thr Ser Leu Ala Glu Glu Lys Ala Met Asn
1               5                   10                  15

Thr Ser Ser Arg Asn Ala Ser Leu Gly Ser Ser His Pro Pro Ile Pro
            20                  25                  30

Ile Val His Trp Val Ile Met Ser Ile Ser Pro Leu Gly Phe Val Glu
        35                  40                  45

Asn Gly Ile Leu Leu Trp Phe Leu Cys Phe Arg Met Arg Arg Asn Pro
    50                  55                  60

Phe Thr Val Tyr Ile Thr His Leu Ser Ile Ala Asp Ile Ser Leu Leu
65                  70                  75                  80

Phe Cys Ile Phe Ile Leu Ser Ile Asp Tyr Ala Leu Asp Tyr Glu Leu
                85                  90                  95

Ser Ser Gly His His Tyr Thr Ile Val Thr Leu Ser Val Thr Phe Leu
            100                 105                 110

Phe Gly Tyr Asn Thr Gly Leu Tyr Leu Leu Thr Ala Ile Ser Val Glu
        115                 120                 125

Arg Cys Leu Ser Val Leu Tyr Pro Ile Trp Tyr Arg Cys His Arg Pro
    130                 135                 140

Lys His Gln Ser Ala Phe Val Cys Ala Leu Leu Trp Ala Leu Ser Cys
145                 150                 155                 160

Leu Val Thr Thr Met Glu Tyr Val Met Cys Ile Asp Ser Gly Glu Glu
                165                 170                 175

Ser His Ser Arg Ser Asp Cys Arg Ala Val Ile Ile Phe Ile Ala Ile
            180                 185                 190

Leu Ser Phe Leu Val Phe Thr Pro Leu Met Leu Val Ser Ser Thr Ile
        195                 200                 205

Leu Val Val Lys Ile Arg Lys Asn Thr Trp Ala Ser His Ser Ser Lys
    210                 215                 220

Leu Tyr Ile Val Ile Met Val Thr Ile Ile Ile Phe Leu Ile Phe Ala
225                 230                 235                 240

Met Pro Met Arg Val Leu Tyr Leu Leu Tyr Tyr Glu Tyr Trp Ser Ala
                245                 250                 255

Phe Gly Asn Leu His Asn Ile Ser Leu Leu Phe Ser Thr Ile Asn Ser
            260                 265                 270

Ser Ala Asn Pro Phe Ile Tyr Phe Phe Val Gly Ser Ser Lys Lys Lys
        275                 280                 285

Arg Phe Arg Glu Ser Leu Lys Val Val Leu Thr Arg Ala Phe Lys Asp
    290                 295                 300

Glu Met Gln Pro Arg Arg Gln Glu Gly Asn Gly Asn Thr Val Ser Ile
305                 310                 315                 320

Glu Thr Val Val

<210> 110
<211> 267
<212> PRT
<213> Homo sapiens

<400> 110
Met Thr Thr Lys Asn Leu Glu Thr Lys Val Thr Val Thr Ser Ser Pro
  1               5                  10                  15

Thr Arg Gly Ala Gly Asp Gly Met Glu Thr Glu Glu Pro Pro Lys Ser
             20                  25                  30

Val Glu Val Thr Ser Gly Val Gln Ser Arg Lys His His Ser Leu Gln
         35                  40                  45

Ser Pro Trp Lys Lys Ala Val Pro Ser Glu Ser Pro Gly Val Leu Gln
     50                  55                  60

Leu Gly Lys Met Leu Thr Glu Lys Ala Met Glu Val Lys Ala Val Arg
 65                  70                  75                  80

Ile Leu Val Pro Lys Ala Ala Ile Thr His Asp Ile Pro Asn Lys Asn
                 85                  90                  95

Thr Lys Val Lys Ser Leu Gly His His Lys Gly Glu Phe Leu Gly Gln
            100                 105                 110

Ser Glu Gly Val Ile Glu Pro Asn Lys Glu Leu Ser Glu Val Lys Asn
        115                 120                 125

Val Leu Glu Lys Leu Lys Asn Ser Glu Arg Arg Leu Leu Gln Asp Lys
    130                 135                 140

Glu Gly Leu Ser Asn Gln Leu Arg Val Gln Thr Glu Val Asn Arg Glu
145                 150                 155                 160

Leu Lys Lys Leu Leu Val Ala Ser Val Gly Asp Asp Leu Gln Tyr His

165                 170                 175

Phe Glu Arg Leu Ala Arg Glu Lys Asn Gln Leu Ile Leu Glu Asn Glu
            180                 185                 190

Ala Leu Gly Arg Asn Thr Ala Gln Leu Ser Glu Gln Leu Glu Arg Met
        195                 200                 205

Ser Ile Gln Cys Asp Val Trp Arg Ser Lys Phe Leu Ala Ser Arg Val
    210                 215                 220

Met Ala Asp Glu Leu Thr Asn Ser Arg Ala Ala Leu Gln Arg Gln Asn
225                 230                 235                 240

Arg Asp Ala His Gly Ala Ile Gln Asp Leu Leu Ser Glu Arg Glu Gln
                245                 250                 255

Phe Arg Gln Glu Met Ile Ala Thr Gln Lys Phe
            260                 265

<210> 111
<211> 403
<212> PRT
<213> Mus musculus

<400> 111
Met Glu Lys Met Thr Thr Leu Lys Ser Ser Glu Asn Lys Gly Ile Leu
  1               5                  10                  15

Thr Ser Thr Pro Ile Arg Gly Ala Gly Asp Gly Met Glu Thr Glu Glu
             20                  25                  30

Pro Pro Lys Ser Val Glu Val Thr His Gly Val Gln Pro Ile Asn Gln
         35                  40                  45

His Val Leu Pro Ser Pro Arg Lys Lys Val Ser Ser Asp Ser Pro Gly
     50                  55                  60

Val Leu Gln Leu Gly Lys Ile Leu Asn Glu Arg Thr Val Glu Val Glu
 65                  70                  75                  80

Ala Val Arg Ile Phe Val Pro Lys Ala Ala Ile Thr His Asp Ile Pro
                 85                  90                  95

Thr Lys Asn Thr Lys Val Lys Ser Leu Gly His His Arg Glu Glu Leu
            100                 105                 110

His Asn Gln Ala Glu Val Val Thr Asp Pro Arg Lys Glu Leu Ser Glu
        115                 120                 125

Val Lys Lys Val Leu Glu Lys Leu Lys Asn Ser Glu Arg Arg Leu Leu
    130                 135                 140

Gln Asp Lys Glu Gly Leu Ser Asn Gln Leu Arg Val Gln Thr Glu Ile
145                 150                 155                 160

Asn Arg Glu Leu Lys Lys Leu Leu Val Ala Ser Val Gly Asp Asp Pro
                165                 170                 175

Gln Tyr His Phe Glu Arg Leu Ala Arg Glu Lys Asn Gln Leu Ile Leu
            180                 185                 190

Glu Asn Glu Ala Leu Gly Arg Asn Thr Ala Gln Leu Ser Glu Gln Leu
        195                 200                 205

Glu Arg Met Ser Ile Gln Cys Asp Val Trp Arg Ser Lys Phe Leu Ala
    210                 215                 220

Ser Arg Val Met Ala Asp Glu Leu Thr Asn Phe Arg Val Val Leu Gln
225                 230                 235                 240

Arg Gln Asn Arg Asp Ala Gln Ser Ala Ile Gln Asp Leu Leu Ser Glu
                245                 250                 255

Arg Glu Gln Phe Arg Gln Glu Met Thr Ser Thr Gln Lys Phe Leu Glu
            260                 265                 270

Glu Leu Leu Val Ser Leu Gln Trp Gly Arg Glu Gln Thr Tyr Ser Pro
        275                 280                 285

Asn Thr Gln Pro His Ser Thr Ala Asp Leu Ala Leu Thr Asn His Gly
    290                 295                 300

Leu Ala Gln Ala Ile His Ala His Leu Leu Gly Asn Val Gly Ile Ser
305                 310                 315                 320

His Gln Lys Lys Ile Pro Thr Thr Val Glu Phe Cys Ser Thr Pro Ala
                325                 330                 335

Glu Lys Met Ala Glu Lys Val Leu Arg Ile Leu Asp Pro Val Ala Cys
            340                 345                 350

Thr Glu Ser Ser Pro Asp Asn Gln Phe Ala Glu Ser Ser Pro Thr Thr
        355                 360                 365

Leu Leu Thr Thr Lys Lys Asn Ile Gly Arg Phe His Pro Tyr Thr Arg
    370                 375                 380

Tyr Glu Asn Ile Thr Phe Asn Cys Cys Asn His Cys Gln Gly Glu Leu
385                 390                 395                 400

Ile Ala Leu

<210> 112
<211> 35
<212> PRT
<213> Mus musculus

<400> 112
Met Glu Lys Met Thr Thr Leu Lys Ser Ser Glu Asn Lys Gly Lys Gln
1               5                   10                  15

Glu Met Met Lys Gly Lys Lys Met Gln Ser Pro Ser Pro Ala Cys Ser
            20                  25                  30

Pro Ala Arg
        35

<210> 113
<211> 319
<212> PRT
<213> Mus musculus

<400> 113
Met Met Val Leu Arg Val Glu Glu Leu Val Thr Gly Lys Lys Asn Ser
1               5                   10                  15

Asn Gly Ala Ala Gly Glu Phe Leu Pro Gly Glu Phe Arg Asn Gly Glu
            20                  25                  30

Tyr Glu Ala Ala Val Ala Leu Glu Lys Gln Glu Asp Leu Lys Thr Leu
        35                  40                  45

Pro Ala Asn Ser Val Lys Gln Gly Glu Glu Gln Arg Lys Ser Glu Lys
    50                  55                  60

Leu Arg Glu Ala Glu Leu Lys Lys Lys Lys Leu Glu Gln Arg Ser Lys
65                  70                  75                  80

Leu Glu Asn Leu Glu Asp Leu Glu Ile Ile Val Gln Leu Lys Lys Arg

                85                  90                  95

Lys Lys Tyr Lys Lys Thr Lys Val Pro Val Val Lys Glu Pro Glu Pro
            100                 105                 110

Glu Ile Met Thr Glu Pro Val Asp Val Pro Arg Phe Leu Lys Ala Ala
        115                 120                 125

Leu Glu Asn Lys Leu Pro Val Val Glu Lys Phe Leu Ser Asp Lys Asn
    130                 135                 140

Ser Pro Asp Val Cys Asp Glu Tyr Lys Arg Thr Ala Leu His Arg Ala
145                 150                 155                 160

Cys Leu Glu Gly His Leu Ala Ile Val Glu Lys Leu Met Glu Ala Gly
                165                 170                 175

Ala Gln Ile Glu Phe Arg Asp Met Leu Glu Ser Thr Ala Ile His Trp
            180                 185                 190

Ala Cys Arg Gly Gly Asn Ala Asp Val Leu Lys Leu Leu Leu Asn Lys
        195                 200                 205

Gly Ala Lys Ile Ser Ala Arg Asp Lys Leu Leu Ser Thr Ala Leu His
    210                 215                 220

Val Ala Val Arg Thr Gly His Tyr Glu Cys Ala Glu His Leu Ile Ala
225                 230                 235                 240

Cys Glu Ala Asp Leu Asn Ala Lys Asp Arg Glu Gly Asp Thr Pro Leu
                245                 250                 255

His Asp Ala Val Arg Leu Asn Arg Tyr Lys Met Ile Arg Leu Leu Met
            260                 265                 270

Thr Phe Gly Ala Asp Leu Lys Val Lys Asn Cys Ala Gly Lys Thr Pro
        275                 280                 285

Met Asp Leu Val Leu His Trp Gln Ser Gly Thr Lys Ala Ile Phe Asp
    290                 295                 300

Ser Leu Lys Glu Asn Ala Tyr Lys Asn Ser Arg Ile Ala Thr Phe
305                 310                 315

<210> 114
<211> 509
<212> PRT

<213> Homo sapiens

<400> 114
Met Tyr Ser Pro Leu Cys Leu Thr Gln Asp Glu Phe His Pro Phe Ile
1               5                   10                  15

Glu Ala Leu Leu Pro His Val Arg Ala Phe Ala Tyr Thr Trp Phe Asn
            20                  25                  30

Leu Gln Ala Arg Lys Arg Lys Tyr Phe Lys Lys His Glu Lys Arg Met
        35                  40                  45

Ser Lys Glu Glu Glu Arg Ala Val Lys Asp Glu Leu Leu Ser Glu Lys
    50                  55                  60

Pro Glu Val Lys Gln Lys Trp Ala Ser Arg Leu Leu Ala Lys Leu Arg
65                  70                  75                  80

Lys Asp Ile Arg Pro Glu Tyr Arg Glu Asp Phe Val Leu Thr Val Thr
                85                  90                  95

Gly Lys Lys Pro Pro Cys Cys Val Leu Ser Asn Pro Asp Gln Lys Gly
            100                 105                 110

Lys Met Arg Arg Ile Asp Cys Leu Arg Gln Ala Asp Lys Val Trp Arg
        115                 120                 125

Leu Asp Leu Val Met Val Ile Leu Phe Lys Gly Ile Pro Leu Glu Ser
    130                 135                 140

Thr Asp Gly Glu Arg Leu Val Lys Ser Pro Gln Cys Ser Asn Pro Gly
145                 150                 155                 160

Leu Cys Val Gln Pro His His Ile Gly Val Ser Val Lys Glu Leu Asp
                165                 170                 175

Leu Tyr Leu Ala Tyr Phe Val His Ala Ala Asp Ser Ser Gln Ser Glu
            180                 185                 190

Ser Pro Ser Gln Pro Ser Asp Ala Asp Ile Lys Asp Gln Pro Glu Asn
        195                 200                 205

Gly His Leu Gly Phe Gln Asp Ser Phe Val Thr Ser Gly Val Phe Ser
    210                 215                 220

Val Thr Glu Leu Val Arg Val Ser Gln Thr Pro Ile Ala Ala Gly Thr
225                 230                 235                 240

Gly Pro Asn Phe Ser Leu Ser Asp Leu Glu Ser Ser Ser Tyr Tyr Ser
                245                 250                 255

Met Ser Pro Gly Ala Met Arg Arg Ser Leu Pro Ser Thr Ser Ser Thr
            260                 265                 270

Ser Ser Thr Lys Arg Leu Lys Ser Val Glu Asp Glu Met Asp Ser Pro
        275                 280                 285

Gly Glu Glu Pro Phe Tyr Thr Gly Gln Gly Arg Ser Pro Gly Ser Gly
    290                 295                 300

Ser Gln Ser Ser Gly Trp His Glu Val Glu Pro Gly Met Pro Ser Pro
305                 310                 315                 320

Thr Thr Leu Lys Lys Ser Glu Lys Ser Gly Phe Ser Ser Pro Ser Pro
                325                 330                 335

Ser Gln Thr Ser Ser Leu Gly Thr Ala Phe Thr Gln His His Arg Pro
            340                 345                 350

Val Ile Thr Gly Pro Arg Ala Ser Pro His Ala Thr Pro Ser Thr Leu
        355                 360                 365

His Phe Pro Thr Ser Pro Ile Ile Gln Gln Pro Gly Pro Tyr Phe Ser
    370                 375                 380

His Pro Ala Ile Arg Tyr His Pro Gln Glu Thr Leu Lys Glu Phe Val
385                 390                 395                 400

Gln Leu Val Cys Pro Asp Ala Gly Gln Gln Ala Gly Gln Val Gly Phe
                405                 410                 415

Leu Asn Pro Asn Gly Ser Ser Gln Gly Lys Val His Asn Pro Phe Leu
            420                 425                 430

Pro Thr Pro Met Leu Pro Pro Pro Pro Pro Pro Pro Met Ala Arg Pro
        435                 440                 445

Val Pro Leu Pro Val Pro Asp Thr Lys Pro Pro Thr Thr Ser Thr Glu
    450                 455                 460

Gly Gly Ala Ala Ser Pro Thr Ser Pro Thr Tyr Ser Thr Pro Ser Thr
465                 470                 475                 480

Ser Pro Ala Asn Arg Phe Val Ser Val Gly Pro Arg Asp Pro Ser Phe
                485                 490                 495

Val Asn Ile Pro Gln Gln Thr Gln Ser Trp Tyr Leu Gly
            500                 505

<210> 115
<400> 115
000

<210> 116
<211> 1625
<212> DNA
<213> Homo sapiens

<400> 116
agcagcggcg ggggcccgag ggattctgaa ggaagatttc cattaggtaa tttgtttaat 60
cagtgcaagc gaaattaagg gaaaatggat gtagaaaatg agcagatact gaatgtaaac 120
cctgcagatc ctgataactt aagtgactct ctcttttccg gtgatgaaga aaatgctggg 180
actgaggaag taaagaatga aataaatgga aattggattt cagcatcctc cattaacgaa 240
gctagaatta atgccaaggc aaaaaggcga ctaaggaaaa actcatcccg ggactctggc 300
agaggcgatt cggtcagcga cagtgggagt gacgccctta gaagtggatt aactgtgcca 360
accagtccaa agggaaggtt gctggatagg cgatccagat ctgggaaagg aaggggacta 420
ccaaagaaag gtggtgcagg aggcaaaggt gtctggggta cacctggaca ggtgtatgat 480
gtggaggagg tggatgtgaa agatcctaac tatgatgatg accaggagaa ctgtgtttat 540
gaaactgtag ttttgccttt ggatgaaagg gcatttgaga agactttaac accaatcata 600
caggaatatt ttgagcatgg agatactaat gaagttgcgg aaatgttaag agatttaaat 660
cttggtgaaa tgaaaagtgg agtaccagtg ttggcagtat ccttagcatt ggaggggaag 720
gctagtcata gagagatgac atctaagctt ctttctgacc tttgtgggac agtaatgagc 780
acaactgatg tggaaaaatc atttgataaa ttgttgaaag atctacctga attagcactg 840
gatactccta gagcaccaca gttggtgggc cagtttattg ctagagctgt tggagatgga 900
attttatgta atacctatat tgatagttac aaaggaactg tagattgtgt gcaggctaga 960
gctgctctgg ataaggctac cgtgcttctg agtatgtcta aaggtggaaa gcgtaaagat 1020
agtgtgtggg gctctggagg tgggcagcaa tctgtcaatc accttgttaa agagattgat 1080
atgctgctga aagaatattt actctctgga gacatatctg aagctgaaca ttgccttaag 1140
gaactggaag tacctcattt tcaccatgag cttgtatatg aagctattat aatggtttta 1200
gagtcaactg gagaaagtac atttaagatg attttggatt tattaaagtc cctttggaag 1260
tcttctacca ttactgtaga ccaaatgaaa agaggttatg agagaattta caatgaaatt 1320
ccggacatta atctggatgt cccacattca tactctgtgc tggagcggtt tgtagaagaa 1380
tgttttcagg ctggaataat ttccaaacaa ctcagagatc tttgtccttc aaggggcaga 1440
aagcgttttg taagcgaagg agatggaggt cgtcttaaac cagagagcta ctgaatataa 1500
gaactcttgc agtcttagat gttataaaaa tatatatctg aattgtaaga gttgttagca 1560
caagtttttt tttttttttt ttaagcactt gttttgggta caaggcattt ctgacatttt 1620
gggta                                                             1625

<210> 117
<211> 3510
<212> DNA

<213> Mus musculus

<400> 117
gaattcggca cgaggtgtgc taagacctga cggctagcgc tgctttggct actgcacgcc 60
ccgctgtaag gggcccgctg tctccagaag aactggatat tctttgttac aattatggca 120
gacaaattaa caagaattgc tattgtcaac cacgacaaat gcaaacctaa gaaatgtcga 180
caggagtgca aaaagagttg ccctgtggtt cggatgggaa aattgtgcat agaagttaca 240
ccccagagca aaatagcatg gatttctgaa actctctgta ttggttgtgg tatttgtatt 300
aagaaatgtc cctttggcgc cttatcaatt gtcaatttgc caagcaactt ggaaaaagaa 360
acaacacatc gctactgtgc caatgccttc aagcttcaca ggttgcctat ccctcgtcca 420
ggtgaagttt tgggattagt tggaactaat ggtattggaa agtcaactgc actaaaaatt 480
ttagcaggaa aacaaaagcc aaaccttgga aagtatgatg atccacctga ttggcaagag 540
attttgactt atttccgtgg atctgaatta caaaattact ttaccaagat tctcgaagat 600
gacctaaaag ccattatcaa acctcaatat gtagaccaaa ttcccaaggc tgcaaagggg 660
acagtgggct ctattctgga ccgaaaagat gaaacaaaga cacaggcaat tgtatgtcag 720
cagcttgatt taactcacct taaagaacga aatgtcgaag atctttcagg aggagagttg 780
cagagatttg cttgtgctgt cgtttgcata caaaaggctg atatttttat gtttgatgaa 840
ccttctagtt acctcgatgt caagcaacgt ttaaaggctg ccattacgat tcgatctcta 900
ataaatccag atagatatat cattgtggtg gagcatgatc taagtgtatt agactatctc 960
tctgacttca tctgctgtct atatggggta ccgagtgctt atggtgttgt cacgatgcct 1020
tttagtgtaa gagaaggcat aaatatattt ttggatggct atgttccaac agagaacctg 1080
aggttcaggg atgcgtcgct tgtttttaag gtagctgaga cagcaaatga agaagaagtt 1140
aaaaagatgt gcatgtataa atatcccggg atgaagaaaa agatgggaga gttcgagcta 1200
gcaattgtag ctggagagtt cacggactct gagatcatgg tgatgctggg ggagaatggt 1260
acaggtaaaa ctacatttat cagaatgctt gctggaaggc ttaaaccaga tgaaggagga 1320
gaagtgccag ttctaaatgt cagttataag ccacagaaaa tcagtcccaa atcaacagga 1380
agtgttcgcc agttactgca tgaaaagatc agagatgctt acacgcatcc gcagtttgtg 1440
actgatgtaa tgaagcccct acagattgaa aacatcattg accaagaggt acagacattg 1500
tctggtggtg aacttcagcg agtagcttta gctctttgtt tgggcaaacc tgctgacgtc 1560
tatttgattg atgaaccttc tgcatatttg gattctgagc aaagattaat ggcagctcgg 1620
gtcgtcaaac gtttcattct ccatgcaaag aagacagctt ttgttgtaga acatgacttc 1680
atcatggcca cctatctagc agatcgcgtc atcgtttttg atggtgttcc atcaaagaac 1740
acagttgcaa acagtcctca gactcttttg gctggcatga acaaattttt gtctcagctc 1800
gaaattacat tcagaagaga ccccaacaac tacaggccac gaataaataa gctcaattca 1860
atcaaggatg tagaacaaaa gaagagtgga aactactttt tcttggatga ttagattgag 1920
tctgaactta taagccattt acttactggg atttttatca tataaaactt taatcaggtt 1980
ttatggcccc acatactctg gagcttgaag tatgattttc ttaatgtgac atcaaaaagc 2040
cagttgtctt gtaaattgtt gtttaagaac tattttgttc ttaagattaa acctctgtgc 2100
atactacatg aacatggaga catttcagat tatcagttac ctccagattt tcacaatcta 2160
cggggagatt ttcagttgta tattatattc atgtaccaaa tgcttcctgg tgttgcccat 2220
ctttaaaatc ttggataaaa tctgtactta cctgtttgct aagtgtacca gtataacaga 2280
actgccccct tctaaaaagc cagtcacaga cttgctgcca aagtctgata cataagcatc 2340
agggctatat ttgctacttg tgttcagtgt gtgcttccaa ggaacagtgt ctgctacaaa 2400
caccattgtt ggaagatgtt gatgaccaac ttatgcattg tttttaatga gtttttggtg 2460
cttttgaaca tctgtcaaaa tttatttcag tgccctaatt taactttttt ttttagttct 2520
tacattctat aacaacattc acagtaatgt tacttttata aatatataat cactgatcaa 2580
gattagggag gaggcatttc tgaagactat ttgcttcctg cagaactata ggcttgggct 2640
ggggagatgg ttcagtcagt aagtacaagc tgcacgctca tgaggaccat atttgagact 2700

ttagtagaag tgaagtccag cagatcccta aagcccactg gcctgccagc ctaacacagt 2760
ctatgagcac attcagtaga aggtcctgtc ttaaaagcta agtggacagt aattgaggaa 2820
gacactctgg caccaacaca cacacacaaa acccaaccga tacagtcttt atagttggtg 2880
cctaagtatc tggattttag ggtttcggtt ttaattttgt tttgagacag cttgtcgtcc 2940
tatagcccaa gctggcctca aattatgtat ccaaggacaa ccttgaattt ctcagcccac 3000
tacctaggct tggattacac agtgctgaag atcaaaccca gggctttgtg ttgtgctatg 3060
caacactcta acaactgagc tacatcccca gccctacata ttttagactc cagagtcttc 3120
tgttgagcaa ccaaggacaa acctaaaaca acttctccag tgagtgcagg gaaatagcaa 3180
tacaaagtgt ccattgctgc agtgcaatcc tatttgttgg aatcaaagga cattatccag 3240
attttacaat atgccaaact ggaattatga ggaaggtgga ttataatgaa cagggcaggc 3300
agaacaggaa aaattagaag tatttgataa gactttagaa ctttaaataa taggtctgat 3360
taatttgcag acaatgatag aaagtttgtt ttgactgata cccattaggc tttctaaaaa 3420
tgaatttata tatccataaa tatgtttctg ttgttaagga accaagtata tggaataaaa 3480
tctcccttta tcagttaaaa aaaaaaaaaa 3510

<210> 118
<211> 3566
<212> DNA
<213> Homo sapiens

<400> 118
ccggtcctga gacacgctgt gtggctgaaa agtgaaggca agagctcatt tggcctctgt 60
gctcccctcc gcaagggatc gtttctccag aagagctgga tattctttcg cccagttatg 120
gcagacaagt taacgagaat tgctattgtc aaccatgaca aatgtaaacc taagaaatgt 180
cgacaggaat gcaaaaagag ttgtcctgta gttcgaatgg gaaaattatg catagaggtt 240
acaccccaga gcaaaatagc atggatttcc gaaactcttt gtattggttg tggtatctgt 300
attaagaaat gcccctttgg cgccttatca attgtcaatc taccaagcaa cttggaaaaa 360
gaaaccacac atcgatattg tgccaatgcc ttcaaacttc acaggttgcc tatccctcgt 420
ccaggtgaag ttttgggatt agttggaact aatggtattg gaaagtcaac tgctttaaaa 480
attttagcag gaaaacaaaa gccaaacctt ggaaagtacg atgatcctcc tgactggcag 540
gagattttga cttatttccg tggatctgaa ttacaaaatt actttacaaa gattctagaa 600
gatgacctaa aagccatcat caaacctcaa tatgtagacc agattcctaa ggctgcaaag 660
gggacagtgg gatctatttt ggaccgaaaa gatgaaacaa agacacaggc aattgtatgt 720
cagcagcttg atttaaccca cctaaaagaa cgaaatgttg aagatctttc aggaggagag 780
ttgcagagat ttgcttgtgc tgtcgtttgc atacagaaag ctgatatttt catgtttgat 840
gagccttcta gttacctaga tgtcaagcag cgtttaaagg ctgctattac tatacgatct 900
ctaataaatc cagatagata tatcattgtg gtggaacatg atctaagtgt attagactat 960
ctctccgact tcatctgctg tttatatggt gtaccaagcg cctatggagt tgtcactatg 1020
ccttttagtg taagagaagg cataaacatt tttttggatg gctatgttcc aacagaaaac 1080
ttgagattca gagatgcatc acttgttttt aaagtggctg agacagcaaa tgaagaagaa 1140
gttaaaaaga tgtgtatgta taaatatcca ggaatgaaga aaaaaatggg agaatttgag 1200
ctagcaattg tagctggaga gtttacagat tctgaaatta tggtgatgct gggggaaaat 1260
ggaacgggta aaacgacatt tatcagaatg cttgctggaa gacttaaacc tgatgaagga 1320
ggagaagtac cagttctaaa tgtcagttat aagccacaga aaattagtcc caaatcaact 1380
ggaagtgttc gccagttact acatgaaaag ataagagatg cttatactca cccacaattt 1440
gtgaccgatg taatgaagcc tctgcaaatt gaaaacatca ttgatcaaga ggtgcagaca 1500
ttatctggtg gtgaactaca gcgagtagct ttagcccttt gcttgggcaa acctgctgat 1560

gtctatttaa ttgatgaacc atctgcatat ttggattctg agcaaagact gatggcagct 1620
cgagttgtca aacgtttcat actccatgca aaaaagacag cctttgttgt ggaacatgac 1680
ttcatcatgg ccacctatct agcggatcgc gtcatcgttt ttgatggtgt tccatctaag 1740
aacacagttg caaacagtcc tcaaaccctt ttggctggca tgaataaatt tttgtctcag 1800
cttgaaatta cattcagaag agatccaaac aactataggc cacgaataaa caaacttaat 1860
tcaattaagg atgtagaaca aaagaagagt ggaaactact tttcttggga tgattagact 1920
gactctgaga atattgataa gccatttatt aaaaggagta tttactagaa ttttttgtca 1980
tataaaactt gaatcaggat tttatgcccc acatactctg gaacttgaag tataatatac 2040
ttaatataac ataaaaagcc agttgggttc taaattgtag ttgaaacaca gaaaatgcca 2100
cttttctgtt cctgaagagg ctcttttgtg cataatattc taaaatgaag acatttcaag 2160
ctatacaaat tacttccaag ttttcatgat gtatgggaag attttcagta ggtgtattat 2220
attcacggta ccaaatgctg accagtgttg ctccattttt taaatcttga aaagggtttc 2280
tgtacttacc tggtttgcca agtatgccag tgtaatgaaa ctgcccttat tttaaaagcc 2340
agtcaaagat tccactgatt gacatttgat aaataaacat caggattatg tttattgttt 2400
gttttcagtc tttgcactat attaccagta tatggtttcc gaggaagatt atctactgca 2460
aaacaccact gttggaaaaa taggtatttt taaattgttt ttaatctttt ttggtgcttt 2520
taaacatgtt tagcaaaaac caattcagtc cattccccgc aaaaaacccc taactttact 2580
ctgaactttt tttgtttttg cattccatga ggttctgtat tcagtcattc tctaggtaat 2640
gtcatttttg tacacatata tttatataat cactgattga gatttaggaa aaagcatttc 2700
taaagaatat ttgcttccct tagaactaca gactcgaaat ctttaaagat ggtgcctaag 2760
catctatgta ttttttttaa gttccacaga tttttctgtt gggcaggcca aggattataa 2820
accacttccc taaaggcaac attaatgcaa aagtccccag atggcaatac aaagtatccc 2880
ctggtaccac atatattcat ttgtgagttt ggatatagag cacattatct aaaccatttt 2940
gtagttccaa aaacccatct aaatttcttg agttcctgaa ttttgaacag gattacctgg 3000
agcctggagc cactttaagt tgtacttctg actaaactgg aattatgagt gaggaagagt 3060
gtttactaaa taaatgactg gggcaagcaa aattgaggag gaaattagaa actgtttgac 3120
aaactttaag agctacttga aataacagaa gtcttgatta atatgcaaat aatggctaga 3180
aagtatggtt taactggacc ctattatgcc ttttaaaaat aatttcagta acccataaat 3240
acatgttgta aaaaattcaa atatacagaa tggaataaaa aaatgatctc cctttattac 3300
cctcccaaag gttaccagcg tttgaattta ataatgtata ttctttcatg cttttttctg 3360
tgcacttacc taagtgtgaa tatgtaaagg gtttgttttg tatacaaatg ggattatact 3420
aaaataagta atgcctattt ttaaggatag gttaaatttg tgaatgatca tttcaaatat 3480
attgaataaa ataagcaaaa gctattgtta tttactgatc ctgaaaaaaa aaaaaaaaaa 3540
aaaaaaaaaa aaaaaaaaaa aaaaaa 3566

<210> 119
<211> 2321
<212> DNA
<213> Mus musculus

<400> 119
ggtccttgcc ctcgcagctc cggccctcga caccaccggc ccctccgctg gtgacgcccc 60
cgcagaggct tcgccgtcgc cgaggccttc tctccgcgac tcgtcgccgc aggattctac 120
tttcactggg ttccgcccgc acaccgccac cattgacgcc atgtcgagtt attctagtga 180
ccgagaccgc ggccgggatc gagggtttgg tgcacctcga tttggaggga gtagaacagg 240
acccctctct ggaaagaagt ttggaaatcc tggggagaaa ctagttaaaa agaagtggaa 300
tcttgatgag ctgcccaaat ttgagaagaa tttttatcaa gaacaccctg atttggcaag 360

gcgcaccgca caagaggtag atacatacag aagaagcaag gaaattacag ttagaggtca 420
caactgtcca aaacctgttc tgaattttta tgaagcaaac tttcctgcga atgtcatgga 480
tgtgattgca aggcagaact ttactgaacc cactgctatt caagctcagg gctggccagt 540
tgctctcagt ggattggata tggttggagt agctcagact ggatctggga aaacattatc 600
ttatttgctg cctgccattg tacacataaa ccaccagcca ttcctagaga gaggtgatgg 660
gcctatttgc ttggtgctgg caccaactcg agaactggca cagcaggtgc agcaagtggc 720
tgctgaatat tgtcgagctt gtcgcttgaa gtctacttgc atctatggtg gtgctcccaa 780
aggaccacag attcgtgatt tggaaagagg tgtggaaatc tgtattgcaa cacctggaag 840
actgattgac tttttagagt gtgggaaaac caatctgaga agaacaactt accttgtcct 900
tgatgaagct gataggatgc ttgatatggg atttgaaccc cagataagga aaattgtgga 960
tcaaataaga cctgataggc aaacactaat gtggagtgca acttggccaa aagaagtaag 1020
acagcttgct gaagatttcc tgaaagacta tattcatatc aatattggtg cactggaact 1080
gagtgcaaac cataacattc ttcagattgt ggatgtatgt catgatgtcg aaaaggatga 1140
aaagcttatt cgtctgatgg aagaaatcat gagtgagaag gagaataaaa ctattgtttt 1200
tgttgaaacc aaaagaagat gtgatgaact taccagaaaa atgaggagag atgggtggcc 1260
tgccatgggc atccatggtg acaagagtca gcaggaacgt gactgggttc taaatgaatt 1320
caaacatgga aaagctccta ttctgattgc taccgatgtg gcctccagag ggctagatgt 1380
ggaagatgtg aaatttgtca tcaattatga ctaccctaac tcctcagagg attatattca 1440
tcgaattgga agaactgctc gcagtaccaa aacaggcaca gcatacactt tctttacacc 1500
taataacata aagcaagtga gcgaccttat ctctgtgctt cgggaagcta atcaagcaat 1560
taatcccaag ttgcttcagt tggtcgaaga cagaggttca ggtcgttcca ggggtagagg 1620
aggcatgaag gacgatcgtc gtgacagata ctctgcaggc aaaaggggtg gatttaatac 1680
ctttagagac agggaaaact atgacagagg ctactctaat ctgcttaaga gagattttgg 1740
ggctaaaact cagaatggtg tttacagtgc tgcaaattac accaatggga gctttggaag 1800
taattttgta tctgctggca tacagaccag ttttaggact ggtaatccaa cagggactta 1860
ccagaacggt tatgatagca ctcagcaata tggaagtaat gttgcaaata tgcacaatgg 1920
tatgaaccaa caggcatatg catatcctgc taccgcagct gctgcgccta tgattggcta 1980
tcccatgcca acagggtatt ctcaataaga ctttagaagt atatgtaaat gtctgttttt 2040
cataattgct ctttatattg tgtgttatca gacaagatag ttatttaaga aacatgggaa 2100
atgcagaaat gactgcagtg cagcagtaat tatggtgcac ttttttcgct atttaagttg 2160
gatatttctc tacattcctg aaacaatttt taggtttttt tttgtactag aaaatgcagg 2220
cagtgttttc acaaaagcaa ctgtacagtg atttcaaata caataaatga aggcaatgca 2280
tggccttcca ataaaagata tttgaagact gaaaaaaaaa a 2321

<210> 120
<211> 1217
<212> DNA
<213> Mus musculus

<400> 120
tgcttgcaaa gatcacactt tcctaactga gccaccctca cccctcagct gtctctttca 60
tttcaattct tgaaactttc atacatcttg tttgtttgat ttatctacag aaaatgacct 120
gaagtgaccc agggcaagaa ctcctcatgg accagtcaaa tatgacatcc cttgctgaag 180
agaaagccat gaatacctcc agcagaaatg cctccctggg gagctcacac ccacccattc 240
ccatagtgca ctgggtcatc atgagcatct cccctctggg ctttgtggag aatgggatcc 300
tcctctggtt cctctgcttc cggatgagga gaaatccctt cacggtctac attacccact 360
tgtccattgc tgatatctct cttctgttct gtatttttat cctgtccatt gactatgctt 420

tagactatga actctcttct ggccatcact acacaatcgt gacattatcg gtgacttttc 480
tatttggcta caacacgggc ctctatctgc tgacagccat cagtgtggag aggtgcctat 540
cggtcctcta ccccatatgg tacagatgcc accgccccaa gcaccagtca gcattcgtct 600
gtgcccttct gtgggcactt tcgtgcttgg tgaccaccat ggagtatgtc atgtgtattg 660
acagcggaga agagagtcat tctcggagtg actgccgggc ggtcatcatc ttcatagcca 720
tcctcagctt cttggtcttc actccgctca tgttagtgtc cagcaccatc ttggtggtga 780
agatacggaa gaacacgtgg gcctcccatt cttcgaagct gtacattgtc atcatggtca 840
ccattatcat attcctcatc tttgccatgc ccatgcgggt cctctacttg ctgtactacg 900
agtactggtc agcctttggg aacctgcata acatctcctt gcttttctcc accatcaaca 960
gcagcgccaa ccctttcatc tacttttttg tgggcagcag taagaagaag cgcttcaggg 1020
agtccttaaa agtcgtcctg accagggctt tcaaagatga aatgcaaccc aggcgccagg 1080
agggcaacgg caacactgta tccattgaga ctgtggtctg aggacagcag gggaaattgt 1140
ggacagaaac agtggaatgc tggaggcttt tagcttgtgc ttggaatgca atttaagcac 1200
atttaaatct cctaact 1217

<210> 121
<211> 1144
<212> DNA
<213> Homo sapiens

<400> 121
agccccctga aatacggaga ataagaatct tagaggttgt tcagcagaag tcttggagtg 60
cattttcagt ggttaaggtg aaaaaatgac tactaaaaat ttagaaacca aagtcaccgt 120
tacttcatcc ccaacccgag gagcaggaga tggaatggaa actgaggaac cacctaaatc 180
tgttgaagtt acctccggag tccaatctag aaagcatcat agtcttcaga gtccatggaa 240
gaaagcagtt ccatcagaga gcccaggagt tcttcagcta gggaaaatgc tcactgaaaa 300
agcaatggaa gttaaagctg taagaatatt agttcccaaa gctgctataa ctcatgatat 360
ccccaacaaa aatacaaagg ttaagtctct gggacatcat aaaggagaat tccttggtca 420
gtcagaggga gttatagaac ctaataagga actctcagag gtaaagaatg tattggaaaa 480
gctcaagaat tctgaaagaa ggttactaca ggacaaagaa ggtctttcaa accagctccg 540
tgtacagaca gaggtaaatc gtgagttaaa aaagttactg gtggcttctg ttggggatga 600
tcttcagtat cactttgaac gtctagcccg tgagaaaaat cagcttattt tagaaaatga 660
agccctaggt cgaaacacag ctcagctttc tgaacagtta gaacgtatgt caatacagtg 720
tgatgtatgg cgaagtaaat tccttgcaag cagggtaatg gcagatgagt taaccaactc 780
aagagcagct ttacagcgtc aaaaccgtga tgcacacggg gctatacaag atctcctaag 840
tgaacgggaa cagtttcgtc aagaaatgat agctacccag aagttctaag aattttagat 900
ccagttacct gcaaagagag ttcacctgat aatccatttt ttgagtcttc accaaccacc 960
ttacttgcta caaagaaaaa tattggacga tttcatccct atactagata tgaaaatata 1020
actttcaatt gctgcaatca ctgccgggga gaactgattg ccctttaaca gtcaatatgt 1080
tggaggcatg ctaaggtact tccttattac ccaagagtca ttattatttg ggagctgggg 1140
ttct 1144

<210> 122
<211> 1812
<212> DNA
<213> Mus musculus

<400> 122
aattcgtcga caaggggcgg gttggcttca gagtgcsagg ggagtttaca agtcagccgg 60
ctgctgagcg agtgaagagc ctgagcggtg gacattgaag taaggatatc acaggtcttc 120
ggtgaaagtc ttggattgta ttctcattag ttatggaaaa aatgactacc cttaaaagtt 180
cggaaaacaa aggcatcctt acttcgaccc caatccgagg agcaggagat ggaatggaaa 240
cggaggagcc ccctaagtct gttgaagtca cccacggagt ccaacccata aatcaacatg 300
tccttccaag tccacggaag aaagtgtcct cagacagccc gggcgttctt cagctgggga 360
agattcttaa tgaaagaaca gtggaagttg aagctgtcag aatatttgtt cccaaagctg 420
ctatcactca cgacatcccc accaagaaca caaaggttaa gtccctagga catcacagag 480
aagaactcca caatcaggca gaagtggtca cagacccaag gaaggaactc tcagaggtaa 540
agaaggtttt ggaaaagctc aagaattctg aaaggaggct acttcaggac aaagaaggcc 600
tttccaatca gctccgagta cagacagaga taaatcgtga gttaaagaag ttactcgtgg 660
cttctgttgg ggatgacccg cagtatcact ttgaacgtct agcccgggag aagaaccagc 720
tcatcttaga aaatgaagca ctaggtcgga acacagccca gctttctgaa cagctggaac 780
ggatgtcaat acagtgcgat gtctggagaa gtaaattcct tgcaagcagg gtaatggcag 840
atgagttaac aaacttcaga gtggttttac agcgtcagaa ccgagatgcc cagagcgcta 900
tccaggatct cctgagtgaa cgagagcagt ttcgtcagga aatgacctct acccaaaaat 960
ttttggaaga actcttggtc tccttacagt gggggagaga gcagacatac tcccctaaca 1020
cacagcccca tagcacggca gacttagcac tcacgaacca tggactggca caagcaatac 1080
atgcccatct gttggggaat gttggcatta gccatcaaaa gaagattcca acaacagtcg 1140
aattctgcag taccccagct gagaaaatgg ctgaaaaggt tctgcggatt ttggatccag 1200
ttgcctgtac agagagctct ccggacaacc aatttgctga atcttcacca accacattac 1260
tcactacaaa gaaaaatatt ggacgtttcc atccctatac cagatatgaa aatataactt 1320
tcaattgctg caatcactgc cagggggagc tcattgctct ttagtgtgta gtctgttgga 1380
gccatgctca gtacccagag tcattatttg ggagcccgga ttagtaagtt gctagaaata 1440
aaatcaattc atcgtgatat aatacatacc catgaagcta tttcatgtac tggattccag 1500
attatttttt aacaaatatc tcagggtaag aaagtgaagc tttgtagata tatttaaaga 1560
acagaatact tcccagtaca tagtatttat gctaaaaaac taaaaagatt ttataacttt 1620
cttttaggcc ctatattatt attctgttat atatttatat aaactataag caggtttcat 1680
tttggtgcat aaagcaaaac caggcaaact ccaaccatgc attgtaaagg ggtgtggcca 1740
tatccatcct ttaccgctgt ctggctgctt tactgtaggg cagcagagtc ggtgtgtggc 1800
tagcaaagtc ca 1812

<210> 123
<211> 1889
<212> DNA
<213> Mus musculus

<400> 123
aattcgtcga caaggggcgg gttggcttca gagtgcsagg ggagtttaca agtcagccgg 60
ctgctgagcg agtgaagagc ctgagcggtg gacattgaag taaggatatc acaggtcttc 120
ggtgaaagtc ttggattgta ttctcattag ttatggaaaa aatgactacc cttaaaagtt 180
cggaaaacaa agggaaacaa gaaatgatga aaggtaagaa aatgcaaagc cctagccctg 240
cctgctcacc cgcgcgttag ccagagctgg catccttact tcgaccccaa tccgaggagc 300
aggagatgga atggaaacgg aggagccccc taagtctgtt gaagtcaccc acggagtcca 360
acccataaat caacatgtcc ttccaagtcc acggaagaaa gtgtcctcag acagcccggg 420

cgttcttcag ctggggaaga ttcttaatga aagaacagtg gaagttgaag ctgtcagaat 480
atttgttccc aaagctgcta tcactcacga catccccacc aagaacacaa aggttaagtc 540
cctaggacat cacagagaag aactccacaa tcaggcagaa gtggtcacag acccaaggaa 600
ggaactctca gaggtaaaga aggttttgga aaagctcaag aattctgaaa ggaggctact 660
tcaggacaaa gaaggccttt ccaatcagct ccgagtacag acagagataa atcgtgagtt 720
aaagaagtta ctcgtggctt ctgttgggga tgacccgcag tatcactttg aacgtctagc 780
ccgggagaag aaccagctca tcttagaaaa tgaagcacta ggtcggaaca cagcccagct 840
ttctgaacag ctggaacgga tgtcaataca gtgcgatgtc tggagaagta aattccttgc 900
aagcagggta atggcagatg agttaacaaa cttcagagtg gttttacagc gtcagaaccg 960
agatgcccag agcgctatcc aggatctcct gagtgaacga gagcagtttc gtcaggaaat 1020
gacctctacc caaaaatttt tggaagaact cttggtctcc ttacagtggg ggagagagca 1080
gacatactcc cctaacacac agccccatag cacggcagac ttagcactca cgaaccatgg 1140
actggcacaa gcaatacatg cccatctgtt ggggaatgtt ggcattagcc atcaaaagaa 1200
gattccaaca acagtcgaat tctgcagtac cccagctgag aaaatggctg aaaaggttct 1260
gcggattttg gatccagttg cctgtacaga gagctctccg gacaaccaat ttgctgaatc 1320
ttcaccaacc acattactca ctacaaagaa aaatattgga cgtttccatc cctataccag 1380
atatgaaaat ataactttca attgctgcaa tcactgccag ggggagctca ttgctcttta 1440
gtgtgtagtc tgttggagcc atgctcagta cccagagtca ttatttggga gcccggatta 1500
gtaagttgct agaaataaaa tcaattcatc gtgatataat acatacccat gaagctattt 1560
catgtactgg attccagatt attttttaac aaatatctca gggtaagaaa gtgaagcttt 1620
gtagatatat ttaaagaaca gaatacttcc cagtacatag tatttatgct aaaaaactaa 1680
aaagatttta taactttctt ttaggcccta tattattatt ctgttatata tttatataaa 1740
ctataagcag gtttcatttt ggtgcataaa gcaaaaccag gcaaactcca accatgcatt 1800
gtaaaggggt gtggccatat ccatccttta ccgctgtctg gctgctttac tgtagggcag 1860
cagagtcggt gtgtggctag caaagtcca 1889

<210> 124
<211> 1758
<212> DNA
<213> Mus musculus

<400> 124
gttccagggg ttmattcsrc aagagagaaa aacatagact cacggctgcc aacatgatgg 60
tactgagagt agaggagctg gtaacaggca aaaagaacag caatggggcc gcaggggaat 120
tccttcctgg ggagttcaga aatggagaat atgaagctgc tgttgctttg gagaagcaag 180
aggacctgaa gacacttccg gccaacagcg tgaagcaggg ggaggaacaa cggaaaagcg 240
agaaactgcg agaggcagag ctcaaaaaga aaaaactaga acaaagatca aagcttgaaa 300
acttagaaga ccttgaaata attgttcaac tgaagaaaag gaaaaaatac aagaaaacca 360
aagttccagt tgtgaaggag ccagaacctg aaattatgac tgaacctgtg gatgtgccga 420
ggtttctgaa agctgcgctg gagaacaaac tgccagttgt agagaaattc ctgtcagaca 480
agaacagccc cgacgtctgc gatgagtata aacggacggc actccaccga gcatgcttag 540
aaggacactt ggcgatcgtg gagaagttaa tggaggctgg agcccagatt gaattccgag 600
atatgcttga atccacagcc atccactggg catgtcgtgg aggaaacgca gatgtcctga 660
aactgttgct gaacaaagga gccaaaatca gtgccagaga caagcttctc agcacagcgc 720
tgcatgtggc ggtgaggact ggtcattacg agtgcgctga gcacctcatc gcctgcgagg 780
ctgatctcaa tgccaaggac agagaaggag acaccccact gcatgatgct gtgaggctga 840
accgctataa gatgattcga ctcttgatga ccttcggtgc ggacctcaag gtcaagaact 900

gtgctgggaa gacccccatg gatctggtgt tgcactggca gagtggaacc aaagcaatat 960
tcgacagcct caaggagaat gcctacaaga actctcgcat agctacattc tgagaaaaga 1020
gactcaacag gagctgttcg caggcatttt ttaaagcatt tcccaacaga agagcaacta 1080
gcaataaaat ccagcttttg tttgccagcc aaatagggag agatgctcaa agaagcttca 1140
agaaagcact gggttgtagg tgttaaaata tcctctagtg atgttcacca cttttattta 1200
ttcctaccta tttatttatt tcatagcacc agtattcagg ctttcatgaa catcattttg 1260
ggggagcaag acttcatctg tatgtacatt cagagtcttc ccatagctct taaaccgtgt 1320
gggtgaatgg tgtctaattg cacccctttg tgttctcact tgcataaagc tattcaaggt 1380
actttttaaa aggcaagggt tgatccccgc agcccttctc tgaattctca ctgctgatat 1440
ctgaatgaag caacagaagg cacgcagtaa cgcagacggg aacggaagca gttccgcagg 1500
actggcgcgr aggctcgcca cccacccaca gatttggttc ggtttggttg tttaccarat 1560
cacatgatkt cgatktccca aggtgaaggc aacgtcagga gtsccgcatg tagggagatg 1620
gagggagtag gcaatggggc caaggaaaac tgagttcgat gaaagaagaa atcaaaagga 1680
cacgcgctgt aaatgtataa atgtatattg tgttgtatgt atattttata tttataataa 1740
atgcaaacta attcccgg 1758

<210> 125
<211> 43
<212> DNA
<213> Künstliche Sequenz

<220>
<223> Beschreibung der künstlichen Sequenz:Antisense
      primer that links the Sp6 promoter with a sequence
      homologous to human MRP-14

<400> 125
atttaggtga cactatagaa taccccgagg cctggcttat ggt 43

<210> 126
<211> 41
<212> DNA
<213> Künstliche Sequenz

<220>
<223> Beschreibung der künstlichen Sequenz:Sense primer
      that links the T3 promoter with a sequence
      homologous to human MRP-14

<400> 126
aattaaccct cactaaaggg ggtggctcct cggctttgac a 41

<210> 127
<211> 30
<212> DNA

<213> Homo sapiens

<400> 127
gagagaggta ccatgccgtc tgaactggag                                        30

<210> 128
<211> 36
<212> DNA
<213> Homo sapiens

<400> 128
gagagagaca cgtgctactc cttgtggctg tctttg                                 36